(19) Europäisches Patentamt
European Patent Office
Office européen des brevets

(11) EP 3 231 813 A1

# (12) EUROPEAN PATENT APPLICATION

(43) Date of publication:
**18.10.2017 Bulletin 2017/42**

(21) Application number: **16162539.7**

(22) Date of filing: **29.03.2016**

(51) Int Cl.:
***C07K 14/705*** *(2006.01)*
***A61K 47/50*** *(2017.01)*
***C07K 16/28*** *(2006.01)*
***C07K 16/30*** *(2006.01)*
***C12N 15/62*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **F. Hoffmann-La Roche AG**
**4070 Basel (CH)**

(72) Inventor: **The designation of the inventor has not yet been filed**

(74) Representative: **Klostermeyer-Rauber, Dörte**
**F. Hoffmann-La Roche AG**
**Corporate Law Patents (CLP)**
**Grenzacherstrasse 124**
**4070 Basel (CH)**

## (54) TRIMERIC COSTIMULATORY TNF FAMILY LIGAND-CONTAINING ANTIGEN BINDING MOLECULES

(57) The invention relates to novel trimeric costimulatory TNF family ligand-containing antigen binding molecules comprising three fusion polypeptides, each of the three fusion polypeptides comprising (a) an ectodomain of a TNF ligand family member or fragments thereof, (b) a trimerization domain, in particular a trimerization domain derived from human cartilage matrix protein (huCMP) of SEQ ID NO:1, and (c) a moiety capable of specific binding to a target cell antigen, and to methods of producing these molecules and to methods of using the same.

EP 3 231 813 A1

SEQUENCE LISTING

<110>  F. Hoffmann-La Roche AG

<120>  Trimeric costimulatory TNF family ligand-containing antigen binding molecules

<130>  P32820-EP

<160>  93

<170>  PatentIn version 3.5

<210>  1
<211>  496
<212>  PRT
<213>  human

<400>  1

```
Met Arg Val Leu Ser Gly Thr Ser Leu Met Leu Cys Ser Leu Leu Leu
1               5                   10                  15

Leu Leu Gln Ala Leu Cys Ser Pro Gly Leu Ala Pro Gln Ser Arg Gly
            20                  25                  30

His Leu Cys Arg Thr Arg Pro Thr Asp Leu Val Phe Val Val Asp Ser
        35                  40                  45

Ser Arg Ser Val Arg Pro Val Glu Phe Glu Lys Val Lys Val Phe Leu
    50                  55                  60

Ser Gln Val Ile Glu Ser Leu Asp Val Gly Pro Asn Ala Thr Arg Val
65                  70                  75                  80

Gly Met Val Asn Tyr Ala Ser Thr Val Lys Gln Glu Phe Ser Leu Arg
                85                  90                  95

Ala His Val Ser Lys Ala Ala Leu Leu Gln Ala Val Arg Arg Ile Gln
            100                 105                 110

Pro Leu Ser Thr Gly Thr Met Thr Gly Leu Ala Ile Gln Phe Ala Ile
        115                 120                 125

Thr Lys Ala Phe Gly Asp Ala Glu Gly Gly Arg Ser Arg Ser Pro Asp
    130                 135                 140

Ile Ser Lys Val Val Ile Val Val Thr Asp Gly Arg Pro Gln Asp Ser
145                 150                 155                 160

Val Gln Asp Val Ser Ala Arg Ala Arg Ala Ser Gly Val Glu Leu Phe
                165                 170                 175
```

```
Ala Ile Gly Val Gly Ser Val Asp Lys Ala Thr Leu Arg Gln Ile Ala
            180                 185                 190

Ser Glu Pro Gln Asp Glu His Val Asp Tyr Val Glu Ser Tyr Ser Val
        195                 200                 205

Ile Glu Lys Leu Ser Arg Lys Phe Gln Glu Ala Phe Cys Val Val Ser
    210                 215                 220

Asp Leu Cys Ala Thr Gly Asp His Asp Cys Glu Gln Val Cys Ile Ser
225                 230                 235                 240

Ser Pro Gly Ser Tyr Thr Cys Ala Cys His Glu Gly Phe Thr Leu Asn
                245                 250                 255

Ser Asp Gly Lys Thr Cys Asn Val Cys Ser Gly Gly Gly Gly Ser Ser
            260                 265                 270

Ala Thr Asp Leu Val Phe Leu Ile Asp Gly Ser Lys Ser Val Arg Pro
        275                 280                 285

Glu Asn Phe Glu Leu Val Lys Lys Phe Ile Ser Gln Ile Val Asp Thr
    290                 295                 300

Leu Asp Val Ser Asp Lys Leu Ala Gln Val Gly Leu Val Gln Tyr Ser
305                 310                 315                 320

Ser Ser Val Arg Gln Glu Phe Pro Leu Gly Arg Phe His Thr Lys Lys
                325                 330                 335

Asp Ile Lys Ala Ala Val Arg Asn Met Ser Tyr Met Glu Lys Gly Thr
            340                 345                 350

Met Thr Gly Ala Ala Leu Lys Tyr Leu Ile Asp Asn Ser Phe Thr Val
        355                 360                 365

Ser Ser Gly Ala Arg Pro Gly Ala Gln Lys Val Gly Ile Val Phe Thr
    370                 375                 380

Asp Gly Arg Ser Gln Asp Tyr Ile Asn Asp Ala Ala Lys Lys Ala Lys
385                 390                 395                 400

Asp Leu Gly Phe Lys Met Phe Ala Val Gly Val Gly Asn Ala Val Glu
                405                 410                 415

Asp Glu Leu Arg Glu Ile Ala Ser Glu Pro Val Ala Glu His Tyr Phe
            420                 425                 430
```

```
Tyr Thr Ala Asp Phe Lys Thr Ile Asn Gln Ile Gly Lys Lys Leu Gln
        435                 440                 445

Lys Lys Ile Cys Val Glu Glu Asp Pro Cys Ala Cys Glu Ser Leu Val
    450                 455                 460

Lys Phe Gln Ala Lys Val Glu Gly Leu Leu Gln Ala Leu Thr Arg Lys
465                 470                 475                 480

Leu Glu Ala Val Ser Lys Arg Leu Ala Ile Leu Glu Asn Thr Val Val
                485                 490                 495


<210>  2
<211>  39
<212>  PRT
<213>  human

<400>  2

Cys Ala Cys Glu Ser Leu Val Lys Phe Gln Ala Lys Val Glu Gly Leu
1               5                   10                  15

Leu Gln Ala Leu Thr Arg Lys Leu Glu Ala Val Ser Lys Arg Leu Ala
            20                  25                  30

Ile Leu Glu Asn Thr Val Val
        35


<210>  3
<211>  184
<212>  PRT
<213>  human

<400>  3

Arg Glu Gly Pro Glu Leu Ser Pro Asp Asp Pro Ala Gly Leu Leu Asp
1               5                   10                  15

Leu Arg Gln Gly Met Phe Ala Gln Leu Val Ala Gln Asn Val Leu Leu
            20                  25                  30

Ile Asp Gly Pro Leu Ser Trp Tyr Ser Asp Pro Gly Leu Ala Gly Val
        35                  40                  45

Ser Leu Thr Gly Gly Leu Ser Tyr Lys Glu Asp Thr Lys Glu Leu Val
    50                  55                  60

Val Ala Lys Ala Gly Val Tyr Tyr Val Phe Phe Gln Leu Glu Leu Arg
65                  70                  75                  80
```

Arg Val Val Ala Gly Glu Gly Ser Gly Ser Val Ser Leu Ala Leu His
85 90 95

Leu Gln Pro Leu Arg Ser Ala Ala Gly Ala Ala Ala Leu Ala Leu Thr
100 105 110

Val Asp Leu Pro Pro Ala Ser Ser Glu Ala Arg Asn Ser Ala Phe Gly
115 120 125

Phe Gln Gly Arg Leu Leu His Leu Ser Ala Gly Gln Arg Leu Gly Val
130 135 140

His Leu His Thr Glu Ala Arg Ala Arg His Ala Trp Gln Leu Thr Gln
145 150 155 160

Gly Ala Thr Val Leu Gly Leu Phe Arg Val Thr Pro Glu Ile Pro Ala
165 170 175

Gly Leu Pro Ser Pro Arg Ser Glu
180

<210> 4
<211> 170
<212> PRT
<213> human

<400> 4

Leu Asp Leu Arg Gln Gly Met Phe Ala Gln Leu Val Ala Gln Asn Val
1 5 10 15

Leu Leu Ile Asp Gly Pro Leu Ser Trp Tyr Ser Asp Pro Gly Leu Ala
20 25 30

Gly Val Ser Leu Thr Gly Gly Leu Ser Tyr Lys Glu Asp Thr Lys Glu
35 40 45

Leu Val Val Ala Lys Ala Gly Val Tyr Tyr Val Phe Phe Gln Leu Glu
50 55 60

Leu Arg Arg Val Val Ala Gly Glu Gly Ser Gly Ser Val Ser Leu Ala
65 70 75 80

Leu His Leu Gln Pro Leu Arg Ser Ala Ala Gly Ala Ala Ala Leu Ala
85 90 95

Leu Thr Val Asp Leu Pro Pro Ala Ser Ser Glu Ala Arg Asn Ser Ala
100 105 110

```
Phe Gly Phe Gln Gly Arg Leu Leu His Leu Ser Ala Gly Gln Arg Leu
        115                 120                 125

Gly Val His Leu His Thr Glu Ala Arg Ala Arg His Ala Trp Gln Leu
    130                 135                 140

Thr Gln Gly Ala Thr Val Leu Gly Leu Phe Arg Val Thr Pro Glu Ile
145                 150                 155                 160

Pro Ala Gly Leu Pro Ser Pro Arg Ser Glu
                165                 170


<210>  5
<211>  175
<212>  PRT
<213>  human

<400>  5

Asp Pro Ala Gly Leu Leu Asp Leu Arg Gln Gly Met Phe Ala Gln Leu
1               5                   10                  15

Val Ala Gln Asn Val Leu Leu Ile Asp Gly Pro Leu Ser Trp Tyr Ser
            20                  25                  30

Asp Pro Gly Leu Ala Gly Val Ser Leu Thr Gly Gly Leu Ser Tyr Lys
        35                  40                  45

Glu Asp Thr Lys Glu Leu Val Val Ala Lys Ala Gly Val Tyr Tyr Val
    50                  55                  60

Phe Phe Gln Leu Glu Leu Arg Arg Val Val Ala Gly Glu Gly Ser Gly
65                  70                  75                  80

Ser Val Ser Leu Ala Leu His Leu Gln Pro Leu Arg Ser Ala Ala Gly
                85                  90                  95

Ala Ala Ala Leu Ala Leu Thr Val Asp Leu Pro Pro Ala Ser Ser Glu
            100                 105                 110

Ala Arg Asn Ser Ala Phe Gly Phe Gln Gly Arg Leu Leu His Leu Ser
        115                 120                 125

Ala Gly Gln Arg Leu Gly Val His Leu His Thr Glu Ala Arg Ala Arg
    130                 135                 140

His Ala Trp Gln Leu Thr Gln Gly Ala Thr Val Leu Gly Leu Phe Arg
145                 150                 155                 160
```

Val Thr Pro Glu Ile Pro Ala Gly Leu Pro Ser Pro Arg Ser Glu
165 170 175

<210> 6
<211> 203
<212> PRT
<213> human

<400> 6

Pro Trp Ala Val Ser Gly Ala Arg Ala Ser Pro Gly Ser Ala Ala Ser
1 5 10 15

Pro Arg Leu Arg Glu Gly Pro Glu Leu Ser Pro Asp Asp Pro Ala Gly
20 25 30

Leu Leu Asp Leu Arg Gln Gly Met Phe Ala Gln Leu Val Ala Gln Asn
35 40 45

Val Leu Leu Ile Asp Gly Pro Leu Ser Trp Tyr Ser Asp Pro Gly Leu
50 55 60

Ala Gly Val Ser Leu Thr Gly Gly Leu Ser Tyr Lys Glu Asp Thr Lys
65 70 75 80

Glu Leu Val Val Ala Lys Ala Gly Val Tyr Tyr Val Phe Phe Gln Leu
85 90 95

Glu Leu Arg Arg Val Val Ala Gly Glu Gly Ser Gly Ser Val Ser Leu
100 105 110

Ala Leu His Leu Gln Pro Leu Arg Ser Ala Ala Gly Ala Ala Ala Leu
115 120 125

Ala Leu Thr Val Asp Leu Pro Pro Ala Ser Ser Glu Ala Arg Asn Ser
130 135 140

Ala Phe Gly Phe Gln Gly Arg Leu Leu His Leu Ser Ala Gly Gln Arg
145 150 155 160

Leu Gly Val His Leu His Thr Glu Ala Arg Ala Arg His Ala Trp Gln
165 170 175

Leu Thr Gln Gly Ala Thr Val Leu Gly Leu Phe Arg Val Thr Pro Glu
180 185 190

Ile Pro Ala Gly Leu Pro Ser Pro Arg Ser Glu
195 200

<210> 7
<211> 178
<212> PRT
<213> human

<400> 7

Arg Glu Gly Pro Glu Leu Ser Pro Asp Asp Pro Ala Gly Leu Leu Asp
1 5 10 15

Leu Arg Gln Gly Met Phe Ala Gln Leu Val Ala Gln Asn Val Leu Leu
20 25 30

Ile Asp Gly Pro Leu Ser Trp Tyr Ser Asp Pro Gly Leu Ala Gly Val
35 40 45

Ser Leu Thr Gly Gly Leu Ser Tyr Lys Glu Asp Thr Lys Glu Leu Val
50 55 60

Val Ala Lys Ala Gly Val Tyr Tyr Val Phe Phe Gln Leu Glu Leu Arg
65 70 75 80

Arg Val Val Ala Gly Glu Gly Ser Gly Ser Val Ser Leu Ala Leu His
85 90 95

Leu Gln Pro Leu Arg Ser Ala Ala Gly Ala Ala Ala Leu Ala Leu Thr
100 105 110

Val Asp Leu Pro Pro Ala Ser Ser Glu Ala Arg Asn Ser Ala Phe Gly
115 120 125

Phe Gln Gly Arg Leu Leu His Leu Ser Ala Gly Gln Arg Leu Gly Val
130 135 140

His Leu His Thr Glu Ala Arg Ala Arg His Ala Trp Gln Leu Thr Gln
145 150 155 160

Gly Ala Thr Val Leu Gly Leu Phe Arg Val Thr Pro Glu Ile Pro Ala
165 170 175

Gly Leu

<210> 8
<211> 164
<212> PRT
<213> human

<400> 8

Leu Asp Leu Arg Gln Gly Met Phe Ala Gln Leu Val Ala Gln Asn Val
1               5                   10                  15

Leu Leu Ile Asp Gly Pro Leu Ser Trp Tyr Ser Asp Pro Gly Leu Ala
            20                  25                  30

Gly Val Ser Leu Thr Gly Gly Leu Ser Tyr Lys Glu Asp Thr Lys Glu
        35                  40                  45

Leu Val Val Ala Lys Ala Gly Val Tyr Tyr Val Phe Phe Gln Leu Glu
    50                  55                  60

Leu Arg Arg Val Val Ala Gly Glu Gly Ser Gly Ser Val Ser Leu Ala
65                  70                  75                  80

Leu His Leu Gln Pro Leu Arg Ser Ala Ala Gly Ala Ala Ala Leu Ala
                85                  90                  95

Leu Thr Val Asp Leu Pro Pro Ala Ser Ser Glu Ala Arg Asn Ser Ala
            100                 105                 110

Phe Gly Phe Gln Gly Arg Leu Leu His Leu Ser Ala Gly Gln Arg Leu
        115                 120                 125

Gly Val His Leu His Thr Glu Ala Arg Ala Arg His Ala Trp Gln Leu
    130                 135                 140

Thr Gln Gly Ala Thr Val Leu Gly Leu Phe Arg Val Thr Pro Glu Ile
145                 150                 155                 160

Pro Ala Gly Leu

<210> 9
<211> 169
<212> PRT
<213> humand

<400> 9

Asp Pro Ala Gly Leu Leu Asp Leu Arg Gln Gly Met Phe Ala Gln Leu
1               5                   10                  15

Val Ala Gln Asn Val Leu Leu Ile Asp Gly Pro Leu Ser Trp Tyr Ser
            20                  25                  30

Asp Pro Gly Leu Ala Gly Val Ser Leu Thr Gly Gly Leu Ser Tyr Lys
        35                  40                  45

```
Glu Asp Thr Lys Glu Leu Val Val Ala Lys Ala Gly Val Tyr Tyr Val
    50                  55                  60

Phe Phe Gln Leu Glu Leu Arg Arg Val Val Ala Gly Glu Gly Ser Gly
65                  70                  75                  80

Ser Val Ser Leu Ala Leu His Leu Gln Pro Leu Arg Ser Ala Ala Gly
                85                  90                  95

Ala Ala Ala Leu Ala Leu Thr Val Asp Leu Pro Pro Ala Ser Ser Glu
            100                 105                 110

Ala Arg Asn Ser Ala Phe Gly Phe Gln Gly Arg Leu Leu His Leu Ser
        115                 120                 125

Ala Gly Gln Arg Leu Gly Val His Leu His Thr Glu Ala Arg Ala Arg
    130                 135                 140

His Ala Trp Gln Leu Thr Gln Gly Ala Thr Val Leu Gly Leu Phe Arg
145                 150                 155                 160

Val Thr Pro Glu Ile Pro Ala Gly Leu
                165


<210>  10
<211>  197
<212>  PRT
<213>  human

<400>  10

Pro Trp Ala Val Ser Gly Ala Arg Ala Ser Pro Gly Ser Ala Ala Ser
1               5                   10                  15

Pro Arg Leu Arg Glu Gly Pro Glu Leu Ser Pro Asp Asp Pro Ala Gly
            20                  25                  30

Leu Leu Asp Leu Arg Gln Gly Met Phe Ala Gln Leu Val Ala Gln Asn
        35                  40                  45

Val Leu Leu Ile Asp Gly Pro Leu Ser Trp Tyr Ser Asp Pro Gly Leu
    50                  55                  60

Ala Gly Val Ser Leu Thr Gly Gly Leu Ser Tyr Lys Glu Asp Thr Lys
65                  70                  75                  80

Glu Leu Val Val Ala Lys Ala Gly Val Tyr Tyr Val Phe Phe Gln Leu
                85                  90                  95
```

Glu Leu Arg Arg Val Val Ala Gly Glu Gly Ser Gly Ser Val Ser Leu
100 105 110

Ala Leu His Leu Gln Pro Leu Arg Ser Ala Ala Gly Ala Ala Ala Leu
115 120 125

Ala Leu Thr Val Asp Leu Pro Pro Ala Ser Ser Glu Ala Arg Asn Ser
130 135 140

Ala Phe Gly Phe Gln Gly Arg Leu Leu His Leu Ser Ala Gly Gln Arg
145 150 155 160

Leu Gly Val His Leu His Thr Glu Ala Arg Ala Arg His Ala Trp Gln
165 170 175

Leu Thr Gln Gly Ala Thr Val Leu Gly Leu Phe Arg Val Thr Pro Glu
180 185 190

Ile Pro Ala Gly Leu
195

<210> 11
<211> 133
<212> PRT
<213> human

<400> 11

Gln Val Ser His Arg Tyr Pro Arg Ile Gln Ser Ile Lys Val Gln Phe
1 5 10 15

Thr Glu Tyr Lys Lys Glu Lys Gly Phe Ile Leu Thr Ser Gln Lys Glu
20 25 30

Asp Glu Ile Met Lys Val Gln Asn Asn Ser Val Ile Ile Asn Cys Asp
35 40 45

Gly Phe Tyr Leu Ile Ser Leu Lys Gly Tyr Phe Ser Gln Glu Val Asn
50 55 60

Ile Ser Leu His Tyr Gln Lys Asp Glu Glu Pro Leu Phe Gln Leu Lys
65 70 75 80

Lys Val Arg Ser Val Asn Ser Leu Met Val Ala Ser Leu Thr Tyr Lys
85 90 95

Asp Lys Val Tyr Leu Asn Val Thr Thr Asp Asn Thr Ser Leu Asp Asp
100 105 110

Phe His Val Asn Gly Gly Glu Leu Ile Leu Ile His Gln Asn Pro Gly
115 120 125

Glu Phe Cys Val Leu
130

<210> 12
<211> 133
<212> PRT
<213> Artificial

<220>
<223> Ox40L with mutations N90D, N114D

<400> 12

Gln Val Ser His Arg Tyr Pro Arg Ile Gln Ser Ile Lys Val Gln Phe
1 5 10 15

Thr Glu Tyr Lys Lys Glu Lys Gly Phe Ile Leu Thr Ser Gln Lys Glu
20 25 30

Asp Glu Ile Met Lys Val Gln Asp Asn Ser Val Ile Ile Asn Cys Asp
35 40 45

Gly Phe Tyr Leu Ile Ser Leu Lys Gly Tyr Phe Ser Gln Glu Val Asp
50 55 60

Ile Ser Leu His Tyr Gln Lys Asp Glu Glu Pro Leu Phe Gln Leu Lys
65 70 75 80

Lys Val Arg Ser Val Asn Ser Leu Met Val Ala Ser Leu Thr Tyr Lys
85 90 95

Asp Lys Val Tyr Leu Asn Val Thr Thr Asp Asn Thr Ser Leu Asp Asp
100 105 110

Phe His Val Asn Gly Gly Glu Leu Ile Leu Ile His Gln Asn Pro Gly
115 120 125

Glu Phe Cys Val Leu
130

<210> 13
<211> 132
<212> PRT
<213> human

<400> 13

Val Ser His Arg Tyr Pro Arg Ile Gln Ser Ile Lys Val Gln Phe Thr
1 5 10 15

Glu Tyr Lys Lys Glu Lys Gly Phe Ile Leu Thr Ser Gln Lys Glu Asp
20 25 30

Glu Ile Met Lys Val Gln Asn Asn Ser Val Ile Ile Asn Cys Asp Gly
35 40 45

Phe Tyr Leu Ile Ser Leu Lys Gly Tyr Phe Ser Gln Glu Val Asn Ile
50 55 60

Ser Leu His Tyr Gln Lys Asp Glu Glu Pro Leu Phe Gln Leu Lys Lys
65 70 75 80

Val Arg Ser Val Asn Ser Leu Met Val Ala Ser Leu Thr Tyr Lys Asp
85 90 95

Lys Val Tyr Leu Asn Val Thr Thr Asp Asn Thr Ser Leu Asp Asp Phe
100 105 110

His Val Asn Gly Gly Glu Leu Ile Leu Ile His Gln Asn Pro Gly Glu
115 120 125

Phe Cys Val Leu
130

<210> 14
<211> 5
<212> PRT
<213> Artificial Sequence

<220>
<223> FAP(28H1) CDR-H1

<400> 14

Ser His Ala Met Ser
1 5

<210> 15
<211> 16
<212> PRT
<213> Artificial Sequence

<220>
<223> FAP(28H1) CDR-H2

<400> 15

Ala Ile Trp Ala Ser Gly Glu Gln Tyr Tyr Ala Asp Ser Val Lys Gly
1 5 10 15

<210> 16

<211> 8
<212> PRT
<213> Artificial Sequence

<220>
<223> FAP(28H1) CDR-H3

<400> 16

Gly Trp Leu Gly Asn Phe Asp Tyr
1 5

<210> 17
<211> 12
<212> PRT
<213> Artificial Sequence

<220>
<223> FAP(28H1) CDR-L1

<400> 17

Arg Ala Ser Gln Ser Val Ser Arg Ser Tyr Leu Ala
1 5 10

<210> 18
<211> 7
<212> PRT
<213> Artificial Sequence

<220>
<223> FAP(28H1) CDR-L2

<400> 18

Gly Ala Ser Thr Arg Ala Thr
1 5

<210> 19
<211> 9
<212> PRT
<213> Artificial Sequence

<220>
<223> FAP(28H1) CDR-L3

<400> 19

Gln Gln Gly Gln Val Ile Pro Pro Thr
1 5

<210> 20
<211> 5
<212> PRT
<213> Artificial Sequence

<220>
<223> FAP(4B9) CDR-H1

<400> 20

Ser Tyr Ala Met Ser
1 5

<210> 21
<211> 17
<212> PRT
<213> Artificial Sequence

<220>
<223> FAP(4B9) CDR-H2

<400> 21

Ala Ile Ile Gly Ser Gly Ala Ser Thr Tyr Tyr Ala Asp Ser Val Lys
1 5 10 15

Gly

<210> 22
<211> 8
<212> PRT
<213> Artificial Sequence

<220>
<223> FAP(4B9) CDR-H3

<400> 22

Gly Trp Phe Gly Gly Phe Asn Tyr
1 5

<210> 23
<211> 12
<212> PRT
<213> Artificial Sequence

<220>
<223> FAP(4B9) CDR-L1

<400> 23

Arg Ala Ser Gln Ser Val Ser Arg Ser Tyr Leu Ala
1 5 10

<210> 24
<211> 7
<212> PRT
<213> Artificial Sequence

<220>
<223> FAP(4B9) CDR-L2

<400> 24

Val Gly Ser Arg Arg Ala Thr

1 5

<210> 25
<211> 9
<212> PRT
<213> Artificial Sequence

<220>
<223> FAP(4B9) CDR-L3

<400> 25

Gln Gln Gly Ile Met Leu Pro Pro Thr
1 5

<210> 26
<211> 116
<212> PRT
<213> Artificial Sequence

<220>
<223> FAP(28H1) VH

<400> 26

Glu Val Gln Leu Leu Glu Ser Gly Gly Gly Leu Val Gln Pro Gly Gly
1 5 10 15

Ser Leu Arg Leu Ser Cys Ala Ala Ser Gly Phe Thr Phe Ser Ser His
20 25 30

Ala Met Ser Trp Val Arg Gln Ala Pro Gly Lys Gly Leu Glu Trp Val
35 40 45

Ser Ala Ile Trp Ala Ser Gly Glu Gln Tyr Tyr Ala Asp Ser Val Lys
50 55 60

Gly Arg Phe Thr Ile Ser Arg Asp Asn Ser Lys Asn Thr Leu Tyr Leu
65 70 75 80

Gln Met Asn Ser Leu Arg Ala Glu Asp Thr Ala Val Tyr Tyr Cys Ala
85 90 95

Lys Gly Trp Leu Gly Asn Phe Asp Tyr Trp Gly Gln Gly Thr Leu Val
100 105 110

Thr Val Ser Ser
115

<210> 27
<211> 108
<212> PRT
<213> Artificial Sequence

<220>
<223> FAP(28H1) VL

<400> 27

Glu Ile Val Leu Thr Gln Ser Pro Gly Thr Leu Ser Leu Ser Pro Gly
1 5 10 15

Glu Arg Ala Thr Leu Ser Cys Arg Ala Ser Gln Ser Val Ser Arg Ser
20 25 30

Tyr Leu Ala Trp Tyr Gln Gln Lys Pro Gly Gln Ala Pro Arg Leu Leu
35 40 45

Ile Ile Gly Ala Ser Thr Arg Ala Thr Gly Ile Pro Asp Arg Phe Ser
50 55 60

Gly Ser Gly Ser Gly Thr Asp Phe Thr Leu Thr Ile Ser Arg Leu Glu
65 70 75 80

Pro Glu Asp Phe Ala Val Tyr Tyr Cys Gln Gln Gly Gln Val Ile Pro
85 90 95

Pro Thr Phe Gly Gln Gly Thr Lys Val Glu Ile Lys
100 105

<210> 28
<211> 117
<212> PRT
<213> Artificial Sequence

<220>
<223> FAP(4B9) VH

<400> 28

Glu Val Gln Leu Leu Glu Ser Gly Gly Gly Leu Val Gln Pro Gly Gly
1 5 10 15

Ser Leu Arg Leu Ser Cys Ala Ala Ser Gly Phe Thr Phe Ser Ser Tyr
20 25 30

Ala Met Ser Trp Val Arg Gln Ala Pro Gly Lys Gly Leu Glu Trp Val
35 40 45

Ser Ala Ile Ile Gly Ser Gly Ala Ser Thr Tyr Tyr Ala Asp Ser Val
50 55 60

Lys Gly Arg Phe Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Ala Lys Gly Trp Phe Gly Gly Phe Asn Tyr Trp Gly Gln Gly Thr Leu
100 105 110

Val Thr Val Ser Ser
115

<210> 29
<211> 108
<212> PRT
<213> Artificial Sequence

<220>
<223> FAP(4B9) VL

<400> 29

Glu Ile Val Leu Thr Gln Ser Pro Gly Thr Leu Ser Leu Ser Pro Gly
1 5 10 15

Glu Arg Ala Thr Leu Ser Cys Arg Ala Ser Gln Ser Val Thr Ser Ser
20 25 30

Tyr Leu Ala Trp Tyr Gln Gln Lys Pro Gly Gln Ala Pro Arg Leu Leu
35 40 45

Ile Asn Val Gly Ser Arg Arg Ala Thr Gly Ile Pro Asp Arg Phe Ser
50 55 60

Gly Ser Gly Ser Gly Thr Asp Phe Thr Leu Thr Ile Ser Arg Leu Glu
65 70 75 80

Pro Glu Asp Phe Ala Val Tyr Tyr Cys Gln Gln Gly Ile Met Leu Pro
85 90 95

Pro Thr Phe Gly Gln Gly Thr Lys Val Glu Ile Lys
100 105

<210> 30
<211> 760
<212> PRT
<213> human

<400> 30

Met Lys Thr Trp Val Lys Ile Val Phe Gly Val Ala Thr Ser Ala Val
1 5 10 15

Leu Ala Leu Leu Val Met Cys Ile Val Leu Arg Pro Ser Arg Val His

```
            20                  25                  30

Asn Ser Glu Glu Asn Thr Met Arg Ala Leu Thr Leu Lys Asp Ile Leu
        35                  40                  45

Asn Gly Thr Phe Ser Tyr Lys Thr Phe Phe Pro Asn Trp Ile Ser Gly
    50                  55                  60

Gln Glu Tyr Leu His Gln Ser Ala Asp Asn Asn Ile Val Leu Tyr Asn
65                  70                  75                  80

Ile Glu Thr Gly Gln Ser Tyr Thr Ile Leu Ser Asn Arg Thr Met Lys
                85                  90                  95

Ser Val Asn Ala Ser Asn Tyr Gly Leu Ser Pro Asp Arg Gln Phe Val
            100                 105                 110

Tyr Leu Glu Ser Asp Tyr Ser Lys Leu Trp Arg Tyr Ser Tyr Thr Ala
        115                 120                 125

Thr Tyr Tyr Ile Tyr Asp Leu Ser Asn Gly Glu Phe Val Arg Gly Asn
    130                 135                 140

Glu Leu Pro Arg Pro Ile Gln Tyr Leu Cys Trp Ser Pro Val Gly Ser
145                 150                 155                 160

Lys Leu Ala Tyr Val Tyr Gln Asn Asn Ile Tyr Leu Lys Gln Arg Pro
                165                 170                 175

Gly Asp Pro Pro Phe Gln Ile Thr Phe Asn Gly Arg Glu Asn Lys Ile
            180                 185                 190

Phe Asn Gly Ile Pro Asp Trp Val Tyr Glu Glu Glu Met Leu Ala Thr
        195                 200                 205

Lys Tyr Ala Leu Trp Trp Ser Pro Asn Gly Lys Phe Leu Ala Tyr Ala
    210                 215                 220

Glu Phe Asn Asp Thr Asp Ile Pro Val Ile Ala Tyr Ser Tyr Tyr Gly
225                 230                 235                 240

Asp Glu Gln Tyr Pro Arg Thr Ile Asn Ile Pro Tyr Pro Lys Ala Gly
                245                 250                 255

Ala Lys Asn Pro Val Val Arg Ile Phe Ile Ile Asp Thr Thr Tyr Pro
            260                 265                 270
```

```
Ala Tyr Val Gly Pro Gln Glu Val Pro Val Pro Ala Met Ile Ala Ser
        275                 280                 285

Ser Asp Tyr Tyr Phe Ser Trp Leu Thr Trp Val Thr Asp Glu Arg Val
    290                 295                 300

Cys Leu Gln Trp Leu Lys Arg Val Gln Asn Val Ser Val Leu Ser Ile
305                 310                 315                 320

Cys Asp Phe Arg Glu Asp Trp Gln Thr Trp Asp Cys Pro Lys Thr Gln
                325                 330                 335

Glu His Ile Glu Glu Ser Arg Thr Gly Trp Ala Gly Gly Phe Phe Val
            340                 345                 350

Ser Thr Pro Val Phe Ser Tyr Asp Ala Ile Ser Tyr Tyr Lys Ile Phe
        355                 360                 365

Ser Asp Lys Asp Gly Tyr Lys His Ile His Tyr Ile Lys Asp Thr Val
    370                 375                 380

Glu Asn Ala Ile Gln Ile Thr Ser Gly Lys Trp Glu Ala Ile Asn Ile
385                 390                 395                 400

Phe Arg Val Thr Gln Asp Ser Leu Phe Tyr Ser Ser Asn Glu Phe Glu
                405                 410                 415

Glu Tyr Pro Gly Arg Arg Asn Ile Tyr Arg Ile Ser Ile Gly Ser Tyr
            420                 425                 430

Pro Pro Ser Lys Lys Cys Val Thr Cys His Leu Arg Lys Glu Arg Cys
        435                 440                 445

Gln Tyr Tyr Thr Ala Ser Phe Ser Asp Tyr Ala Lys Tyr Tyr Ala Leu
    450                 455                 460

Val Cys Tyr Gly Pro Gly Ile Pro Ile Ser Thr Leu His Asp Gly Arg
465                 470                 475                 480

Thr Asp Gln Glu Ile Lys Ile Leu Glu Glu Asn Lys Glu Leu Glu Asn
                485                 490                 495

Ala Leu Lys Asn Ile Gln Leu Pro Lys Glu Glu Ile Lys Lys Leu Glu
            500                 505                 510

Val Asp Glu Ile Thr Leu Trp Tyr Lys Met Ile Leu Pro Pro Gln Phe
        515                 520                 525
```

```
Asp Arg Ser Lys Lys Tyr Pro Leu Leu Ile Gln Val Tyr Gly Gly Pro
    530                 535                 540

Cys Ser Gln Ser Val Arg Ser Val Phe Ala Val Asn Trp Ile Ser Tyr
545                 550                 555                 560

Leu Ala Ser Lys Glu Gly Met Val Ile Ala Leu Val Asp Gly Arg Gly
                565                 570                 575

Thr Ala Phe Gln Gly Asp Lys Leu Leu Tyr Ala Val Tyr Arg Lys Leu
            580                 585                 590

Gly Val Tyr Glu Val Glu Asp Gln Ile Thr Ala Val Arg Lys Phe Ile
        595                 600                 605

Glu Met Gly Phe Ile Asp Glu Lys Arg Ile Ala Ile Trp Gly Trp Ser
    610                 615                 620

Tyr Gly Gly Tyr Val Ser Ser Leu Ala Leu Ala Ser Gly Thr Gly Leu
625                 630                 635                 640

Phe Lys Cys Gly Ile Ala Val Ala Pro Val Ser Ser Trp Glu Tyr Tyr
                645                 650                 655

Ala Ser Val Tyr Thr Glu Arg Phe Met Gly Leu Pro Thr Lys Asp Asp
            660                 665                 670

Asn Leu Glu His Tyr Lys Asn Ser Thr Val Met Ala Arg Ala Glu Tyr
        675                 680                 685

Phe Arg Asn Val Asp Tyr Leu Leu Ile His Gly Thr Ala Asp Asp Asn
    690                 695                 700

Val His Phe Gln Asn Ser Ala Gln Ile Ala Lys Ala Leu Val Asn Ala
705                 710                 715                 720

Gln Val Asp Phe Gln Ala Met Trp Tyr Ser Asp Gln Asn His Gly Leu
                725                 730                 735

Ser Gly Leu Ser Thr Asn His Leu Tyr Thr His Met Thr His Phe Leu
            740                 745                 750

Lys Gln Cys Phe Ser Leu Ser Asp
        755                 760
```

<210> 31
<211> 748

<212> PRT
<213> Artificial Sequence

<220>
<223> hu FAP ectodomain+poly-lys-tag+his6-tag

<400> 31

Arg Pro Ser Arg Val His Asn Ser Glu Glu Asn Thr Met Arg Ala Leu
1 5 10 15

Thr Leu Lys Asp Ile Leu Asn Gly Thr Phe Ser Tyr Lys Thr Phe Phe
20 25 30

Pro Asn Trp Ile Ser Gly Gln Glu Tyr Leu His Gln Ser Ala Asp Asn
35 40 45

Asn Ile Val Leu Tyr Asn Ile Glu Thr Gly Gln Ser Tyr Thr Ile Leu
50 55 60

Ser Asn Arg Thr Met Lys Ser Val Asn Ala Ser Asn Tyr Gly Leu Ser
65 70 75 80

Pro Asp Arg Gln Phe Val Tyr Leu Glu Ser Asp Tyr Ser Lys Leu Trp
85 90 95

Arg Tyr Ser Tyr Thr Ala Thr Tyr Tyr Ile Tyr Asp Leu Ser Asn Gly
100 105 110

Glu Phe Val Arg Gly Asn Glu Leu Pro Arg Pro Ile Gln Tyr Leu Cys
115 120 125

Trp Ser Pro Val Gly Ser Lys Leu Ala Tyr Val Tyr Gln Asn Asn Ile
130 135 140

Tyr Leu Lys Gln Arg Pro Gly Asp Pro Pro Phe Gln Ile Thr Phe Asn
145 150 155 160

Gly Arg Glu Asn Lys Ile Phe Asn Gly Ile Pro Asp Trp Val Tyr Glu
165 170 175

Glu Glu Met Leu Ala Thr Lys Tyr Ala Leu Trp Trp Ser Pro Asn Gly
180 185 190

Lys Phe Leu Ala Tyr Ala Glu Phe Asn Asp Thr Asp Ile Pro Val Ile
195 200 205

Ala Tyr Ser Tyr Tyr Gly Asp Glu Gln Tyr Pro Arg Thr Ile Asn Ile
210 215 220

Pro Tyr Pro Lys Ala Gly Ala Lys Asn Pro Val Val Arg Ile Phe Ile
225 230 235 240

Ile Asp Thr Thr Tyr Pro Ala Tyr Val Gly Pro Gln Glu Val Pro Val
245 250 255

Pro Ala Met Ile Ala Ser Ser Asp Tyr Tyr Phe Ser Trp Leu Thr Trp
260 265 270

Val Thr Asp Glu Arg Val Cys Leu Gln Trp Leu Lys Arg Val Gln Asn
275 280 285

Val Ser Val Leu Ser Ile Cys Asp Phe Arg Glu Asp Trp Gln Thr Trp
290 295 300

Asp Cys Pro Lys Thr Gln Glu His Ile Glu Glu Ser Arg Thr Gly Trp
305 310 315 320

Ala Gly Gly Phe Phe Val Ser Thr Pro Val Phe Ser Tyr Asp Ala Ile
325 330 335

Ser Tyr Tyr Lys Ile Phe Ser Asp Lys Asp Gly Tyr Lys His Ile His
340 345 350

Tyr Ile Lys Asp Thr Val Glu Asn Ala Ile Gln Ile Thr Ser Gly Lys
355 360 365

Trp Glu Ala Ile Asn Ile Phe Arg Val Thr Gln Asp Ser Leu Phe Tyr
370 375 380

Ser Ser Asn Glu Phe Glu Glu Tyr Pro Gly Arg Arg Asn Ile Tyr Arg
385 390 395 400

Ile Ser Ile Gly Ser Tyr Pro Pro Ser Lys Lys Cys Val Thr Cys His
405 410 415

Leu Arg Lys Glu Arg Cys Gln Tyr Tyr Thr Ala Ser Phe Ser Asp Tyr
420 425 430

Ala Lys Tyr Tyr Ala Leu Val Cys Tyr Gly Pro Gly Ile Pro Ile Ser
435 440 445

Thr Leu His Asp Gly Arg Thr Asp Gln Glu Ile Lys Ile Leu Glu Glu
450 455 460

Asn Lys Glu Leu Glu Asn Ala Leu Lys Asn Ile Gln Leu Pro Lys Glu
465 470 475 480

Glu Ile Lys Lys Leu Glu Val Asp Glu Ile Thr Leu Trp Tyr Lys Met
485 490 495

Ile Leu Pro Pro Gln Phe Asp Arg Ser Lys Lys Tyr Pro Leu Leu Ile
500 505 510

Gln Val Tyr Gly Gly Pro Cys Ser Gln Ser Val Arg Ser Val Phe Ala
515 520 525

Val Asn Trp Ile Ser Tyr Leu Ala Ser Lys Glu Gly Met Val Ile Ala
530 535 540

Leu Val Asp Gly Arg Gly Thr Ala Phe Gln Gly Asp Lys Leu Leu Tyr
545 550 555 560

Ala Val Tyr Arg Lys Leu Gly Val Tyr Glu Val Glu Asp Gln Ile Thr
565 570 575

Ala Val Arg Lys Phe Ile Glu Met Gly Phe Ile Asp Glu Lys Arg Ile
580 585 590

Ala Ile Trp Gly Trp Ser Tyr Gly Gly Tyr Val Ser Ser Leu Ala Leu
595 600 605

Ala Ser Gly Thr Gly Leu Phe Lys Cys Gly Ile Ala Val Ala Pro Val
610 615 620

Ser Ser Trp Glu Tyr Tyr Ala Ser Val Tyr Thr Glu Arg Phe Met Gly
625 630 635 640

Leu Pro Thr Lys Asp Asp Asn Leu Glu His Tyr Lys Asn Ser Thr Val
645 650 655

Met Ala Arg Ala Glu Tyr Phe Arg Asn Val Asp Tyr Leu Leu Ile His
660 665 670

Gly Thr Ala Asp Asp Asn Val His Phe Gln Asn Ser Ala Gln Ile Ala
675 680 685

Lys Ala Leu Val Asn Ala Gln Val Asp Phe Gln Ala Met Trp Tyr Ser
690 695 700

Asp Gln Asn His Gly Leu Ser Gly Leu Ser Thr Asn His Leu Tyr Thr
705 710 715 720

His Met Thr His Phe Leu Lys Gln Cys Phe Ser Leu Ser Asp Gly Lys

725 730 735

Lys Lys Lys Lys Lys Gly His His His His His His
740 745

<210> 32
<211> 2244
<212> DNA
<213> Artificial Sequence

<220>
<223> nucleotide sequence hu FAP ectodomain+poly-lys-tag+his6-tag

<400> 32
cgcccttcaa gagttcataa ctctgaagaa aatacaatga gagcactcac actgaaggat 60

attttaaatg gaacattttc ttataaaaca tttttccaa actggatttc aggacaagaa 120

tatcttcatc aatctgcaga taacaatata gtactttata atattgaaac aggacaatca 180

tataccattt tgagtaatag aaccatgaaa agtgtgaatg cttcaaatta cggcttatca 240

cctgatcggc aatttgtata tctagaaagt gattattcaa agctttggag atactcttac 300

acagcaacat attacatcta tgaccttagc aatggagaat ttgtaagagg aaatgagctt 360

cctcgtccaa ttcagtattt atgctggtcg cctgttggga gtaaattagc atatgtctat 420

caaaacaata tctatttgaa acaaagacca ggagatccac cttttcaaat aacatttaat 480

ggaagagaaa ataaaatatt taatggaatc ccagactggg tttatgaaga ggaaatgctt 540

gctacaaaat atgctctctg gtggtctcct aatggaaaat ttttggcata tgcggaattt 600

aatgatacgg atataccagt tattgcctat tcctattatg gcgatgaaca atatcctaga 660

acaataaata ttccataccc aaaggctgga gctaagaatc ccgttgttcg gatatttatt 720

atcgatacca cttaccctgc gtatgtaggt ccccaggaag tgcctgttcc agcaatgata 780

gcctcaagtg attattattt cagttggctc acgtgggtta ctgatgaacg agtatgtttg 840

cagtggctaa aaagagtcca gaatgtttcg gtcctgtcta tatgtgactt cagggaagac 900

tggcagacat gggattgtcc aaagacccag gagcatatag aagaaagcag aactggatgg 960

gctggtggat tctttgtttc aacaccagtt ttcagctatg atgccatttc gtactacaaa 1020

atatttagtg acaaggatgg ctacaaacat attcactata tcaaagacac tgtggaaaat 1080

gctattcaaa ttacaagtgg caagtgggag gccataaata tattcagagt aacacaggat 1140

tcactgtttt attctagcaa tgaatttgaa gaataccctg gaagaagaaa catctacaga 1200

attagcattg gaagctatcc tccaagcaag aagtgtgtta cttgccatct aaggaaagaa 1260

aggtgccaat attacacagc aagtttcagc gactacgcca agtactatgc acttgtctgc 1320

tacggcccag gcatccccat ttccaccctt catgatggac gcactgatca agaaattaaa 1380

atcctggaag aaaacaagga attggaaaat gctttgaaaa atatccagct gcctaaagag 1440

gaaattaaga aacttgaagt agatgaaatt actttatggt acaagatgat tcttcctcct 1500

caatttgaca gatcaaagaa gtatcccttg ctaattcaag tgtatggtgg tccctgcagt 1560

cagagtgtaa ggtctgtatt tgctgttaat tggatatctt atcttgcaag taaggaaggg 1620

atggtcattg ccttggtgga tggtcgagga acagctttcc aaggtgacaa actcctctat 1680

gcagtgtatc gaaagctggg tgtttatgaa gttgaagacc agattacagc tgtcagaaaa 1740

ttcatagaaa tgggtttcat tgatgaaaaa agaatagcca tatggggctg gtcctatgga 1800

ggatacgttt catcactggc ccttgcatct ggaactggtc ttttcaaatg tggtatagca 1860

gtggctccag tctccagctg ggaatattac gcgtctgtct acacagagag attcatgggt 1920

ctcccaacaa aggatgataa tcttgagcac tataagaatt caactgtgat ggcaagagca 1980

gaatatttca gaaatgtaga ctatcttctc atccacggaa cagcagatga taatgtgcac 2040

tttcaaaact cagcacagat tgctaaagct ctggttaatg cacaagtgga tttccaggca 2100

atgtggtact ctgaccagaa ccacggctta tccggcctgt ccacgaacca cttatacacc 2160

cacatgaccc acttcctaaa gcagtgtttc tctttgtcag acggcaaaaa gaaaaagaaa 2220

aagggccacc accatcacca tcac 2244

<210> 33
<211> 761
<212> PRT
<213> murine

<400> 33

Met Lys Thr Trp Leu Lys Thr Val Phe Gly Val Thr Thr Leu Ala Ala
1 5 10 15

Leu Ala Leu Val Val Ile Cys Ile Val Leu Arg Pro Ser Arg Val Tyr
20 25 30

Lys Pro Glu Gly Asn Thr Lys Arg Ala Leu Thr Leu Lys Asp Ile Leu
35 40 45

Asn Gly Thr Phe Ser Tyr Lys Thr Tyr Phe Pro Asn Trp Ile Ser Glu
50 55 60

Gln Glu Tyr Leu His Gln Ser Glu Asp Asp Asn Ile Val Phe Tyr Asn
65 70 75 80

Ile Glu Thr Arg Glu Ser Tyr Ile Ile Leu Ser Asn Ser Thr Met Lys
85 90 95

Ser Val Asn Ala Thr Asp Tyr Gly Leu Ser Pro Asp Arg Gln Phe Val
100 105 110

```
Tyr Leu Glu Ser Asp Tyr Ser Lys Leu Trp Arg Tyr Ser Tyr Thr Ala
        115                 120                 125

Thr Tyr Tyr Ile Tyr Asp Leu Gln Asn Gly Glu Phe Val Arg Gly Tyr
    130                 135                 140

Glu Leu Pro Arg Pro Ile Gln Tyr Leu Cys Trp Ser Pro Val Gly Ser
145                 150                 155                 160

Lys Leu Ala Tyr Val Tyr Gln Asn Asn Ile Tyr Leu Lys Gln Arg Pro
                165                 170                 175

Gly Asp Pro Pro Phe Gln Ile Thr Tyr Thr Gly Arg Glu Asn Arg Ile
            180                 185                 190

Phe Asn Gly Ile Pro Asp Trp Val Tyr Glu Glu Glu Met Leu Ala Thr
        195                 200                 205

Lys Tyr Ala Leu Trp Trp Ser Pro Asp Gly Lys Phe Leu Ala Tyr Val
    210                 215                 220

Glu Phe Asn Asp Ser Asp Ile Pro Ile Ile Ala Tyr Ser Tyr Tyr Gly
225                 230                 235                 240

Asp Gly Gln Tyr Pro Arg Thr Ile Asn Ile Pro Tyr Pro Lys Ala Gly
                245                 250                 255

Ala Lys Asn Pro Val Val Arg Val Phe Ile Val Asp Thr Thr Tyr Pro
            260                 265                 270

His His Val Gly Pro Met Glu Val Pro Val Pro Glu Met Ile Ala Ser
        275                 280                 285

Ser Asp Tyr Tyr Phe Ser Trp Leu Thr Trp Val Ser Ser Glu Arg Val
    290                 295                 300

Cys Leu Gln Trp Leu Lys Arg Val Gln Asn Val Ser Val Leu Ser Ile
305                 310                 315                 320

Cys Asp Phe Arg Glu Asp Trp His Ala Trp Glu Cys Pro Lys Asn Gln
                325                 330                 335

Glu His Val Glu Glu Ser Arg Thr Gly Trp Ala Gly Gly Phe Phe Val
            340                 345                 350

Ser Thr Pro Ala Phe Ser Gln Asp Ala Thr Ser Tyr Tyr Lys Ile Phe
```

355 360 365

Ser Asp Lys Asp Gly Tyr Lys His Ile His Tyr Ile Lys Asp Thr Val
370 375 380

Glu Asn Ala Ile Gln Ile Thr Ser Gly Lys Trp Glu Ala Ile Tyr Ile
385 390 395 400

Phe Arg Val Thr Gln Asp Ser Leu Phe Tyr Ser Ser Asn Glu Phe Glu
405 410 415

Gly Tyr Pro Gly Arg Arg Asn Ile Tyr Arg Ile Ser Ile Gly Asn Ser
420 425 430

Pro Pro Ser Lys Lys Cys Val Thr Cys His Leu Arg Lys Glu Arg Cys
435 440 445

Gln Tyr Tyr Thr Ala Ser Phe Ser Tyr Lys Ala Lys Tyr Tyr Ala Leu
450 455 460

Val Cys Tyr Gly Pro Gly Leu Pro Ile Ser Thr Leu His Asp Gly Arg
465 470 475 480

Thr Asp Gln Glu Ile Gln Val Leu Glu Glu Asn Lys Glu Leu Glu Asn
485 490 495

Ser Leu Arg Asn Ile Gln Leu Pro Lys Val Glu Ile Lys Lys Leu Lys
500 505 510

Asp Gly Gly Leu Thr Phe Trp Tyr Lys Met Ile Leu Pro Pro Gln Phe
515 520 525

Asp Arg Ser Lys Lys Tyr Pro Leu Leu Ile Gln Val Tyr Gly Gly Pro
530 535 540

Cys Ser Gln Ser Val Lys Ser Val Phe Ala Val Asn Trp Ile Thr Tyr
545 550 555 560

Leu Ala Ser Lys Glu Gly Ile Val Ile Ala Leu Val Asp Gly Arg Gly
565 570 575

Thr Ala Phe Gln Gly Asp Lys Phe Leu His Ala Val Tyr Arg Lys Leu
580 585 590

Gly Val Tyr Glu Val Glu Asp Gln Leu Thr Ala Val Arg Lys Phe Ile
595 600 605

Glu Met Gly Phe Ile Asp Glu Glu Arg Ile Ala Ile Trp Gly Trp Ser
610 615 620

Tyr Gly Gly Tyr Val Ser Ser Leu Ala Leu Ala Ser Gly Thr Gly Leu
625 630 635 640

Phe Lys Cys Gly Ile Ala Val Ala Pro Val Ser Ser Trp Glu Tyr Tyr
645 650 655

Ala Ser Ile Tyr Ser Glu Arg Phe Met Gly Leu Pro Thr Lys Asp Asp
660 665 670

Asn Leu Glu His Tyr Lys Asn Ser Thr Val Met Ala Arg Ala Glu Tyr
675 680 685

Phe Arg Asn Val Asp Tyr Leu Leu Ile His Gly Thr Ala Asp Asp Asn
690 695 700

Val His Phe Gln Asn Ser Ala Gln Ile Ala Lys Ala Leu Val Asn Ala
705 710 715 720

Gln Val Asp Phe Gln Ala Met Trp Tyr Ser Asp Gln Asn His Gly Ile
725 730 735

Ser Ser Gly Arg Ser Gln Asn His Leu Tyr Thr His Met Thr His Phe
740 745 750

Leu Lys Gln Cys Phe Ser Leu Ser Asp
755 760

<210> 34
<211> 749
<212> PRT
<213> Artificial Sequence

<220>
<223> Murine FAP ectodomain+poly-lys-tag+his6-tag

<400> 34

Arg Pro Ser Arg Val Tyr Lys Pro Glu Gly Asn Thr Lys Arg Ala Leu
1 5 10 15

Thr Leu Lys Asp Ile Leu Asn Gly Thr Phe Ser Tyr Lys Thr Tyr Phe
20 25 30

Pro Asn Trp Ile Ser Glu Gln Glu Tyr Leu His Gln Ser Glu Asp Asp
35 40 45

Asn Ile Val Phe Tyr Asn Ile Glu Thr Arg Glu Ser Tyr Ile Ile Leu

50 55 60

Ser Asn Ser Thr Met Lys Ser Val Asn Ala Thr Asp Tyr Gly Leu Ser
65 70 75 80

Pro Asp Arg Gln Phe Val Tyr Leu Glu Ser Asp Tyr Ser Lys Leu Trp
85 90 95

Arg Tyr Ser Tyr Thr Ala Thr Tyr Tyr Ile Tyr Asp Leu Gln Asn Gly
100 105 110

Glu Phe Val Arg Gly Tyr Glu Leu Pro Arg Pro Ile Gln Tyr Leu Cys
115 120 125

Trp Ser Pro Val Gly Ser Lys Leu Ala Tyr Val Tyr Gln Asn Asn Ile
130 135 140

Tyr Leu Lys Gln Arg Pro Gly Asp Pro Pro Phe Gln Ile Thr Tyr Thr
145 150 155 160

Gly Arg Glu Asn Arg Ile Phe Asn Gly Ile Pro Asp Trp Val Tyr Glu
165 170 175

Glu Glu Met Leu Ala Thr Lys Tyr Ala Leu Trp Trp Ser Pro Asp Gly
180 185 190

Lys Phe Leu Ala Tyr Val Glu Phe Asn Asp Ser Asp Ile Pro Ile Ile
195 200 205

Ala Tyr Ser Tyr Tyr Gly Asp Gly Gln Tyr Pro Arg Thr Ile Asn Ile
210 215 220

Pro Tyr Pro Lys Ala Gly Ala Lys Asn Pro Val Val Arg Val Phe Ile
225 230 235 240

Val Asp Thr Thr Tyr Pro His His Val Gly Pro Met Glu Val Pro Val
245 250 255

Pro Glu Met Ile Ala Ser Ser Asp Tyr Tyr Phe Ser Trp Leu Thr Trp
260 265 270

Val Ser Ser Glu Arg Val Cys Leu Gln Trp Leu Lys Arg Val Gln Asn
275 280 285

Val Ser Val Leu Ser Ile Cys Asp Phe Arg Glu Asp Trp His Ala Trp
290 295 300

```
Glu Cys Pro Lys Asn Gln Glu His Val Glu Glu Ser Arg Thr Gly Trp
305                 310                 315                 320

Ala Gly Gly Phe Phe Val Ser Thr Pro Ala Phe Ser Gln Asp Ala Thr
                325                 330                 335

Ser Tyr Tyr Lys Ile Phe Ser Asp Lys Asp Gly Tyr Lys His Ile His
            340                 345                 350

Tyr Ile Lys Asp Thr Val Glu Asn Ala Ile Gln Ile Thr Ser Gly Lys
        355                 360                 365

Trp Glu Ala Ile Tyr Ile Phe Arg Val Thr Gln Asp Ser Leu Phe Tyr
    370                 375                 380

Ser Ser Asn Glu Phe Glu Gly Tyr Pro Gly Arg Arg Asn Ile Tyr Arg
385                 390                 395                 400

Ile Ser Ile Gly Asn Ser Pro Pro Ser Lys Lys Cys Val Thr Cys His
                405                 410                 415

Leu Arg Lys Glu Arg Cys Gln Tyr Tyr Thr Ala Ser Phe Ser Tyr Lys
            420                 425                 430

Ala Lys Tyr Tyr Ala Leu Val Cys Tyr Gly Pro Gly Leu Pro Ile Ser
        435                 440                 445

Thr Leu His Asp Gly Arg Thr Asp Gln Glu Ile Gln Val Leu Glu Glu
    450                 455                 460

Asn Lys Glu Leu Glu Asn Ser Leu Arg Asn Ile Gln Leu Pro Lys Val
465                 470                 475                 480

Glu Ile Lys Lys Leu Lys Asp Gly Gly Leu Thr Phe Trp Tyr Lys Met
                485                 490                 495

Ile Leu Pro Pro Gln Phe Asp Arg Ser Lys Lys Tyr Pro Leu Leu Ile
            500                 505                 510

Gln Val Tyr Gly Gly Pro Cys Ser Gln Ser Val Lys Ser Val Phe Ala
        515                 520                 525

Val Asn Trp Ile Thr Tyr Leu Ala Ser Lys Glu Gly Ile Val Ile Ala
    530                 535                 540

Leu Val Asp Gly Arg Gly Thr Ala Phe Gln Gly Asp Lys Phe Leu His
545                 550                 555                 560
```

Ala Val Tyr Arg Lys Leu Gly Val Tyr Glu Val Glu Asp Gln Leu Thr
565 570 575

Ala Val Arg Lys Phe Ile Glu Met Gly Phe Ile Asp Glu Glu Arg Ile
580 585 590

Ala Ile Trp Gly Trp Ser Tyr Gly Gly Tyr Val Ser Ser Leu Ala Leu
595 600 605

Ala Ser Gly Thr Gly Leu Phe Lys Cys Gly Ile Ala Val Ala Pro Val
610 615 620

Ser Ser Trp Glu Tyr Tyr Ala Ser Ile Tyr Ser Glu Arg Phe Met Gly
625 630 635 640

Leu Pro Thr Lys Asp Asp Asn Leu Glu His Tyr Lys Asn Ser Thr Val
645 650 655

Met Ala Arg Ala Glu Tyr Phe Arg Asn Val Asp Tyr Leu Leu Ile His
660 665 670

Gly Thr Ala Asp Asp Asn Val His Phe Gln Asn Ser Ala Gln Ile Ala
675 680 685

Lys Ala Leu Val Asn Ala Gln Val Asp Phe Gln Ala Met Trp Tyr Ser
690 695 700

Asp Gln Asn His Gly Ile Leu Ser Gly Arg Ser Gln Asn His Leu Tyr
705 710 715 720

Thr His Met Thr His Phe Leu Lys Gln Cys Phe Ser Leu Ser Asp Gly
725 730 735

Lys Lys Lys Lys Lys Lys Gly His His His His His His
740 745

<210> 35
<211> 2247
<212> DNA
<213> Artificial Sequence

<220>
<223> nucleotide sequence of murine FAP
ectodomain+poly-lys-tag+his6-tag

<400> 35
cgtccctcaa gagtttacaa acctgaagga aacacaaaga gagctcttac cttgaaggat 60

attttaaatg gaacattctc atataaaaca tattttccca actggatttc agaacaagaa 120

tatcttcatc aatctgagga tgataacata gtattttata atattgaaac aagagaatca 180
tatatcattt tgagtaatag caccatgaaa agtgtgaatg ctacagatta tggtttgtca 240
cctgatcggc aatttgtgta tctagaaagt gattattcaa agctctggcg atattcatac 300
acagcgacat actacatcta cgaccttcag aatggggaat ttgtaagagg atacgagctc 360
cctcgtccaa ttcagtatct atgctggtcg cctgttggga gtaaattagc atatgtatat 420
caaaacaata tttatttgaa acaaagacca ggagatccac cttttcaaat aacttatact 480
ggaagagaaa atagaatatt taatggaata ccagactggg tttatgaaga ggaaatgctt 540
gccacaaaat atgctctttg gtggtctcca gatggaaaat tttggcatat tgtagaattt 600
aatgattcag atataccaat tattgcctat tcttattatg gtgatggaca gtatcctaga 660
actataaata ttccatatcc aaaggctggg gctaagaatc cggttgttcg tgtttttatt 720
gttgacacca cctaccctca ccacgtgggc ccaatggaag tgccagttcc agaaatgata 780
gcctcaagtg actattattt cagctggctc acatgggtgt ccagtgaacg agtatgcttg 840
cagtggctaa aaagagtgca gaatgtctca gtcctgtcta tatgtgattt cagggaagac 900
tggcatgcat gggaatgtcc aaagaaccag gagcatgtag aagaaagcag aacaggatgg 960
gctggtggat tctttgtttc gacaccagct tttagccagg atgccacttc ttactacaaa 1020
atatttagcg acaaggatgg ttacaaacat attcactaca tcaaagacac tgtggaaaat 1080
gctattcaaa ttacaagtgg caagtgggag gccatatata tattccgcgt aacacaggat 1140
tcactgtttt attctagcaa tgaatttgaa ggttaccctg gaagaagaaa catctacaga 1200
attagcattg gaaactctcc tccgagcaag aagtgtgtta cttgccatct aaggaaagaa 1260
aggtgccaat attacacagc aagtttcagc tacaaagcca agtactatgc actcgtctgc 1320
tatggccctg gcctccccat ttccaccctc catgatggcc gcacagacca agaaatacaa 1380
gtattagaag aaaacaaaga actggaaaat tctctgagaa atatccagct gcctaaagtg 1440
gagattaaga agctcaaaga cgggggactg actttctggt acaagatgat tctgcctcct 1500
cagtttgaca gatcaaagaa gtaccctttg ctaattcaag tgtatggtgg tccttgtagc 1560
cagagtgtta agtctgtgtt tgctgttaat tggataactt atctcgcaag taaggagggg 1620
atagtcattg ccctggtaga tggtcggggc actgctttcc aaggtgacaa attcctgcat 1680
gccgtgtatc gaaaactggg tgtatatgaa gttgaggacc agctcacagc tgtcagaaaa 1740
ttcatagaaa tgggtttcat tgatgaagaa agaatagcca tatggggctg gtcctacgga 1800
ggttatgttt catccctggc ccttgcatct ggaactggtc ttttcaaatg tggcatagca 1860
gtggctccag tctccagctg ggaatattac gcatctatct actcagagag attcatgggc 1920
ctcccaacaa aggacgacaa tctcgaacac tataaaaatt caactgtgat ggcaagagca 1980
gaatatttca gaaatgtaga ctatcttctc atccacggaa cagcagatga taatgtgcac 2040

tttcagaact cagcacagat tgctaaagct ttggttaatg cacaagtgga tttccaggcg 2100

atgtggtact ctgaccagaa ccatggtata ttatctgggc gctcccagaa tcatttatat 2160

acccacatga cgcacttcct caagcaatgc ttttctttat cagacggcaa aaagaaaaag 2220

aaaaagggcc accaccatca ccatcac 2247

<210> 36
<211> 748
<212> PRT
<213> Artificial Sequence

<220>
<223> Cynomolgus FAP ectodomain+poly-lys-tag+his6-tag

<400> 36

```
Arg Pro Pro Arg Val His Asn Ser Glu Glu Asn Thr Met Arg Ala Leu
1               5                   10                  15

Thr Leu Lys Asp Ile Leu Asn Gly Thr Phe Ser Tyr Lys Thr Phe Phe
            20                  25                  30

Pro Asn Trp Ile Ser Gly Gln Glu Tyr Leu His Gln Ser Ala Asp Asn
        35                  40                  45

Asn Ile Val Leu Tyr Asn Ile Glu Thr Gly Gln Ser Tyr Thr Ile Leu
    50                  55                  60

Ser Asn Arg Thr Met Lys Ser Val Asn Ala Ser Asn Tyr Gly Leu Ser
65                  70                  75                  80

Pro Asp Arg Gln Phe Val Tyr Leu Glu Ser Asp Tyr Ser Lys Leu Trp
                85                  90                  95

Arg Tyr Ser Tyr Thr Ala Thr Tyr Tyr Ile Tyr Asp Leu Ser Asn Gly
            100                 105                 110

Glu Phe Val Arg Gly Asn Glu Leu Pro Arg Pro Ile Gln Tyr Leu Cys
        115                 120                 125

Trp Ser Pro Val Gly Ser Lys Leu Ala Tyr Val Tyr Gln Asn Asn Ile
    130                 135                 140

Tyr Leu Lys Gln Arg Pro Gly Asp Pro Pro Phe Gln Ile Thr Phe Asn
145                 150                 155                 160

Gly Arg Glu Asn Lys Ile Phe Asn Gly Ile Pro Asp Trp Val Tyr Glu
                165                 170                 175
```

```
Glu Glu Met Leu Ala Thr Lys Tyr Ala Leu Trp Trp Ser Pro Asn Gly
            180                 185                 190

Lys Phe Leu Ala Tyr Ala Glu Phe Asn Asp Thr Asp Ile Pro Val Ile
        195                 200                 205

Ala Tyr Ser Tyr Tyr Gly Asp Glu Gln Tyr Pro Arg Thr Ile Asn Ile
    210                 215                 220

Pro Tyr Pro Lys Ala Gly Ala Lys Asn Pro Phe Val Arg Ile Phe Ile
225                 230                 235                 240

Ile Asp Thr Thr Tyr Pro Ala Tyr Val Gly Pro Gln Glu Val Pro Val
                245                 250                 255

Pro Ala Met Ile Ala Ser Ser Asp Tyr Tyr Phe Ser Trp Leu Thr Trp
            260                 265                 270

Val Thr Asp Glu Arg Val Cys Leu Gln Trp Leu Lys Arg Val Gln Asn
        275                 280                 285

Val Ser Val Leu Ser Ile Cys Asp Phe Arg Glu Asp Trp Gln Thr Trp
    290                 295                 300

Asp Cys Pro Lys Thr Gln Glu His Ile Glu Glu Ser Arg Thr Gly Trp
305                 310                 315                 320

Ala Gly Gly Phe Phe Val Ser Thr Pro Val Phe Ser Tyr Asp Ala Ile
                325                 330                 335

Ser Tyr Tyr Lys Ile Phe Ser Asp Lys Asp Gly Tyr Lys His Ile His
            340                 345                 350

Tyr Ile Lys Asp Thr Val Glu Asn Ala Ile Gln Ile Thr Ser Gly Lys
        355                 360                 365

Trp Glu Ala Ile Asn Ile Phe Arg Val Thr Gln Asp Ser Leu Phe Tyr
    370                 375                 380

Ser Ser Asn Glu Phe Glu Asp Tyr Pro Gly Arg Arg Asn Ile Tyr Arg
385                 390                 395                 400

Ile Ser Ile Gly Ser Tyr Pro Pro Ser Lys Lys Cys Val Thr Cys His
                405                 410                 415

Leu Arg Lys Glu Arg Cys Gln Tyr Tyr Thr Ala Ser Phe Ser Asp Tyr
            420                 425                 430
```

Ala Lys Tyr Tyr Ala Leu Val Cys Tyr Gly Pro Gly Ile Pro Ile Ser
435 440 445

Thr Leu His Asp Gly Arg Thr Asp Gln Glu Ile Lys Ile Leu Glu Glu
450 455 460

Asn Lys Glu Leu Glu Asn Ala Leu Lys Asn Ile Gln Leu Pro Lys Glu
465 470 475 480

Glu Ile Lys Lys Leu Glu Val Asp Glu Ile Thr Leu Trp Tyr Lys Met
485 490 495

Ile Leu Pro Pro Gln Phe Asp Arg Ser Lys Lys Tyr Pro Leu Leu Ile
500 505 510

Gln Val Tyr Gly Gly Pro Cys Ser Gln Ser Val Arg Ser Val Phe Ala
515 520 525

Val Asn Trp Ile Ser Tyr Leu Ala Ser Lys Glu Gly Met Val Ile Ala
530 535 540

Leu Val Asp Gly Arg Gly Thr Ala Phe Gln Gly Asp Lys Leu Leu Tyr
545 550 555 560

Ala Val Tyr Arg Lys Leu Gly Val Tyr Glu Val Glu Asp Gln Ile Thr
565 570 575

Ala Val Arg Lys Phe Ile Glu Met Gly Phe Ile Asp Glu Lys Arg Ile
580 585 590

Ala Ile Trp Gly Trp Ser Tyr Gly Gly Tyr Val Ser Ser Leu Ala Leu
595 600 605

Ala Ser Gly Thr Gly Leu Phe Lys Cys Gly Ile Ala Val Ala Pro Val
610 615 620

Ser Ser Trp Glu Tyr Tyr Ala Ser Val Tyr Thr Glu Arg Phe Met Gly
625 630 635 640

Leu Pro Thr Lys Asp Asp Asn Leu Glu His Tyr Lys Asn Ser Thr Val
645 650 655

Met Ala Arg Ala Glu Tyr Phe Arg Asn Val Asp Tyr Leu Leu Ile His
660 665 670

Gly Thr Ala Asp Asp Asn Val His Phe Gln Asn Ser Ala Gln Ile Ala

```
        675                 680                 685

Lys Ala Leu Val Asn Ala Gln Val Asp Phe Gln Ala Met Trp Tyr Ser
    690                 695                 700

Asp Gln Asn His Gly Leu Ser Gly Leu Ser Thr Asn His Leu Tyr Thr
705                 710                 715                 720

His Met Thr His Phe Leu Lys Gln Cys Phe Ser Leu Ser Asp Gly Lys
                725                 730                 735

Lys Lys Lys Lys Lys Gly His His His His His His
            740                 745
```

<210>  37
<211>  2244
<212>  DNA
<213>  Artificial Sequence

<220>
<223>  nucleotide sequence of cynomolgus FAP
       ectodomain+poly-lys-tag+his6-tag

<400>  37

```
cgccctccaa gagttcataa ctctgaagaa aatacaatga gagcactcac actgaaggat      60

attttaaatg ggacattttc ttataaaaca ttttttccaa actggatttc aggacaagaa     120

tatcttcatc aatctgcaga taacaatata gtactttata atattgaaac aggacaatca     180

tataccattt tgagtaacag aaccatgaaa agtgtgaatg cttcaaatta tggcttatca     240

cctgatcggc aatttgtata tctagaaagt gattattcaa agctttggag atactcttac     300

acagcaacat attacatcta tgaccttagc aatggagaat ttgtaagagg aaatgagctt     360

cctcgtccaa ttcagtattt atgctggtcg cctgttggga gtaaattagc atatgtctat     420

caaaacaata tctatttgaa acaaagacca ggagatccac cttttcaaat aacatttaat     480

ggaagagaaa ataaaatatt taatggaatc ccagactggg tttatgaaga ggaaatgctt     540

gctacaaaat atgctctctg gtggtctcct aatggaaaat tttggcatat tgcggaattt     600

aatgatacag atataccagt tattgcctat tcctattatg gcgatgaaca atatcccaga     660

acaataaata ttccataccc aaaggccgga gctaagaatc cttttgttcg gatatttatt     720

atcgatacca cttaccctgc gtatgtaggt ccccaggaag tgcctgttcc agcaatgata     780

gcctcaagtg attattattt cagttggctc acgtgggtta ctgatgaacg agtatgtttg     840

cagtggctaa aaagagtcca gaatgtttcg gtcttgtcta tatgtgattt cagggaagac     900

tggcagacat gggattgtcc aaagacccag gagcatatag aagaaagcag aactggatgg     960

gctggtggat tctttgtttc aacaccagtt ttcagctatg atgccatttc atactacaaa    1020
```

atatttagtg acaaggatgg ctacaaacat attcactata tcaaagacac tgtggaaaat 1080

gctattcaaa ttacaagtgg caagtgggag gccataaata tattcagagt aacacaggat 1140

tcactgtttt attctagcaa tgaatttgaa gattaccctg gaagaagaaa catctacaga 1200

attagcattg gaagctatcc tccaagcaag aagtgtgtta cttgccatct aaggaaagaa 1260

aggtgccaat attacacagc aagtttcagc gactacgcca agtactatgc acttgtctgc 1320

tatggcccag gcatccccat ttccacccctt catgacggac gcactgatca agaaattaaa 1380

atcctggaag aaaacaagga attggaaaat gctttgaaaa atatccagct gcctaaagag 1440

gaaattaaga aacttgaagt agatgaaatt actttatggt acaagatgat tcttcctcct 1500

caatttgaca gatcaaagaa gtatcccttg ctaattcaag tgtatggtgg tccctgcagt 1560

cagagtgtaa ggtctgtatt tgctgttaat tggatatctt atcttgcaag taaggaaggg 1620

atggtcattg ccttggtgga tggtcgggga acagctttcc aaggtgacaa actcctgtat 1680

gcagtgtatc gaaagctggg tgtttatgaa gttgaagacc agattacagc tgtcagaaaa 1740

ttcatagaaa tgggtttcat tgatgaaaaa agaatagcca tatggggctg gtcctatgga 1800

ggatatgttt catcactggc ccttgcatct ggaactggtc ttttcaaatg tgggatagca 1860

gtggctccag tctccagctg ggaatattac gcgtctgtct acacagagag attcatgggt 1920

ctcccaacaa aggatgataa tcttgagcac tataagaatt caactgtgat ggcaagagca 1980

gaatatttca gaaatgtaga ctatcttctc atccacggaa cagcagatga taatgtgcac 2040

tttcaaaact cagcacagat tgctaaagct ctggttaatg cacaagtgga tttccaggca 2100

atgtggtact ctgaccagaa ccacggctta tccggcctgt ccacgaacca cttatacacc 2160

cacatgaccc acttcctaaa gcagtgtttc tctttgtcag acggcaaaaa gaaaaagaaa 2220

aagggccacc accatcacca tcac 2244

<210> 38
<211> 702
<212> PRT
<213> human

<400> 38

Met Glu Ser Pro Ser Ala Pro Pro His Arg Trp Cys Ile Pro Trp Gln
1 5 10 15

Arg Leu Leu Leu Thr Ala Ser Leu Leu Thr Phe Trp Asn Pro Pro Thr
20 25 30

Thr Ala Lys Leu Thr Ile Glu Ser Thr Pro Phe Asn Val Ala Glu Gly
35 40 45

Lys Glu Val Leu Leu Leu Val His Asn Leu Pro Gln His Leu Phe Gly

```
        50                  55                  60

Tyr Ser Trp Tyr Lys Gly Glu Arg Val Asp Gly Asn Arg Gln Ile Ile
65                  70                  75                  80

Gly Tyr Val Ile Gly Thr Gln Gln Ala Thr Pro Gly Pro Ala Tyr Ser
                85                  90                  95

Gly Arg Glu Ile Ile Tyr Pro Asn Ala Ser Leu Leu Ile Gln Asn Ile
            100                 105                 110

Ile Gln Asn Asp Thr Gly Phe Tyr Thr Leu His Val Ile Lys Ser Asp
        115                 120                 125

Leu Val Asn Glu Glu Ala Thr Gly Gln Phe Arg Val Tyr Pro Glu Leu
    130                 135                 140

Pro Lys Pro Ser Ile Ser Ser Asn Asn Ser Lys Pro Val Glu Asp Lys
145                 150                 155                 160

Asp Ala Val Ala Phe Thr Cys Glu Pro Glu Thr Gln Asp Ala Thr Tyr
                165                 170                 175

Leu Trp Trp Val Asn Asn Gln Ser Leu Pro Val Ser Pro Arg Leu Gln
            180                 185                 190

Leu Ser Asn Gly Asn Arg Thr Leu Thr Leu Phe Asn Val Thr Arg Asn
        195                 200                 205

Asp Thr Ala Ser Tyr Lys Cys Glu Thr Gln Asn Pro Val Ser Ala Arg
    210                 215                 220

Arg Ser Asp Ser Val Ile Leu Asn Val Leu Tyr Gly Pro Asp Ala Pro
225                 230                 235                 240

Thr Ile Ser Pro Leu Asn Thr Ser Tyr Arg Ser Gly Glu Asn Leu Asn
                245                 250                 255

Leu Ser Cys His Ala Ala Ser Asn Pro Pro Ala Gln Tyr Ser Trp Phe
            260                 265                 270

Val Asn Gly Thr Phe Gln Gln Ser Thr Gln Glu Leu Phe Ile Pro Asn
        275                 280                 285

Ile Thr Val Asn Asn Ser Gly Ser Tyr Thr Cys Gln Ala His Asn Ser
    290                 295                 300
```

Asp Thr Gly Leu Asn Arg Thr Thr Val Thr Thr Ile Thr Val Tyr Ala
305 310 315 320

Glu Pro Pro Lys Pro Phe Ile Thr Ser Asn Asn Ser Asn Pro Val Glu
325 330 335

Asp Glu Asp Ala Val Ala Leu Thr Cys Glu Pro Glu Ile Gln Asn Thr
340 345 350

Thr Tyr Leu Trp Trp Val Asn Asn Gln Ser Leu Pro Val Ser Pro Arg
355 360 365

Leu Gln Leu Ser Asn Asp Asn Arg Thr Leu Thr Leu Leu Ser Val Thr
370 375 380

Arg Asn Asp Val Gly Pro Tyr Glu Cys Gly Ile Gln Asn Lys Leu Ser
385 390 395 400

Val Asp His Ser Asp Pro Val Ile Leu Asn Val Leu Tyr Gly Pro Asp
405 410 415

Asp Pro Thr Ile Ser Pro Ser Tyr Thr Tyr Tyr Arg Pro Gly Val Asn
420 425 430

Leu Ser Leu Ser Cys His Ala Ala Ser Asn Pro Pro Ala Gln Tyr Ser
435 440 445

Trp Leu Ile Asp Gly Asn Ile Gln Gln His Thr Gln Glu Leu Phe Ile
450 455 460

Ser Asn Ile Thr Glu Lys Asn Ser Gly Leu Tyr Thr Cys Gln Ala Asn
465 470 475 480

Asn Ser Ala Ser Gly His Ser Arg Thr Thr Val Lys Thr Ile Thr Val
485 490 495

Ser Ala Glu Leu Pro Lys Pro Ser Ile Ser Ser Asn Asn Ser Lys Pro
500 505 510

Val Glu Asp Lys Asp Ala Val Ala Phe Thr Cys Glu Pro Glu Ala Gln
515 520 525

Asn Thr Thr Tyr Leu Trp Trp Val Asn Gly Gln Ser Leu Pro Val Ser
530 535 540

Pro Arg Leu Gln Leu Ser Asn Gly Asn Arg Thr Leu Thr Leu Phe Asn
545 550 555 560

Val Thr Arg Asn Asp Ala Arg Ala Tyr Val Cys Gly Ile Gln Asn Ser
565 570 575

Val Ser Ala Asn Arg Ser Asp Pro Val Thr Leu Asp Val Leu Tyr Gly
580 585 590

Pro Asp Thr Pro Ile Ile Ser Pro Pro Asp Ser Ser Tyr Leu Ser Gly
595 600 605

Ala Asn Leu Asn Leu Ser Cys His Ser Ala Ser Asn Pro Ser Pro Gln
610 615 620

Tyr Ser Trp Arg Ile Asn Gly Ile Pro Gln Gln His Thr Gln Val Leu
625 630 635 640

Phe Ile Ala Lys Ile Thr Pro Asn Asn Asn Gly Thr Tyr Ala Cys Phe
645 650 655

Val Ser Asn Leu Ala Thr Gly Arg Asn Asn Ser Ile Val Lys Ser Ile
660 665 670

Thr Val Ser Ala Ser Gly Thr Ser Pro Gly Leu Ser Ala Gly Ala Thr
675 680 685

Val Gly Ile Met Ile Gly Val Leu Val Gly Val Ala Leu Ile
690 695 700

<210> 39
<211> 2322
<212> PRT
<213> human

<400> 39

Met Gln Ser Gly Pro Arg Pro Pro Leu Pro Ala Pro Gly Leu Ala Leu
1 5 10 15

Ala Leu Thr Leu Thr Met Leu Ala Arg Leu Ala Ser Ala Ala Ser Phe
20 25 30

Phe Gly Glu Asn His Leu Glu Val Pro Val Ala Thr Ala Leu Thr Asp
35 40 45

Ile Asp Leu Gln Leu Gln Phe Ser Thr Ser Gln Pro Glu Ala Leu Leu
50 55 60

Leu Leu Ala Ala Gly Pro Ala Asp His Leu Leu Leu Gln Leu Tyr Ser
65 70 75 80

```
Gly Arg Leu Gln Val Arg Leu Val Leu Gly Gln Glu Glu Leu Arg Leu
                85                  90                  95

Gln Thr Pro Ala Glu Thr Leu Leu Ser Asp Ser Ile Pro His Thr Val
            100                 105                 110

Val Leu Thr Val Val Glu Gly Trp Ala Thr Leu Ser Val Asp Gly Phe
        115                 120                 125

Leu Asn Ala Ser Ser Ala Val Pro Gly Ala Pro Leu Glu Val Pro Tyr
    130                 135                 140

Gly Leu Phe Val Gly Gly Thr Gly Thr Leu Gly Leu Pro Tyr Leu Arg
145                 150                 155                 160

Gly Thr Ser Arg Pro Leu Arg Gly Cys Leu His Ala Ala Thr Leu Asn
                165                 170                 175

Gly Arg Ser Leu Leu Arg Pro Leu Thr Pro Asp Val His Glu Gly Cys
            180                 185                 190

Ala Glu Glu Phe Ser Ala Ser Asp Asp Val Ala Leu Gly Phe Ser Gly
        195                 200                 205

Pro His Ser Leu Ala Ala Phe Pro Ala Trp Gly Thr Gln Asp Glu Gly
    210                 215                 220

Thr Leu Glu Phe Thr Leu Thr Thr Gln Ser Arg Gln Ala Pro Leu Ala
225                 230                 235                 240

Phe Gln Ala Gly Gly Arg Arg Gly Asp Phe Ile Tyr Val Asp Ile Phe
                245                 250                 255

Glu Gly His Leu Arg Ala Val Val Glu Lys Gly Gln Gly Thr Val Leu
            260                 265                 270

Leu His Asn Ser Val Pro Val Ala Asp Gly Gln Pro His Glu Val Ser
        275                 280                 285

Val His Ile Asn Ala His Arg Leu Glu Ile Ser Val Asp Gln Tyr Pro
    290                 295                 300

Thr His Thr Ser Asn Arg Gly Val Leu Ser Tyr Leu Glu Pro Arg Gly
305                 310                 315                 320

Ser Leu Leu Leu Gly Gly Leu Asp Ala Glu Ala Ser Arg His Leu Gln
                325                 330                 335
```

```
Glu His Arg Leu Gly Leu Thr Pro Glu Ala Thr Asn Ala Ser Leu Leu
            340                 345                 350

Gly Cys Met Glu Asp Leu Ser Val Asn Gly Gln Arg Arg Gly Leu Arg
        355                 360                 365

Glu Ala Leu Leu Thr Arg Asn Met Ala Ala Gly Cys Arg Leu Glu Glu
    370                 375                 380

Glu Glu Tyr Glu Asp Asp Ala Tyr Gly His Tyr Glu Ala Phe Ser Thr
385                 390                 395                 400

Leu Ala Pro Glu Ala Trp Pro Ala Met Glu Leu Pro Glu Pro Cys Val
                405                 410                 415

Pro Glu Pro Gly Leu Pro Pro Val Phe Ala Asn Phe Thr Gln Leu Leu
            420                 425                 430

Thr Ile Ser Pro Leu Val Val Ala Glu Gly Gly Thr Ala Trp Leu Glu
        435                 440                 445

Trp Arg His Val Gln Pro Thr Leu Asp Leu Met Glu Ala Glu Leu Arg
    450                 455                 460

Lys Ser Gln Val Leu Phe Ser Val Thr Arg Gly Ala Arg His Gly Glu
465                 470                 475                 480

Leu Glu Leu Asp Ile Pro Gly Ala Gln Ala Arg Lys Met Phe Thr Leu
                485                 490                 495

Leu Asp Val Val Asn Arg Lys Ala Arg Phe Ile His Asp Gly Ser Glu
            500                 505                 510

Asp Thr Ser Asp Gln Leu Val Leu Glu Val Ser Val Thr Ala Arg Val
        515                 520                 525

Pro Met Pro Ser Cys Leu Arg Arg Gly Gln Thr Tyr Leu Leu Pro Ile
    530                 535                 540

Gln Val Asn Pro Val Asn Asp Pro Pro His Ile Ile Phe Pro His Gly
545                 550                 555                 560

Ser Leu Met Val Ile Leu Glu His Thr Gln Lys Pro Leu Gly Pro Glu
                565                 570                 575

Val Phe Gln Ala Tyr Asp Pro Asp Ser Ala Cys Glu Gly Leu Thr Phe
```

580 585 590

Gln Val Leu Gly Thr Ser Ser Gly Leu Pro Val Glu Arg Arg Asp Gln
595 600 605

Pro Gly Glu Pro Ala Thr Glu Phe Ser Cys Arg Glu Leu Glu Ala Gly
610 615 620

Ser Leu Val Tyr Val His Arg Gly Gly Pro Ala Gln Asp Leu Thr Phe
625 630 635 640

Arg Val Ser Asp Gly Leu Gln Ala Ser Pro Pro Ala Thr Leu Lys Val
645 650 655

Val Ala Ile Arg Pro Ala Ile Gln Ile His Arg Ser Thr Gly Leu Arg
660 665 670

Leu Ala Gln Gly Ser Ala Met Pro Ile Leu Pro Ala Asn Leu Ser Val
675 680 685

Glu Thr Asn Ala Val Gly Gln Asp Val Ser Val Leu Phe Arg Val Thr
690 695 700

Gly Ala Leu Gln Phe Gly Glu Leu Gln Lys Gln Gly Ala Gly Gly Val
705 710 715 720

Glu Gly Ala Glu Trp Trp Ala Thr Gln Ala Phe His Gln Arg Asp Val
725 730 735

Glu Gln Gly Arg Val Arg Tyr Leu Ser Thr Asp Pro Gln His His Ala
740 745 750

Tyr Asp Thr Val Glu Asn Leu Ala Leu Glu Val Gln Val Gly Gln Glu
755 760 765

Ile Leu Ser Asn Leu Ser Phe Pro Val Thr Ile Gln Arg Ala Thr Val
770 775 780

Trp Met Leu Arg Leu Glu Pro Leu His Thr Gln Asn Thr Gln Gln Glu
785 790 795 800

Thr Leu Thr Thr Ala His Leu Glu Ala Thr Leu Glu Glu Ala Gly Pro
805 810 815

Ser Pro Pro Thr Phe His Tyr Glu Val Val Gln Ala Pro Arg Lys Gly
820 825 830

```
Asn Leu Gln Leu Gln Gly Thr Arg Leu Ser Asp Gly Gln Gly Phe Thr
        835                 840                 845

Gln Asp Asp Ile Gln Ala Gly Arg Val Thr Tyr Gly Ala Thr Ala Arg
    850                 855                 860

Ala Ser Glu Ala Val Glu Asp Thr Phe Arg Phe Arg Val Thr Ala Pro
865                 870                 875                 880

Pro Tyr Phe Ser Pro Leu Tyr Thr Phe Pro Ile His Ile Gly Gly Asp
                885                 890                 895

Pro Asp Ala Pro Val Leu Thr Asn Val Leu Leu Val Val Pro Glu Gly
            900                 905                 910

Gly Glu Gly Val Leu Ser Ala Asp His Leu Phe Val Lys Ser Leu Asn
        915                 920                 925

Ser Ala Ser Tyr Leu Tyr Glu Val Met Glu Arg Pro Arg His Gly Arg
    930                 935                 940

Leu Ala Trp Arg Gly Thr Gln Asp Lys Thr Thr Met Val Thr Ser Phe
945                 950                 955                 960

Thr Asn Glu Asp Leu Leu Arg Gly Arg Leu Val Tyr Gln His Asp Asp
                965                 970                 975

Ser Glu Thr Thr Glu Asp Asp Ile Pro Phe Val Ala Thr Arg Gln Gly
            980                 985                 990

Glu Ser Ser Gly Asp Met Ala Trp  Glu Glu Val Arg Gly  Val Phe Arg
        995                 1000                 1005

Val Ala  Ile Gln Pro Val Asn  Asp His Ala Pro Val  Gln Thr Ile
    1010                 1015                 1020

Ser Arg  Ile Phe His Val Ala  Arg Gly Gly Arg Arg  Leu Leu Thr
    1025                 1030                 1035

Thr Asp  Asp Val Ala Phe Ser  Asp Ala Asp Ser Gly  Phe Ala Asp
    1040                 1045                 1050

Ala Gln  Leu Val Leu Thr Arg  Lys Asp Leu Leu Phe  Gly Ser Ile
    1055                 1060                 1065

Val Ala  Val Asp Glu Pro Thr  Arg Pro Ile Tyr Arg  Phe Thr Gln
    1070                 1075                 1080
```

Glu Asp Leu Arg Lys Arg Arg Val Leu Phe Val His Ser Gly Ala
1085 1090 1095

Asp Arg Gly Trp Ile Gln Leu Gln Val Ser Asp Gly Gln His Gln
1100 1105 1110

Ala Thr Ala Leu Leu Glu Val Gln Ala Ser Glu Pro Tyr Leu Arg
1115 1120 1125

Val Ala Asn Gly Ser Ser Leu Val Val Pro Gln Gly Gly Gln Gly
1130 1135 1140

Thr Ile Asp Thr Ala Val Leu His Leu Asp Thr Asn Leu Asp Ile
1145 1150 1155

Arg Ser Gly Asp Glu Val His Tyr His Val Thr Ala Gly Pro Arg
1160 1165 1170

Trp Gly Gln Leu Val Arg Ala Gly Gln Pro Ala Thr Ala Phe Ser
1175 1180 1185

Gln Gln Asp Leu Leu Asp Gly Ala Val Leu Tyr Ser His Asn Gly
1190 1195 1200

Ser Leu Ser Pro Arg Asp Thr Met Ala Phe Ser Val Glu Ala Gly
1205 1210 1215

Pro Val His Thr Asp Ala Thr Leu Gln Val Thr Ile Ala Leu Glu
1220 1225 1230

Gly Pro Leu Ala Pro Leu Lys Leu Val Arg His Lys Lys Ile Tyr
1235 1240 1245

Val Phe Gln Gly Glu Ala Ala Glu Ile Arg Arg Asp Gln Leu Glu
1250 1255 1260

Ala Ala Gln Glu Ala Val Pro Pro Ala Asp Ile Val Phe Ser Val
1265 1270 1275

Lys Ser Pro Pro Ser Ala Gly Tyr Leu Val Met Val Ser Arg Gly
1280 1285 1290

Ala Leu Ala Asp Glu Pro Pro Ser Leu Asp Pro Val Gln Ser Phe
1295 1300 1305

Ser Gln Glu Ala Val Asp Thr Gly Arg Val Leu Tyr Leu His Ser
1310 1315 1320

```
Arg Pro  Glu Ala Trp Ser Asp  Ala Phe Ser Leu Asp  Val Ala Ser
    1325                 1330                 1335

Gly Leu  Gly Ala Pro Leu Glu  Gly Val Leu Val Glu  Leu Glu Val
    1340                 1345                 1350

Leu Pro  Ala Ala Ile Pro Leu  Glu Ala Gln Asn Phe  Ser Val Pro
    1355                 1360                 1365

Glu Gly  Gly Ser Leu Thr Leu  Ala Pro Pro Leu Leu  Arg Val Ser
    1370                 1375                 1380

Gly Pro  Tyr Phe Pro Thr Leu  Leu Gly Leu Ser Leu  Gln Val Leu
    1385                 1390                 1395

Glu Pro  Pro Gln His Gly Ala  Leu Gln Lys Glu Asp  Gly Pro Gln
    1400                 1405                 1410

Ala Arg  Thr Leu Ser Ala Phe  Ser Trp Arg Met Val  Glu Glu Gln
    1415                 1420                 1425

Leu Ile  Arg Tyr Val His Asp  Gly Ser Glu Thr Leu  Thr Asp Ser
    1430                 1435                 1440

Phe Val  Leu Met Ala Asn Ala  Ser Glu Met Asp Arg  Gln Ser His
    1445                 1450                 1455

Pro Val  Ala Phe Thr Val Thr  Val Leu Pro Val Asn  Asp Gln Pro
    1460                 1465                 1470

Pro Ile  Leu Thr Thr Asn Thr  Gly Leu Gln Met Trp  Glu Gly Ala
    1475                 1480                 1485

Thr Ala  Pro Ile Pro Ala Glu  Ala Leu Arg Ser Thr  Asp Gly Asp
    1490                 1495                 1500

Ser Gly  Ser Glu Asp Leu Val  Tyr Thr Ile Glu Gln  Pro Ser Asn
    1505                 1510                 1515

Gly Arg  Val Val Leu Arg Gly  Ala Pro Gly Thr Glu  Val Arg Ser
    1520                 1525                 1530

Phe Thr  Gln Ala Gln Leu Asp  Gly Gly Leu Val Leu  Phe Ser His
    1535                 1540                 1545

Arg Gly  Thr Leu Asp Gly Gly  Phe Arg Phe Arg Leu  Ser Asp Gly
```

1550 1555 1560

Glu His Thr Ser Pro Gly His Phe Phe Arg Val Thr Ala Gln Lys
1565 1570 1575

Gln Val Leu Leu Ser Leu Lys Gly Ser Gln Thr Leu Thr Val Cys
1580 1585 1590

Pro Gly Ser Val Gln Pro Leu Ser Ser Gln Thr Leu Arg Ala Ser
1595 1600 1605

Ser Ser Ala Gly Thr Asp Pro Gln Leu Leu Leu Tyr Arg Val Val
1610 1615 1620

Arg Gly Pro Gln Leu Gly Arg Leu Phe His Ala Gln Gln Asp Ser
1625 1630 1635

Thr Gly Glu Ala Leu Val Asn Phe Thr Gln Ala Glu Val Tyr Ala
1640 1645 1650

Gly Asn Ile Leu Tyr Glu His Glu Met Pro Pro Glu Pro Phe Trp
1655 1660 1665

Glu Ala His Asp Thr Leu Glu Leu Gln Leu Ser Ser Pro Pro Ala
1670 1675 1680

Arg Asp Val Ala Ala Thr Leu Ala Val Ala Val Ser Phe Glu Ala
1685 1690 1695

Ala Cys Pro Gln Arg Pro Ser His Leu Trp Lys Asn Lys Gly Leu
1700 1705 1710

Trp Val Pro Glu Gly Gln Arg Ala Arg Ile Thr Val Ala Ala Leu
1715 1720 1725

Asp Ala Ser Asn Leu Leu Ala Ser Val Pro Ser Pro Gln Arg Ser
1730 1735 1740

Glu His Asp Val Leu Phe Gln Val Thr Gln Phe Pro Ser Arg Gly
1745 1750 1755

Gln Leu Leu Val Ser Glu Glu Pro Leu His Ala Gly Gln Pro His
1760 1765 1770

Phe Leu Gln Ser Gln Leu Ala Ala Gly Gln Leu Val Tyr Ala His
1775 1780 1785

Gly Gly Gly Gly Thr Gln Gln Asp Gly Phe His Phe Arg Ala His
1790 1795 1800

Leu Gln Gly Pro Ala Gly Ala Ser Val Ala Gly Pro Gln Thr Ser
1805 1810 1815

Glu Ala Phe Ala Ile Thr Val Arg Asp Val Asn Glu Arg Pro Pro
1820 1825 1830

Gln Pro Gln Ala Ser Val Pro Leu Arg Leu Thr Arg Gly Ser Arg
1835 1840 1845

Ala Pro Ile Ser Arg Ala Gln Leu Ser Val Val Asp Pro Asp Ser
1850 1855 1860

Ala Pro Gly Glu Ile Glu Tyr Glu Val Gln Arg Ala Pro His Asn
1865 1870 1875

Gly Phe Leu Ser Leu Val Gly Gly Gly Leu Gly Pro Val Thr Arg
1880 1885 1890

Phe Thr Gln Ala Asp Val Asp Ser Gly Arg Leu Ala Phe Val Ala
1895 1900 1905

Asn Gly Ser Ser Val Ala Gly Ile Phe Gln Leu Ser Met Ser Asp
1910 1915 1920

Gly Ala Ser Pro Pro Leu Pro Met Ser Leu Ala Val Asp Ile Leu
1925 1930 1935

Pro Ser Ala Ile Glu Val Gln Leu Arg Ala Pro Leu Glu Val Pro
1940 1945 1950

Gln Ala Leu Gly Arg Ser Ser Leu Ser Gln Gln Gln Leu Arg Val
1955 1960 1965

Val Ser Asp Arg Glu Glu Pro Glu Ala Ala Tyr Arg Leu Ile Gln
1970 1975 1980

Gly Pro Gln Tyr Gly His Leu Leu Val Gly Gly Arg Pro Thr Ser
1985 1990 1995

Ala Phe Ser Gln Phe Gln Ile Asp Gln Gly Glu Val Val Phe Ala
2000 2005 2010

Phe Thr Asn Phe Ser Ser Ser His Asp His Phe Arg Val Leu Ala
2015 2020 2025

Leu Ala Arg Gly Val Asn Ala Ser Ala Val Val Asn Val Thr Val
2030 2035 2040

Arg Ala Leu Leu His Val Trp Ala Gly Gly Pro Trp Pro Gln Gly
2045 2050 2055

Ala Thr Leu Arg Leu Asp Pro Thr Val Leu Asp Ala Gly Glu Leu
2060 2065 2070

Ala Asn Arg Thr Gly Ser Val Pro Arg Phe Arg Leu Leu Glu Gly
2075 2080 2085

Pro Arg His Gly Arg Val Val Arg Val Pro Arg Ala Arg Thr Glu
2090 2095 2100

Pro Gly Gly Ser Gln Leu Val Glu Gln Phe Thr Gln Gln Asp Leu
2105 2110 2115

Glu Asp Gly Arg Leu Gly Leu Glu Val Gly Arg Pro Glu Gly Arg
2120 2125 2130

Ala Pro Gly Pro Ala Gly Asp Ser Leu Thr Leu Glu Leu Trp Ala
2135 2140 2145

Gln Gly Val Pro Pro Ala Val Ala Ser Leu Asp Phe Ala Thr Glu
2150 2155 2160

Pro Tyr Asn Ala Ala Arg Pro Tyr Ser Val Ala Leu Leu Ser Val
2165 2170 2175

Pro Glu Ala Ala Arg Thr Glu Ala Gly Lys Pro Glu Ser Ser Thr
2180 2185 2190

Pro Thr Gly Glu Pro Gly Pro Met Ala Ser Ser Pro Glu Pro Ala
2195 2200 2205

Val Ala Lys Gly Gly Phe Leu Ser Phe Leu Glu Ala Asn Met Phe
2210 2215 2220

Ser Val Ile Ile Pro Met Cys Leu Val Leu Leu Leu Leu Ala Leu
2225 2230 2235

Ile Leu Pro Leu Leu Phe Tyr Leu Arg Lys Arg Asn Lys Thr Gly
2240 2245 2250

Lys His Asp Val Gln Val Leu Thr Ala Lys Pro Arg Asn Gly Leu
2255 2260 2265

Ala Gly Asp Thr Glu Thr Phe Arg Lys Val Glu Pro Gly Gln Ala
2270 2275 2280

Ile Pro Leu Thr Ala Val Pro Gly Gln Gly Pro Pro Pro Gly Gly
2285 2290 2295

Gln Pro Asp Pro Glu Leu Leu Gln Phe Cys Arg Thr Pro Asn Pro
2300 2305 2310

Ala Leu Lys Asn Gly Gln Tyr Trp Val
2315 2320

<210> 40
<211> 1210
<212> PRT
<213> human

<400> 40

Met Arg Pro Ser Gly Thr Ala Gly Ala Ala Leu Leu Ala Leu Leu Ala
1 5 10 15

Ala Leu Cys Pro Ala Ser Arg Ala Leu Glu Glu Lys Lys Val Cys Gln
20 25 30

Gly Thr Ser Asn Lys Leu Thr Gln Leu Gly Thr Phe Glu Asp His Phe
35 40 45

Leu Ser Leu Gln Arg Met Phe Asn Asn Cys Glu Val Val Leu Gly Asn
50 55 60

Leu Glu Ile Thr Tyr Val Gln Arg Asn Tyr Asp Leu Ser Phe Leu Lys
65 70 75 80

Thr Ile Gln Glu Val Ala Gly Tyr Val Leu Ile Ala Leu Asn Thr Val
85 90 95

Glu Arg Ile Pro Leu Glu Asn Leu Gln Ile Ile Arg Gly Asn Met Tyr
100 105 110

Tyr Glu Asn Ser Tyr Ala Leu Ala Val Leu Ser Asn Tyr Asp Ala Asn
115 120 125

Lys Thr Gly Leu Lys Glu Leu Pro Met Arg Asn Leu Gln Glu Ile Leu
130 135 140

His Gly Ala Val Arg Phe Ser Asn Asn Pro Ala Leu Cys Asn Val Glu
145 150 155 160

```
Ser Ile Gln Trp Arg Asp Ile Val Ser Ser Asp Phe Leu Ser Asn Met
                    165                 170                 175

Ser Met Asp Phe Gln Asn His Leu Gly Ser Cys Gln Lys Cys Asp Pro
                180                 185                 190

Ser Cys Pro Asn Gly Ser Cys Trp Gly Ala Gly Glu Glu Asn Cys Gln
        195                 200                 205

Lys Leu Thr Lys Ile Ile Cys Ala Gln Gln Cys Ser Gly Arg Cys Arg
    210                 215                 220

Gly Lys Ser Pro Ser Asp Cys Cys His Asn Gln Cys Ala Ala Gly Cys
225                 230                 235                 240

Thr Gly Pro Arg Glu Ser Asp Cys Leu Val Cys Arg Lys Phe Arg Asp
                    245                 250                 255

Glu Ala Thr Cys Lys Asp Thr Cys Pro Pro Leu Met Leu Tyr Asn Pro
                260                 265                 270

Thr Thr Tyr Gln Met Asp Val Asn Pro Glu Gly Lys Tyr Ser Phe Gly
        275                 280                 285

Ala Thr Cys Val Lys Lys Cys Pro Arg Asn Tyr Val Val Thr Asp His
    290                 295                 300

Gly Ser Cys Val Arg Ala Cys Gly Ala Asp Ser Tyr Glu Met Glu Glu
305                 310                 315                 320

Asp Gly Val Arg Lys Cys Lys Lys Cys Glu Gly Pro Cys Arg Lys Val
                    325                 330                 335

Cys Asn Gly Ile Gly Ile Gly Glu Phe Lys Asp Ser Leu Ser Ile Asn
                340                 345                 350

Ala Thr Asn Ile Lys His Phe Lys Asn Cys Thr Ser Ile Ser Gly Asp
        355                 360                 365

Leu His Ile Leu Pro Val Ala Phe Arg Gly Asp Ser Phe Thr His Thr
    370                 375                 380

Pro Pro Leu Asp Pro Gln Glu Leu Asp Ile Leu Lys Thr Val Lys Glu
385                 390                 395                 400

Ile Thr Gly Phe Leu Leu Ile Gln Ala Trp Pro Glu Asn Arg Thr Asp
```

```
                405                 410                 415

Leu His Ala Phe Glu Asn Leu Glu Ile Ile Arg Gly Arg Thr Lys Gln
            420                 425                 430

His Gly Gln Phe Ser Leu Ala Val Val Ser Leu Asn Ile Thr Ser Leu
        435                 440                 445

Gly Leu Arg Ser Leu Lys Glu Ile Ser Asp Gly Asp Val Ile Ile Ser
    450                 455                 460

Gly Asn Lys Asn Leu Cys Tyr Ala Asn Thr Ile Asn Trp Lys Lys Leu
465                 470                 475                 480

Phe Gly Thr Ser Gly Gln Lys Thr Lys Ile Ile Ser Asn Arg Gly Glu
                485                 490                 495

Asn Ser Cys Lys Ala Thr Gly Gln Val Cys His Ala Leu Cys Ser Pro
            500                 505                 510

Glu Gly Cys Trp Gly Pro Glu Pro Arg Asp Cys Val Ser Cys Arg Asn
        515                 520                 525

Val Ser Arg Gly Arg Glu Cys Val Asp Lys Cys Asn Leu Leu Glu Gly
    530                 535                 540

Glu Pro Arg Glu Phe Val Glu Asn Ser Glu Cys Ile Gln Cys His Pro
545                 550                 555                 560

Glu Cys Leu Pro Gln Ala Met Asn Ile Thr Cys Thr Gly Arg Gly Pro
                565                 570                 575

Asp Asn Cys Ile Gln Cys Ala His Tyr Ile Asp Gly Pro His Cys Val
            580                 585                 590

Lys Thr Cys Pro Ala Gly Val Met Gly Glu Asn Asn Thr Leu Val Trp
        595                 600                 605

Lys Tyr Ala Asp Ala Gly His Val Cys His Leu Cys His Pro Asn Cys
    610                 615                 620

Thr Tyr Gly Cys Thr Gly Pro Gly Leu Glu Gly Cys Pro Thr Asn Gly
625                 630                 635                 640

Pro Lys Ile Pro Ser Ile Ala Thr Gly Met Val Gly Ala Leu Leu Leu
                645                 650                 655
```

Leu Leu Val Val Ala Leu Gly Ile Gly Leu Phe Met Arg Arg Arg His
660 665 670

Ile Val Arg Lys Arg Thr Leu Arg Arg Leu Leu Gln Glu Arg Glu Leu
675 680 685

Val Glu Pro Leu Thr Pro Ser Gly Glu Ala Pro Asn Gln Ala Leu Leu
690 695 700

Arg Ile Leu Lys Glu Thr Glu Phe Lys Lys Ile Lys Val Leu Gly Ser
705 710 715 720

Gly Ala Phe Gly Thr Val Tyr Lys Gly Leu Trp Ile Pro Glu Gly Glu
725 730 735

Lys Val Lys Ile Pro Val Ala Ile Lys Glu Leu Arg Glu Ala Thr Ser
740 745 750

Pro Lys Ala Asn Lys Glu Ile Leu Asp Glu Ala Tyr Val Met Ala Ser
755 760 765

Val Asp Asn Pro His Val Cys Arg Leu Leu Gly Ile Cys Leu Thr Ser
770 775 780

Thr Val Gln Leu Ile Thr Gln Leu Met Pro Phe Gly Cys Leu Leu Asp
785 790 795 800

Tyr Val Arg Glu His Lys Asp Asn Ile Gly Ser Gln Tyr Leu Leu Asn
805 810 815

Trp Cys Val Gln Ile Ala Lys Gly Met Asn Tyr Leu Glu Asp Arg Arg
820 825 830

Leu Val His Arg Asp Leu Ala Ala Arg Asn Val Leu Val Lys Thr Pro
835 840 845

Gln His Val Lys Ile Thr Asp Phe Gly Leu Ala Lys Leu Leu Gly Ala
850 855 860

Glu Glu Lys Glu Tyr His Ala Glu Gly Gly Lys Val Pro Ile Lys Trp
865 870 875 880

Met Ala Leu Glu Ser Ile Leu His Arg Ile Tyr Thr His Gln Ser Asp
885 890 895

Val Trp Ser Tyr Gly Val Thr Val Trp Glu Leu Met Thr Phe Gly Ser
900 905 910

Lys Pro Tyr Asp Gly Ile Pro Ala Ser Glu Ile Ser Ser Ile Leu Glu
915 920 925

Lys Gly Glu Arg Leu Pro Gln Pro Pro Ile Cys Thr Ile Asp Val Tyr
930 935 940

Met Ile Met Val Lys Cys Trp Met Ile Asp Ala Asp Ser Arg Pro Lys
945 950 955 960

Phe Arg Glu Leu Ile Ile Glu Phe Ser Lys Met Ala Arg Asp Pro Gln
965 970 975

Arg Tyr Leu Val Ile Gln Gly Asp Glu Arg Met His Leu Pro Ser Pro
980 985 990

Thr Asp Ser Asn Phe Tyr Arg Ala Leu Met Asp Glu Glu Asp Met Asp
995 1000 1005

Asp Val Val Asp Ala Asp Glu Tyr Leu Ile Pro Gln Gln Gly Phe
1010 1015 1020

Phe Ser Ser Pro Ser Thr Ser Arg Thr Pro Leu Leu Ser Ser Leu
1025 1030 1035

Ser Ala Thr Ser Asn Asn Ser Thr Val Ala Cys Ile Asp Arg Asn
1040 1045 1050

Gly Leu Gln Ser Cys Pro Ile Lys Glu Asp Ser Phe Leu Gln Arg
1055 1060 1065

Tyr Ser Ser Asp Pro Thr Gly Ala Leu Thr Glu Asp Ser Ile Asp
1070 1075 1080

Asp Thr Phe Leu Pro Val Pro Glu Tyr Ile Asn Gln Ser Val Pro
1085 1090 1095

Lys Arg Pro Ala Gly Ser Val Gln Asn Pro Val Tyr His Asn Gln
1100 1105 1110

Pro Leu Asn Pro Ala Pro Ser Arg Asp Pro His Tyr Gln Asp Pro
1115 1120 1125

His Ser Thr Ala Val Gly Asn Pro Glu Tyr Leu Asn Thr Val Gln
1130 1135 1140

Pro Thr Cys Val Asn Ser Thr Phe Asp Ser Pro Ala His Trp Ala
1145 1150 1155

Gln Lys Gly Ser His Gln Ile Ser Leu Asp Asn Pro Asp Tyr Gln
1160 1165 1170

Gln Asp Phe Phe Pro Lys Glu Ala Lys Pro Asn Gly Ile Phe Lys
1175 1180 1185

Gly Ser Thr Ala Glu Asn Ala Glu Tyr Leu Arg Val Ala Pro Gln
1190 1195 1200

Ser Ser Glu Phe Ile Gly Ala
1205 1210

<210> 41
<211> 556
<212> PRT
<213> human

<400> 41

Met Pro Pro Pro Arg Leu Leu Phe Phe Leu Leu Phe Leu Thr Pro Met
1 5 10 15

Glu Val Arg Pro Glu Glu Pro Leu Val Val Lys Val Glu Glu Gly Asp
20 25 30

Asn Ala Val Leu Gln Cys Leu Lys Gly Thr Ser Asp Gly Pro Thr Gln
35 40 45

Gln Leu Thr Trp Ser Arg Glu Ser Pro Leu Lys Pro Phe Leu Lys Leu
50 55 60

Ser Leu Gly Leu Pro Gly Leu Gly Ile His Met Arg Pro Leu Ala Ile
65 70 75 80

Trp Leu Phe Ile Phe Asn Val Ser Gln Gln Met Gly Gly Phe Tyr Leu
85 90 95

Cys Gln Pro Gly Pro Pro Ser Glu Lys Ala Trp Gln Pro Gly Trp Thr
100 105 110

Val Asn Val Glu Gly Ser Gly Glu Leu Phe Arg Trp Asn Val Ser Asp
115 120 125

Leu Gly Gly Leu Gly Cys Gly Leu Lys Asn Arg Ser Ser Glu Gly Pro
130 135 140

Ser Ser Pro Ser Gly Lys Leu Met Ser Pro Lys Leu Tyr Val Trp Ala
145 150 155 160

```
Lys Asp Arg Pro Glu Ile Trp Glu Gly Glu Pro Pro Cys Leu Pro Pro
                165                 170                 175

Arg Asp Ser Leu Asn Gln Ser Leu Ser Gln Asp Leu Thr Met Ala Pro
            180                 185                 190

Gly Ser Thr Leu Trp Leu Ser Cys Gly Val Pro Pro Asp Ser Val Ser
        195                 200                 205

Arg Gly Pro Leu Ser Trp Thr His Val His Pro Lys Gly Pro Lys Ser
    210                 215                 220

Leu Leu Ser Leu Glu Leu Lys Asp Asp Arg Pro Ala Arg Asp Met Trp
225                 230                 235                 240

Val Met Glu Thr Gly Leu Leu Leu Pro Arg Ala Thr Ala Gln Asp Ala
                245                 250                 255

Gly Lys Tyr Tyr Cys His Arg Gly Asn Leu Thr Met Ser Phe His Leu
            260                 265                 270

Glu Ile Thr Ala Arg Pro Val Leu Trp His Trp Leu Leu Arg Thr Gly
        275                 280                 285

Gly Trp Lys Val Ser Ala Val Thr Leu Ala Tyr Leu Ile Phe Cys Leu
    290                 295                 300

Cys Ser Leu Val Gly Ile Leu His Leu Gln Arg Ala Leu Val Leu Arg
305                 310                 315                 320

Arg Lys Arg Lys Arg Met Thr Asp Pro Thr Arg Arg Phe Phe Lys Val
                325                 330                 335

Thr Pro Pro Pro Gly Ser Gly Pro Gln Asn Gln Tyr Gly Asn Val Leu
            340                 345                 350

Ser Leu Pro Thr Pro Thr Ser Gly Leu Gly Arg Ala Gln Arg Trp Ala
        355                 360                 365

Ala Gly Leu Gly Gly Thr Ala Pro Ser Tyr Gly Asn Pro Ser Ser Asp
    370                 375                 380

Val Gln Ala Asp Gly Ala Leu Gly Ser Arg Ser Pro Pro Gly Val Gly
385                 390                 395                 400

Pro Glu Glu Glu Glu Gly Glu Gly Tyr Glu Glu Pro Asp Ser Glu Glu
```

405 410 415

Asp Ser Glu Phe Tyr Glu Asn Asp Ser Asn Leu Gly Gln Asp Gln Leu
420 425 430

Ser Gln Asp Gly Ser Gly Tyr Glu Asn Pro Glu Asp Glu Pro Leu Gly
435 440 445

Pro Glu Asp Glu Asp Ser Phe Ser Asn Ala Glu Ser Tyr Glu Asn Glu
450 455 460

Asp Glu Glu Leu Thr Gln Pro Val Ala Arg Thr Met Asp Phe Leu Ser
465 470 475 480

Pro His Gly Ser Ala Trp Asp Pro Ser Arg Glu Ala Thr Ser Leu Gly
485 490 495

Ser Gln Ser Tyr Glu Asp Met Arg Gly Ile Leu Tyr Ala Ala Pro Gln
500 505 510

Leu Arg Ser Ile Arg Gly Gln Pro Gly Pro Asn His Glu Glu Asp Ala
515 520 525

Asp Ser Tyr Glu Asn Met Asp Asn Pro Asp Gly Pro Asp Pro Ala Trp
530 535 540

Gly Gly Gly Gly Arg Met Gly Thr Trp Ser Thr Arg
545 550 555

<210> 42
<211> 297
<212> PRT
<213> human

<400> 42

Met Thr Thr Pro Arg Asn Ser Val Asn Gly Thr Phe Pro Ala Glu Pro
1 5 10 15

Met Lys Gly Pro Ile Ala Met Gln Ser Gly Pro Lys Pro Leu Phe Arg
20 25 30

Arg Met Ser Ser Leu Val Gly Pro Thr Gln Ser Phe Phe Met Arg Glu
35 40 45

Ser Lys Thr Leu Gly Ala Val Gln Ile Met Asn Gly Leu Phe His Ile
50 55 60

Ala Leu Gly Gly Leu Leu Met Ile Pro Ala Gly Ile Tyr Ala Pro Ile

```
65                  70                  75                  80

Cys Val Thr Val Trp Tyr Pro Leu Trp Gly Gly Ile Met Tyr Ile Ile
                85                  90                  95

Ser Gly Ser Leu Leu Ala Ala Thr Glu Lys Asn Ser Arg Lys Cys Leu
            100                 105                 110

Val Lys Gly Lys Met Ile Met Asn Ser Leu Ser Leu Phe Ala Ala Ile
        115                 120                 125

Ser Gly Met Ile Leu Ser Ile Met Asp Ile Leu Asn Ile Lys Ile Ser
    130                 135                 140

His Phe Leu Lys Met Glu Ser Leu Asn Phe Ile Arg Ala His Thr Pro
145                 150                 155                 160

Tyr Ile Asn Ile Tyr Asn Cys Glu Pro Ala Asn Pro Ser Glu Lys Asn
                165                 170                 175

Ser Pro Ser Thr Gln Tyr Cys Tyr Ser Ile Gln Ser Leu Phe Leu Gly
            180                 185                 190

Ile Leu Ser Val Met Leu Ile Phe Ala Phe Phe Gln Glu Leu Val Ile
        195                 200                 205

Ala Gly Ile Val Glu Asn Glu Trp Lys Arg Thr Cys Ser Arg Pro Lys
    210                 215                 220

Ser Asn Ile Val Leu Leu Ser Ala Glu Glu Lys Lys Glu Gln Thr Ile
225                 230                 235                 240

Glu Ile Lys Glu Glu Val Val Gly Leu Thr Glu Thr Ser Ser Gln Pro
                245                 250                 255

Lys Asn Glu Glu Asp Ile Glu Ile Ile Pro Ile Gln Glu Glu Glu Glu
            260                 265                 270

Glu Glu Thr Glu Thr Asn Phe Pro Glu Pro Pro Gln Asp Gln Glu Ser
        275                 280                 285

Ser Pro Ile Glu Asn Asp Ser Ser Pro
    290                 295
```

<210> 43
<211> 364
<212> PRT
<213> human

<400> 43

Met Pro Leu Leu Leu Leu Leu Pro Leu Leu Trp Ala Gly Ala Leu Ala
1               5                   10                  15

Met Asp Pro Asn Phe Trp Leu Gln Val Gln Glu Ser Val Thr Val Gln
            20                  25                  30

Glu Gly Leu Cys Val Leu Val Pro Cys Thr Phe Phe His Pro Ile Pro
        35                  40                  45

Tyr Tyr Asp Lys Asn Ser Pro Val His Gly Tyr Trp Phe Arg Glu Gly
    50                  55                  60

Ala Ile Ile Ser Arg Asp Ser Pro Val Ala Thr Asn Lys Leu Asp Gln
65                  70                  75                  80

Glu Val Gln Glu Glu Thr Gln Gly Arg Phe Arg Leu Leu Gly Asp Pro
                85                  90                  95

Ser Arg Asn Asn Cys Ser Leu Ser Ile Val Asp Ala Arg Arg Arg Asp
            100                 105                 110

Asn Gly Ser Tyr Phe Phe Arg Met Glu Arg Gly Ser Thr Lys Tyr Ser
        115                 120                 125

Tyr Lys Ser Pro Gln Leu Ser Val His Val Thr Asp Leu Thr His Arg
    130                 135                 140

Pro Lys Ile Leu Ile Pro Gly Thr Leu Glu Pro Gly His Ser Lys Asn
145                 150                 155                 160

Leu Thr Cys Ser Val Ser Trp Ala Cys Glu Gln Gly Thr Pro Pro Ile
                165                 170                 175

Phe Ser Trp Leu Ser Ala Ala Pro Thr Ser Leu Gly Pro Arg Thr Thr
            180                 185                 190

His Ser Ser Val Leu Ile Ile Thr Pro Arg Pro Gln Asp His Gly Thr
        195                 200                 205

Asn Leu Thr Cys Gln Val Lys Phe Ala Gly Ala Gly Val Thr Thr Glu
    210                 215                 220

Arg Thr Ile Gln Leu Asn Val Thr Tyr Val Pro Gln Asn Pro Thr Thr
225                 230                 235                 240

```
Gly Ile Phe Pro Gly Asp Gly Ser Gly Lys Gln Glu Thr Arg Ala Gly
                245                 250                 255

Val Val His Gly Ala Ile Gly Gly Ala Gly Val Thr Ala Leu Leu Ala
            260                 265                 270

Leu Cys Leu Cys Leu Ile Phe Phe Ile Val Lys Thr His Arg Arg Lys
        275                 280                 285

Ala Ala Arg Thr Ala Val Gly Arg Asn Asp Thr His Pro Thr Thr Gly
    290                 295                 300

Ser Ala Ser Pro Lys His Gln Lys Lys Ser Lys Leu His Gly Pro Thr
305                 310                 315                 320

Glu Thr Ser Ser Cys Ser Gly Ala Ala Pro Thr Val Glu Met Asp Glu
                325                 330                 335

Glu Leu His Tyr Ala Ser Leu Asn Phe His Gly Met Asn Pro Ser Lys
            340                 345                 350

Asp Thr Ser Thr Glu Tyr Ser Glu Val Arg Thr Gln
        355                 360


<210>  44
<211>  183
<212>  PRT
<213>  human

<400>  44

Met Glu Arg Val Gln Pro Leu Glu Glu Asn Val Gly Asn Ala Ala Arg
1               5                   10                  15

Pro Arg Phe Glu Arg Asn Lys Leu Leu Leu Val Ala Ser Val Ile Gln
            20                  25                  30

Gly Leu Gly Leu Leu Leu Cys Phe Thr Tyr Ile Cys Leu His Phe Ser
        35                  40                  45

Ala Leu Gln Val Ser His Arg Tyr Pro Arg Ile Gln Ser Ile Lys Val
    50                  55                  60

Gln Phe Thr Glu Tyr Lys Lys Glu Lys Gly Phe Ile Leu Thr Ser Gln
65                  70                  75                  80

Lys Glu Asp Glu Ile Met Lys Val Gln Asn Asn Ser Val Ile Ile Asn
                85                  90                  95
```

Cys Asp Gly Phe Tyr Leu Ile Ser Leu Lys Gly Tyr Phe Ser Gln Glu
100 105 110

Val Asn Ile Ser Leu His Tyr Gln Lys Asp Glu Glu Pro Leu Phe Gln
115 120 125

Leu Lys Lys Val Arg Ser Val Asn Ser Leu Met Val Ala Ser Leu Thr
130 135 140

Tyr Lys Asp Lys Val Tyr Leu Asn Val Thr Thr Asp Asn Thr Ser Leu
145 150 155 160

Asp Asp Phe His Val Asn Gly Gly Glu Leu Ile Leu Ile His Gln Asn
165 170 175

Pro Gly Glu Phe Cys Val Leu
180

<210> 45
<211> 193
<212> PRT
<213> human

<400> 45

Met Pro Glu Glu Gly Ser Gly Cys Ser Val Arg Arg Arg Pro Tyr Gly
1 5 10 15

Cys Val Leu Arg Ala Ala Leu Val Pro Leu Val Ala Gly Leu Val Ile
20 25 30

Cys Leu Val Val Cys Ile Gln Arg Phe Ala Gln Ala Gln Gln Gln Leu
35 40 45

Pro Leu Glu Ser Leu Gly Trp Asp Val Ala Glu Leu Gln Leu Asn His
50 55 60

Thr Gly Pro Gln Gln Asp Pro Arg Leu Tyr Trp Gln Gly Gly Pro Ala
65 70 75 80

Leu Gly Arg Ser Phe Leu His Gly Pro Glu Leu Asp Lys Gly Gln Leu
85 90 95

Arg Ile His Arg Asp Gly Ile Tyr Met Val His Ile Gln Val Thr Leu
100 105 110

Ala Ile Cys Ser Ser Thr Thr Ala Ser Arg His His Pro Thr Thr Leu
115 120 125

Ala Val Gly Ile Cys Ser Pro Ala Ser Arg Ser Ile Ser Leu Leu Arg
130 135 140

Leu Ser Phe His Gln Gly Cys Thr Ile Ala Ser Gln Arg Leu Thr Pro
145 150 155 160

Leu Ala Arg Gly Asp Thr Leu Cys Thr Asn Leu Thr Gly Thr Leu Leu
165 170 175

Pro Ser Arg Asn Thr Asp Glu Thr Phe Phe Gly Val Gln Trp Val Arg
180 185 190

Pro

<210> 46
<211> 234
<212> PRT
<213> human

<400> 46

Met Asp Pro Gly Leu Gln Gln Ala Leu Asn Gly Met Ala Pro Pro Gly
1 5 10 15

Asp Thr Ala Met His Val Pro Ala Gly Ser Val Ala Ser His Leu Gly
20 25 30

Thr Thr Ser Arg Ser Tyr Phe Tyr Leu Thr Thr Ala Thr Leu Ala Leu
35 40 45

Cys Leu Val Phe Thr Val Ala Thr Ile Met Val Leu Val Val Gln Arg
50 55 60

Thr Asp Ser Ile Pro Asn Ser Pro Asp Asn Val Pro Leu Lys Gly Gly
65 70 75 80

Asn Cys Ser Glu Asp Leu Leu Cys Ile Leu Lys Arg Ala Pro Phe Lys
85 90 95

Lys Ser Trp Ala Tyr Leu Gln Val Ala Lys His Leu Asn Lys Thr Lys
100 105 110

Leu Ser Trp Asn Lys Asp Gly Ile Leu His Gly Val Arg Tyr Gln Asp
115 120 125

Gly Asn Leu Val Ile Gln Phe Pro Gly Leu Tyr Phe Ile Ile Cys Gln
130 135 140

Leu Gln Phe Leu Val Gln Cys Pro Asn Asn Ser Val Asp Leu Lys Leu
145 150 155 160

Glu Leu Leu Ile Asn Lys His Ile Lys Lys Gln Ala Leu Val Thr Val
165 170 175

Cys Glu Ser Gly Met Gln Thr Lys His Val Tyr Gln Asn Leu Ser Gln
180 185 190

Phe Leu Leu Asp Tyr Leu Gln Val Asn Thr Thr Ile Ser Val Asn Val
195 200 205

Asp Thr Phe Gln Tyr Ile Asp Thr Ser Thr Phe Pro Leu Glu Asn Val
210 215 220

Leu Ser Ile Phe Leu Tyr Ser Asn Ser Asp
225 230

<210> 47
<211> 254
<212> PRT
<213> human

<400> 47

Met Glu Tyr Ala Ser Asp Ala Ser Leu Asp Pro Glu Ala Pro Trp Pro
1 5 10 15

Pro Ala Pro Arg Ala Arg Ala Cys Arg Val Leu Pro Trp Ala Leu Val
20 25 30

Ala Gly Leu Leu Leu Leu Leu Leu Leu Ala Ala Ala Cys Ala Val Phe
35 40 45

Leu Ala Cys Pro Trp Ala Val Ser Gly Ala Arg Ala Ser Pro Gly Ser
50 55 60

Ala Ala Ser Pro Arg Leu Arg Glu Gly Pro Glu Leu Ser Pro Asp Asp
65 70 75 80

Pro Ala Gly Leu Leu Asp Leu Arg Gln Gly Met Phe Ala Gln Leu Val
85 90 95

Ala Gln Asn Val Leu Leu Ile Asp Gly Pro Leu Ser Trp Tyr Ser Asp
100 105 110

Pro Gly Leu Ala Gly Val Ser Leu Thr Gly Gly Leu Ser Tyr Lys Glu
115 120 125

```
Asp Thr Lys Glu Leu Val Val Ala Lys Ala Gly Val Tyr Tyr Val Phe
    130                 135                 140

Phe Gln Leu Glu Leu Arg Arg Val Val Ala Gly Glu Gly Ser Gly Ser
145                 150                 155                 160

Val Ser Leu Ala Leu His Leu Gln Pro Leu Arg Ser Ala Ala Gly Ala
                165                 170                 175

Ala Ala Leu Ala Leu Thr Val Asp Leu Pro Pro Ala Ser Ser Glu Ala
            180                 185                 190

Arg Asn Ser Ala Phe Gly Phe Gln Gly Arg Leu Leu His Leu Ser Ala
        195                 200                 205

Gly Gln Arg Leu Gly Val His Leu His Thr Glu Ala Arg Ala Arg His
    210                 215                 220

Ala Trp Gln Leu Thr Gln Gly Ala Thr Val Leu Gly Leu Phe Arg Val
225                 230                 235                 240

Thr Pro Glu Ile Pro Ala Gly Leu Pro Ser Pro Arg Ser Glu
                245                 250


<210>  48
<211>  240
<212>  PRT
<213>  human

<400>  48

Met Glu Glu Ser Val Val Arg Pro Ser Val Phe Val Val Asp Gly Gln
1               5                   10                  15

Thr Asp Ile Pro Phe Thr Arg Leu Gly Arg Ser His Arg Arg Gln Ser
            20                  25                  30

Cys Ser Val Ala Arg Val Gly Leu Gly Leu Leu Leu Leu Leu Met Gly
        35                  40                  45

Ala Gly Leu Ala Val Gln Gly Trp Phe Leu Leu Gln Leu His Trp Arg
    50                  55                  60

Leu Gly Glu Met Val Thr Arg Leu Pro Asp Gly Pro Ala Gly Ser Trp
65                  70                  75                  80

Glu Gln Leu Ile Gln Glu Arg Arg Ser His Glu Val Asn Pro Ala Ala
                85                  90                  95
```

```
His Leu Thr Gly Ala Asn Ser Ser Leu Thr Gly Ser Gly Gly Pro Leu
            100                 105                 110

Leu Trp Glu Thr Gln Leu Gly Leu Ala Phe Leu Arg Gly Leu Ser Tyr
        115                 120                 125

His Asp Gly Ala Leu Val Val Thr Lys Ala Gly Tyr Tyr Tyr Ile Tyr
    130                 135                 140

Ser Lys Val Gln Leu Gly Gly Val Gly Cys Pro Leu Gly Leu Ala Ser
145                 150                 155                 160

Thr Ile Thr His Gly Leu Tyr Lys Arg Thr Pro Arg Tyr Pro Glu Glu
                165                 170                 175

Leu Glu Leu Leu Val Ser Gln Gln Ser Pro Cys Gly Arg Ala Thr Ser
            180                 185                 190

Ser Ser Arg Val Trp Trp Asp Ser Ser Phe Leu Gly Gly Val Val His
        195                 200                 205

Leu Glu Ala Gly Glu Lys Val Val Val Arg Val Leu Asp Glu Arg Leu
    210                 215                 220

Val Arg Leu Arg Asp Gly Thr Arg Ser Tyr Phe Gly Ala Phe Met Val
225                 230                 235                 240


<210>  49
<211>  199
<212>  PRT
<213>  human

<400>  49

Met Thr Leu His Pro Ser Pro Ile Thr Cys Glu Phe Leu Phe Ser Thr
1               5                   10                  15

Ala Leu Ile Ser Pro Lys Met Cys Leu Ser His Leu Glu Asn Met Pro
            20                  25                  30

Leu Ser His Ser Arg Thr Gln Gly Ala Gln Arg Ser Ser Trp Lys Leu
        35                  40                  45

Trp Leu Phe Cys Ser Ile Val Met Leu Leu Phe Leu Cys Ser Phe Ser
    50                  55                  60

Trp Leu Ile Phe Ile Phe Leu Gln Leu Glu Thr Ala Lys Glu Pro Cys
65                  70                  75                  80
```

Met Ala Lys Phe Gly Pro Leu Pro Ser Lys Trp Gln Met Ala Ser Ser
85 90 95

Glu Pro Pro Cys Val Asn Lys Val Ser Asp Trp Lys Leu Glu Ile Leu
100 105 110

Gln Asn Gly Leu Tyr Leu Ile Tyr Gly Gln Val Ala Pro Asn Ala Asn
115 120 125

Tyr Asn Asp Val Ala Pro Phe Glu Val Arg Leu Tyr Lys Asn Lys Asp
130 135 140

Met Ile Gln Thr Leu Thr Asn Lys Ser Lys Ile Gln Asn Val Gly Gly
145 150 155 160

Thr Tyr Glu Leu His Val Gly Asp Thr Ile Asp Leu Ile Phe Asn Ser
165 170 175

Glu His Gln Val Leu Lys Asn Asn Thr Tyr Trp Gly Ile Ile Leu Leu
180 185 190

Ala Asn Pro Gln Phe Ile Ser
195

<210> 50
<211> 205
<212> PRT
<213> human

<400> 50

Ala Cys Pro Trp Ala Val Ser Gly Ala Arg Ala Ser Pro Gly Ser Ala
1 5 10 15

Ala Ser Pro Arg Leu Arg Glu Gly Pro Glu Leu Ser Pro Asp Asp Pro
20 25 30

Ala Gly Leu Leu Asp Leu Arg Gln Gly Met Phe Ala Gln Leu Val Ala
35 40 45

Gln Asn Val Leu Leu Ile Asp Gly Pro Leu Ser Trp Tyr Ser Asp Pro
50 55 60

Gly Leu Ala Gly Val Ser Leu Thr Gly Gly Leu Ser Tyr Lys Glu Asp
65 70 75 80

Thr Lys Glu Leu Val Val Ala Lys Ala Gly Val Tyr Tyr Val Phe Phe
85 90 95

Gln Leu Glu Leu Arg Arg Val Val Ala Gly Glu Gly Ser Gly Ser Val
100 105 110

Ser Leu Ala Leu His Leu Gln Pro Leu Arg Ser Ala Ala Gly Ala Ala
115 120 125

Ala Leu Ala Leu Thr Val Asp Leu Pro Pro Ala Ser Ser Glu Ala Arg
130 135 140

Asn Ser Ala Phe Gly Phe Gln Gly Arg Leu Leu His Leu Ser Ala Gly
145 150 155 160

Gln Arg Leu Gly Val His Leu His Thr Glu Ala Arg Ala Arg His Ala
165 170 175

Trp Gln Leu Thr Gln Gly Ala Thr Val Leu Gly Leu Phe Arg Val Thr
180 185 190

Pro Glu Ile Pro Ala Gly Leu Pro Ser Pro Arg Ser Glu
195 200 205

<210> 51
<211> 5
<212> PRT
<213> Artificial Sequence

<220>
<223> Peptide linker G4S

<400> 51

Gly Gly Gly Gly Ser
1 5

<210> 52
<211> 10
<212> PRT
<213> Artificial Sequence

<220>
<223> Peptide linker (G4S)2

<400> 52

Gly Gly Gly Gly Ser Gly Gly Gly Gly Ser
1 5 10

<210> 53
<211> 10
<212> PRT
<213> Artificial Sequence

<220>
<223> Peptide linker (SG4)2

<400> 53

Ser Gly Gly Gly Gly Ser Gly Gly Gly Gly
1               5                   10

<210> 54
<211> 15
<212> PRT
<213> Artificial Sequence

<220>
<223> Peptide linker (G4S)3

<400> 54

Gly Gly Gly Gly Ser Gly Gly Gly Gly Ser Gly Gly Gly Gly Ser
1               5                   10                  15

<210> 55
<211> 14
<212> PRT
<213> Artificial Sequence

<220>
<223> Peptide linker G4(SG4)2

<400> 55

Gly Gly Gly Gly Ser Gly Gly Gly Gly Ser Gly Gly Gly Gly
1               5                   10

<210> 56
<211> 20
<212> PRT
<213> Artificial Sequence

<220>
<223> Peptide linker (G4S)4

<400> 56

Gly Gly Gly Gly Ser Gly Gly Gly Gly Ser Gly Gly Gly Gly Ser Gly
1               5                   10                  15

Gly Gly Gly Ser
            20

<210> 57
<211> 10
<212> PRT
<213> Artificial Sequence

<220>
<223> Peptide linker

<400> 57

Gly Ser Pro Gly Ser Ser Ser Ser Gly Ser
1 5 10

<210> 58
<211> 8
<212> PRT
<213> Artificial Sequence

<220>
<223> Peptide linker

<400> 58

Gly Ser Gly Ser Gly Ser Gly Ser
1 5

<210> 59
<211> 8
<212> PRT
<213> Artificial Sequence

<220>
<223> Peptide linker

<400> 59

Gly Ser Gly Ser Gly Asn Gly Ser
1 5

<210> 60
<211> 8
<212> PRT
<213> Artificial Sequence

<220>
<223> Peptide linker

<400> 60

Gly Gly Ser Gly Ser Gly Ser Gly
1 5

<210> 61
<211> 6
<212> PRT
<213> Artificial Sequence

<220>
<223> Peptide linker

<400> 61

Gly Gly Ser Gly Ser Gly
1 5

<210> 62
<211> 4
<212> PRT

<213> Artificial Sequence

<220>
<223> Peptide linker

<400> 62

Gly Gly Ser Gly
1

<210> 63
<211> 8
<212> PRT
<213> Artificial Sequence

<220>
<223> Peptide linker

<400> 63

Gly Gly Ser Gly Asn Gly Ser Gly
1 5

<210> 64
<211> 8
<212> PRT
<213> Artificial Sequence

<220>
<223> Peptide linker

<400> 64

Gly Gly Asn Gly Ser Gly Ser Gly
1 5

<210> 65
<211> 6
<212> PRT
<213> Artificial Sequence

<220>
<223> Peptide linker

<400> 65

Gly Gly Asn Gly Ser Gly
1 5

<210> 66
<211> 501
<212> PRT
<213> human

<400> 66

Met Ala Ala Ala Ser Val Thr Pro Pro Gly Ser Leu Glu Leu Leu Gln
1 5 10 15

```
Pro Gly Phe Ser Lys Thr Leu Leu Gly Thr Lys Leu Glu Ala Lys Tyr
            20                  25                  30

Leu Cys Ser Ala Cys Arg Asn Val Leu Arg Arg Pro Phe Gln Ala Gln
        35                  40                  45

Cys Gly His Arg Tyr Cys Ser Phe Cys Leu Ala Ser Ile Leu Ser Ser
    50                  55                  60

Gly Pro Gln Asn Cys Ala Ala Cys Val His Glu Gly Ile Tyr Glu Glu
65                  70                  75                  80

Gly Ile Ser Ile Leu Glu Ser Ser Ser Ala Phe Pro Asp Asn Ala Ala
                85                  90                  95

Arg Arg Glu Val Glu Ser Leu Pro Ala Val Cys Pro Ser Asp Gly Cys
            100                 105                 110

Thr Trp Lys Gly Thr Leu Lys Glu Tyr Glu Ser Cys His Glu Gly Arg
        115                 120                 125

Cys Pro Leu Met Leu Thr Glu Cys Pro Ala Cys Lys Gly Leu Val Arg
    130                 135                 140

Leu Gly Glu Lys Glu Arg His Leu Glu His Glu Cys Pro Glu Arg Ser
145                 150                 155                 160

Leu Ser Cys Arg His Cys Arg Ala Pro Cys Cys Gly Ala Asp Val Lys
                165                 170                 175

Ala His His Glu Val Cys Pro Lys Phe Pro Leu Thr Cys Asp Gly Cys
            180                 185                 190

Gly Lys Lys Lys Ile Pro Arg Glu Lys Phe Gln Asp His Val Lys Thr
        195                 200                 205

Cys Gly Lys Cys Arg Val Pro Cys Arg Phe His Ala Ile Gly Cys Leu
    210                 215                 220

Glu Thr Val Glu Gly Glu Lys Gln Gln Glu His Glu Val Gln Trp Leu
225                 230                 235                 240

Arg Glu His Leu Ala Met Leu Leu Ser Ser Val Leu Glu Ala Lys Pro
                245                 250                 255

Leu Leu Gly Asp Gln Ser His Ala Gly Ser Glu Leu Leu Gln Arg Cys
            260                 265                 270
```

```
Glu Ser Leu Glu Lys Lys Thr Ala Thr Phe Glu Asn Ile Val Cys Val
        275                 280                 285

Leu Asn Arg Glu Val Glu Arg Val Ala Met Thr Ala Glu Ala Cys Ser
    290                 295                 300

Arg Gln His Arg Leu Asp Gln Asp Lys Ile Glu Ala Leu Ser Ser Lys
305                 310                 315                 320

Val Gln Gln Leu Glu Arg Ser Ile Gly Leu Lys Asp Leu Ala Met Ala
                325                 330                 335

Asp Leu Glu Gln Lys Val Leu Glu Met Glu Ala Ser Thr Tyr Asp Gly
            340                 345                 350

Val Phe Ile Trp Lys Ile Ser Asp Phe Ala Arg Lys Arg Gln Glu Ala
        355                 360                 365

Val Ala Gly Arg Ile Pro Ala Ile Phe Ser Pro Ala Phe Tyr Thr Ser
    370                 375                 380

Arg Tyr Gly Tyr Lys Met Cys Leu Arg Ile Tyr Leu Asn Gly Asp Gly
385                 390                 395                 400

Thr Gly Arg Gly Thr His Leu Ser Leu Phe Phe Val Val Met Lys Gly
                405                 410                 415

Pro Asn Asp Ala Leu Leu Arg Trp Pro Phe Asn Gln Lys Val Thr Leu
            420                 425                 430

Met Leu Leu Asp Gln Asn Asn Arg Glu His Val Ile Asp Ala Phe Arg
        435                 440                 445

Pro Asp Val Thr Ser Ser Ser Phe Gln Arg Pro Val Asn Asp Met Asn
    450                 455                 460

Ile Ala Ser Gly Cys Pro Leu Phe Cys Pro Val Ser Lys Met Glu Ala
465                 470                 475                 480

Lys Asn Ser Tyr Val Arg Asp Asp Ala Ile Phe Ile Lys Ala Ile Val
                485                 490                 495

Asp Leu Thr Gly Leu
            500
```

<210> 67

<211> 1170
<212> PRT
<213> human

<400> 67

```
Met Gly Leu Ala Trp Gly Leu Gly Val Leu Phe Leu Met His Val Cys
1               5                   10                  15

Gly Thr Asn Arg Ile Pro Glu Ser Gly Gly Asp Asn Ser Val Phe Asp
            20                  25                  30

Ile Phe Glu Leu Thr Gly Ala Ala Arg Lys Gly Ser Gly Arg Arg Leu
        35                  40                  45

Val Lys Gly Pro Asp Pro Ser Ser Pro Ala Phe Arg Ile Glu Asp Ala
    50                  55                  60

Asn Leu Ile Pro Pro Val Pro Asp Asp Lys Phe Gln Asp Leu Val Asp
65                  70                  75                  80

Ala Val Arg Ala Glu Lys Gly Phe Leu Leu Leu Ala Ser Leu Arg Gln
                85                  90                  95

Met Lys Lys Thr Arg Gly Thr Leu Leu Ala Leu Glu Arg Lys Asp His
            100                 105                 110

Ser Gly Gln Val Phe Ser Val Val Ser Asn Gly Lys Ala Gly Thr Leu
        115                 120                 125

Asp Leu Ser Leu Thr Val Gln Gly Lys Gln His Val Val Ser Val Glu
    130                 135                 140

Glu Ala Leu Leu Ala Thr Gly Gln Trp Lys Ser Ile Thr Leu Phe Val
145                 150                 155                 160

Gln Glu Asp Arg Ala Gln Leu Tyr Ile Asp Cys Glu Lys Met Glu Asn
                165                 170                 175

Ala Glu Leu Asp Val Pro Ile Gln Ser Val Phe Thr Arg Asp Leu Ala
            180                 185                 190

Ser Ile Ala Arg Leu Arg Ile Ala Lys Gly Gly Val Asn Asp Asn Phe
        195                 200                 205

Gln Gly Val Leu Gln Asn Val Arg Phe Val Phe Gly Thr Thr Pro Glu
    210                 215                 220

Asp Ile Leu Arg Asn Lys Gly Cys Ser Ser Ser Thr Ser Val Leu Leu
```

```
225                 230                 235                 240

Thr Leu Asp Asn Asn Val Val Asn Gly Ser Ser Pro Ala Ile Arg Thr
                245                 250                 255

Asn Tyr Ile Gly His Lys Thr Lys Asp Leu Gln Ala Ile Cys Gly Ile
            260                 265                 270

Ser Cys Asp Glu Leu Ser Ser Met Val Leu Glu Leu Arg Gly Leu Arg
        275                 280                 285

Thr Ile Val Thr Thr Leu Gln Asp Ser Ile Arg Lys Val Thr Glu Glu
    290                 295                 300

Asn Lys Glu Leu Ala Asn Glu Leu Arg Arg Pro Pro Leu Cys Tyr His
305                 310                 315                 320

Asn Gly Val Gln Tyr Arg Asn Asn Glu Glu Trp Thr Val Asp Ser Cys
                325                 330                 335

Thr Glu Cys His Cys Gln Asn Ser Val Thr Ile Cys Lys Lys Val Ser
            340                 345                 350

Cys Pro Ile Met Pro Cys Ser Asn Ala Thr Val Pro Asp Gly Glu Cys
        355                 360                 365

Cys Pro Arg Cys Trp Pro Ser Asp Ser Ala Asp Asp Gly Trp Ser Pro
    370                 375                 380

Trp Ser Glu Trp Thr Ser Cys Ser Thr Ser Cys Gly Asn Gly Ile Gln
385                 390                 395                 400

Gln Arg Gly Arg Ser Cys Asp Ser Leu Asn Asn Arg Cys Glu Gly Ser
                405                 410                 415

Ser Val Gln Thr Arg Thr Cys His Ile Gln Glu Cys Asp Lys Arg Phe
            420                 425                 430

Lys Gln Asp Gly Gly Trp Ser His Trp Ser Pro Trp Ser Ser Cys Ser
        435                 440                 445

Val Thr Cys Gly Asp Gly Val Ile Thr Arg Ile Arg Leu Cys Asn Ser
    450                 455                 460

Pro Ser Pro Gln Met Asn Gly Lys Pro Cys Glu Gly Glu Ala Arg Glu
465                 470                 475                 480
```

```
Thr Lys Ala Cys Lys Lys Asp Ala Cys Pro Ile Asn Gly Gly Trp Gly
                485                 490                 495

Pro Trp Ser Pro Trp Asp Ile Cys Ser Val Thr Cys Gly Gly Gly Val
            500                 505                 510

Gln Lys Arg Ser Arg Leu Cys Asn Asn Pro Thr Pro Gln Phe Gly Gly
        515                 520                 525

Lys Asp Cys Val Gly Asp Val Thr Glu Asn Gln Ile Cys Asn Lys Gln
    530                 535                 540

Asp Cys Pro Ile Asp Gly Cys Leu Ser Asn Pro Cys Phe Ala Gly Val
545                 550                 555                 560

Lys Cys Thr Ser Tyr Pro Asp Gly Ser Trp Lys Cys Gly Ala Cys Pro
                565                 570                 575

Pro Gly Tyr Ser Gly Asn Gly Ile Gln Cys Thr Asp Val Asp Glu Cys
            580                 585                 590

Lys Glu Val Pro Asp Ala Cys Phe Asn His Asn Gly Glu His Arg Cys
        595                 600                 605

Glu Asn Thr Asp Pro Gly Tyr Asn Cys Leu Pro Cys Pro Pro Arg Phe
    610                 615                 620

Thr Gly Ser Gln Pro Phe Gly Gln Gly Val Glu His Ala Thr Ala Asn
625                 630                 635                 640

Lys Gln Val Cys Lys Pro Arg Asn Pro Cys Thr Asp Gly Thr His Asp
                645                 650                 655

Cys Asn Lys Asn Ala Lys Cys Asn Tyr Leu Gly His Tyr Ser Asp Pro
            660                 665                 670

Met Tyr Arg Cys Glu Cys Lys Pro Gly Tyr Ala Gly Asn Gly Ile Ile
        675                 680                 685

Cys Gly Glu Asp Thr Asp Leu Asp Gly Trp Pro Asn Glu Asn Leu Val
    690                 695                 700

Cys Val Ala Asn Ala Thr Tyr His Cys Lys Lys Asp Asn Cys Pro Asn
705                 710                 715                 720

Leu Pro Asn Ser Gly Gln Glu Asp Tyr Asp Lys Asp Gly Ile Gly Asp
                725                 730                 735
```

```
Ala Cys Asp Asp Asp Asp Asp Asn Asp Lys Ile Pro Asp Asp Arg Asp
            740                 745                 750

Asn Cys Pro Phe His Tyr Asn Pro Ala Gln Tyr Asp Tyr Asp Arg Asp
        755                 760                 765

Asp Val Gly Asp Arg Cys Asp Asn Cys Pro Tyr Asn His Asn Pro Asp
    770                 775                 780

Gln Ala Asp Thr Asp Asn Asn Gly Glu Gly Asp Ala Cys Ala Ala Asp
785                 790                 795                 800

Ile Asp Gly Asp Gly Ile Leu Asn Glu Arg Asp Asn Cys Gln Tyr Val
                805                 810                 815

Tyr Asn Val Asp Gln Arg Asp Thr Asp Met Asp Gly Val Gly Asp Gln
            820                 825                 830

Cys Asp Asn Cys Pro Leu Glu His Asn Pro Asp Gln Leu Asp Ser Asp
        835                 840                 845

Ser Asp Arg Ile Gly Asp Thr Cys Asp Asn Asn Gln Asp Ile Asp Glu
    850                 855                 860

Asp Gly His Gln Asn Asn Leu Asp Asn Cys Pro Tyr Val Pro Asn Ala
865                 870                 875                 880

Asn Gln Ala Asp His Asp Lys Asp Gly Lys Gly Asp Ala Cys Asp His
                885                 890                 895

Asp Asp Asp Asn Asp Gly Ile Pro Asp Asp Lys Asp Asn Cys Arg Leu
            900                 905                 910

Val Pro Asn Pro Asp Gln Lys Asp Ser Asp Gly Asp Gly Arg Gly Asp
        915                 920                 925

Ala Cys Lys Asp Asp Phe Asp His Asp Ser Val Pro Asp Ile Asp Asp
    930                 935                 940

Ile Cys Pro Glu Asn Val Asp Ile Ser Glu Thr Asp Phe Arg Arg Phe
945                 950                 955                 960

Gln Met Ile Pro Leu Asp Pro Lys Gly Thr Ser Gln Asn Asp Pro Asn
                965                 970                 975

Trp Val Val Arg His Gln Gly Lys Glu Leu Val Gln Thr Val Asn Cys
            980                 985                 990
```

```
Asp Pro Gly Leu Ala Val Gly Tyr  Asp Glu Phe Asn Ala  Val Asp Phe
        995                 1000                 1005

Ser Gly  Thr Phe Phe Ile Asn  Thr Glu Arg Asp Asp  Asp Tyr Ala
    1010                 1015                 1020

Gly Phe  Val Phe Gly Tyr Gln  Ser Ser Ser Arg Phe  Tyr Val Val
    1025                 1030                 1035

Met Trp  Lys Gln Val Thr Gln  Ser Tyr Trp Asp Thr  Asn Pro Thr
    1040                 1045                 1050

Arg Ala  Gln Gly Tyr Ser Gly  Leu Ser Val Lys Val  Val Asn Ser
    1055                 1060                 1065

Thr Thr  Gly Pro Gly Glu His  Leu Arg Asn Ala Leu  Trp His Thr
    1070                 1075                 1080

Gly Asn  Thr Pro Gly Gln Val  Arg Thr Leu Trp His  Asp Pro Arg
    1085                 1090                 1095

His Ile  Gly Trp Lys Asp Phe  Thr Ala Tyr Arg Trp  Arg Leu Ser
    1100                 1105                 1110

His Arg  Pro Lys Thr Gly Phe  Ile Arg Val Val Met  Tyr Glu Gly
    1115                 1120                 1125

Lys Lys  Ile Met Ala Asp Ser  Gly Pro Ile Tyr Asp  Lys Thr Tyr
    1130                 1135                 1140

Ala Gly  Gly Arg Leu Gly Leu  Phe Val Phe Ser Gln  Glu Met Val
    1145                 1150                 1155

Phe Phe  Ser Asp Leu Lys Tyr  Glu Cys Arg Asp Pro
    1160                 1165                 1170

<210>  68
<211>  622
<212>  PRT
<213>  human

<400>  68

Met Arg Gly Leu Leu Cys Trp Pro Val Leu Leu Leu Leu Leu Gln Pro
1               5                   10                  15

Trp Glu Thr Gln Leu Gln Leu Thr Gly Pro Arg Cys His Thr Gly Pro
            20                  25                  30
```

```
Leu Asp Leu Val Phe Val Ile Asp Ser Ser Arg Ser Val Arg Pro Phe
        35                  40                  45

Glu Phe Glu Thr Met Arg Gln Phe Leu Met Gly Leu Leu Arg Gly Leu
    50                  55                  60

Asn Val Gly Pro Asn Ala Thr Arg Val Gly Val Ile Gln Tyr Ser Ser
65                  70                  75                  80

Gln Val Gln Ser Val Phe Pro Leu Arg Ala Phe Ser Arg Arg Glu Asp
                85                  90                  95

Met Glu Arg Ala Ile Arg Asp Leu Val Pro Leu Ala Gln Gly Thr Met
            100                 105                 110

Thr Gly Leu Ala Ile Gln Tyr Ala Met Asn Val Ala Phe Ser Val Ala
        115                 120                 125

Glu Gly Ala Arg Pro Pro Glu Glu Arg Val Pro Arg Val Ala Val Ile
    130                 135                 140

Val Thr Asp Gly Arg Pro Gln Asp Arg Val Ala Glu Val Ala Ala Gln
145                 150                 155                 160

Ala Arg Ala Arg Gly Ile Glu Ile Tyr Ala Val Gly Val Gln Arg Ala
                165                 170                 175

Asp Val Gly Ser Leu Arg Ala Met Ala Ser Pro Pro Leu Asp Glu His
            180                 185                 190

Val Phe Leu Val Glu Ser Phe Asp Leu Ile Gln Glu Phe Gly Leu Gln
        195                 200                 205

Phe Gln Ser Arg Leu Cys Gly Lys Asp Gln Cys Ala Glu Gly Gly His
    210                 215                 220

Gly Cys Gln His Gln Cys Val Asn Ala Trp Ala Met Phe His Cys Thr
225                 230                 235                 240

Cys Asn Pro Gly Tyr Lys Leu Ala Ala Asp Asn Lys Ser Cys Leu Ala
                245                 250                 255

Ile Asp Leu Cys Ala Glu Gly Thr His Gly Cys Glu His His Cys Val
            260                 265                 270

Asn Ser Pro Gly Ser Tyr Phe Cys His Cys Gln Val Gly Phe Val Leu
```

275 280 285

Gln Gln Asp Gln Arg Ser Cys Arg Ala Ile Asp Tyr Cys Ser Phe Gly
290 295 300

Asn His Ser Cys Gln His Glu Cys Val Ser Thr Pro Gly Gly Pro Arg
305 310 315 320

Cys His Cys Arg Glu Gly His Asp Leu Gln Pro Asp Gly Arg Ser Cys
325 330 335

Gln Val Arg Asp Leu Cys Asn Gly Val Asp His Gly Cys Glu Phe Gln
340 345 350

Cys Val Ser Glu Gly Leu Ser Tyr Arg Cys Leu Cys Pro Glu Gly Arg
355 360 365

Gln Leu Gln Ala Asp Gly Lys Ser Cys Asn Arg Cys Arg Glu Gly His
370 375 380

Val Asp Leu Val Leu Leu Val Asp Gly Ser Lys Ser Val Arg Pro Gln
385 390 395 400

Asn Phe Glu Leu Val Lys Arg Phe Val Asn Gln Ile Val Asp Phe Leu
405 410 415

Asp Val Ser Pro Glu Gly Thr Arg Val Gly Leu Val Gln Phe Ser Ser
420 425 430

Arg Val Arg Thr Glu Phe Pro Leu Gly Arg Tyr Gly Thr Ala Ala Glu
435 440 445

Val Lys Gln Ala Val Leu Ala Val Glu Tyr Met Glu Arg Gly Thr Met
450 455 460

Thr Gly Leu Ala Leu Arg His Met Val Glu His Ser Phe Ser Glu Ala
465 470 475 480

Gln Gly Ala Arg Pro Arg Ala Leu Asn Val Pro Arg Val Gly Leu Val
485 490 495

Phe Thr Asp Gly Arg Ser Gln Asp Asp Ile Ser Val Trp Ala Ala Arg
500 505 510

Ala Lys Glu Glu Gly Ile Val Met Tyr Ala Val Gly Val Gly Lys Ala
515 520 525

Val Glu Ala Glu Leu Arg Glu Ile Ala Ser Glu Pro Ala Glu Leu His
    530                 535                 540

Val Ser Tyr Ala Pro Asp Phe Gly Thr Met Thr His Leu Leu Glu Asn
545                 550                 555                 560

Leu Arg Gly Ser Ile Cys Pro Glu Glu Gly Ile Ser Ala Gly Thr Glu
                565                 570                 575

Leu Arg Ser Pro Cys Glu Cys Glu Ser Leu Val Glu Phe Gln Gly Arg
            580                 585                 590

Thr Leu Gly Ala Leu Glu Ser Leu Thr Leu Asn Leu Ala Gln Leu Thr
        595                 600                 605

Ala Arg Leu Glu Asp Leu Glu Asn Gln Leu Ala Asn Gln Lys
    610                 615                 620

<210> 69
<211> 3623
<212> PRT
<213> human

<400> 69

Met Met Asn Met Ser Leu Pro Phe Leu Trp Ser Leu Leu Thr Leu Leu
1               5                   10                  15

Ile Phe Ala Glu Val Asn Gly Glu Ala Gly Glu Leu Glu Leu Gln Arg
            20                  25                  30

Gln Lys Arg Ser Ile Asn Leu Gln Gln Pro Arg Met Ala Thr Glu Arg
        35                  40                  45

Gly Asn Leu Val Phe Leu Thr Gly Ser Ala Gln Asn Ile Glu Phe Arg
    50                  55                  60

Thr Gly Ser Leu Gly Lys Ile Lys Leu Asn Asp Glu Asp Leu Ser Glu
65                  70                  75                  80

Cys Leu His Gln Ile Gln Lys Asn Lys Glu Asp Ile Ile Glu Leu Lys
                85                  90                  95

Gly Ser Ala Ile Gly Leu Pro Gln Asn Ile Ser Ser Gln Ile Tyr Gln
            100                 105                 110

Leu Asn Ser Lys Leu Val Asp Leu Glu Arg Lys Phe Gln Gly Leu Gln
        115                 120                 125

Gln Thr Val Asp Lys Lys Val Cys Ser Ser Asn Pro Cys Gln Asn Gly
130 135 140

Gly Thr Cys Leu Asn Leu His Asp Ser Phe Phe Cys Ile Cys Pro Pro
145 150 155 160

Gln Trp Lys Gly Pro Leu Cys Ser Ala Asp Val Asn Glu Cys Glu Ile
165 170 175

Tyr Ser Gly Thr Pro Leu Ser Cys Gln Asn Gly Gly Thr Cys Val Asn
180 185 190

Thr Met Gly Ser Tyr Ser Cys His Cys Pro Pro Glu Thr Tyr Gly Pro
195 200 205

Gln Cys Ala Ser Lys Tyr Asp Asp Cys Glu Gly Gly Ser Val Ala Arg
210 215 220

Cys Val His Gly Ile Cys Glu Asp Leu Met Arg Glu Gln Ala Gly Glu
225 230 235 240

Pro Lys Tyr Ser Cys Val Cys Asp Ala Gly Trp Met Phe Ser Pro Asn
245 250 255

Ser Pro Ala Cys Thr Leu Asp Arg Asp Glu Cys Ser Phe Gln Pro Gly
260 265 270

Pro Cys Ser Thr Leu Val Gln Cys Phe Asn Thr Gln Gly Ser Phe Tyr
275 280 285

Cys Gly Ala Cys Pro Thr Gly Trp Gln Gly Asn Gly Tyr Ile Cys Glu
290 295 300

Asp Ile Asn Glu Cys Glu Ile Asn Asn Gly Gly Cys Ser Val Ala Pro
305 310 315 320

Pro Val Glu Cys Val Asn Thr Pro Gly Ser Ser His Cys Gln Ala Cys
325 330 335

Pro Pro Gly Tyr Gln Gly Asp Gly Arg Val Cys Thr Leu Thr Asp Ile
340 345 350

Cys Ser Val Ser Asn Gly Gly Cys His Pro Asp Ala Ser Cys Ser Ser
355 360 365

Thr Leu Gly Ser Leu Pro Leu Cys Thr Cys Leu Pro Gly Tyr Thr Gly
370 375 380

Asn Gly Tyr Gly Pro Asn Gly Cys Val Gln Leu Ser Asn Ile Cys Leu
385 390 395 400

Ser His Pro Cys Leu Asn Gly Gln Cys Ile Asp Thr Val Ser Gly Tyr
405 410 415

Phe Cys Lys Cys Asp Ser Gly Trp Thr Gly Val Asn Cys Thr Glu Asn
420 425 430

Ile Asn Glu Cys Leu Ser Asn Pro Cys Leu Asn Gly Gly Thr Cys Val
435 440 445

Asp Gly Val Asp Ser Phe Ser Cys Glu Cys Thr Arg Leu Trp Thr Gly
450 455 460

Ala Leu Cys Gln Val Pro Gln Gln Val Cys Gly Glu Ser Leu Ser Gly
465 470 475 480

Ile Asn Gly Ser Phe Ser Tyr Arg Ser Pro Asp Val Gly Tyr Val His
485 490 495

Asp Val Asn Cys Phe Trp Val Ile Lys Thr Glu Met Gly Lys Val Leu
500 505 510

Arg Ile Thr Phe Thr Phe Phe Arg Leu Glu Ser Met Asp Asn Cys Pro
515 520 525

His Glu Phe Leu Gln Val Tyr Asp Gly Asp Ser Ser Ser Ala Phe Gln
530 535 540

Leu Gly Arg Phe Cys Gly Ser Ser Leu Pro His Glu Leu Leu Ser Ser
545 550 555 560

Asp Asn Ala Leu Tyr Phe His Leu Tyr Ser Glu His Leu Arg Asn Gly
565 570 575

Arg Gly Phe Thr Val Arg Trp Glu Thr Gln Gln Pro Glu Cys Gly Gly
580 585 590

Ile Leu Thr Gly Pro Tyr Gly Ser Ile Lys Ser Pro Gly Tyr Pro Gly
595 600 605

Asn Tyr Pro Pro Gly Arg Asp Cys Val Trp Ile Val Val Thr Ser Pro
610 615 620

Asp Leu Leu Val Thr Phe Thr Phe Gly Thr Leu Ser Leu Glu His His
625 630 635 640

```
Asp Asp Cys Asn Lys Asp Tyr Leu Glu Ile Arg Asp Gly Pro Leu Tyr
                645                 650                 655

Gln Asp Pro Leu Leu Gly Lys Phe Cys Thr Thr Phe Ser Val Pro Pro
            660                 665                 670

Leu Gln Thr Thr Gly Pro Phe Ala Arg Ile His Phe His Ser Asp Ser
        675                 680                 685

Gln Ile Ser Asp Gln Gly Phe His Ile Thr Tyr Leu Thr Ser Pro Ser
    690                 695                 700

Asp Leu Arg Cys Gly Gly Asn Tyr Thr Asp Pro Glu Gly Glu Leu Phe
705                 710                 715                 720

Leu Pro Glu Leu Ser Gly Pro Phe Thr His Thr Arg Gln Cys Val Tyr
                725                 730                 735

Met Met Lys Gln Pro Gln Gly Glu Gln Ile Gln Ile Asn Phe Thr His
            740                 745                 750

Val Glu Leu Gln Cys Gln Ser Asp Ser Ser Gln Asn Tyr Ile Glu Val
        755                 760                 765

Arg Asp Gly Glu Thr Leu Leu Gly Lys Val Cys Gly Asn Gly Thr Ile
    770                 775                 780

Ser His Ile Lys Ser Ile Thr Asn Ser Val Trp Ile Arg Phe Lys Ile
785                 790                 795                 800

Asp Ala Ser Val Glu Lys Ala Ser Phe Arg Ala Val Tyr Gln Val Ala
                805                 810                 815

Cys Gly Asp Glu Leu Thr Gly Glu Gly Val Ile Arg Ser Pro Phe Phe
            820                 825                 830

Pro Asn Val Tyr Pro Gly Glu Arg Thr Cys Arg Trp Thr Ile His Gln
        835                 840                 845

Pro Gln Ser Gln Val Ile Leu Leu Asn Phe Thr Val Phe Glu Ile Gly
    850                 855                 860

Ser Ser Ala His Cys Glu Thr Asp Tyr Val Glu Ile Gly Ser Ser Ser
865                 870                 875                 880

Ile Leu Gly Ser Pro Glu Asn Lys Lys Tyr Cys Gly Thr Asp Ile Pro
```

885 890 895

Ser Phe Ile Thr Ser Val Tyr Asn Phe Leu Tyr Val Thr Phe Val Lys
900 905 910

Ser Ser Ser Thr Glu Asn His Gly Phe Met Ala Lys Phe Ser Ala Glu
915 920 925

Asp Leu Ala Cys Gly Glu Ile Leu Thr Glu Ser Thr Gly Thr Ile Gln
930 935 940

Ser Pro Gly His Pro Asn Val Tyr Pro His Gly Ile Asn Cys Thr Trp
945 950 955 960

His Ile Leu Val Gln Pro Asn His Leu Ile His Leu Met Phe Glu Thr
965 970 975

Phe His Leu Glu Phe His Tyr Asn Cys Thr Asn Asp Tyr Leu Glu Val
980 985 990

Tyr Asp Thr Asp Ser Glu Thr Ser Leu Gly Arg Tyr Cys Gly Lys Ser
995 1000 1005

Ile Pro Pro Ser Leu Thr Ser Ser Gly Asn Ser Leu Met Leu Val
1010 1015 1020

Phe Val Thr Asp Ser Asp Leu Ala Tyr Glu Gly Phe Leu Ile Asn
1025 1030 1035

Tyr Glu Ala Ile Ser Ala Ala Thr Ala Cys Leu Gln Asp Tyr Thr
1040 1045 1050

Asp Asp Leu Gly Thr Phe Thr Ser Pro Asn Phe Pro Asn Asn Tyr
1055 1060 1065

Pro Asn Asn Trp Glu Cys Ile Tyr Arg Ile Thr Val Arg Thr Gly
1070 1075 1080

Gln Leu Ile Ala Val His Phe Thr Asn Phe Ser Leu Glu Glu Ala
1085 1090 1095

Ile Gly Asn Tyr Tyr Thr Asp Phe Leu Glu Ile Arg Asp Gly Gly
1100 1105 1110

Tyr Glu Lys Ser Pro Leu Leu Gly Ile Phe Tyr Gly Ser Asn Leu
1115 1120 1125

Pro Pro Thr Ile Ile Ser His Ser Asn Lys Leu Trp Leu Lys Phe
1130 1135 1140

Lys Ser Asp Gln Ile Asp Thr Arg Ser Gly Phe Ser Ala Tyr Trp
1145 1150 1155

Asp Gly Ser Ser Thr Gly Cys Gly Gly Asn Leu Thr Thr Ser Ser
1160 1165 1170

Gly Thr Phe Ile Ser Pro Asn Tyr Pro Met Pro Tyr Tyr His Ser
1175 1180 1185

Ser Glu Cys Tyr Trp Trp Leu Lys Ser Ser His Gly Ser Ala Phe
1190 1195 1200

Glu Leu Glu Phe Lys Asp Phe His Leu Glu His His Pro Asn Cys
1205 1210 1215

Thr Leu Asp Tyr Leu Ala Val Tyr Asp Gly Pro Ser Ser Asn Ser
1220 1225 1230

His Leu Leu Thr Gln Leu Cys Gly Asp Glu Lys Pro Pro Leu Ile
1235 1240 1245

Arg Ser Ser Gly Asp Ser Met Phe Ile Lys Leu Arg Thr Asp Glu
1250 1255 1260

Gly Gln Gln Gly Arg Gly Phe Lys Ala Glu Tyr Arg Gln Thr Cys
1265 1270 1275

Glu Asn Val Val Ile Val Asn Gln Thr Tyr Gly Ile Leu Glu Ser
1280 1285 1290

Ile Gly Tyr Pro Asn Pro Tyr Ser Glu Asn Gln His Cys Asn Trp
1295 1300 1305

Thr Ile Arg Ala Thr Thr Gly Asn Thr Val Asn Tyr Thr Phe Leu
1310 1315 1320

Ala Phe Asp Leu Glu His His Ile Asn Cys Ser Thr Asp Tyr Leu
1325 1330 1335

Glu Leu Tyr Asp Gly Pro Arg Gln Met Gly Arg Tyr Cys Gly Val
1340 1345 1350

Asp Leu Pro Pro Pro Gly Ser Thr Thr Ser Ser Lys Leu Gln Val
1355 1360 1365

Leu Leu Leu Thr Asp Gly Val Gly Arg Arg Glu Lys Gly Phe Gln
1370 1375 1380

Met Gln Trp Phe Val Tyr Gly Cys Gly Gly Glu Leu Ser Gly Ala
1385 1390 1395

Thr Gly Ser Phe Ser Ser Pro Gly Phe Pro Asn Arg Tyr Pro Pro
1400 1405 1410

Asn Lys Glu Cys Ile Trp Tyr Ile Arg Thr Asp Pro Gly Ser Ser
1415 1420 1425

Ile Gln Leu Thr Ile His Asp Phe Asp Val Glu Tyr His Ser Arg
1430 1435 1440

Cys Asn Phe Asp Val Leu Glu Ile Tyr Gly Gly Pro Asp Phe His
1445 1450 1455

Ser Pro Arg Ile Ala Gln Leu Cys Thr Gln Arg Ser Pro Glu Asn
1460 1465 1470

Pro Met Gln Val Ser Ser Thr Gly Asn Glu Leu Ala Ile Arg Phe
1475 1480 1485

Lys Thr Asp Leu Ser Ile Asn Gly Arg Gly Phe Asn Ala Ser Trp
1490 1495 1500

Gln Ala Val Thr Gly Gly Cys Gly Gly Ile Phe Gln Ala Pro Ser
1505 1510 1515

Gly Glu Ile His Ser Pro Asn Tyr Pro Ser Pro Tyr Arg Ser Asn
1520 1525 1530

Thr Asp Cys Ser Trp Val Ile Arg Val Asp Arg Asn His Arg Val
1535 1540 1545

Leu Leu Asn Phe Thr Asp Phe Asp Leu Glu Pro Gln Asp Ser Cys
1550 1555 1560

Ile Met Ala Tyr Asp Gly Leu Ser Ser Thr Met Ser Arg Leu Ala
1565 1570 1575

Arg Thr Cys Gly Arg Glu Gln Leu Ala Asn Pro Ile Val Ser Ser
1580 1585 1590

Gly Asn Ser Leu Phe Leu Arg Phe Gln Ser Gly Pro Ser Arg Gln
1595 1600 1605

Asn Arg Gly Phe Arg Ala Gln Phe Arg Gln Ala Cys Gly Gly His
1610 1615 1620

Ile Leu Thr Ser Ser Phe Asp Thr Val Ser Ser Pro Arg Phe Pro
1625 1630 1635

Ala Asn Tyr Pro Asn Asn Gln Asn Cys Ser Trp Ile Ile Gln Ala
1640 1645 1650

Gln Pro Pro Leu Asn His Ile Thr Leu Ser Phe Thr His Phe Glu
1655 1660 1665

Leu Glu Arg Ser Thr Thr Cys Ala Arg Asp Phe Val Glu Ile Leu
1670 1675 1680

Asp Gly Gly His Glu Asp Ala Pro Leu Arg Gly Arg Tyr Cys Gly
1685 1690 1695

Thr Asp Met Pro His Pro Ile Thr Ser Phe Ser Ser Ala Leu Thr
1700 1705 1710

Leu Arg Phe Val Ser Asp Ser Ser Ile Ser Ala Gly Gly Phe His
1715 1720 1725

Thr Thr Val Thr Ala Ser Val Ser Ala Cys Gly Gly Thr Phe Tyr
1730 1735 1740

Met Ala Glu Gly Ile Phe Asn Ser Pro Gly Tyr Pro Asp Ile Tyr
1745 1750 1755

Pro Pro Asn Val Glu Cys Val Trp Asn Ile Val Ser Ser Pro Gly
1760 1765 1770

Asn Arg Leu Gln Leu Ser Phe Ile Ser Phe Gln Leu Glu Asp Ser
1775 1780 1785

Gln Asp Cys Ser Arg Asp Phe Val Glu Ile Arg Glu Gly Asn Ala
1790 1795 1800

Thr Gly His Leu Val Gly Arg Tyr Cys Gly Asn Ser Phe Pro Leu
1805 1810 1815

Asn Tyr Ser Ser Ile Val Gly His Thr Leu Trp Val Arg Phe Ile
1820 1825 1830

Ser Asp Gly Ser Gly Ser Gly Thr Gly Phe Gln Ala Thr Phe Met

1835 1840 1845

Lys Ile Phe Gly Asn Asp Asn Ile Val Gly Thr His Gly Lys Val
1850 1855 1860

Ala Ser Pro Phe Trp Pro Glu Asn Tyr Pro His Asn Ser Asn Tyr
1865 1870 1875

Gln Trp Thr Val Asn Val Asn Ala Ser His Val Val His Gly Arg
1880 1885 1890

Ile Leu Glu Met Asp Ile Glu Glu Ile Gln Asn Cys Tyr Tyr Asp
1895 1900 1905

Lys Leu Arg Ile Tyr Asp Gly Pro Ser Ile His Ala Arg Leu Ile
1910 1915 1920

Gly Ala Tyr Cys Gly Thr Gln Thr Glu Ser Phe Ser Ser Thr Gly
1925 1930 1935

Asn Ser Leu Thr Phe His Phe Tyr Ser Asp Ser Ser Ile Ser Gly
1940 1945 1950

Lys Gly Phe Leu Leu Glu Trp Phe Ala Val Asp Ala Pro Asp Gly
1955 1960 1965

Val Leu Pro Thr Ile Ala Pro Gly Ala Cys Gly Gly Phe Leu Arg
1970 1975 1980

Thr Gly Asp Ala Pro Val Phe Leu Phe Ser Pro Gly Trp Pro Asp
1985 1990 1995

Ser Tyr Ser Asn Arg Val Asp Cys Thr Trp Leu Ile Gln Ala Pro
2000 2005 2010

Asp Ser Thr Val Glu Leu Asn Ile Leu Ser Leu Asp Ile Glu Ser
2015 2020 2025

His Arg Thr Cys Ala Tyr Asp Ser Leu Val Ile Arg Asp Gly Asp
2030 2035 2040

Asn Asn Leu Ala Gln Gln Leu Ala Val Leu Cys Gly Arg Glu Ile
2045 2050 2055

Pro Gly Pro Ile Arg Ser Thr Gly Glu Tyr Met Phe Ile Arg Phe
2060 2065 2070

Thr Ser Asp Ser Ser Val Thr Arg Ala Gly Phe Asn Ala Ser Phe
2075 2080 2085

His Lys Ser Cys Gly Gly Tyr Leu His Ala Asp Arg Gly Ile Ile
2090 2095 2100

Thr Ser Pro Lys Tyr Pro Glu Thr Tyr Pro Ser Asn Leu Asn Cys
2105 2110 2115

Ser Trp His Val Leu Val Gln Ser Gly Leu Thr Ile Ala Val His
2120 2125 2130

Phe Glu Gln Pro Phe Gln Ile Pro Asn Gly Asp Ser Ser Cys Asn
2135 2140 2145

Gln Gly Asp Tyr Leu Val Leu Arg Asn Gly Pro Asp Ile Cys Ser
2150 2155 2160

Pro Pro Leu Gly Pro Pro Gly Gly Asn Gly His Phe Cys Gly Ser
2165 2170 2175

His Ala Ser Ser Thr Leu Phe Thr Ser Asp Asn Gln Met Phe Val
2180 2185 2190

Gln Phe Ile Ser Asp His Ser Asn Glu Gly Gln Gly Phe Lys Ile
2195 2200 2205

Lys Tyr Glu Ala Lys Ser Leu Ala Cys Gly Gly Asn Val Tyr Ile
2210 2215 2220

His Asp Ala Asp Ser Ala Gly Tyr Val Thr Ser Pro Asn His Pro
2225 2230 2235

His Asn Tyr Pro Pro His Ala Asp Cys Ile Trp Ile Leu Ala Ala
2240 2245 2250

Pro Pro Glu Thr Arg Ile Gln Leu Gln Phe Glu Asp Arg Phe Asp
2255 2260 2265

Ile Glu Val Thr Pro Asn Cys Thr Ser Asn Tyr Leu Glu Leu Arg
2270 2275 2280

Asp Gly Val Asp Ser Asp Ala Pro Ile Leu Ser Lys Phe Cys Gly
2285 2290 2295

Thr Ser Leu Pro Ser Ser Gln Trp Ser Ser Gly Glu Val Met Tyr
2300 2305 2310

Leu Arg Phe Arg Ser Asp Asn Ser Pro Thr His Val Gly Phe Lys
2315 2320 2325

Ala Lys Tyr Ser Ile Ala Gln Cys Gly Gly Arg Val Pro Gly Gln
2330 2335 2340

Ser Gly Val Val Glu Ser Ile Gly His Pro Thr Leu Pro Tyr Arg
2345 2350 2355

Asp Asn Leu Phe Cys Glu Trp His Leu Gln Gly Leu Ser Gly His
2360 2365 2370

Tyr Leu Thr Ile Ser Phe Glu Asp Phe Asn Leu Gln Asn Ser Ser
2375 2380 2385

Gly Cys Glu Lys Asp Phe Val Glu Ile Trp Asp Asn His Thr Ser
2390 2395 2400

Gly Asn Ile Leu Gly Arg Tyr Cys Gly Asn Thr Ile Pro Asp Ser
2405 2410 2415

Ile Asp Thr Ser Ser Asn Thr Ala Val Val Arg Phe Val Thr Asp
2420 2425 2430

Gly Ser Val Thr Ala Ser Gly Phe Arg Leu Arg Phe Glu Ser Ser
2435 2440 2445

Met Glu Glu Cys Gly Gly Asp Leu Gln Gly Ser Ile Gly Thr Phe
2450 2455 2460

Thr Ser Pro Asn Tyr Pro Asn Pro Asn Pro His Gly Arg Ile Cys
2465 2470 2475

Glu Trp Arg Ile Thr Ala Pro Glu Gly Arg Arg Ile Thr Leu Met
2480 2485 2490

Phe Asn Asn Leu Arg Leu Ala Thr His Pro Ser Cys Asn Asn Glu
2495 2500 2505

His Val Ile Val Phe Asn Gly Ile Arg Ser Asn Ser Pro Gln Leu
2510 2515 2520

Glu Lys Leu Cys Ser Ser Val Asn Val Ser Asn Glu Ile Lys Ser
2525 2530 2535

Ser Gly Asn Thr Met Lys Val Ile Phe Phe Thr Asp Gly Ser Arg
2540 2545 2550

Pro Tyr Gly Gly Phe Thr Ala Ser Tyr Thr Ser Ser Glu Asp Ala
2555 2560 2565

Val Cys Gly Gly Ser Leu Pro Asn Thr Pro Glu Gly Asn Phe Thr
2570 2575 2580

Ser Pro Gly Tyr Asp Gly Val Arg Asn Tyr Ser Arg Asn Leu Asn
2585 2590 2595

Cys Glu Trp Thr Leu Ser Asn Pro Asn Gln Gly Asn Ser Ser Ile
2600 2605 2610

Ser Ile His Phe Glu Asp Phe Tyr Leu Glu Ser His Gln Asp Cys
2615 2620 2625

Gln Phe Asp Val Leu Glu Phe Arg Val Gly Asp Ala Asp Gly Pro
2630 2635 2640

Leu Met Trp Arg Leu Cys Gly Pro Ser Lys Pro Thr Leu Pro Leu
2645 2650 2655

Val Ile Pro Tyr Ser Gln Val Trp Ile His Phe Val Thr Asn Glu
2660 2665 2670

Arg Val Glu His Ile Gly Phe His Ala Lys Tyr Ser Phe Thr Asp
2675 2680 2685

Cys Gly Gly Ile Gln Ile Gly Asp Ser Gly Val Ile Thr Ser Pro
2690 2695 2700

Asn Tyr Pro Asn Ala Tyr Asp Ser Leu Thr His Cys Ser Ser Leu
2705 2710 2715

Leu Glu Ala Pro Gln Gly His Thr Ile Thr Leu Thr Phe Ser Asp
2720 2725 2730

Phe Asp Ile Glu Pro His Thr Thr Cys Ala Trp Asp Ser Val Thr
2735 2740 2745

Val Arg Asn Gly Gly Ser Pro Glu Ser Pro Ile Ile Gly Gln Tyr
2750 2755 2760

Cys Gly Asn Ser Asn Pro Arg Thr Ile Gln Ser Gly Ser Asn Gln
2765 2770 2775

Leu Val Val Thr Phe Asn Ser Asp His Ser Leu Gln Gly Gly Gly

2780 2785 2790

Phe Tyr Ala Thr Trp Asn Thr Gln Thr Leu Gly Cys Gly Gly Ile
2795 2800 2805

Phe His Ser Asp Asn Gly Thr Ile Arg Ser Pro His Trp Pro Gln
2810 2815 2820

Asn Phe Pro Glu Asn Ser Arg Cys Ser Trp Thr Ala Ile Thr His
2825 2830 2835

Lys Ser Lys His Leu Glu Ile Ser Phe Asp Asn Asn Phe Leu Ile
2840 2845 2850

Pro Ser Gly Asp Gly Gln Cys Gln Asn Ser Phe Val Lys Val Trp
2855 2860 2865

Ala Gly Thr Glu Glu Val Asp Lys Ala Leu Leu Ala Thr Gly Cys
2870 2875 2880

Gly Asn Val Ala Pro Gly Pro Val Ile Thr Pro Ser Asn Thr Phe
2885 2890 2895

Thr Ala Val Phe Gln Ser Gln Glu Ala Pro Ala Gln Gly Phe Ser
2900 2905 2910

Ala Ser Phe Val Ser Arg Cys Gly Ser Asn Phe Thr Gly Pro Ser
2915 2920 2925

Gly Tyr Ile Ile Ser Pro Asn Tyr Pro Lys Gln Tyr Asp Asn Asn
2930 2935 2940

Met Asn Cys Thr Tyr Val Ile Glu Ala Asn Pro Leu Ser Val Val
2945 2950 2955

Leu Leu Thr Phe Val Ser Phe His Leu Glu Ala Arg Ser Ala Val
2960 2965 2970

Thr Gly Ser Cys Val Asn Asp Gly Val His Ile Ile Arg Gly Tyr
2975 2980 2985

Ser Val Met Ser Thr Pro Phe Ala Thr Val Cys Gly Asp Glu Met
2990 2995 3000

Pro Ala Pro Leu Thr Ile Ala Gly Pro Val Leu Leu Asn Phe Tyr
3005 3010 3015

Ser Asn Glu Gln Ile Thr Asp Phe Gly Phe Lys Phe Ser Tyr Arg
3020 3025 3030

Ile Ile Ser Cys Gly Gly Val Phe Asn Phe Ser Ser Gly Ile Ile
3035 3040 3045

Thr Ser Pro Ala Tyr Ser Tyr Ala Asp Tyr Pro Asn Asp Met His
3050 3055 3060

Cys Leu Tyr Thr Ile Thr Val Ser Asp Asp Lys Val Ile Glu Leu
3065 3070 3075

Lys Phe Ser Asp Phe Asp Val Val Pro Ser Thr Ser Cys Ser His
3080 3085 3090

Asp Tyr Leu Ala Ile Tyr Asp Gly Ala Asn Thr Ser Asp Pro Leu
3095 3100 3105

Leu Gly Lys Phe Cys Gly Ser Lys Arg Pro Pro Asn Val Lys Ser
3110 3115 3120

Ser Asn Asn Ser Met Leu Leu Val Phe Lys Thr Asp Ser Phe Gln
3125 3130 3135

Thr Ala Lys Gly Trp Lys Met Ser Phe Arg Gln Thr Leu Gly Pro
3140 3145 3150

Gln Gln Gly Cys Gly Gly Tyr Leu Thr Gly Ser Asn Asn Thr Phe
3155 3160 3165

Ala Ser Pro Asp Ser Asp Ser Asn Gly Met Tyr Asp Lys Asn Leu
3170 3175 3180

Asn Cys Val Trp Ile Ile Ile Ala Pro Val Asn Lys Val Ile His
3185 3190 3195

Leu Thr Phe Asn Thr Phe Ala Leu Glu Ala Ala Ser Thr Arg Gln
3200 3205 3210

Arg Cys Leu Tyr Asp Tyr Val Lys Leu Tyr Asp Gly Asp Ser Glu
3215 3220 3225

Asn Ala Asn Leu Ala Gly Thr Phe Cys Gly Ser Thr Val Pro Ala
3230 3235 3240

Pro Phe Ile Ser Ser Gly Asn Phe Leu Thr Val Gln Phe Ile Ser
3245 3250 3255

Asp Leu Thr Leu Glu Arg Glu Gly Phe Asn Ala Thr Tyr Thr Ile
3260 3265 3270

Met Asp Met Pro Cys Gly Gly Thr Tyr Asn Ala Thr Trp Thr Pro
3275 3280 3285

Gln Asn Ile Ser Ser Pro Asn Ser Ser Asp Pro Asp Val Pro Phe
3290 3295 3300

Ser Ile Cys Thr Trp Val Ile Asp Ser Pro Pro His Gln Gln Val
3305 3310 3315

Lys Ile Thr Val Trp Ala Leu Gln Leu Thr Ser Gln Asp Cys Thr
3320 3325 3330

Gln Asn Tyr Leu Gln Leu Gln Asp Ser Pro Gln Gly His Gly Asn
3335 3340 3345

Ser Arg Phe Gln Phe Cys Gly Arg Asn Ala Ser Ala Val Pro Val
3350 3355 3360

Phe Tyr Ser Ser Met Ser Thr Ala Met Val Ile Phe Lys Ser Gly
3365 3370 3375

Val Val Asn Arg Asn Ser Arg Met Ser Phe Thr Tyr Gln Ile Ala
3380 3385 3390

Asp Cys Asn Arg Asp Tyr His Lys Ala Phe Gly Asn Leu Arg Ser
3395 3400 3405

Pro Gly Trp Pro Asp Asn Tyr Asp Asn Asp Lys Asp Cys Thr Val
3410 3415 3420

Thr Leu Thr Ala Pro Gln Asn His Thr Ile Ser Leu Phe Phe His
3425 3430 3435

Ser Leu Gly Ile Glu Asn Ser Val Glu Cys Arg Asn Asp Phe Leu
3440 3445 3450

Glu Val Arg Asn Gly Ser Asn Ser Asn Ser Pro Leu Leu Gly Lys
3455 3460 3465

Tyr Cys Gly Thr Leu Leu Pro Asn Pro Val Phe Ser Gln Asn Asn
3470 3475 3480

Glu Leu Tyr Leu Arg Phe Lys Ser Asp Ser Val Thr Ser Asp Arg
3485 3490 3495

Gly Tyr Glu Ile Ile Trp Thr Ser Ser Pro Ser Gly Cys Gly Gly
3500 3505 3510

Thr Leu Tyr Gly Asp Arg Gly Ser Phe Thr Ser Pro Gly Tyr Pro
3515 3520 3525

Gly Thr Tyr Pro Asn Asn Thr Tyr Cys Glu Trp Val Leu Val Ala
3530 3535 3540

Pro Ala Gly Arg Leu Val Thr Ile Asn Phe Tyr Phe Ile Ser Ile
3545 3550 3555

Asp Asp Pro Gly Asp Cys Val Gln Asn Tyr Leu Thr Leu Tyr Asp
3560 3565 3570

Gly Pro Asn Ala Ser Ser Pro Ser Ser Gly Pro Tyr Cys Gly Gly
3575 3580 3585

Asp Thr Ser Ile Ala Pro Phe Val Ala Ser Ser Asn Gln Val Phe
3590 3595 3600

Ile Lys Phe His Ala Asp Tyr Ala Arg Arg Pro Ser Ala Phe Arg
3605 3610 3615

Leu Thr Trp Asp Ser
3620

<210> 70
<211> 32
<212> PRT
<213> Artificial Sequence

<220>
<223> isoleucine zipper domain

<400> 70

Ile Lys Gln Ile Glu Asp Lys Ile Glu Glu Ile Leu Ser Lys Ile Tyr
1 5 10 15

His Ile Glu Asn Glu Ile Ala Arg Ile Lys Lys Leu Ile Gly Glu Arg
20 25 30

<210> 71
<211> 478
<212> PRT
<213> Artificial Sequence

<220>
<223> FAP(28H1)(VHCH1)-CMPtd-4-1BBL(71-254)

<400> 71

Glu Val Gln Leu Leu Glu Ser Gly Gly Gly Leu Val Gln Pro Gly Gly
1 5 10 15

Ser Leu Arg Leu Ser Cys Ala Ala Ser Gly Phe Thr Phe Ser Ser His
20 25 30

Ala Met Ser Trp Val Arg Gln Ala Pro Gly Lys Gly Leu Glu Trp Val
35 40 45

Ser Ala Ile Trp Ala Ser Gly Glu Gln Tyr Tyr Ala Asp Ser Val Lys
50 55 60

Gly Arg Phe Thr Ile Ser Arg Asp Asn Ser Lys Asn Thr Leu Tyr Leu
65 70 75 80

Gln Met Asn Ser Leu Arg Ala Glu Asp Thr Ala Val Tyr Tyr Cys Ala
85 90 95

Lys Gly Trp Leu Gly Asn Phe Asp Tyr Trp Gly Gln Gly Thr Leu Val
100 105 110

Thr Val Ser Ser Ala Ser Thr Lys Gly Pro Ser Val Phe Pro Leu Ala
115 120 125

Pro Ser Ser Lys Ser Thr Ser Gly Gly Thr Ala Ala Leu Gly Cys Leu
130 135 140

Val Lys Asp Tyr Phe Pro Glu Pro Val Thr Val Ser Trp Asn Ser Gly
145 150 155 160

Ala Leu Thr Ser Gly Val His Thr Phe Pro Ala Val Leu Gln Ser Ser
165 170 175

Gly Leu Tyr Ser Leu Ser Ser Val Val Thr Val Pro Ser Ser Ser Leu
180 185 190

Gly Thr Gln Thr Tyr Ile Cys Asn Val Asn His Lys Pro Ser Asn Thr
195 200 205

Lys Val Asp Lys Lys Val Glu Pro Lys Ser Cys Asp Gly Gly Gly Gly
210 215 220

Ser Gly Gly Gly Gly Ser Glu Glu Asp Pro Cys Ala Cys Glu Ser Leu
225 230 235 240

Val Lys Phe Gln Ala Lys Val Glu Gly Leu Leu Gln Ala Leu Thr Arg
245 250 255

Lys Leu Glu Ala Val Ser Lys Arg Leu Ala Ile Leu Glu Asn Thr Val
260 265 270

Val Ser Gly Gly Gly Gly Ser Gly Gly Gly Gly Ser Gly Gly Gly Gly
275 280 285

Ser Gly Gly Gly Gly Ser Arg Glu Gly Pro Glu Leu Ser Pro Asp Asp
290 295 300

Pro Ala Gly Leu Leu Asp Leu Arg Gln Gly Met Phe Ala Gln Leu Val
305 310 315 320

Ala Gln Asn Val Leu Leu Ile Asp Gly Pro Leu Ser Trp Tyr Ser Asp
325 330 335

Pro Gly Leu Ala Gly Val Ser Leu Thr Gly Gly Leu Ser Tyr Lys Glu
340 345 350

Asp Thr Lys Glu Leu Val Val Ala Lys Ala Gly Val Tyr Tyr Val Phe
355 360 365

Phe Gln Leu Glu Leu Arg Arg Val Val Ala Gly Glu Gly Ser Gly Ser
370 375 380

Val Ser Leu Ala Leu His Leu Gln Pro Leu Arg Ser Ala Ala Gly Ala
385 390 395 400

Ala Ala Leu Ala Leu Thr Val Asp Leu Pro Pro Ala Ser Ser Glu Ala
405 410 415

Arg Asn Ser Ala Phe Gly Phe Gln Gly Arg Leu Leu His Leu Ser Ala
420 425 430

Gly Gln Arg Leu Gly Val His Leu His Thr Glu Ala Arg Ala Arg His
435 440 445

Ala Trp Gln Leu Thr Gln Gly Ala Thr Val Leu Gly Leu Phe Arg Val
450 455 460

Thr Pro Glu Ile Pro Ala Gly Leu Pro Ser Pro Arg Ser Glu
465 470 475

<210> 72
<211> 427
<212> PRT

<213> Artificial Sequence

<220>
<223> FAP(VHCH1)-CMPtd-Ox40L(51-183)

<400> 72

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser His
            20                  25                  30

Ala Met Ser Trp Val Arg Gln Ala Pro Gly Lys Gly Leu Glu Trp Val
        35                  40                  45

Ser Ala Ile Trp Ala Ser Gly Glu Gln Tyr Tyr Ala Asp Ser Val Lys
    50                  55                  60

Gly Arg Phe Thr Ile Ser Arg Asp Asn Ser Lys Asn Thr Leu Tyr Leu
65                  70                  75                  80

Gln Met Asn Ser Leu Arg Ala Glu Asp Thr Ala Val Tyr Tyr Cys Ala
                85                  90                  95

Lys Gly Trp Leu Gly Asn Phe Asp Tyr Trp Gly Gln Gly Thr Leu Val
            100                 105                 110

Thr Val Ser Ser Ala Ser Thr Lys Gly Pro Ser Val Phe Pro Leu Ala
        115                 120                 125

Pro Ser Ser Lys Ser Thr Ser Gly Gly Thr Ala Ala Leu Gly Cys Leu
    130                 135                 140

Val Lys Asp Tyr Phe Pro Glu Pro Val Thr Val Ser Trp Asn Ser Gly
145                 150                 155                 160

Ala Leu Thr Ser Gly Val His Thr Phe Pro Ala Val Leu Gln Ser Ser
                165                 170                 175

Gly Leu Tyr Ser Leu Ser Ser Val Val Thr Val Pro Ser Ser Ser Leu
            180                 185                 190

Gly Thr Gln Thr Tyr Ile Cys Asn Val Asn His Lys Pro Ser Asn Thr
        195                 200                 205

Lys Val Asp Lys Lys Val Glu Pro Lys Ser Cys Asp Gly Gly Gly Gly
    210                 215                 220

Ser Gly Gly Gly Gly Ser Glu Glu Asp Pro Cys Ala Cys Glu Ser Leu
225 230 235 240

Val Lys Phe Gln Ala Lys Val Glu Gly Leu Leu Gln Ala Leu Thr Arg
245 250 255

Lys Leu Glu Ala Val Ser Lys Arg Leu Ala Ile Leu Glu Asn Thr Val
260 265 270

Val Ser Gly Gly Gly Gly Ser Gly Gly Gly Gly Ser Gly Gly Gly Gly
275 280 285

Ser Gly Gly Gly Gly Ser Gln Val Ser His Arg Tyr Pro Arg Ile Gln
290 295 300

Ser Ile Lys Val Gln Phe Thr Glu Tyr Lys Lys Glu Lys Gly Phe Ile
305 310 315 320

Leu Thr Ser Gln Lys Glu Asp Glu Ile Met Lys Val Gln Asp Asn Ser
325 330 335

Val Ile Ile Asn Cys Asp Gly Phe Tyr Leu Ile Ser Leu Lys Gly Tyr
340 345 350

Phe Ser Gln Glu Val Asp Ile Ser Leu His Tyr Gln Lys Asp Glu Glu
355 360 365

Pro Leu Phe Gln Leu Lys Lys Val Arg Ser Val Asn Ser Leu Met Val
370 375 380

Ala Ser Leu Thr Tyr Lys Asp Lys Val Tyr Leu Asn Val Thr Thr Asp
385 390 395 400

Asn Thr Ser Leu Asp Asp Phe His Val Asn Gly Gly Glu Leu Ile Leu
405 410 415

Ile His Gln Asn Pro Gly Glu Phe Cys Val Leu
420 425

<210> 73
<211> 1434
<212> DNA
<213> Artificial Sequence

<220>
<223> nucleotide sequence of FAP(28H1)(VHCH1)-CMPtd-4-1BBL(71-254)

<400> 73
gaagtgcagc tgctggaatc cggcggaggc ctggtgcagc ctggcggatc tctgagactg 60

```
tcctgcgccg cctccggctt caccttctcc tcccacgcca tgtcctgggt ccgacaggct      120
cctggcaaag gcctggaatg ggtgtccgcc atctgggcct ccggcgagca gtactacgcc      180
gactctgtga agggccggtt caccatctcc cgggacaact ccaagaacac cctgtacctg      240
cagatgaact ccctgcgggc cgaggacacc gccgtgtact actgtgccaa gggctggctg      300
ggcaacttcg actactgggg acagggcacc ctggtcaccg tgtccagcgc tagcacaaag      360
ggacctagcg tgttccccct ggcccccagc agcaagtcta catctggcgg aacagccgcc      420
ctgggctgcc tcgtgaagga ctactttccc gagcccgtga ccgtgtcctg gaactctggc      480
gctctgacaa gcggcgtgca cacctttcca gccgtgctgc agagcagcgg cctgtactct      540
ctgagcagcg tcgtgacagt gcccagcagc tctctgggca cccagaccta catctgcaac      600
gtgaaccaca agcccagcaa caccaaggtg gacaagaagg tggaacccaa gagctgcgac      660
ggcggagggg gatctggcgg cggaggatcc gaggaagatc cttgcgcctg cgagagcctc      720
gtgaagttcc aggccaaggt ggaaggactg ctgcaggccc tgacccggaa actggaagcc      780
gtgtccaagc ggctggccat cctggaaaac accgtggtgt ccggaggcgg aggatctggc      840
ggcggaggaa gtggcggagg cggatctggc ggcggaggat ctagagaggg acccgaactg      900
tcccctgacg atccagccgg gctgctggat ctgagacagg gaatgttcgc ccagctggtg      960
gctcagaatg tgctgctgat tgacggacct ctgagctggt actccgaccc agggctggca     1020
ggggtgtccc tgactggggg actgtcctac aaagaagata caaaagaact ggtggtggct     1080
aaagctgggg tgtactatgt gtttttttcag ctggaactga ggcgggtggt ggctggggag    1140
ggctcaggat ctgtgtccct ggctctgcat ctgcagccac tgcgctctgc tgctggcgca     1200
gctgcactgg ctctgactgt ggacctgcca ccagcctcta gcgaggccag aaacagcgcc     1260
ttcgggttcc aaggacgcct gctgcatctg agcgccggac agcgcctggg agtgcatctg     1320
catactgaag ccagagcccg gcatgcttgg cagctgactc agggggcaac tgtgctggga     1380
ctgtttcgcg tgacacctga gatccctgcc ggactgccaa gccctagatc agaa           1434
```

```
<210>  74
<211>  1281
<212>  DNA
<213>  Artificial Sequence

<220>
<223>  nucleotide sequence of FAP(28H1)(VHCH1)-CMPtd-Ox40L(51-183)

<400>  74
gaagtgcagc tgctggaatc cggcggaggc ctggtgcagc ctggcggatc tctgagactg       60
tcctgcgccg cctccggctt caccttctcc tcccacgcca tgtcctgggt ccgacaggct      120
cctggcaaag gcctggaatg ggtgtccgcc atctgggcct ccggcgagca gtactacgcc      180
gactctgtga agggccggtt caccatctcc cgggacaact ccaagaacac cctgtacctg      240
```

cagatgaact ccctgcgggc cgaggacacc gccgtgtact actgtgccaa gggctggctg 300

ggcaacttcg actactgggg acagggcacc ctggtcaccg tgtccagcgc tagcacaaag 360

ggacctagcg tgttcccccct ggcccccagc agcaagtcta catctggcgg aacagccgcc 420

ctgggctgcc tcgtgaagga ctactttccc gagcccgtga ccgtgtcctg gaactctggc 480

gctctgacaa gcggcgtgca cacctttcca gccgtgctgc agagcagcgg cctgtactct 540

ctgagcagcg tcgtgacagt gcccagcagc tctctgggca cccagaccta catctgcaac 600

gtgaaccaca agcccagcaa caccaaggtg gacaagaagg tggaacccaa gagctgcgac 660

ggcggagggg gatctggcgg cggaggatcc gaggaagatc cttgcgcctg cgagagcctc 720

gtgaagttcc aggccaaggt ggaaggactg ctgcaggccc tgacccggaa actggaagcc 780

gtgtccaagc ggctggccat cctggaaaac accgtggtgt ccggaggcgg aggatctggc 840

ggcggaggaa gtggcggagg gggatctggg ggaggcggat ctcaggtgtc ccacagatac 900

ccccggatcc agagcatcaa ggtgcagttc accgagtaca agaaagagaa gggcttcatc 960

ctgaccagcc agaaagagga cgagatcatg aaggtgcagg acaacagcgt gatcatcaac 1020

tgcgacggct tctacctgat cagcctgaag ggctacttca gccaggaagt ggacatcagc 1080

ctgcactacc agaaggacga ggaacccctg ttccagctga agaaagtgcg gagcgtgaac 1140

agcctgatgg tggccagcct gacctacaag gacaaggtgt acctgaacgt gaccaccgac 1200

aacaccagcc tggacgactt ccacgtgaac ggcggcgagc tgatcctgat tcaccagaac 1260

cccggcgagt tctgcgtgct c 1281

<210> 75
<211> 645
<212> DNA
<213> Artificial Sequence

<220>
<223> nucleotide sequence of Antianti-FAP(28H1) light chain

<400> 75
gagatcgtgc tgacccagtc ccccggcacc ctgtctctga gccctggcga gagagccacc 60

ctgtcctgca gagcctccca gtccgtgtcc cggtcctacc tcgcctggta tcagcagaag 120

cccggccagg cccctcggct gctgatcatc ggcgcctcta ccagagccac cggcatccct 180

gaccggttct ccggctctgg ctccggcacc gacttcaccc tgaccatctc ccggctggaa 240

cccgaggact tcgccgtgta ctactgccag cagggccagg tcatccctcc cacctttggc 300

cagggcacca aggtggaaat caagcgtacg gtggctgcac catctgtctt catcttcccg 360

ccatctgatg agcagttgaa atctggaact gcctctgttg tgtgcctgct gaataacttc 420

tatcccagag aggccaaagt acagtggaag gtggataacg ccctccaatc gggtaactcc 480

caggagagtg tcacagagca ggacagcaag gacagcacct acagcctcag cagcaccctg 540

acgctgagca aagcagacta cgagaaacac aaagtctacg cctgcgaagt cacccatcag 600

ggcctgagct cgcccgtcac aaagagcttc aacaggggag agtgt 645

<210> 76
<211> 215
<212> PRT
<213> Artificial Sequence

<220>
<223> Anti-FAP(28H1) light chain

<400> 76

```
Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Ser Val Ser Arg Ser
            20                  25                  30

Tyr Leu Ala Trp Tyr Gln Gln Lys Pro Gly Gln Ala Pro Arg Leu Leu
        35                  40                  45

Ile Ile Gly Ala Ser Thr Arg Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Gly Gln Val Ile Pro
                85                  90                  95

Pro Thr Phe Gly Gln Gly Thr Lys Val Glu Ile Lys Arg Thr Val Ala
            100                 105                 110

Ala Pro Ser Val Phe Ile Phe Pro Pro Ser Asp Glu Gln Leu Lys Ser
        115                 120                 125

Gly Thr Ala Ser Val Val Cys Leu Leu Asn Asn Phe Tyr Pro Arg Glu
    130                 135                 140

Ala Lys Val Gln Trp Lys Val Asp Asn Ala Leu Gln Ser Gly Asn Ser
145                 150                 155                 160

Gln Glu Ser Val Thr Glu Gln Asp Ser Lys Asp Ser Thr Tyr Ser Leu
                165                 170                 175

Ser Ser Thr Leu Thr Leu Ser Lys Ala Asp Tyr Glu Lys His Lys Val
            180                 185                 190
```

Tyr Ala Cys Glu Val Thr His Gln Gly Leu Ser Ser Pro Val Thr Lys
195 200 205

Ser Phe Asn Arg Gly Glu Cys
210 215

<210> 77
<211> 1431
<212> DNA
<213> Artificial Sequence

<220>
<223> nucleotide sequence of DP47 (VHCH1)-CMPtd-4-1BBL(71-254)

<400> 77
gaggtgcaat tgttggagtc tgggggaggc ttggtacagc ctggggggtc cctgagactc 60
tcctgtgcag cctccggatt cacctttagc agttatgcca tgagctgggt ccgccaggct 120
ccagggaagg ggctggagtg ggtctcagct attagtggta gtggtggtag cacatactac 180
gcagactccg tgaagggccg gttcaccatc tccagagaca attccaagaa cacgctgtat 240
ctgcagatga acagcctgag agccgaggac acggccgtat attactgtgc gaaaggcagc 300
ggatttgact actggggcca aggaaccctg gtcaccgtct cgagtgctag cacaaaggga 360
cctagcgtgt tccccctggc ccccagcagc aagtctacat ctggcggaac agccgccctg 420
ggctgcctcg tgaaggacta ctttcccgag cccgtgaccg tgtcctggaa ctctggcgct 480
ctgacaagcg gcgtgcacac ctttccagcc gtgctgcaga gcagcggcct gtactctctg 540
agcagcgtcg tgacagtgcc cagcagctct ctgggcaccc agacctacat ctgcaacgtg 600
aaccacaagc ccagcaacac caaggtggac aagaaggtgg aacccaagag ctgcgacggc 660
ggagggggat ctggcggcgg aggatccgag gaagatcctt gcgcctgcga gagcctcgtg 720
aagttccagg ccaaggtgga aggactgctg caggccctga cccggaaact ggaagccgtg 780
tccaagcggc tggccatcct ggaaaacacc gtggtgtccg gaggcggagg atctggcggc 840
ggaggaagtg gcggaggcgg atctggcggc ggaggatcta gagagggacc cgaactgtcc 900
cctgacgatc cagccgggct gctggatctg agacagggaa tgttcgccca gctggtggct 960
cagaatgtgc tgctgattga cggacctctg agctggtact ccgacccagg gctggcaggg 1020
gtgtccctga ctggggggact gtcctacaaa gaagatacaa aagaactggt ggtggctaaa 1080
gctggggtgt actatgtgtt ttttcagctg gaactgaggc gggtggtggc tggggagggc 1140
tcaggatctg tgtccctggc tctgcatctg cagccactgc gctctgctgc tggcgcagct 1200
gcactggctc tgactgtgga cctgccacca gcctctagcg aggccagaaa cagcgccttc 1260
gggttccaag gacgcctgct gcatctgagc gccggacagc gcctgggagt gcatctgcat 1320

actgaagcca gagcccggca tgcttggcag ctgactcagg gggcaactgt gctgggactg 1380

tttcgcgtga cacctgagat ccctgccgga ctgccaagcc ctagatcaga a 1431

<210> 78
<211> 645
<212> DNA
<213> Artificial Sequence

<220>
<223> nucleotide sequence of DP47 light chain

<400> 78
gaaatcgtgt taacgcagtc tccaggcacc ctgtctttgt ctccagggga aagagccacc 60

ctctcttgca gggccagtca gagtgttagc agcagctact tagcctggta ccagcagaaa 120

cctggccagg ctcccaggct cctcatctat ggagcatcca gcagggccac tggcatccca 180

gacaggttca gtggcagtgg atccgggaca gacttcactc tcaccatcag cagactggag 240

cctgaagatt ttgcagtgta ttactgtcag cagtatggta gctcaccgct gacgttcggc 300

caggggacca aagtggaaat caaacgtacg gtggctgcac catctgtctt catcttcccg 360

ccatctgatg agcagttgaa atctggaact gcctctgttg tgtgcctgct gaataacttc 420

tatcccagag aggccaaagt acagtggaag gtggataacg ccctccaatc gggtaactcc 480

caggagagtg tcacagagca ggacagcaag gacagcacct acagcctcag cagcaccctg 540

acgctgagca aagcagacta cgagaaacac aaagtctacg cctgcgaagt cacccatcag 600

ggcctgagct cgcccgtcac aaagagcttc aacaggggag agtgt 645

<210> 79
<211> 477
<212> PRT
<213> Artificial Sequence

<220>
<223> DP47(VHCH1)-CMPtd-4-1BBL(71-254)

<400> 79

Glu Val Gln Leu Leu Glu Ser Gly Gly Gly Leu Val Gln Pro Gly Gly
1 5 10 15

Ser Leu Arg Leu Ser Cys Ala Ala Ser Gly Phe Thr Phe Ser Ser Tyr
20 25 30

Ala Met Ser Trp Val Arg Gln Ala Pro Gly Lys Gly Leu Glu Trp Val
35 40 45

Ser Ala Ile Ser Gly Ser Gly Gly Ser Thr Tyr Tyr Ala Asp Ser Val
50 55 60

```
Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gly Ser Gly Phe Asp Tyr Trp Gly Gln Gly Thr Leu Val Thr
            100                 105                 110

Val Ser Ser Ala Ser Thr Lys Gly Pro Ser Val Phe Pro Leu Ala Pro
        115                 120                 125

Ser Ser Lys Ser Thr Ser Gly Gly Thr Ala Ala Leu Gly Cys Leu Val
    130                 135                 140

Lys Asp Tyr Phe Pro Glu Pro Val Thr Val Ser Trp Asn Ser Gly Ala
145                 150                 155                 160

Leu Thr Ser Gly Val His Thr Phe Pro Ala Val Leu Gln Ser Ser Gly
                165                 170                 175

Leu Tyr Ser Leu Ser Ser Val Val Thr Val Pro Ser Ser Ser Leu Gly
            180                 185                 190

Thr Gln Thr Tyr Ile Cys Asn Val Asn His Lys Pro Ser Asn Thr Lys
        195                 200                 205

Val Asp Lys Lys Val Glu Pro Lys Ser Cys Asp Gly Gly Gly Gly Ser
    210                 215                 220

Gly Gly Gly Gly Ser Glu Glu Asp Pro Cys Ala Cys Glu Ser Leu Val
225                 230                 235                 240

Lys Phe Gln Ala Lys Val Glu Gly Leu Leu Gln Ala Leu Thr Arg Lys
                245                 250                 255

Leu Glu Ala Val Ser Lys Arg Leu Ala Ile Leu Glu Asn Thr Val Val
            260                 265                 270

Ser Gly Gly Gly Gly Ser Gly Gly Gly Gly Ser Gly Gly Gly Gly Ser
        275                 280                 285

Gly Gly Gly Gly Ser Arg Glu Gly Pro Glu Leu Ser Pro Asp Asp Pro
    290                 295                 300

Ala Gly Leu Leu Asp Leu Arg Gln Gly Met Phe Ala Gln Leu Val Ala
305                 310                 315                 320
```

Gln Asn Val Leu Leu Ile Asp Gly Pro Leu Ser Trp Tyr Ser Asp Pro
325 330 335

Gly Leu Ala Gly Val Ser Leu Thr Gly Gly Leu Ser Tyr Lys Glu Asp
340 345 350

Thr Lys Glu Leu Val Val Ala Lys Ala Gly Val Tyr Tyr Val Phe Phe
355 360 365

Gln Leu Glu Leu Arg Arg Val Val Ala Gly Glu Gly Ser Gly Ser Val
370 375 380

Ser Leu Ala Leu His Leu Gln Pro Leu Arg Ser Ala Ala Gly Ala Ala
385 390 395 400

Ala Leu Ala Leu Thr Val Asp Leu Pro Pro Ala Ser Ser Glu Ala Arg
405 410 415

Asn Ser Ala Phe Gly Phe Gln Gly Arg Leu Leu His Leu Ser Ala Gly
420 425 430

Gln Arg Leu Gly Val His Leu His Thr Glu Ala Arg Ala Arg His Ala
435 440 445

Trp Gln Leu Thr Gln Gly Ala Thr Val Leu Gly Leu Phe Arg Val Thr
450 455 460

Pro Glu Ile Pro Ala Gly Leu Pro Ser Pro Arg Ser Glu
465 470 475

<210> 80
<211> 215
<212> PRT
<213> Artificial Sequence

<220>
<223> DP47 light chain

<400> 80

Glu Ile Val Leu Thr Gln Ser Pro Gly Thr Leu Ser Leu Ser Pro Gly
1 5 10 15

Glu Arg Ala Thr Leu Ser Cys Arg Ala Ser Gln Ser Val Ser Ser Ser
20 25 30

Tyr Leu Ala Trp Tyr Gln Gln Lys Pro Gly Gln Ala Pro Arg Leu Leu
35 40 45

```
Ile Tyr Gly Ala Ser Ser Arg Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Tyr Gly Ser Ser Pro
                85                  90                  95

Leu Thr Phe Gly Gln Gly Thr Lys Val Glu Ile Lys Arg Thr Val Ala
            100                 105                 110

Ala Pro Ser Val Phe Ile Phe Pro Pro Ser Asp Glu Gln Leu Lys Ser
        115                 120                 125

Gly Thr Ala Ser Val Val Cys Leu Leu Asn Asn Phe Tyr Pro Arg Glu
    130                 135                 140

Ala Lys Val Gln Trp Lys Val Asp Asn Ala Leu Gln Ser Gly Asn Ser
145                 150                 155                 160

Gln Glu Ser Val Thr Glu Gln Asp Ser Lys Asp Ser Thr Tyr Ser Leu
                165                 170                 175

Ser Ser Thr Leu Thr Leu Ser Lys Ala Asp Tyr Glu Lys His Lys Val
            180                 185                 190

Tyr Ala Cys Glu Val Thr His Gln Gly Leu Ser Ser Pro Val Thr Lys
        195                 200                 205

Ser Phe Asn Arg Gly Glu Cys
    210                 215
```

<210> 81
<211> 1335
<212> DNA
<213> Artificial Sequence

<220>
<223> nucleotide sequence of DP47 heavy chain (hu IgG1 PGLALA)

<400> 81

```
gaggtgcaat tgttggagtc tgggggaggc ttggtacagc ctggggggtc cctgagactc      60

tcctgtgcag cctccggatt cacctttagc agttatgcca tgagctgggt ccgccaggct     120

ccagggaagg ggctggagtg ggtctcagct attagtggta gtggtggtag cacatactac     180

gcagactccg tgaagggccg gttcaccatc tccagagaca attccaagaa cacgctgtat     240

ctgcagatga acagcctgag agccgaggac acggccgtat attactgtgc gaaaggcagc     300
```

ggatttgact actggggcca aggaaccctg gtcaccgtct cgagtgctag caccaagggc 360

ccatcggtct tccccctggc accctcctcc aagagcacct ctgggggcac agcggccctg 420

ggctgcctgg tcaaggacta cttccccgaa ccggtgacgg tgtcgtggaa ctcaggcgcc 480

ctgaccagcg gcgtgcacac cttcccggct gtcctacagt cctcaggact ctactccctc 540

agcagcgtgg tgaccgtgcc ctccagcagc ttgggcaccc agacctacat ctgcaacgtg 600

aatcacaagc ccagcaacac caaggtggac aagaaagttg agcccaaatc ttgtgacaaa 660

actcacacat gcccaccgtg cccagcacct gaagctgcag ggggaccgtc agtcttcctc 720

ttccccccaa aacccaagga caccctcatg atctcccgga cccctgaggt cacatgcgtg 780

gtggtggacg tgagccacga agaccctgag gtcaagttca actggtacgt ggacggcgtg 840

gaggtgcata atgccaagac aaagccgcgg gaggagcagt acaacagcac gtaccgtgtg 900

gtcagcgtcc tcaccgtcct gcaccaggac tggctgaatg gcaaggagta caagtgcaag 960

gtctccaaca aagccctcgg cgcccccatc gagaaaacca tctccaaagc caaagggcag 1020

ccccgagaac cacaggtgta caccctgccc ccatcccggg atgagctgac caagaaccag 1080

gtcagcctga cctgcctggt caaaggcttc tatcccagcg acatcgccgt ggagtgggag 1140

agcaatgggc agccggagaa caactacaag accacgcctc ccgtgctgga ctccgacggc 1200

tccttcttcc tctacagcaa gctcaccgtg gacaagagca ggtggcagca ggggaacgtc 1260

ttctcatgct ccgtgatgca tgaggctctg cacaaccact acacgcagaa gagcctctcc 1320

ctgtctccgg gtaaa 1335

<210> 82
<211> 445
<212> PRT
<213> Artificial Sequence

<220>
<223> DP47 heavy chain (hu IgG1 PGLALA)

<400> 82

```
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60
```

```
Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gly Ser Gly Phe Asp Tyr Trp Gly Gln Gly Thr Leu Val Thr
            100                 105                 110

Val Ser Ser Ala Ser Thr Lys Gly Pro Ser Val Phe Pro Leu Ala Pro
        115                 120                 125

Ser Ser Lys Ser Thr Ser Gly Gly Thr Ala Ala Leu Gly Cys Leu Val
    130                 135                 140

Lys Asp Tyr Phe Pro Glu Pro Val Thr Val Ser Trp Asn Ser Gly Ala
145                 150                 155                 160

Leu Thr Ser Gly Val His Thr Phe Pro Ala Val Leu Gln Ser Ser Gly
                165                 170                 175

Leu Tyr Ser Leu Ser Ser Val Val Thr Val Pro Ser Ser Ser Leu Gly
            180                 185                 190

Thr Gln Thr Tyr Ile Cys Asn Val Asn His Lys Pro Ser Asn Thr Lys
        195                 200                 205

Val Asp Lys Lys Val Glu Pro Lys Ser Cys Asp Lys Thr His Thr Cys
    210                 215                 220

Pro Pro Cys Pro Ala Pro Glu Ala Ala Gly Gly Pro Ser Val Phe Leu
225                 230                 235                 240

Phe Pro Pro Lys Pro Lys Asp Thr Leu Met Ile Ser Arg Thr Pro Glu
                245                 250                 255

Val Thr Cys Val Val Val Asp Val Ser His Glu Asp Pro Glu Val Lys
            260                 265                 270

Phe Asn Trp Tyr Val Asp Gly Val Glu Val His Asn Ala Lys Thr Lys
        275                 280                 285

Pro Arg Glu Glu Gln Tyr Asn Ser Thr Tyr Arg Val Val Ser Val Leu
    290                 295                 300

Thr Val Leu His Gln Asp Trp Leu Asn Gly Lys Glu Tyr Lys Cys Lys
305                 310                 315                 320
```

Val Ser Asn Lys Ala Leu Gly Ala Pro Ile Glu Lys Thr Ile Ser Lys
325 330 335

Ala Lys Gly Gln Pro Arg Glu Pro Gln Val Tyr Thr Leu Pro Pro Ser
340 345 350

Arg Asp Glu Leu Thr Lys Asn Gln Val Ser Leu Thr Cys Leu Val Lys
355 360 365

Gly Phe Tyr Pro Ser Asp Ile Ala Val Glu Trp Glu Ser Asn Gly Gln
370 375 380

Pro Glu Asn Asn Tyr Lys Thr Thr Pro Pro Val Leu Asp Ser Asp Gly
385 390 395 400

Ser Phe Phe Leu Tyr Ser Lys Leu Thr Val Asp Lys Ser Arg Trp Gln
405 410 415

Gln Gly Asn Val Phe Ser Cys Ser Val Met His Glu Ala Leu His Asn
420 425 430

His Tyr Thr Gln Lys Ser Leu Ser Leu Ser Pro Gly Lys
435 440 445

<210> 83
<211> 163
<212> PRT
<213> Homo sapiens

<400> 83

Leu Gln Asp Pro Cys Ser Asn Cys Pro Ala Gly Thr Phe Cys Asp Asn
1 5 10 15

Asn Arg Asn Gln Ile Cys Ser Pro Cys Pro Pro Asn Ser Phe Ser Ser
20 25 30

Ala Gly Gly Gln Arg Thr Cys Asp Ile Cys Arg Gln Cys Lys Gly Val
35 40 45

Phe Arg Thr Arg Lys Glu Cys Ser Ser Thr Ser Asn Ala Glu Cys Asp
50 55 60

Cys Thr Pro Gly Phe His Cys Leu Gly Ala Gly Cys Ser Met Cys Glu
65 70 75 80

Gln Asp Cys Lys Gln Gly Gln Glu Leu Thr Lys Lys Gly Cys Lys Asp
85 90 95

Cys Cys Phe Gly Thr Phe Asn Asp Gln Lys Arg Gly Ile Cys Arg Pro
            100                 105                 110

Trp Thr Asn Cys Ser Leu Asp Gly Lys Ser Val Leu Val Asn Gly Thr
        115                 120                 125

Lys Glu Arg Asp Val Val Cys Gly Pro Ser Pro Ala Asp Leu Ser Pro
    130                 135                 140

Gly Ala Ser Ser Val Thr Pro Pro Ala Pro Ala Arg Glu Pro Gly His
145                 150                 155                 160

Ser Pro Gln

<210> 84
<211> 163
<212> PRT
<213> cynomolgus

<400> 84

Leu Gln Asp Leu Cys Ser Asn Cys Pro Ala Gly Thr Phe Cys Asp Asn
1               5                   10                  15

Asn Arg Ser Gln Ile Cys Ser Pro Cys Pro Pro Asn Ser Phe Ser Ser
            20                  25                  30

Ala Gly Gly Gln Arg Thr Cys Asp Ile Cys Arg Gln Cys Lys Gly Val
        35                  40                  45

Phe Lys Thr Arg Lys Glu Cys Ser Ser Thr Ser Asn Ala Glu Cys Asp
    50                  55                  60

Cys Ile Ser Gly Tyr His Cys Leu Gly Ala Glu Cys Ser Met Cys Glu
65                  70                  75                  80

Gln Asp Cys Lys Gln Gly Gln Glu Leu Thr Lys Lys Gly Cys Lys Asp
                85                  90                  95

Cys Cys Phe Gly Thr Phe Asn Asp Gln Lys Arg Gly Ile Cys Arg Pro
            100                 105                 110

Trp Thr Asn Cys Ser Leu Asp Gly Lys Ser Val Leu Val Asn Gly Thr
        115                 120                 125

Lys Glu Arg Asp Val Val Cys Gly Pro Ser Pro Ala Asp Leu Ser Pro
    130                 135                 140

Gly Ala Ser Ser Ala Thr Pro Pro Ala Pro Ala Arg Glu Pro Gly His
145                 150                 155                 160

Ser Pro Gln

<210> 85
<211> 164
<212> PRT
<213> Mus musculus

<400> 85

Val Gln Asn Ser Cys Asp Asn Cys Gln Pro Gly Thr Phe Cys Arg Lys
1               5                   10                  15

Tyr Asn Pro Val Cys Lys Ser Cys Pro Pro Ser Thr Phe Ser Ser Ile
            20                  25                  30

Gly Gly Gln Pro Asn Cys Asn Ile Cys Arg Val Cys Ala Gly Tyr Phe
        35                  40                  45

Arg Phe Lys Lys Phe Cys Ser Ser Thr His Asn Ala Glu Cys Glu Cys
    50                  55                  60

Ile Glu Gly Phe His Cys Leu Gly Pro Gln Cys Thr Arg Cys Glu Lys
65                  70                  75                  80

Asp Cys Arg Pro Gly Gln Glu Leu Thr Lys Gln Gly Cys Lys Thr Cys
                85                  90                  95

Ser Leu Gly Thr Phe Asn Asp Gln Asn Gly Thr Gly Val Cys Arg Pro
            100                 105                 110

Trp Thr Asn Cys Ser Leu Asp Gly Arg Ser Val Leu Lys Thr Gly Thr
        115                 120                 125

Thr Glu Lys Asp Val Val Cys Gly Pro Pro Val Val Ser Phe Ser Pro
    130                 135                 140

Ser Thr Thr Ile Ser Val Thr Pro Glu Gly Gly Pro Gly Gly His Ser
145                 150                 155                 160

Leu Gln Val Leu

<210> 86
<211> 681
<212> DNA

<213>  Artificial sequence

<220>
<223>  Fc hole chain

<400>  86
gacaaaactc acacatgccc accgtgccca gcacctgaac tcctgggggg accgtcagtc        60

ttcctcttcc ccccaaaacc caaggacacc ctcatgatct cccggacccc tgaggtcaca       120

tgcgtggtgg tggacgtgag ccacgaagac cctgaggtca agttcaactg gtacgtggac       180

ggcgtggagg tgcataatgc caagacaaag ccgcgggagg agcagtacaa cagcacgtac       240

cgtgtggtca gcgtcctcac cgtcctgcac caggactggc tgaatggcaa ggagtacaag       300

tgcaaggtct ccaacaaagc cctcccagcc cccatcgaga aaaccatctc caaagccaaa       360

gggcagcccc gagaaccaca ggtgtgcacc ctgcccccat cccgggatga gctgaccaag       420

aaccaggtca gcctctcgtg cgcagtcaaa ggcttctatc ccagcgacat cgccgtggag       480

tgggagagca atgggcagcc ggagaacaac tacaagacca cgcctcccgt gctggactcc       540

gacggctcct tcttcctcgt gagcaagctc accgtggaca agagcaggtg gcagcagggg       600

aacgtcttct catgctccgt gatgcatgag gctctgcaca accactacac gcagaagagc       660

ctctccctgt ctccgggtaa a                                                 681

<210>  87
<211>  1266
<212>  DNA
<213>  Artificial sequence

<220>
<223>  human 4-1BB Fc knob chain

<400>  87
ctgcaggacc cctgcagcaa ctgccctgcc ggcaccttct gcgacaacaa ccggaaccag        60

atctgcagcc cctgcccccc caacagcttc agctctgccg gcggacagcg gacctgcgac       120

atctgcagac agtgcaaggg cgtgttcaga acccggaaag agtgcagcag caccagcaac       180

gccgagtgcg actgcacccc cggcttccat tgtctgggag ccggctgcag catgtgcgag       240

caggactgca agcagggcca ggaactgacc aagaagggct gcaaggactg ctgcttcggc       300

accttcaacg accagaagcg gggcatctgc cggccctgga ccaactgtag cctggacggc       360

aagagcgtgc tggtcaacgg caccaaagaa cgggacgtcg tgtgcggccc cagccctgct       420

gatctgtctc ctggggccag cagcgtgacc cctcctgccc ctgccagaga gcctggccac       480

tctcctcagg tcgacgaaca gttatatttt cagggcggct cacccaaatc tgcagacaaa       540

actcacacat gcccaccgtg cccagcacct gaactcctgg ggggaccgtc agtcttcctc       600

ttccccccaa aacccaagga caccctcatg atctcccgga cccctgaggt cacatgcgtg       660

gtggtggacg tgagccacga agaccctgag gtcaagttca actggtacgt ggacggcgtg       720

```
gaggtgcata atgccaagac aaagccgcgg gaggagcagt acaacagcac gtaccgtgtg      780
gtcagcgtcc tcaccgtcct gcaccaggac tggctgaatg gcaaggagta caagtgcaag      840
gtctccaaca aagccctccc agcccccatc gagaaaacca tctccaaagc caaagggcag      900
ccccgagaac cacaggtgta caccctgccc ccatgccggg atgagctgac caagaaccag      960
gtcagcctgt ggtgcctggt caaaggcttc tatcccagcg acatcgccgt ggagtgggag     1020
agcaatgggc agccggagaa caactacaag accacgcctc ccgtgctgga ctccgacggc     1080
tccttcttcc tctacagcaa gctcaccgtg gacaagagca ggtggcagca ggggaacgtc     1140
ttctcatgct ccgtgatgca tgaggctctg cacaaccact acacgcagaa gagcctctcc     1200
ctgtctccgg gtaaatccgg aggcctgaac gacatcttcg aggcccagaa gattgaatgg     1260
cacgag                                                                1266
```

<210> 88
<211> 1266
<212> DNA
<213> Artificial sequence

<220>
<223> cynomolgus 4-1BB Fc knob chain

<400> 88

```
ttgcaggatc tgtgtagtaa ctgcccagct ggtacattct gtgataataa caggagtcag       60
atttgcagtc cctgtcctcc aaatagtttc tccagcgcag gtggacaaag gacctgtgac      120
atatgcaggc agtgtaaagg tgttttcaag accaggaagg agtgttcctc caccagcaat      180
gcagagtgtg actgcatttc agggtatcac tgcctggggg cagagtgcag catgtgtgaa      240
caggattgta aacaaggtca agaattgaca aaaaaaggtt gtaaagactg ttgctttggg      300
acatttaatg accagaaacg tggcatctgt cgcccctgga caaactgttc tttggatgga      360
aagtctgtgc ttgtgaatgg gacgaaggag agggacgtgg tctgcggacc atctccagcc      420
gacctctctc caggagcatc ctctgcgacc ccgcctgccc ctgcgagaga gccaggacac      480
tctccgcagg tcgacgaaca gttatatttt cagggcggct cacccaaatc tgcagacaaa      540
actcacacat gcccaccgtg cccagcacct gaactcctgg ggggaccgtc agtcttcctc      600
ttccccccaa aacccaagga caccctcatg atctcccgga cccctgaggt cacatgcgtg      660
gtggtggacg tgagccacga agaccctgag gtcaagttca actggtacgt ggacggcgtg      720
gaggtgcata atgccaagac aaagccgcgg gaggagcagt acaacagcac gtaccgtgtg      780
gtcagcgtcc tcaccgtcct gcaccaggac tggctgaatg gcaaggagta caagtgcaag      840
gtctccaaca aagccctccc agcccccatc gagaaaacca tctccaaagc caaagggcag      900
ccccgagaac cacaggtgta caccctgccc ccatgccggg atgagctgac caagaaccag      960
```

gtcagcctgt ggtgcctggt caaaggcttc tatcccagcg acatcgccgt ggagtgggag 1020

agcaatgggc agccggagaa caactacaag accacgcctc ccgtgctgga ctccgacggc 1080

tccttcttcc tctacagcaa gctcaccgtg gacaagagca ggtggcagca ggggaacgtc 1140

ttctcatgct ccgtgatgca tgaggctctg cacaaccact acacgcagaa gagcctctcc 1200

ctgtctccgg gtaaatccgg aggcctgaac gacatcttcg aggcccagaa gattgaatgg 1260

cacgag 1266

<210> 89
<211> 1269
<212> DNA
<213> Artificial sequence

<220>
<223> Murine 4-1BB Fc knob chain

<400> 89
gtgcagaaca gctgcgacaa ctgccagccc ggcaccttct gccggaagta caaccccgtg 60

tgcaagagct gcccccccag caccttcagc agcatcggcg gccagcccaa ctgcaacatc 120

tgcagagtgt gcgccggcta cttccggttc aagaagttct gcagcagcac ccacaacgcc 180

gagtgcgagt gcatcgaggg cttccactgc ctgggccccc agtgcaccag atgcgagaag 240

gactgcagac ccggccagga actgaccaag cagggctgta agacctgcag cctgggcacc 300

ttcaacgacc agaacgggac cggcgtgtgc cggccttgga ccaattgcag cctggacggg 360

agaagcgtgc tgaaaaccgg caccaccgag aaggacgtcg tgtgcggccc tcccgtggtg 420

tccttcagcc ctagcaccac catcagcgtg acccctgaag gcggccctgg cggacactct 480

ctgcaggtcc tggtcgacga acagttatat tttcagggcg gctcacccaa atctgcagac 540

aaaactcaca catgcccacc gtgcccagca cctgaactcc tggggggacc gtcagtcttc 600

ctcttccccc caaaacccaa ggacaccctc atgatctccc ggacccctga ggtcacatgc 660

gtggtggtgg acgtgagcca cgaagaccct gaggtcaagt tcaactggta cgtggacggc 720

gtggaggtgc ataatgccaa gacaaagccg cgggaggagc agtacaacag cacgtaccgt 780

gtggtcagcg tcctcaccgt cctgcaccag gactggctga atggcaagga gtacaagtgc 840

aaggtctcca acaaagccct cccagccccc atcgagaaaa ccatctccaa agccaaaggg 900

cagccccgag aaccacaggt gtacaccctg cccccatgcc gggatgagct gaccaagaac 960

caggtcagcc tgtggtgcct ggtcaaaggc ttctatccca gcgacatcgc cgtggagtgg 1020

gagagcaatg ggcagccgga gaacaactac aagaccacgc ctcccgtgct ggactccgac 1080

ggctccttct tcctctacag caagctcacc gtggacaaga gcaggtggca gcaggggaac 1140

gtcttctcat gctccgtgat gcatgaggct ctgcacaacc actacacgca gaagagcctc 1200

tccctgtctc cgggtaaatc cggaggcctg aacgacatct tcgaggccca gaagattgaa 1260

tggcacgag 1269

<210> 90
<211> 227
<212> PRT
<213> Artificial sequence

<220>
<223> Fc hole chain

<400> 90

Asp Lys Thr His Thr Cys Pro Pro Cys Pro Ala Pro Glu Leu Leu Gly
1               5                   10                  15

Gly Pro Ser Val Phe Leu Phe Pro Pro Lys Pro Lys Asp Thr Leu Met
            20                  25                  30

Ile Ser Arg Thr Pro Glu Val Thr Cys Val Val Val Asp Val Ser His
        35                  40                  45

Glu Asp Pro Glu Val Lys Phe Asn Trp Tyr Val Asp Gly Val Glu Val
    50                  55                  60

His Asn Ala Lys Thr Lys Pro Arg Glu Glu Gln Tyr Asn Ser Thr Tyr
65                  70                  75                  80

Arg Val Val Ser Val Leu Thr Val Leu His Gln Asp Trp Leu Asn Gly
                85                  90                  95

Lys Glu Tyr Lys Cys Lys Val Ser Asn Lys Ala Leu Pro Ala Pro Ile
            100                 105                 110

Glu Lys Thr Ile Ser Lys Ala Lys Gly Gln Pro Arg Glu Pro Gln Val
        115                 120                 125

Cys Thr Leu Pro Pro Ser Arg Asp Glu Leu Thr Lys Asn Gln Val Ser
    130                 135                 140

Leu Ser Cys Ala Val Lys Gly Phe Tyr Pro Ser Asp Ile Ala Val Glu
145                 150                 155                 160

Trp Glu Ser Asn Gly Gln Pro Glu Asn Asn Tyr Lys Thr Thr Pro Pro
                165                 170                 175

Val Leu Asp Ser Asp Gly Ser Phe Phe Leu Val Ser Lys Leu Thr Val
            180                 185                 190

Asp Lys Ser Arg Trp Gln Gln Gly Asn Val Phe Ser Cys Ser Val Met

195 200 205

His Glu Ala Leu His Asn His Tyr Thr Gln Lys Ser Leu Ser Leu Ser
210 215 220

Pro Gly Lys
225

<210> 91
<211> 422
<212> PRT
<213> Artificial sequence

<220>
<223> Human 4-1BB Fc knob chain

<400> 91

Leu Gln Asp Pro Cys Ser Asn Cys Pro Ala Gly Thr Phe Cys Asp Asn
1 5 10 15

Asn Arg Asn Gln Ile Cys Ser Pro Cys Pro Pro Asn Ser Phe Ser Ser
20 25 30

Ala Gly Gly Gln Arg Thr Cys Asp Ile Cys Arg Gln Cys Lys Gly Val
35 40 45

Phe Arg Thr Arg Lys Glu Cys Ser Ser Thr Ser Asn Ala Glu Cys Asp
50 55 60

Cys Thr Pro Gly Phe His Cys Leu Gly Ala Gly Cys Ser Met Cys Glu
65 70 75 80

Gln Asp Cys Lys Gln Gly Gln Glu Leu Thr Lys Lys Gly Cys Lys Asp
85 90 95

Cys Cys Phe Gly Thr Phe Asn Asp Gln Lys Arg Gly Ile Cys Arg Pro
100 105 110

Trp Thr Asn Cys Ser Leu Asp Gly Lys Ser Val Leu Val Asn Gly Thr
115 120 125

Lys Glu Arg Asp Val Val Cys Gly Pro Ser Pro Ala Asp Leu Ser Pro
130 135 140

Gly Ala Ser Ser Val Thr Pro Pro Ala Pro Ala Arg Glu Pro Gly His
145 150 155 160

Ser Pro Gln Val Asp Glu Gln Leu Tyr Phe Gln Gly Gly Ser Pro Lys
165 170 175

```
Ser Ala Asp Lys Thr His Thr Cys Pro Pro Cys Pro Ala Pro Glu Leu
            180                 185                 190

Leu Gly Gly Pro Ser Val Phe Leu Phe Pro Pro Lys Pro Lys Asp Thr
        195                 200                 205

Leu Met Ile Ser Arg Thr Pro Glu Val Thr Cys Val Val Val Asp Val
    210                 215                 220

Ser His Glu Asp Pro Glu Val Lys Phe Asn Trp Tyr Val Asp Gly Val
225                 230                 235                 240

Glu Val His Asn Ala Lys Thr Lys Pro Arg Glu Glu Gln Tyr Asn Ser
                245                 250                 255

Thr Tyr Arg Val Val Ser Val Leu Thr Val Leu His Gln Asp Trp Leu
            260                 265                 270

Asn Gly Lys Glu Tyr Lys Cys Lys Val Ser Asn Lys Ala Leu Pro Ala
        275                 280                 285

Pro Ile Glu Lys Thr Ile Ser Lys Ala Lys Gly Gln Pro Arg Glu Pro
    290                 295                 300

Gln Val Tyr Thr Leu Pro Pro Cys Arg Asp Glu Leu Thr Lys Asn Gln
305                 310                 315                 320

Val Ser Leu Trp Cys Leu Val Lys Gly Phe Tyr Pro Ser Asp Ile Ala
                325                 330                 335

Val Glu Trp Glu Ser Asn Gly Gln Pro Glu Asn Asn Tyr Lys Thr Thr
            340                 345                 350

Pro Pro Val Leu Asp Ser Asp Gly Ser Phe Phe Leu Tyr Ser Lys Leu
        355                 360                 365

Thr Val Asp Lys Ser Arg Trp Gln Gln Gly Asn Val Phe Ser Cys Ser
    370                 375                 380

Val Met His Glu Ala Leu His Asn His Tyr Thr Gln Lys Ser Leu Ser
385                 390                 395                 400

Leu Ser Pro Gly Lys Ser Gly Gly Leu Asn Asp Ile Phe Glu Ala Gln
                405                 410                 415

Lys Ile Glu Trp His Glu
```

420

<210> 92
<211> 422
<212> PRT
<213> Artificial sequence

<220>
<223> Cynomolgus 4-1BB Fc knob chain

<400> 92

Leu Gln Asp Leu Cys Ser Asn Cys Pro Ala Gly Thr Phe Cys Asp Asn
1               5                   10                  15

Asn Arg Ser Gln Ile Cys Ser Pro Cys Pro Pro Asn Ser Phe Ser Ser
            20                  25                  30

Ala Gly Gly Gln Arg Thr Cys Asp Ile Cys Arg Gln Cys Lys Gly Val
        35                  40                  45

Phe Lys Thr Arg Lys Glu Cys Ser Ser Thr Ser Asn Ala Glu Cys Asp
    50                  55                  60

Cys Ile Ser Gly Tyr His Cys Leu Gly Ala Glu Cys Ser Met Cys Glu
65                  70                  75                  80

Gln Asp Cys Lys Gln Gly Gln Glu Leu Thr Lys Lys Gly Cys Lys Asp
                85                  90                  95

Cys Cys Phe Gly Thr Phe Asn Asp Gln Lys Arg Gly Ile Cys Arg Pro
            100                 105                 110

Trp Thr Asn Cys Ser Leu Asp Gly Lys Ser Val Leu Val Asn Gly Thr
        115                 120                 125

Lys Glu Arg Asp Val Val Cys Gly Pro Ser Pro Ala Asp Leu Ser Pro
    130                 135                 140

Gly Ala Ser Ser Ala Thr Pro Pro Ala Pro Ala Arg Glu Pro Gly His
145                 150                 155                 160

Ser Pro Gln Val Asp Glu Gln Leu Tyr Phe Gln Gly Gly Ser Pro Lys
                165                 170                 175

Ser Ala Asp Lys Thr His Thr Cys Pro Pro Cys Pro Ala Pro Glu Leu
            180                 185                 190

Leu Gly Gly Pro Ser Val Phe Leu Phe Pro Pro Lys Pro Lys Asp Thr
        195                 200                 205

```
Leu Met Ile Ser Arg Thr Pro Glu Val Thr Cys Val Val Val Asp Val
    210                 215                 220

Ser His Glu Asp Pro Glu Val Lys Phe Asn Trp Tyr Val Asp Gly Val
225                 230                 235                 240

Glu Val His Asn Ala Lys Thr Lys Pro Arg Glu Glu Gln Tyr Asn Ser
                245                 250                 255

Thr Tyr Arg Val Val Ser Val Leu Thr Val Leu His Gln Asp Trp Leu
            260                 265                 270

Asn Gly Lys Glu Tyr Lys Cys Lys Val Ser Asn Lys Ala Leu Pro Ala
        275                 280                 285

Pro Ile Glu Lys Thr Ile Ser Lys Ala Lys Gly Gln Pro Arg Glu Pro
    290                 295                 300

Gln Val Tyr Thr Leu Pro Pro Cys Arg Asp Glu Leu Thr Lys Asn Gln
305                 310                 315                 320

Val Ser Leu Trp Cys Leu Val Lys Gly Phe Tyr Pro Ser Asp Ile Ala
                325                 330                 335

Val Glu Trp Glu Ser Asn Gly Gln Pro Glu Asn Asn Tyr Lys Thr Thr
            340                 345                 350

Pro Pro Val Leu Asp Ser Asp Gly Ser Phe Phe Leu Tyr Ser Lys Leu
        355                 360                 365

Thr Val Asp Lys Ser Arg Trp Gln Gln Gly Asn Val Phe Ser Cys Ser
    370                 375                 380

Val Met His Glu Ala Leu His Asn His Tyr Thr Gln Lys Ser Leu Ser
385                 390                 395                 400

Leu Ser Pro Gly Lys Ser Gly Gly Leu Asn Asp Ile Phe Glu Ala Gln
                405                 410                 415

Lys Ile Glu Trp His Glu
            420
```

<210> 93
<211> 423
<212> PRT
<213> Artificial sequence

<220>
<223> Murine 4-1BB Fc knob chain

<400> 93

Val Gln Asn Ser Cys Asp Asn Cys Gln Pro Gly Thr Phe Cys Arg Lys
1               5                   10                  15

Tyr Asn Pro Val Cys Lys Ser Cys Pro Pro Ser Thr Phe Ser Ser Ile
            20                  25                  30

Gly Gly Gln Pro Asn Cys Asn Ile Cys Arg Val Cys Ala Gly Tyr Phe
        35                  40                  45

Arg Phe Lys Lys Phe Cys Ser Ser Thr His Asn Ala Glu Cys Glu Cys
    50                  55                  60

Ile Glu Gly Phe His Cys Leu Gly Pro Gln Cys Thr Arg Cys Glu Lys
65                  70                  75                  80

Asp Cys Arg Pro Gly Gln Glu Leu Thr Lys Gln Gly Cys Lys Thr Cys
                85                  90                  95

Ser Leu Gly Thr Phe Asn Asp Gln Asn Gly Thr Gly Val Cys Arg Pro
            100                 105                 110

Trp Thr Asn Cys Ser Leu Asp Gly Arg Ser Val Leu Lys Thr Gly Thr
        115                 120                 125

Thr Glu Lys Asp Val Val Cys Gly Pro Pro Val Val Ser Phe Ser Pro
    130                 135                 140

Ser Thr Thr Ile Ser Val Thr Pro Glu Gly Gly Pro Gly Gly His Ser
145                 150                 155                 160

Leu Gln Val Leu Val Asp Glu Gln Leu Tyr Phe Gln Gly Gly Ser Pro
                165                 170                 175

Lys Ser Ala Asp Lys Thr His Thr Cys Pro Pro Cys Pro Ala Pro Glu
            180                 185                 190

Leu Leu Gly Gly Pro Ser Val Phe Leu Phe Pro Pro Lys Pro Lys Asp
        195                 200                 205

Thr Leu Met Ile Ser Arg Thr Pro Glu Val Thr Cys Val Val Val Asp
    210                 215                 220

Val Ser His Glu Asp Pro Glu Val Lys Phe Asn Trp Tyr Val Asp Gly
225                 230                 235                 240

```
Val Glu Val His Asn Ala Lys Thr Lys Pro Arg Glu Glu Gln Tyr Asn
                245                 250                 255

Ser Thr Tyr Arg Val Val Ser Val Leu Thr Val Leu His Gln Asp Trp
            260                 265                 270

Leu Asn Gly Lys Glu Tyr Lys Cys Lys Val Ser Asn Lys Ala Leu Pro
        275                 280                 285

Ala Pro Ile Glu Lys Thr Ile Ser Lys Ala Lys Gly Gln Pro Arg Glu
    290                 295                 300

Pro Gln Val Tyr Thr Leu Pro Pro Cys Arg Asp Glu Leu Thr Lys Asn
305                 310                 315                 320

Gln Val Ser Leu Trp Cys Leu Val Lys Gly Phe Tyr Pro Ser Asp Ile
                325                 330                 335

Ala Val Glu Trp Glu Ser Asn Gly Gln Pro Glu Asn Asn Tyr Lys Thr
            340                 345                 350

Thr Pro Pro Val Leu Asp Ser Asp Gly Ser Phe Phe Leu Tyr Ser Lys
        355                 360                 365

Leu Thr Val Asp Lys Ser Arg Trp Gln Gln Gly Asn Val Phe Ser Cys
    370                 375                 380

Ser Val Met His Glu Ala Leu His Asn His Tyr Thr Gln Lys Ser Leu
385                 390                 395                 400

Ser Leu Ser Pro Gly Lys Ser Gly Gly Leu Asn Asp Ile Phe Glu Ala
                405                 410                 415

Gln Lys Ile Glu Trp His Glu
            420
```

## Description

## FIELD OF THE INVENTION

The invention relates to novel trimeric costimulatory TNF family ligand-containing antigen binding molecules comprising three fusion polypeptides, each of the three fusion polypeptides comprising (a) an ectodomain of a TNF ligand family member or fragments thereof, (b) a trimerization domain, in particular a trimerization domain derived from human cartilage matrix protein (hCMP, SEQ ID NO: 1), and (c) a moiety capable of specific binding to a target cell antigen, and to methods of producing these molecules and to methods of using the same. The invention further relates to methods of producing these molecules and to methods of using the same.

## BACKGROUND

Ligands interacting with molecules of the TNF (tumor necrosis factor) receptor superfamily have pivotal roles in the organization and function of the immune system. While regulating normal functions such as immune responses, hematopoiesis and morphogenesis, the TNF family ligands (also called cytokines) play a role in tumorgenesis, transplant rejection, septic shock, viral replication, bone resorption, rheumatoid arthritis and diabetes (Aggarwal, 2003). The TNF ligand family comprises 18 genes encoding 19 type II (i.e. intracellular N terminus and extracellular C-terminus) transmembrane proteins, characterized by the presence of a conserved C-terminal domain coined the 'TNF homology domain' (THD). This domain is responsible for receptor binding and is thus critical for the biological activity of the TNF ligand family members. The sequence identity between family members is ~20-30% (Bodmer, 2002). Members of the TNF ligand family exert their biological function as self-assembling, noncovalent trimers (Banner et al, 1993). Thus, the TNF family ligands need to form a trimer that is able to bind to and to activate the corresponding receptors of TNFR superfamily.

Several approaches have been described to generate artificial multimers with defined stoichiometries of recombinant antibodies or other proteins. WO 01/49866 discloses recombinant fusion proteins comprising a TNF cytokine and a multimerization component, particularly proteins from the C1q protein family such as ACRP30 or a collectin. A disadvantage of these fusion proteins is, however, that the trimerization domain usually has a large molecular weight and/or that the trimerization is rather inefficient. WO 2009/000538 and Wyzgol et al. (2009) disclose fusion proteins comprising a TNF cytokine and a trimerization domain from the chicken protein tenascin. Biological activity could be strongly enhanced. However, a trimerization domain that is derived from a non-human origin, could have the disadvantage of causing immunogenicity reactions in the human body. Human cartilage matix protein (huCMP) is a major extracellular matrix protein localized specifically in cartilage. Its C-terminal region forms a three-stranded alpha-helical coiled-coil structure (Beck, 1996).

WO 2006/121810 relates to a trimeric OX40 ligand fusion polypeptide including a ligand domain, a trimerization domain and an immunoglobulin Fc domain (single chain CH2, CH3 and hinge). Upon assembly of the OX-40L fusion polypeptide into a trimer, two Fc domains dimerize and the third, unpaired Fc domain associates with an unpaired Fc domain of a second OX-40L fusion protein trimer so that a stable OX40L fusion polypeptide hexamer is provided. The trimerization domain is an isoleucine zipper domain derived from yeast GCN4. A similar hexamer comprising a human TRAF2 trimerization domain instead of the isoleucine zipper domain is disclosed in WO 2015/183902.

Some members of the TNF ligand family have co stimulatory effects on T-cells, meaning that they sustain T-cell responses after initial T cell activation (Watts, 2005). 4-1BBL, OX40L, GITRL, CD70, CD30L and LIGHT belong to this group of costimulatory TNF family ligands.

Among several costimulatory molecules, the tumor necrosis factor (TNF) receptor family member OX40 (CD 134) plays a key role in the survival and homeostasis of effector and memory T cells. OX40 (CD134) regulates immune responses against infections, tumors and self-antigens and its expression has been demonstrated on the surface of T-, NKT-, NK-cells as well as neutrophils and shown to be strictly inducible or strongly upregulated in response to various stimulatory signals. Combined with T-cell receptor triggering, OX40 engagement on T-cells by its natural ligand or agonistic antibodies leads to synergistic activation of the PI3K and NFκB signalling pathways. In turn, this results in enhanced proliferation, increased cytokine receptor and cytokine production and better survival of activated T-cells.

4-1BB (CD137), a member of the TNF receptor superfamily, has been first identified as a molecule whose expression is induced by T-cell activation (Kwon and Weissman, 1989). Subsequent studies demonstrated expression of 4-1BB in T- and B-lymphocytes (Snell et al., 2011; Zhang et al., 2010), NK-cells (Lin et al., 2008), NKT-cells (Kim et al., 2008), monocytes (Kienzle and von Kempis, 2000; Schwarz et al., 1995), neutrophils (Heinisch et al., 2000), mast (Nishimoto et al., 2005) and dendritic cells as well as cells of non-hematopoietic origin such as endothelial and smooth muscle cells (Broll et al., 2001; Olofsson et al., 2008). Expression of 4-1BB in different cell types is mostly inducible and driven by various stimulatory signals, such as T-cell receptor (TCR) or B-cell receptor triggering, as well as signaling induced through co-stimulatory molecules or receptors of pro-inflammatory cytokines (Diehl et al., 2002; von Kempis et al., 1997; Zhang et al., 2010).

Expression of 4-1BB ligand (4-1BBL or CD137L) is more restricted and is observed on professional antigen presenting cells (APC) such as B-cells, dendritic cells (DCs) and macrophages. Inducible expression of 4-1BBL is characteristic for T-cells, including both $\alpha\beta$ and $\gamma\delta$ T-cell subsets, and endothelial cells (reviewed in Shao and Schwarz, 2011).

CD 137 signaling is known to stimulate IFN$\gamma$ secretion and proliferation of NK cells (Buechele et al., 2012; Lin et al., 2008; Melero et al., 1998) as well as to promote DC activation as indicated by their increased survival and capacity to secret cytokines and upregulate co-stimulatory molecules (Choi et al., 2009; Futagawa et al., 2002; Wilcox et al., 2002). However, CD 137 is best characterized as a co-stimulatory molecule which modulates TCR-induced activation in both the CD4+ and CD8+ subsets of T-cells. In combination with TCR triggering, agonistic 4-1BB-specific antibodies enhance proliferation of T-cells, stimulate lymphokine secretion and decrease sensitivity of T-lymphocytes to activation-induced cells death (reviewed in Snell et al., 2011).

In line with these co-stimulatory effects of 4-1BB antibodies on T-cells in vitro, their administration to tumor bearing mice leads to potent anti-tumor effects in many experimental tumor models (Melero et al., 1997; Narazaki et al., 2010). However, 4-1BB usually exhibits its potency as an anti-tumor agent only when administered in combination with other immunomodulatory compounds (Curran et al., 2011; Guo et al., 2013; Morales-Kastresana et al., 2013; Teng et al., 2009; Wei et al., 2013), chemotherapeutic reagents (Ju et al., 2008; Kim et al., 2009), tumor-specific vaccination (Cuadros et al., 2005; Lee et al., 2011) or radiotherapy (Shi and Siemann, 2006). In vivo depletion experiments demonstrated that CD8+ T-cells play the most critical role in anti-tumoral effect of 4-1BB-specific antibodies. However, depending on the tumor model or combination therapy, which includes anti-4-1BB, contributions of other types of cells such as DCs, NK-cells or CD4+ T-cells have been reported (Melero et al., 1997; Murillo et al., 2009; Narazaki et al., 2010; Stagg et al., 2011).

In addition to their direct effects on different lymphocyte subsets, 4-1BB agonists can also induce infiltration and retention of activated T-cells in the tumor through 4-1BB-mediated upregulation of intercellular adhesion molecule 1 (ICAM1) and vascular cell adhesion molecule 1 (VCAM1) on tumor vascular endothelium (Palazon et al., 2011). 4-1BB triggering may also reverse the state of T-cell anergy induced by exposure to soluble antigen that may contribute to disruption of immunological tolerance in the tumor micro-environment or during chronic infections (Wilcox et al., 2004).

It appears that the immunomodulatory properties of 4-1BB agonistic antibodies in vivo require the presence of the wild type Fc-portion on the antibody molecule thereby implicating Fc-receptor binding as an important event required for the pharmacological activity of such reagents as has been described for agonistic antibodies specific to other apoptosis-inducing or immunomodulatory members of the TNFR-superfamily (Li and Ravetch, 2011; Teng et al., 2009). However, systemic administration of 4-1BB-specific agonistic antibodies with the functionally active Fc domain also induces expansion of CD8+ T-cells associated with liver toxicity (Dubrot et al., 2010) that is diminished or significantly ameliorated in the absence of functional Fc-receptors in mice. In human clinical trials (ClinicalTrials.gov, NCT00309023), Fc-competent 4-1BB agonistic antibodies (BMS-663513) administered once every three weeks for 12 weeks induced stabilization of the disease in patients with melanoma, ovarian or renal cell carcinoma. However, the same antibody given in another trial (NCT00612664) caused grade 4 hepatitis leading to termination of the trial (Simeone and Ascierto, 2012).

Collectively, the available pre-clinical and clinical data clearly demonstrate that there is a high clinical need for effective 4-1BB agonists. However, new generation drug candidates should not only effectively engage 4-1BB on the surface of hematopoietic and endothelial cells but also be capable of achieving that through mechanisms other than binding to Fc-receptors in order to avoid uncontrollable side effects. The latter may be accomplished through preferential binding to and oligomerization on tumor-specific or tumor-associated moieties.

The novel trimeric antigen binding molecules of this invention consist of fusion polypeptides combining a costimulatory TNF ligand and a moiety capable of specific binding to a tumor-specific target cell antigen and are stable as the fusion polypeptides are covalently linked to each other by a huCMP trimerization domain. The stable TNF ligand trimers are able to trigger TNF receptors not only effectively, but also very selectively at the desired site based on the fact that they comprise three moieties capable of specific binding to a target cell antigen. Side effects may therefore be drastically reduced.

## SUMMARY OF THE INVENTION

The invention relates to a trimeric costimulatory TNF family ligand-containing antigen binding molecule comprising three fusion polypeptides, each of the three fusion polypeptides comprising

(a) an ectodomain of a costimulatory TNF ligand family member or fragments thereof,
(b) a trimerization domain, and
(c) a moiety capable of specific binding to a target cell antigen.

The trimerization domain is derived from a human polypeptide, in particular it can be derived from a polypeptide derived from a protein selected from the group consisting of human TRAF2, human thrombospondin 1, human matrilin-4, human cartilage matrix protein (huCMP) and human cubilin. It can also be an isoleucine zipper domain, for example the peptide with the amino acid sequence of SEQ ID NO:70.

In one aspect, the invention provides a trimeric co stimulatory TNF family ligand-containing antigen binding molecule comprising three fusion polypeptides, each of the three fusion polypeptides comprising

(a) an ectodomain of a co stimulatory TNF ligand family member or fragments thereof,
(b) a trimerization domain derived from human cartilage matrix protein (huCMP) of amino acid sequence of SEQ ID NO:1, and
(c) a moiety capable of specific binding to a target cell antigen.

In a particular aspect, the trimeric costimulatory TNF family ligand-containing antigen binding molecule comprises three fusion polypeptides, each of the three fusion polypeptides comprising a trimerization domain comprising an amino acid sequence having at least 95% identity to SEQ ID NO:2. More particularly, the trimerization domain comprises the amino acid sequence of SEQ ID NO:2.

In particular, the costimulatory TNF family ligand member is selected from the group consisting of 4-1BBL, OX40L, GITRL, CD70, CD30L and LIGHT, more particularly the costimulatory TNF family ligand member is selected from 4-1BBL and OX40L.

In one aspect, the costimulatory TNF ligand family member is 4-1BBL. In particular, the trimeric costimulatory TNF family ligand-containing antigen binding molecule comprises three fusion polypeptides, wherein in each of the three fusion polypeptides the ectodomain of a TNF ligand family member comprises the amino acid sequence selected from the group consisting of SEQ ID NO:3, SEQ ID NO:4, SEQ ID NO:5, SEQ ID NO:6, SEQ ID NO:7, SEQ ID NO:8, SEQ ID NO:9 and SEQ ID NO: 10, particularly the amino acid sequence of SEQ ID NO:3 or SEQ ID NO:7. In a further aspect, the trimeric costimulatory TNF family ligand-containing antigen binding molecule comprises three fusion polypeptides, each of the three fusion polypeptides comprising (a) an ectodomain of a TNF ligand family member comprising the amino acid sequence selected from the group consisting of SEQ ID NO:3, SEQ ID NO:4, SEQ ID NO:5, SEQ ID NO:6, SEQ ID NO:7, SEQ ID NO:8, SEQ ID NO:9 and SEQ ID NO: 10, (b) a trimerization domain comprising the amino acid sequence of SEQ ID NO:2 and (c) a moiety capable of specific binding to a target cell antigen.

In another aspect, the costimulatory TNF ligand family member is OX40L. In particular, the trimeric costimulatory TNF family ligand-containing antigen binding molecule comprises three fusion polypeptides, wherein in each of the three fusion polypeptides the ectodomain of a TNF ligand family member comprises the amino acid sequence selected from the group consisting of SEQ ID NO: 11, SEQ ID NO: 12 and SEQ ID NO: 13. In a further aspect, the trimeric costimulatory TNF family ligand-containing antigen binding molecule comprises three fusion polypeptides, wherein each of the three fusion polypeptides comprises (a) an ectodomain of a TNF ligand family member comprising the amino acid sequence selected from the group consisting of SEQ ID NO: 11, SEQ ID NO: 12 and SEQ ID NO: 13, (b) a trimerization domain comprising the amino acid sequence of SEQ ID NO:2 and (c) a moiety capable of specific binding to a target cell antigen.

In another aspect, provided is a trimeric costimulatory TNF family ligand-containing antigen binding molecule comprising three fusion polypeptides as described before, wherein the ectodomain of a TNF ligand family member or a fragment thereof is fused at the N-terminal amino acid to the C-terminal amino acid of the trimerization domain, optionally through a peptide linker.

In a further aspect, provided is a trimeric costimulatory TNF family ligand-containing antigen binding molecule comprising three fusion polypeptides as described before, wherein the moiety capable of specific binding to a target cell antigen is fused at the C-terminal amino acid to the N-terminal amino acid of the trimerization domain, optionally through a peptide linker.

In another aspect, the invention provides a trimeric costimulatory TNF family ligand-containing antigen binding molecule comprising three fusion polypeptides as described before, wherein the moiety capable of specific binding to a target cell antigen is selected from the group consisting of an antibody fragment, a Fab molecule, a crossover Fab molecule, a single chain Fab molecule, a Fv molecule, a scFv molecule, a single domain antibody, an aVH and a scaffold antigen binding protein. In a particular aspect, the moiety capable of specific binding to a target cell antigen is a Fab molecule capable of specific binding to a target cell antigen. In a further aspect, said Fab molecule is fused at the C-terminal amino acid of the CHI domain to the N-terminal amino acid of the trimerization domain, optionally through a peptide linker.

In a further aspect, provided is a trimeric costimulatory TNF family ligand-containing antigen binding molecule comprising three fusion polypeptides as described before, wherein the target cell antigen is selected from the group consisting of Fibroblast Activation Protein (FAP), Melanoma-associated Chondroitin Sulfate Proteoglycan (MCSP), Epidermal Growth Factor Receptor (EGFR), Carcinoembryonic Antigen (CEA), CD 19, CD20 and CD33. In a particular aspect, the target cell antigen is Fibroblast Activation Protein (FAP).

In a further aspect, provided is a trimeric costimulatory TNF family ligand-containing antigen binding molecule comprising three fusion polypeptides as described before, wherein the moiety capable of specific binding to FAP comprises

(a) a VH domain comprising (i) CDR-H1 comprising the amino acid sequence of SEQ ID NO:14, (ii) CDR-H2 comprising the amino acid sequence of SEQ ID NO:15 and (iii) CDR-H3 comprising the amino acid sequence of SEQ ID NO:16, and a VL domain comprising (iv) CDR-L1 comprising the amino acid sequence of SEQ ID NO: 17, (v) CDR-L2 comprising the amino acid sequence of SEQ ID NO:18 and (vi) CDR-L3 comprising the amino acid sequence of SEQ ID NO:19 or
(b) a VH domain comprising (i) CDR-H1 comprising the amino acid sequence of SEQ ID NO:20, (ii) CDR-H2 comprising the amino acid sequence of SEQ ID NO:21 and (iii) CDR-H3 comprising the amino acid sequence of SEQ ID NO:22, and a VL domain comprising (iv) CDR-L1 comprising the amino acid sequence of SEQ ID NO:23, (v) CDR-L2 comprising the amino acid sequence of SEQ ID NO:24 and (vi) CDR-L3 comprising the amino acid sequence of SEQ ID NO:25.

In a further aspect, the moiety capable of specific binding to FAP comprises (a) a VH domain comprising the amino acid sequence of SEQ ID NO:26 and a VL domain comprising the amino acid sequence of SEQ ID NO:27, or (b) a VH domain comprising the amino acid sequence of SEQ ID NO:28 and a VL domain comprising the amino acid sequence of SEQ ID NO:29.

In one aspect, provided is a trimeric costimulatory TNF family ligand-containing antigen binding molecule comprising three fusion polypeptides as described before, wherein said three fusion polypeptides are identical.

In another aspect, the invention provides a fusion polypeptide comprising (a) an ectodomain of a costimulatory TNF ligand family member or a fragment thereof and (b) a trimerization domain derived from human cartilage matrix protein (huCMP) of the amino acid sequence of SEQ ID NO:2, wherein said trimerization domain is capable of mediating stable association of said fusion polypeptide with two further such fusion polypeptides. In a further aspect, the fusion polypeptide further comprises (c) a moiety capable of specific binding to a target cell antigen.

In yet a further aspect, provided is a fusion polypeptide, wherein the fusion polypeptide comprises

(a) an ectodomain of a TNF ligand family comprising the amino acid sequence selected from the group consisting of SEQ ID NO:3, SEQ ID NO:4, SEQ ID NO:5, SEQ ID NO:6, SEQ ID NO:7, SEQ ID NO:8, SEQ ID NO:9 and SEQ ID NO: 10,
(b) a trimerization domain comprising the amino acid sequence of SEQ ID NO:2 and
(c) a VH domain comprising (i) CDR-H1 comprising the amino acid sequence of SEQ ID NO:14, (ii) CDR-H2 comprising the amino acid sequence of SEQ ID NO:15 and (iii) CDR-H3 comprising the amino acid sequence of SEQ ID NO:16 or a VH domain comprising (i) CDR-H1 comprising the amino acid sequence of SEQ ID NO:20,(ii) CDR-H2 comprising the amino acid sequence of SEQ ID NO:21 and (iii) CDR-H3 comprising the amino acid sequence of SEQ ID NO:22.

According to another aspect of the invention, there is provided an isolated polynucleotide encoding a trimeric costimulatory TNF family ligand-containing antigen binding molecule as defined herein before or a fusion polypeptide as described herein before. The invention further provides a vector, particularly an expression vector, comprising the isolated polynucleotide of the invention and a host cell comprising the isolated polynucleotide or the vector of the invention. In some embodiments the host cell is a eukaryotic cell, particularly a mammalian cell.

In another aspect, provided is a method for producing the trimeric costimulatory TNF family ligand-containing antigen binding molecule of the invention, comprising the steps of (i) culturing the host cell of the invention under conditions suitable for expression of said antigen binding molecule, and (ii) isolating said trimeric antigen binding molecule. The invention also encompasses a trimeric costimulatory TNF family ligand-containing antigen binding molecule produced by the method of the invention.

The invention further provides a pharmaceutical composition comprising trimeric costimulatory TNF family ligand-containing antigen binding molecule of the invention and at least one pharmaceutically acceptable excipient.

Also encompassed by the invention is the trimeric costimulatory TNF family ligand-containing antigen binding molecule of the invention, or the pharmaceutical composition of the invention, for use as medicament. In one aspect, provided is a trimeric costimulatory TNF family ligand-containing antigen binding molecule of the invention, or the pharmaceutical composition of the invention, for use in the treatment of a disease in an individual in need thereof. In a specific embodiment, provided is the trimeric costimulatory TNF family ligand-containing antigen binding molecule of the invention, or the pharmaceutical composition of the invention, for use in the treatment of cancer.

Also provided is the use of the trimeric costimulatory TNF family ligand-containing antigen binding molecule of the invention for the manufacture of a medicament for the treatment of a disease in an individual in need thereof, in particular for the manufacture of a medicament for the treatment of cancer, as well as a method of treating a disease in an individual, comprising administering to said individual a therapeutically effective amount of a composition comprising the trimeric costimulatory TNF family ligand-containing antigen binding molecule of the invention in a pharmaceutically acceptable form. In a specific embodiment, the disease is cancer. In any of the above embodiments the individual is preferably a mammal, particularly a human.

## BRIEF DESCRIPTION OF THE DRAWINGS

**Figure 1A** shows the chain encoding a FAP-targeted 4-1BB ligand-containing fusion polypeptide and the light chain with FAP specificity. Disulfide bonds are formed between three FAP-targeted 4-1BB ligand-containing fusion poylpeptide chains leading to the formation of covalently linked CMP-trimeric 4-1BB ligand-containing antigen binding molecules, targeted to FAP. **Figure 1B** shows the trimeric 4-1BBL-containing antigen binding molecule comprising three Fabs capable of specific binding to FAP (FAP-targeted). **Figure 1C** is a drawing of the monomeric 4-1BB Fc(kih) molecules as prepared in Example 3.

**Figure 2** shows the binding of the trimeric FAP-targeted-CMP- 4-1BB ligand-containing antigen binding molecule to recombinant 4-1BB Fc (kih) receptor as assessed by surface plasmon resonance. In **Figure 2A** binding to human 4-1BB Fc (kih) and in **Figure 2B** the setup of the assay is shown, 4-1BB Fc (kih) is immobilized on a CM5 chip. **Figure 2C** shows binding to cynomolgus 4-1BB Fc (kih), the setup of the assay is illustrated in **Figure 2D.** In **Figure 2E** the binding to murine 4-1BB Fc (kih) and in **Figure 2F** the assay setup is shown.

**Figure 3** illustrates the binding of recombinant 4-1BB Fc(kih) to FAP-targeted-CMP- 4-1BB ligand-containing antigen binding molecule. In **Figure 3A** the setup of the affinity measurement is shown. **Figure 3B** shows the binding of human 4-1BB Fc(kih) to the FAP-targeted-CMP- 4-1BB ligand-containing antigen binding molecule and **Figure 3C** shows the binding of cynomolgus 4-1BB Fc(kih) to FAP-targeted-CMP- 4-1BB ligand-containing antigen binding molecule.

**Figures 4A to 4D** relate to the binding of FAP-targeted CMP trimeric 4-1BB ligand-containing antigen binding molecules (filled circles) or DP47-targeted CMP trimeric 4-1BB ligand-containing antigen binding molecules (open circles) or DP47 hu IgG P329G control (filled triangle) to resting (naive) $CD8^+$ and $CD4^+$ T cells shown in the upper panels **(Figures 4A** and **4B)** or activated $CD8^+$ and $CD4^+$ T cells shown in the lower panels **(Figures 4C** and **4D).** Shown is the binding as median of fluorescence intensity (MFI) of red macrophytic algae Phycoerythrin (R-PE)-labeled anti-human IgG F(ab')2-specific goat IgG F(ab')2 fragment which is used as secondary detection antibody versus the concentration of FAP- or DP47-targeted CMP-trimeric 4-1BB ligand-containing antigen binding molecules. MFI was measured by flow cytometry and baseline corrected by subtracting the MFI of the blank control.

**Figures 5A to 5C** show the binding of FAP-targeted CMP-trimeric 4-1BB ligand-containing antigen binding molecules (filled circles) or DP47-targeted CMP-trimeric 4-1BB ligand-containing antigen binding molecules (open circles) to human fibroblast activation protein (FAP)-expressing transfected mouse embryonic fibroblast cell line 3T3-huFAP clone 39. The binding is characterized by plotting the MFI of polyclonal FITC-labeled anti-human IgG H+L-specific goat IgG F(ab')2 fragment (Figure 5A) or the MFI of FITC-labeled monoclonal anti-human IgG κ-light chain-specific mouse IgGl κ (clone G18-145) (Figure 5B) or the MFI of PE-labeled anti-human 4-1BBL mouse IgG2b (Figure 5C) which are used as secondary detection antibodies versus the concentration in nM of tested CMP-trimeric 4-1BB ligand constructs. MFI was measured by flow cytometry and baseline corrected by subtracting the MFI of the blank control.

The scheme in **Figure 6** illustrates the general principal of the NF-κB activity assay described in Example 5.1.2 using a reporter cell line. The ratio of FAP-expressing tumor cells to the reporter cell line HeLa-huCD137-NF-κB-luciferase was 5 to 1.

**Figure 7A to 7C** show the activation of the NF-κB signaling pathway by FAP-targeted CMP-trimeric 4-1BB ligand-containing antigen binding molecule (FAP-CMP-4-1BBL, filled circles) is strictly dependent on its binding to FAP-expressing target cells and an optimal concentration inducing maximal crosslinking (bell shape curve). NF-κB reporter HeLa cells were co-cultured with the indicated tumor cells (3T3-huFAP clone 39 in **Figure 7A,** MV-3 in **Figure 7B** and WM-266-4 in **Figure 7C)** exhibiting different levels of cell surface FAP expression. Luciferase activity was assessed as described in Example 5.1.2 after culturing cells in the absence or presence of 4-1BBL-containing antigen binding molecules at the indicated concentrations. Open circles refer to DP47 untargeted CMP-trimeric 4-1BB ligand-containing antigen binding molecule. Activity is characterized by blotting the units of released light (URL) measured during 0.5 s versus the concentration in nM of tested constructs. URL are emitted due to luciferase-mediated oxidation of luciferin to oxyluciferin.

The scheme in **Figure 8** illustrates the general principal of the antigen-specific $CD8^+$ T cell activation assay as described in Example 5.1.3 using NLV-specific $CD8^+$ T cells. The ratio of FAP-expressing tumor cells to NLV-specific CD8 T cells was 8 to 1.

**Figures 9A to 9F** show the prolonged IFNγ secretion and CD137 (4-1BB) expression of NLV-specific $CD8^+$ T cells is strictly dependent on simultaneous activation of T-cells via recognition of NLV-HLA-A2 complexes (signal 1) and 4-1BB-triggering by FAP-targeted huCMP trimeric 4-1BB ligand-containing antigen binding molecules (signal 2). Filled circles symbolize FAP-targeted CMP-trimeric 4-1BB ligand-containing antigen binding molecules; open circles symbolize DP47-targeted huCMP-trimeric 4-1BB ligand-containing antigen binding molecules. The effect of 4-1BB upregulation is shown in graphs of **Figures 9A, 9B** and **9C,** whereas the effect of INFγ expression of $CD8^+$ T cells is presented in graphs of **Figures 9D, 9E** and **9F.** Shown is always the frequency in percentage of positive cells in the total $CD8^+$ T cell population. All T cell activation curves are bell-shaped and show that the co-stimulation via FAP-targeted huCMP-trimeric 4-1BB ligand-containing antigen binding molecule is restricted to a defined concentration window of around 0.6 nM.

**Figure 10** shows the binding of FAP-targeted huCMP trimeric OX40 ligand-containing antigen binding molecules to FAP positive WM-266-4 cells. WM-266-4 cells express high levels of human fibroblast activation protein (huFAP). Only FAP-targeted huCMP trimeric OX40 ligand-containing antigen binding molecule (filled circle) but not the untargeted DP47 huIgG P329G control antibody (Control F, filled diamond) bound to WM-266-4 cells. Shown is the binding as median of fluorescence intensity (MFI) of FITC labeled anti-human IgG F(ab')2 specific goat IgG F(ab')2 fragment which is used as secondary detection antibody. MFI was measured by flow cytometry. The x-axis shows the concentration of antigen binding molecules.

**Figures 11A** and **11B** show the binding of FAP-targeted huCMP trimeric OX40 ligand-containing antigen binding molecules to resting and activated human $CD4^+$ T cells, respectively. OX40 is not expressed on resting human $CD4^+$ T cells. In the absence of human OX40 expressing cells no binding was observed **(Figure 11A).** After activation of human PBMCs OX40 is up-regulated on $CD4^+$ T cells **(Figure 11B).** FAP-targeted huCMP trimeric OX40 ligand-containing antigen binding molecules bound to OX40+ activated $CD4^+$ T cells. Shown is the binding as median of fluorescence intensity (MFI) of FITC labeled anti-human IgG $F(ab')_2$ specific goat IgG F(ab')2 fragment which is used as secondary detection antibody. MFI was measured by flow cytometry and baseline corrected by subtracting the MFI of the blank control. The x-axis shows the concentration of antigen binding molecules. In **Figures 11C** and **11D** is shown the binding of FAP-targeted huCMP trimeric OX40 ligand-containing antigen binding molecules to resting and activated human $CD8^+$ T cells, respectively. OX40 is not expressed on resting human $CD8^+$ T cells. In the absence of human OX40 expressing cells no binding was observed **(Figure 11C).** After activation of human PBMCs OX40 is up-regulated on $CD8^+$ T cells **(Figure 11D).** OX40 expression on human $CD8^+$ T cells is lower than on $CD4^+$ T cells and varies between donors and time points. Expression of OX40 was low on the depicted $CD8^+$ T cells. FAP-targeted huCMP trimeric OX40 ligand-containing antigen binding molecules bound to OX40+ activated $CD8^+$ T cells. Shown is the binding as median of fluorescence intensity (MFI) of FITC labeled anti-human IgG F(ab')2 specific goat IgG F(ab')2 fragment which is used as secondary detection antibody. MFI was measured by flow cytometry and baseline corrected by subtracting the MFI of the blank control. The x-axis shows the concentration of antigen binding molecules.

**Figures 12A** and **12B** show the activation of NFκB by FAP-targeted huCMP trimeric OX40 ligand-containing antigen binding molecules in HeLa_hOx40_NFkB_Luc1 reporter cells in the absence or presence of FAP positive tumor cells, respectively. Shown is the activation of NF-κB signaling pathway in the reporter cells by FAP-targeted huCMP trimeric OX40 ligand-containing antigen binding molecules in the presence of low FAP expressing expressing NIH-3T3 human FAP cells (ratio 3 FAP+ tumor cells to 1 reporter cell). The NF-κB-mediated luciferase activity was characterized by blotting the units of released light (URL), measured during 0.5 s, versus the concentration in nM of tested compounds. URLs are emitted due to luciferase-mediated oxidation of luciferin to oxyluciferin. Values are baseline corrected by subtracting the URLs of the blank control.

## DETAILED DESCRIPTION OF THE INVENTION

### Definitions

Unless defined otherwise, technical and scientific terms used herein have the same meaning as generally used in the art to which this invention belongs. For purposes of interpreting this specification, the following definitions will apply and whenever appropriate, terms used in the singular will also include the plural and vice versa.

As used herein, the term **"antigen binding molecule"** refers in its broadest sense to a molecule that specifically binds an antigenic determinant. Examples of antigen binding molecules are antibodies, antibody fragments and scaffold antigen binding proteins.

As used herein, the term **"moiety capable of specific binding to a target cell antigen"** refers to a polypeptide molecule that specifically binds to an antigenic determinant. In one aspect, the antigen binding moiety is able to activate signaling through its target cell antigen. In a particular aspect, the antigen binding moiety is able to direct the entity to which it is attached (e.g. the TNF family ligand trimer) to a target site, for example to a specific type of tumor cell or tumor stroma bearing the antigenic determinant. Moieties capable of specific binding to a target cell antigen include antibodies and fragments thereof as further defined herein. In addition, moieties capable of specific binding to a target cell antigen include scaffold antigen binding proteins as further defined herein, e.g. binding domains which are based on designed repeat proteins or designed repeat domains (see e.g. WO 2002/020565).

In relation to an antibody or fragment thereof, the term "moiety capable of specific binding to a target cell antigen" refers to the part of the molecule that comprises the area which specifically binds to and is complementary to part or all of an antigen. A moiety capable of specific antigen binding may be provided, for example, by one or more antibody variable domains (also called antibody variable regions). Particularly, a moiety capable of specific antigen binding comprises an antibody light chain variable region (VL) and an antibody heavy chain variable region (VH).

The term **"antibody"** herein is used in the broadest sense and encompasses various antibody structures, including but not limited to monoclonal antibodies, polyclonal antibodies, monospecific and multispecific antibodies (e.g., bispecific antibodies), and antibody fragments so long as they exhibit the desired antigen-binding activity.

The term **"monoclonal antibody"** as used herein refers to an antibody obtained from a population of substantially homogeneous antibodies, i.e., the individual antibodies comprising the population are identical and/or bind the same epitope, except for possible variant antibodies, e.g. containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen.

The term **"monospecific"** antibody as used herein denotes an antibody that has one or more binding sites each of which bind to the same epitope of the same antigen. The term **"bispecific"** means that the antigen binding molecule is able to specifically bind to at least two distinct antigenic determinants. Typically, a bispecific antigen binding molecule comprises two antigen binding sites, each of which is specific for a different antigenic determinant. In certain embodiments the bispecific antigen binding molecule is capable of simultaneously binding two antigenic determinants, particularly two antigenic determinants expressed on two distinct cells.

The term **"valent"** as used within the current application denotes the presence of a specified number of binding sites in an antigen binding molecule. As such, the terms "bivalent", "tetravalent", and "hexavalent" denote the presence of two binding sites, four binding sites, and six binding sites, respectively, in an antigen binding molecule.

The terms "full length antibody", "intact antibody", and "whole antibody" are used herein interchangeably to refer to an antibody having a structure substantially similar to a native antibody structure. **"Native antibodies"** refer to naturally occurring immunoglobulin molecules with varying structures. For example, native IgG-class antibodies are heterotetrameric glycoproteins of about 150,000 daltons, composed of two light chains and two heavy chains that are disulfide-bonded. From N- to C-terminus, each heavy chain has a variable region (VH), also called a variable heavy domain or a heavy chain variable domain, followed by three constant domains (CH1, CH2, and CH3), also called a heavy chain constant region. Similarly, from N- to C-terminus, each light chain has a variable region (VL), also called a variable light domain or a light chain variable domain, followed by a light chain constant domain (CL), also called a light chain constant region. The heavy chain of an antibody may be assigned to one of five types, called $\alpha$ (IgA), $\delta$ (IgD), $\varepsilon$ (IgE), $\gamma$ (IgG), or $\mu$ (IgM), some of which may be further divided into subtypes, e.g. $\gamma$l (IgG1), $\gamma$2 (IgG2), $\gamma$3 (IgG3), $\gamma$4 (IgG4), $\alpha$l (IgA1) and $\alpha$2 (IgA2). The light chain of an antibody may be assigned to one of two types, called kappa ($\kappa$) and lambda ($\lambda$), based on the amino acid sequence of its constant domain.

An **"antibody fragment"** refers to a molecule other than an intact antibody that comprises a portion of an intact antibody that binds the antigen to which the intact antibody binds. Examples of antibody fragments include but are not limited to Fv, Fab, Fab', Fab'-SH, $F(ab')_2$; diabodies, triabodies, tetrabodies, cross-Fab fragments; linear antibodies; single-chain antibody molecules (e.g. scFv); and single domain antibodies. For a review of certain antibody fragments, see Hudson et al., Nat Med 9, 129-134 (2003). For a review of scFv fragments, see e.g. Plückthun, in The Pharmacology of Monoclonal Antibodies, vol. 113, Rosenburg and Moore eds., Springer-Verlag, New York, pp. 269-315 (1994); see also WO 93/16185; and U.S. Patent Nos. 5,571,894 and 5,587,458. For discussion of Fab and F(ab')2 fragments comprising salvage receptor binding epitope residues and having increased in vivo half-life, see U.S. Patent No. 5,869,046. Diabodies are antibody fragments with two antigen-binding sites that may be bivalent or bispecific, see, for example, EP 404,097; WO 1993/01161; Hudson et al., Nat Med 9, 129-134 (2003); and Hollinger et al., Proc Natl Acad Sci USA 90, 6444-6448 (1993). Triabodies and tetrabodies are also described in Hudson et al., Nat Med 9, 129-134 (2003). Single-domain antibodies are antibody fragments comprising all or a portion of the heavy chain variable domain or all or a portion of the light chain variable domain of an antibody. In certain embodiments, a single-domain antibody is a human single-domain antibody (Domantis, Inc., Waltham, MA; see e.g. U.S. Patent No. 6,248,516 B1). Antibody fragments can be made by various techniques, including but not limited to proteolytic digestion of an intact antibody as well as production by recombinant host cells (e.g. E. coli or phage), as described herein.

Papain digestion of intact antibodies produces two identical antigen-binding fragments, called "Fab" fragments containing each the heavy- and light-chain variable domains and also the constant domain of the light chain and the first constant domain (CH1) of the heavy chain. As used herein, Thus, the term **"Fab fragment"** refers to an antibody fragment comprising a light chain fragment comprising a VL domain and a constant domain of a light chain (CL), and a VH domain and a first constant domain (CH1) of a heavy chain. Fab' fragments differ from Fab fragments by the addition of a few residues at the carboxy terminus of the heavy chain CH1 domain including one or more cysteins from the antibody hinge region. Fab'-SH are Fab' fragments in which the cysteine residue(s) of the constant domains bear a free thiol group. Pepsin treatment yields an F(ab')2 fragment that has two antigen-combining sites (two Fab fragments) and a part of the Fc region.

The term **"cross-Fab fragment"** or "xFab fragment" or "crossover Fab fragment" refers to a Fab fragment, wherein either the variable regions or the constant regions of the heavy and light chain are exchanged. Two different chain compositions of a crossover Fab molecule are possible and comprised in the bispecific antibodies of the invention: On the one hand, the variable regions of the Fab heavy and light chain are exchanged, i.e. the crossover Fab molecule comprises a peptide chain composed of the light chain variable region (VL) and the heavy chain constant region (CH1), and a peptide chain composed of the heavy chain variable region (VH) and the light chain constant region (CL). This crossover Fab molecule is also referred to as CrossFab (VLVH). On the other hand, when the constant regions of the Fab heavy and light chain are exchanged, the crossover Fab molecule comprises a peptide chain composed of the heavy chain variable region (VH) and the light chain constant region (CL), and a peptide chain composed of the light chain variable region (VL) and the heavy chain constant region (CH1). This crossover Fab molecule is also referred to as CrossFab (CLCH1).

A "single chain Fab fragment" or **"scFab"** is a polypeptide consisting of an antibody heavy chain variable domain (VH), an antibody constant domain 1 (CH1), an antibody light chain variable domain (VL), an antibody light chain constant domain (CL) and a linker, wherein said antibody domains and said linker have one of the following orders in N-terminal to C-terminal direction: a) VH-CH1-linker-VL-CL, b) VL-CL-linker-VH-CH1, c) VH-CL-linker-VL-CH1 or d) VL-CH1-linker-VH-CL; and wherein said linker is a polypeptide of at least 30 amino acids, preferably between 32 and 50 amino acids. Said single chain Fab fragments are stabilized via the natural disulfide bond between the CL domain and the CH1 domain. In addition, these single chain Fab molecules might be further stabilized by generation of interchain disulfide bonds via insertion of cysteine residues (e.g. position 44 in the variable heavy chain and position 100 in the variable light chain according to Kabat numbering).

A "crossover single chain Fab fragment" or **"x-scFab"** is a is a polypeptide consisting of an antibody heavy chain variable domain (VH), an antibody constant domain 1 (CH1), an antibody light chain variable domain (VL), an antibody light chain constant domain (CL) and a linker, wherein said antibody domains and said linker have one of the following orders in N-terminal to C-terminal direction: a) VH-CL-linker-VL-CH1 and b) VL-CH1-linker-VH-CL; wherein VH and VL form together an antigen-binding site which binds specifically to an antigen and wherein said linker is a polypeptide of at least 30 amino acids. In addition, these x-scFab molecules might be further stabilized by generation of interchain disulfide bonds via insertion of cysteine residues (e.g. position 44 in the variable heavy chain and position 100 in the variable light chain according to Kabat numbering).

A **"single-chain variable fragment (scFv)"** is a fusion protein of the variable regions of the heavy ($V_H$) and light chains ($V_L$) of an antibody, connected with a short linker peptide of ten to about 25 amino acids. The linker is usually rich in glycine for flexibility, as well as serine or threonine for solubility, and can either connect the N-terminus of the $V_H$ with the C-terminus of the $V_L$, or *vice versa.* This protein retains the specificity of the original antibody, despite removal of the constant regions and the introduction of the linker. scFv antibodies are, e.g. described in Houston, J.S., Methods in Enzymol. 203 (1991) 46-96). In addition, antibody fragments comprise single chain polypeptides having the characteristics of a VH domain, namely being able to assemble together with a VL domain, or of a VL domain, namely being able to assemble together with a VH domain to a functional antigen binding site and thereby providing the antigen binding property of full length antibodies.

**"Scaffold antigen binding proteins"** are known in the art, for example, fibronectin and designed ankyrin repeat proteins (DARPins) have been used as alternative scaffolds for antigen-binding domains, see, e.g., Gebauer and Skerra, Engineered protein scaffolds as next-generation antibody therapeutics. Curr Opin Chem Biol 13:245-255 (2009) and Stumpp et al., Darpins: A new generation of protein therapeutics. Drug Discovery Today 13: 695-701 (2008). In one aspect of the invention, a scaffold antigen binding protein is selected from the group consisting of CTLA-4 (Evibody), Lipocalins (Anticalin), a Protein A-derived molecule such as Z-domain of Protein A (Affibody), an A-domain (Avimer/Maxibody), a serum transferrin (*trans*-body); a designed ankyrin repeat protein (DARPin), a variable domain of antibody light chain or heavy chain (single-domain antibody, sdAb), a variable domain of antibody heavy chain (nanobody, aVH), $V_{NAR}$ fragments, a fibronectin (AdNectin), a C-type lectin domain (Tetranectin); a variable domain of a new antigen receptor beta-lactamase ($V_{NAR}$ fragments), a human gamma-crystallin or ubiquitin (Affilin molecules); a kunitz type domain of human protease inhibitors, microbodies such as the proteins from the knottin family, peptide aptamers and fibronectin (adnectin).

CTLA-4 (Cytotoxic T Lymphocyte-associated Antigen 4) is a CD28-family receptor expressed on mainly CD4+ T-cells. Its extracellular domain has a variable domain- like Ig fold. Loops corresponding to CDRs of antibodies can be substituted with heterologous sequence to confer different binding properties. CTLA-4 molecules engineered to have different binding specificities are also known as Evibodies (e.g. US7166697B1). Evibodies are around the same size as the isolated variable region of an antibody (e.g. a domain antibody). For further details see Journal of Immunological Methods 248 (1-2), 31-45 (2001).

Lipocalins are a family of extracellular proteins which transport small hydrophobic molecules such as steroids, bilins, retinoids and lipids. They have a rigid beta-sheet secondary structure with a number of loops at the open end of the conical structure which can be engineered to bind to different target antigens. Anticalins are between 160-180 amino acids in size, and are derived from lipocalins. For further details see Biochim Biophys Acta 1482: 337-350 (2000), US7250297B1 and US20070224633.

An affibody is a scaffold derived from Protein A of Staphylococcus aureus which can be engineered to bind to antigen. The domain consists of a three-helical bundle of approximately 58 amino acids. Libraries have been generated by randomization of surface residues. For further details see Protein Eng. Des. Sel. 17, 455-462 (2004) and EP 1641818A1.

Avimers are multidomain proteins derived from the A-domain scaffold family. The native domains of approximately 35 amino acids adopt a defined disulfide bonded structure. Diversity is generated by shuffling of the natural variation exhibited by the family of A-domains. For further details see Nature Biotechnology 23(12), 1556 - 1561 (2005) and Expert Opinion on Investigational Drugs 16(6), 909-917 (June 2007).

A transfeitin is a monomeric serum transport glycoprotein. Transferring can be engineered to bind different target antigens by insertion of peptide sequences in a permissive surface loop. Examples of engineered transfeitin scaffolds include the Trans-body. For further details see J. Biol. Chem 274, 24066-24073 (1999).

Designed Ankyrin Repeat Proteins (DARPins) are derived from Ankyrin which is a family of proteins that mediate attachment of integral membrane proteins to the cytoskeleton. A single ankyrin repeat is a 33 residue motif consisting of two alpha-helices and a beta-turn. They can be engineered to bind different target antigens by randomizing residues in the first alpha-helix and a beta-turn of each repeat. Their binding interface can be increased by increasing the number of modules (a method of affinity maturation). For further details see J. Mol. Biol. 332, 489-503 (2003), PNAS 100(4), 1700-1705 (2003) and J. Mol. Biol. 369, 1015-1028 (2007) and US20040132028A1.

A single-domain antibody is an antibody fragment consisting of a single monomeric variable antibody domain. The first single domains were derived from the variable domain of the antibody heavy chain from camelids (nanobodies or $V_HH$ fragments). Furthermore, the term single-domain antibody includes an autonomous human heavy chain variable domain (aVH) or $V_{NAR}$ fragments derived from sharks.

Fibronectin is a scaffold which can be engineered to bind to antigen. Adnectins consists of a backbone of the natural amino acid sequence of the 10th domain of the 15 repeating units of human fibronectin type III (FN3). Three loops at one end of the .beta.-sandwich can be engineered to enable an Adnectin to specifically recognize a therapeutic target of interest. For further details see Protein Eng. Des. Sel. 18, 435- 444 (2005), US20080139791, WO2005056764 and US6818418B1.

Peptide aptamers are combinatorial recognition molecules that consist of a constant scaffold protein, typically thioredoxin (TrxA) which contains a constrained variable peptide loop inserted at the active site. For further details see Expert Opin. Biol. Ther. 5, 783-797 (2005).

Microbodies are derived from naturally occurring microproteins of 25-50 amino acids in length which contain 3-4 cysteine bridges - examples of microproteins include KalataBI and conotoxin and knottins. The microproteins have a loop which can beengineered to include upto 25 amino acids without affecting the overall fold of the microprotein. For further details of engineered knottin domains, see WO2008098796.

An **"antigen binding molecule that binds to the same epitope"** as a reference molecule refers to an antigen binding molecule that blocks binding of the reference molecule to its antigen in a competition assay by 50% or more, and conversely, the reference molecule blocks binding of the antigen binding molecule to its antigen in a competition assay by 50% or more.

The term **"antigen binding domain"** refers to the part of an antigen binding molecule that comprises the area which specifically binds to and is complementary to part or all of an antigen. Where an antigen is large, an antigen binding molecule may only bind to a particular part of the antigen, which part is termed an epitope. An antigen binding domain may be provided by, for example, one or more variable domains (also called variable regions). Preferably, an antigen binding domain comprises an antibody light chain variable region (VL) and an antibody heavy chain variable region (VH).

As used herein, the term **"antigenic determinant"** is synonymous with "antigen" and "epitope," and refers to a site (e.g. a contiguous stretch of amino acids or a conformational configuration made up of different regions of non-contiguous amino acids) on a polypeptide macromolecule to which an antigen binding moiety binds, forming an antigen binding moiety-antigen complex. Useful antigenic determinants can be found, for example, on the surfaces of tumor cells, on the surfaces of virus-infected cells, on the surfaces of other diseased cells, on the surface of immune cells, free in blood serum, and/or in the extracellular matrix (ECM). The proteins useful as antigens herein can be any native form the proteins from any vertebrate source, including mammals such as primates (e.g. humans) and rodents (e.g. mice and rats), unless otherwise indicated. In a particular embodiment the antigen is a human protein. Where reference is made to a specific protein herein, the term encompasses the "full-length", unprocessed protein as well as any form of the protein that results from processing in the cell. The term also encompasses naturally occurring variants of the protein, e.g. splice variants or allelic variants.

By **"specific binding"** is meant that the binding is selective for the antigen and can be discriminated from unwanted or non-specific interactions. The ability of an antigen binding molecule to bind to a specific antigen can be measured either through an enzyme-linked immunosorbent assay (ELISA) or other techniques familiar to one of skill in the art, e.g. Surface Plasmon Resonance (SPR) technique (analyzed on a BIAcore instrument) (Liljeblad et al., Glyco J 17, 323-329 (2000)), and traditional binding assays (Heeley, Endocr Res 28, 217-229 (2002)). In one embodiment, the extent of binding of an antigen binding molecule to an unrelated protein is less than about 10% of the binding of the antigen binding molecule to the antigen as measured, e.g. by SPR. In certain embodiments, an molecule that binds to the antigen has a dissociation constant (Kd) of $\leq 1$ μM, $\leq 100$ nM, $\leq 10$ nM, $\leq 1$ nM, $\leq 0.1$ nM, $\leq 0.01$ nM, or $\leq 0.001$ nM (e.g. $10^{-8}$ M or less, e.g. from $10^{-8}$ M to $10^{-13}$ M, e.g. from $10^{-9}$ M to $10^{-13}$ M).

**"Affinity"** or "binding affinity" refers to the strength of the sum total of non-covalent interactions between a single binding site of a molecule (e.g. an antibody) and its binding partner (e.g. an antigen). Unless indicated otherwise, as used herein, "binding affinity" refers to intrinsic binding affinity which reflects a 1:1 interaction between members of a binding pair (e.g. antibody and antigen). The affinity of a molecule X for its partner Y can generally be represented by the dissociation constant (Kd), which is the ratio of dissociation and association rate constants (koff and kon, respectively). Thus, equivalent affinities may comprise different rate constants, as long as the ratio of the rate constants remains the same. Affinity can be measured by common methods known in the art, including those described herein. A particular method for measuring affinity is Surface Plasmon Resonance (SPR).

A **"target cell antigen"** as used herein refers to an antigenic determinant presented on the surface of a target cell, for example a cell in a tumor such as a cancer cell or a cell of the tumor stroma. In certain embodiments, the target cell antigen is an antigen on the surface of a tumor cell. In one embodiment, target cell antigen is selected from the group consisting of Fibroblast Activation Protein (FAP), Carcinoembryonic Antigen (CEA), Melanoma-associated Chondroitin Sulfate Proteoglycan (MCSP), Epidermal Growth Factor Receptor (EGFR), CD19, CD20 and CD33. In particular, the target cell antigen is Fibroblast Activation Protein (FAP).

The term **"Fibroblast activation protein (FAP)",** also known as Prolyl endopeptidase FAP or Seprase (EC 3.4.21), refers to any native FAP from any vertebrate source, including mammals such as primates (e.g. humans) non-human primates (e.g. cynomolgus monkeys) and rodents (e.g. mice and rats), unless otherwise indicated. The term encompasses "full-length," unprocessed FAP as well as any form of FAP that results from processing in the cell. The term also encompasses naturally occurring variants of FAP, e.g., splice variants or allelic variants. In one embodiment, the antigen binding molecule of the invention is capable of specific binding to human, mouse and/or cynomolgus FAP. The amino acid sequence of human FAP is shown in UniProt (www.uniprot.org) accession no. Q12884 (version 149, SEQ ID NO:30), or NCBI (www.ncbi.nlm.nih.gov/) RefSeq NP_004451.2. The extracellular domain (ECD) of human FAP extends from amino acid position 26 to 760. The amino acid and nucleotide sequences of a His-tagged human FAP ECD is shown in SEQ ID NOs 31 and 32, respectively. The amino acid sequence of mouse FAP is shown in UniProt accession no. P97321 (version 126, SEQ ID NO:33), or NCBI RefSeq NP_032012.1. The extracellular domain (ECD) of mouse FAP extends from amino acid position 26 to 761. SEQ ID NOs 34 and 35 show the amino acid and nucleotide sequences, respectively, of a His-tagged mouse FAP ECD. SEQ ID NOs 36 and 37 show the amino acid and nucleotide sequences, respectively, of a His-tagged cynomolgus FAP ECD. Preferably, an anti-FAP binding molecule of the invention binds to the extracellular domain of FAP. Exemplary anti-FAP binding molecules are described in International Patent Application No. WO 2012/020006 A2.

The term **"Carcinoembroynic antigen (CEA)",** also known as Carcinoembryonic antigen-related cell adhesion molecule 5 (CEACAM5), refers to any native CEA from any vertebrate source, including mammals such as primates (e.g. humans) non-human primates (e.g. cynomolgus monkeys) and rodents (e.g. mice and rats), unless otherwise indicated. The amino acid sequence of human CEA is shown in UniProt accession no. P06731 (version 151, SEQ ID NO:38). CEA has long been identified as a tumor-associated antigen (Gold and Freedman, J Exp Med., 121:439-462, 1965; Berinstein N. L., J Clin Oncol., 20:2197-2207, 2002). Originally classified as a protein expressed only in fetal tissue, CEA has now been identified in several normal adult tissues. These tissues are primarily epithelial in origin, including cells of the gastrointestinal, respiratory, and urogential tracts, and cells of colon, cervix, sweat glands, and prostate (Nap et al., Tumour Biol., 9(2-3):145-53, 1988; Nap et al., Cancer Res., 52(8):2329-23339, 1992). Tumors of epithelial origin, as well as their metastases, contain CEA as a tumor associated antigen. While the presence of CEA itself does not indicate transformation to a cancerous cell, the distribution of CEA is indicative. In normal tissue, CEA is generally expressed on the apical surface of the cell (Hammarström S., Semin Cancer Biol. 9(2):67-81 (1999)), making it inaccessible to antibody in the blood stream. In contrast to normal tissue, CEA tends to be expressed over the entire surface of cancerous cells (Hammarström S., Semin Cancer Biol. 9(2):67-81 (1999)). This change of expression pattern makes CEA accessible to antibody binding in cancerous cells. In addition, CEA expression increases in cancerous cells. Furthermore, increased CEA expression promotes increased intercellular adhesions, which may lead to metastasis (Marshall J., Semin Oncol., 30(a Suppl. 8):30-6, 2003). The prevalence of CEA expression in various tumor entities is generally very high. In concordance with published data, own analyses performed in tissue samples confirmed its high prevalence, with approximately 95% in colorectal carcinoma (CRC), 90% in pancreatic cancer, 80% in gastric cancer, 60% in non-small cell lung cancer (NSCLC, where it is co-expressed with HER3), and 40% in breast cancer; low expression was found in small cell lung cancer and glioblastoma.

CEA is readily cleaved from the cell surface and shed into the blood stream from tumors, either directly or via the lymphatics. Because of this property, the level of serum CEA has been used as a clinical marker for diagnosis of cancers and screening for recurrence of cancers, particularly colorectal cancer (Goldenberg D M., The International Journal of Biological Markers, 7:183-188, 1992; Chau I., et al., J Clin Oncol., 22:1420-1429, 2004; Flamini et al., Clin Cancer Res; 12(23):6985-6988, 2006).

The term **"Melanoma-associated Chondroitin Sulfate Proteoglycan (MCSP)",** also known as Chondroitin Sulfate Proteoglycan 4 (CSPG4) refers to any native MCSP from any vertebrate source, including mammals such as primates (e.g. humans) non-human primates (e.g. cynomolgus monkeys) and rodents (e.g. mice and rats), unless otherwise indicated. The amino acid sequence of human MCSP is shown in UniProt accession no. Q6UVK1 (version 103, SEQ ID NO:39). The term **"Epidermal Growth Factor Receptor (EGFR)",** also named Proto-oncogene c-ErbB-1 or Receptor tyrosine-protein kinase erbB-1, refers to any native EGFR from any vertebrate source, including mammals such as primates (e.g. humans) non-human primates (e.g. cynomolgus monkeys) and rodents (e.g. mice and rats), unless otherwise indicated. The amino acid sequence of human EGFR is shown in UniProt accession no. P00533 (version 211, SEQ ID NO:40).

The term **"CD19"** refers to B-lymphocyte antigen CD19, also known as B-lymphocyte surface antigen B4 or T-cell surface antigen Leu-12 and includes any native CD19 from any vertebrate source, including mammals such as primates (e.g. humans) non-human primates (e.g. cynomolgus monkeys) and rodents (e.g. mice and rats), unless otherwise indicated. The amino acid sequence of human CD19 is shown in Uniprot accession no. P15391 (version 160, SEQ ID NO:41). The term encompasses "full-length" unprocessed human CD19 as well as any form of human CD19 that results from processing in the cell as long as the antibody as reported herein binds thereto. CD19 is a structurally distinct cell surface receptor expressed on the surface of human B cells, including, but not limited to, pre-B cells, B cells in early development {i.e., immature B cells), mature B cells through terminal differentiation into plasma cells, and malignant B cells. CD19 is expressed by most pre-B acute lymphoblastic leukemias (ALL), non-Hodgkin's lymphomas, B cell chronic lymphocytic leukemias (CLL), pro-lymphocytic leukemias, hairy cell leukemias, common acute lymphocytic leukemias, and some Null-acute lymphoblastic leukemias. The expression of CD19 on plasma cells further suggests it may be expressed on differentiated B cell tumors such as multiple myeloma. Therefore, the CD19 antigen is a target for immunotherapy in the treatment of non-Hodgkin's lymphoma, chronic lymphocytic leukemia and/or acute lymphoblastic leukemia.

**"CD20"** refers to B-lymphocyte antigen CD20, also known as membrane-spanning 4-domains subfamily A member 1 (MS4A1), B-lymphocyte surface antigen B1 or Leukocyte surface antigen Leu-16, and includes any native CD20 from any vertebrate source, including mammals such as primates (e.g. humans) non-human primates (e.g. cynomolgus monkeys) and rodents (e.g. mice and rats), unless otherwise indicated. The amino acid sequence of human CD20 is shown in Uniprot accession no. P11836 (version 149, SEQ ID NO:42). **"CD33"** refers to Myeloid cell surface antigen CD33, also known as SIGLEC3 or gp67, and includes any native CD33 from any vertebrate source, including mammals such as primates (e.g. humans) non-human primates (e.g. cynomolgus monkeys) and rodents (e.g. mice and rats), unless otherwise indicated. The amino acid sequence of human CD33 is shown in Uniprot accession no. P20138 (version 157, SEQ ID NO:43).

The term **"variable region"** or "variable domain" refers to the domain of an antibody heavy or light chain that is involved in binding the antigen binding molecule to antigen. The variable domains of the heavy chain and light chain (VH and VL, respectively) of a native antibody generally have similar structures, with each domain comprising four conserved framework regions (FRs) and three hypervariable regions (HVRs). See, e.g., Kindt et al., Kuby Immunology, 6th ed., W.H. Freeman and Co., page 91 (2007). A single VH or VL domain may be sufficient to confer antigen-binding specificity.

The term **"hypervariable region"** or "HVR," as used herein refers to each of the regions of an antibody variable domain which are hypervariable in sequence and/or form structurally defined loops ("hypervariable loops"). Generally, native four-chain antibodies comprise six HVRs; three in the VH (H1, H2, H3), and three in the VL (L1, L2, L3). HVRs generally comprise amino acid residues from the hypervariable loops and/or from the "complementarity determining regions" (CDRs), the latter being of highest sequence variability and/or involved in antigen recognition. Exemplary hypervariable loops occur at amino acid residues 26-32 (LI), 50-52 (L2), 91-96 (L3), 26-32 (H1), 53-55 (H2), and 96-101 (H3). (Chothia and Lesk, J. Mol. Biol. 196:901-917 (1987).) Exemplary CDRs (CDR-L1, CDR-L2, CDR-L3, CDR-H1, CDR-H2, and CDR-H3) occur at amino acid residues 24-34 of L1, 50-56 of L2, 89-97 of L3, 31-35B of H1, 50-65 of H2, and 95-102 of H3. (Kabat et al., Sequences of Proteins of Immunological Interest, 5th Ed. Public Health Service, National Institutes of Health, Bethesda, MD (1991).) Hypervariable regions (HVRs) are also referred to as complementarity determining regions (CDRs), and these terms are used herein interchangeably in reference to portions of the variable region that form the antigen binding regions. This particular region has been described by Kabat et al., U.S. Dept. of Health and Human Services, "Sequences of Proteins of Immunological Interest" (1983) and by Chothia et al., J. Mol. Biol. 196:901-917 (1987), where the definitions include overlapping or subsets of amino acid residues when compared against each other. Nevertheless, application of either definition to refer to a CDR of an antibody or variants thereof is intended to be within the scope of the term as defined and used herein. The appropriate amino acid residues which encompass the CDRs as defined by each of the above cited references are set forth below in Table A as a comparison. The exact residue numbers which encompass a particular CDR will vary depending on the sequence and size of the CDR. Those skilled in the art can routinely determine which residues comprise a particular CDR given the variable region amino acid sequence of the antibody

TABLE A. CDR Definitions[1]

| CDR | Kabat | Chothia | AbM[2] |
|---|---|---|---|
| $V_H$ CDR1 | 31-35 | 26-32 | 26-35 |
| $V_H$ CDR2 | 50-65 | 52-58 | 50-58 |
| $V_H$ CDR3 | 95-102 | 95-102 | 95-102 |
| $V_L$ CDR 1 | 24-34 | 26-32 | 24-34 |
| $V_L$ CDR2 | 50-56 | 50-52 | 50-56 |
| $V_L$ CDR3 | 89-97 | 91-96 | 89-97 |

[1] Numbeung of all CDR definitions in Table A is according to the numbering conventions set forth by Kabat et al. (see below).
[2] "AbM" with a lowercase "b" as used in Table A refers to the CDRs as defined by Oxford Molecular's "AbM" antibody modeling software.

Kabat *et al.* also defined a numbering system for variable region sequences that is applicable to any antibody. One of ordinary skill in the art can unambiguously assign this system of "Kabat numbering" to any variable region sequence, without reliance on any experimental data beyond the sequence itself. As used herein, "Kabat numbering" refers to the numbering system set forth by Kabat et al., U.S. Dept. of Health and Human Services, "Sequence of Proteins of Immunological Interest" (1983). Unless otherwise specified, references to the numbering of specific amino acid residue positions in an antibody variable region are according to the Kabat numbering system.

With the exception of CDR1 in VH, CDRs generally comprise the amino acid residues that form the hypervariable loops. CDRs also comprise "specificity determining residues," or "SDRs," which are residues that contact antigen. SDRs are contained within regions of the CDRs called abbreviated-CDRs, or a-CDRs. Exemplary a-CDRs (a-CDR-L1, a-CDR-L2, a-CDR-L3, a-CDR-H1, a-CDR-H2, and a-CDR-H3) occur at amino acid residues 31-34 of L1, 50-55 of L2, 89-96 of L3, 31-35B of H1, 50-58 of H2, and 95-102 of H3. (See Almagro and Fransson, Front. Biosci. 13:1619-1633 (2008).) Unless otherwise indicated, HVR residues and other residues in the variable domain (e.g., FR residues) are numbered herein according to Kabat et al., *supra.*

As used herein, the term **"affinity matured"** in the context of antigen binding molecules (e.g., antibodies) refers to an antigen binding molecule that is derived from a reference antigen binding molecule, e.g., by mutation, binds to the same antigen, preferably binds to the same epitope, as the reference antibody; and has a higher affinity for the antigen than that of the reference antigen binding molecule. Affinity maturation generally involves modification of one or more amino acid residues in one or more CDRs of the antigen binding molecule. Typically, the affinity matured antigen binding molecule binds to the same epitope as the initial reference antigen binding molecule.

**"Framework"** or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following sequence in VH (or VL): FR1-H1(L1)-FR2-H2(L2)-FR3-H3(L3)-FR4.

An **"acceptor human framework"** for the purposes herein is a framework comprising the amino acid sequence of a light chain variable domain (VL) framework or a heavy chain variable domain (VH) framework derived from a human immunoglobulin framework or a human consensus framework, as defined below. An acceptor human framework "derived from" a human immunoglobulin framework or a human consensus framework may comprise the same amino acid sequence thereof, or it may contain amino acid sequence changes. In some embodiments, the number of amino acid changes are 10 or less, 9 or less, 8 or less, 7 or less, 6 or less, 5 or less, 4 or less, 3 or less, or 2 or less. In some embodiments, the VL acceptor human framework is identical in sequence to the VL human immunoglobulin framework sequence or human consensus framework sequence.

The term **"chimeric"** antibody refers to an antibody in which a portion of the heavy and/or light chain is derived from a particular source or species, while the remainder of the heavy and/or light chain is derived from a different source or species.

The **"class"** of an antibody refers to the type of constant domain or constant region possessed by its heavy chain. There are five major classes of antibodies: IgA, IgD, IgE, IgG, and IgM, and several of these may be further divided into subclasses (isotypes), e.g. $IgG_1$, $IgG_2$, $IgG_3$, $IgG_4$, $IgA_1$, and $IgG_2$. The heavy chain constant domains that correspond to the different classes of immunoglobulins are called $\alpha$, $\delta$, $\varepsilon$, $\gamma$, and $\mu$ respectively..

A **"humanized"** antibody refers to a chimeric antibody comprising amino acid residues from non-human HVRs and amino acid residues from human FRs. In certain embodiments, a humanized antibody will comprise substantially all of at least one, and typically two, variable domains, in which all or substantially all of the HVRs (e.g., CDRs) correspond to those of a non-human antibody, and all or substantially all of the FRs correspond to those of a human antibody. A humanized antibody optionally may comprise at least a portion of an antibody constant region derived from a human antibody. A **"humanized form"** of an antibody, e.g., a non-human antibody, refers to an antibody that has undergone humanization. Other forms of "humanized antibodies" encompassed by the present invention are those in which the constant region has been additionally modified or changed from that of the original antibody to generate the properties according to the invention, especially in regard to C1q binding and/or Fc receptor (FcR) binding.

A **"human"** antibody is one which possesses an amino acid sequence which corresponds to that of an antibody produced by a human or a human cell or derived from a non-human source that utilizes human antibody repertoires or other human antibody-encoding sequences. This definition of a human antibody specifically excludes a humanized antibody comprising non-human antigen-binding residues.

The term "Fc domain" or **"Fc region"** herein is used to define a C-terminal region of an antibody heavy chain that contains at least a portion of the constant region. The term includes native sequence Fc regions and variant Fc regions. An IgG Fc region comprises an IgG CH2 and an IgG CH3 domain. The "CH2 domain" of a human IgG Fc region usually extends from an amino acid residue at about position 231 to an amino acid residue at about position 340. In one embodiment, a carbohydrate chain is attached to the CH2 domain. The CH2 domain herein may be a native sequence CH2 domain or variant CH2 domain. The "CH3 domain" comprises the stretch of residues C-terminal to a CH2 domain in an Fc region (i.e. from an amino acid residue at about position 341 to an amino acid residue at about position 447 of an IgG). The CH3 region herein may be a native sequence CH3 domain or a variant CH3 domain (e.g. a CH3 domain with an introduced "protuberance" ("knob") in one chain thereof and a corresponding introduced "cavity" ("hole") in the other chain thereof; see US Patent No. 5,821,333, expressly incorporated herein by reference). Such variant CH3 domains may be used to promote heterodimerization of two non-identical antibody heavy chains as herein described. In one embodiment, a human IgG heavy chain Fc region extends from Cys226, or from Pro230, to the carboxyl-terminus of the heavy chain. However, the C-terminal lysine (Lys447) of the Fc region may or may not be present. Unless otherwise specified herein, numbering of amino acid residues in the Fc region or constant region is according to the EU numbering system, also called the EU index, as described in Kabat et al., Sequences of Proteins of Immunological Interest, 5th Ed. Public Health Service, National Institutes of Health, Bethesda, MD, 1991.

The term **"TNF ligand family member"** or "TNF family ligand" refers to a proinflammatory cytokine. Cytokines in general, and in particular the members of the TNF ligand family, play a crucial role in the stimulation and coordination of the immune system. At present, nineteen cytokines have been identified as members of the TNF (tumour necrosis factor) ligand superfamily on the basis of sequence, functional, and structural similarities. All these ligands are type II transmembrane proteins with a C-terminal extracellular domain (ectodomain), N-terminal intracellular domain and a single transmembrane domain. The C-terminal extracellular domain, known as TNF homology domain (THD), has 20-30% amino acid identity between the superfamily members and is responsible for binding to the receptor. The TNF ectodomain is also responsible for the TNF ligands to form trimeric complexes that are recognized by their specific receptors. Members of the TNF ligand family are selected from the group consisting of Lymphotoxin $\alpha$ (also known as LTA or TNFSF1), TNF (also known as TNFSF2), LT$\beta$ (also known as TNFSF3), OX40L (also known as TNFSF4), CD40L (also known as CD154 or TNFSF5), FasL (also known as CD95L, CD178 or TNFSF6), CD27L (also known as CD70 or TNFSF7), CD30L (also known as CD153 or TNFSF8), 4-1BBL (also known as TNFSF9), TRAIL (also known as APO2L, CD253 or TNFSF10), RANKL (also known as CD254 or TNFSF11), TWEAK (also known as TNFSF12), APRIL (also known as CD256 or TNFSF13), BAFF (also known as CD257 or TNFSF13B), LIGHT (also known as CD258 or TNFSF14), TL1A (also known as VEGI or TNFSF15), GITRL (also known as TNFSF18), EDA-A1 (also known as ectodysplasin A1) and EDA-A2 (also known as ectodysplasin A2). The term refers to any native TNF family ligand from any vertebrate source, including mammals such as primates (e.g. humans), non-human primates (e.g. cynomolgus monkeys) and rodents (e.g. mice and rats), unless otherwise indicated.

The term **"costimulatory TNF ligand family member"** or "costimulatory TNF family ligand" refers to a subgroup of TNF ligand family members, which are able to costimulate proliferation and cytokine production of T-cells. These TNF family ligands can costimulate TCR signals upon interaction with their corresponding TNF receptors and the interaction with their receptors leads to recruitment of TNFR-associated factors (TRAF), which initiate signalling cascades that result in T-cell activation. Costimulatory TNF family ligands are selected from the group consisting of 4-1BBL, OX40L, GITRL, CD70, CD30L and LIGHT, more particularly the costimulatory TNF ligand family member is selected from 4-1BBL and OX40L.

Further information, in particular sequences, of the costimulatory TNF ligand family members may be obtained from publically accessible databases such as Uniprot (www.uniprot.org). For instance, the human TNF ligands have the following amino acid sequences: human OX40L (UniProt accession no. P23510, SEQ ID NO:44), human CD27L (CD70, UniProt accession no. P32970, SEQ ID NO:45), human CD30L (UniProt accession no. P32971, SEQ ID NO:46), 4-1BBL (UniProt accession no. P41273, SEQ ID NO:47), LIGHT (UniProt accession no. 043557, SEQ ID NO:48), and GITRL (UniProt accession no. Q9UNG2, SEQ ID NO:49).

An **"ectodomain"** is the domain of a membrane protein that extends into the extracellular space (i.e. the space outside the target cell). Ectodomains are usually the parts of proteins that initiate contact with surfaces, which leads to signal transduction. The ectodomain of TNF ligand family member as defined herein thus refers to the part of the TNF ligand protein that extends into the extracellular space (the extracellular domain), but also includes shorter parts or fragments thereof that are responsible for the trimerization and for the binding to the corresponding TNF receptor. The term "ectodomain of a TNF ligand family member or a fragment thereof" thus refers to the extracellular domain of the TNF ligand family member that forms the extracellular domain or to parts thereof that are still able to bind to the receptor (receptor binding domain).

As described herein before, 4-1BBL is a type II transmembrane protein and one member of the TNF ligand family. Complete or full length 4-1BBL having the amino acid sequence of SEQ ID NO:47 has been described to form trimers on the surface of cells. The formation of trimers is enabled by specific motives of the ectodomain of 4-1BBL. Said motives are designated herein as "trimerization region". The amino acids 50-254 of the human 4-1BBL sequence (SEQ ID NO:50) form the extracellular domain of 4-1BBL, but even fragments thereof are able to form the trimers. In specific embodiments of the invention, the term "ectodomain of 4-1BBL or a fragment thereof' refers to a polypeptide having an amino acid sequence selected from SEQ ID NO: 6 (amino acids 52-254 of human 4-1BBL), SEQ ID NO:3 (amino acids 71-254 of human 4-1BBL), SEQ ID NO:5 (amino acids 80-254 of human 4-1BBL) and SEQ ID NO:4 (amino acids 85-254 of human 4-1BBL) or a polypeptide having an amino acid sequence selected from SEQ ID NO:7 (amino acids 71-248 of human 4-1BBL), SEQ ID NO:10 (amino acids 52-248 of human 4-1BBL), SEQ ID NO:9 (amino acids 80-248 of human 4-1BBL) and SEQ ID NO:8 (amino acids 85-248 of human 4-1BBL), but also other fragments of the ectodomain capable of trimerization are included herein.

As described herein before, OX40L is another type II transmembrane protein and a further member of the TNF ligand family. Complete or full length human OX40L has the amino acid sequence of SEQ ID NO:44. The amino acids 51-183 of the human OX40L sequence (SEQ ID NO: 11) form the extracellular domain of OX40L, but even fragments thereof that are able to form the trimers. In specific embodiments of the invention, the term "ectodomain of OX40L or a fragment thereof' refers to a polypeptide having an amino acid sequence selected from SEQ ID NO:11 (amino acids 51-183 of human OX40L) or SEQ ID NO:13 (amino acids 52-183 of human OX40L), but also other fragments of the ectodomain capable of trimerization are included herein.

The term **"peptide linker"** refers to a peptide comprising one or more amino acids, typically about 2 to 20 amino acids. Peptide linkers are known in the art or are described herein. Suitable, non-immunogenic linker peptides are, for example, $(G_4S)_n$, $(SG_4)_n$ or $G_4(SG_4)_n$ peptide linkers, wherein "n" is generally a number between 1 and 10, typically between 1 and 4, in particular 2, i.e. the peptides selected from the group consisting of GGGGS (SEQ ID NO:51), GGGGSGGGGS (SEQ ID NO:52), SGGGGSGGGG (SEQ ID NO:53), $(G_4S)_3$ or GGGGSGGGGSGGGGS (SEQ ID NO:54), GGGGSGGGGSGGGG or G4(SG4)$_2$ (SEQ ID NO:55), and $(G_4S)_4$ or GGGGSGGGGSGGGGSGGGGS (SEQ

ID NO:56), but also include the sequences GSPGSSSSGS (SEQ ID NO:57), GSGSGSGS (SEQ ID NO:58), GSGSGNGS (SEQ ID NO:59), GGSGSGSG (SEQ ID NO:60), GGSGSG (SEQ ID NO:61), GGSG (SEQ ID NO:62), GGSGNGSG (SEQ ID NO:63), GGNGSGSG (SEQ ID NO:64) and GGNGSG (SEQ ID N0:65). Peptide linkers of particular interest are $(G4S)_1$ or GGGGS (SEQ ID NO:51), $(G_4S)_2$ or GGGGSGGGGS (SEQ ID NO:52), $(G_4S)_3$ or GGGGSGGGGSGGGGS (SEQ ID NO:54) and $(G_4S)_4$ or GGGGSGGGGSGGGGSGGGGS (SEQ ID NO:56).

The term **"amino acid"** as used within this application denotes the group of naturally occurring carboxy $\alpha$-amino acids comprising alanine (three letter code: ala, one letter code: A), arginine (arg, R), asparagine (asn, N), aspartic acid (asp, D), cysteine (cys, C), glutamine (gln, Q), glutamic acid (glu, E), glycine (gly, G), histidine (his, H), isoleucine (ile, I), leucine (leu, L), lysine (lys, K), methionine (met, M), phenylalanine (phe, F), proline (pro, P), serine (ser, S), threonine (thr, T), tryptophan (trp, W), tyrosine (tyr, Y), and valine (val, V).

A **"fusion polypeptide"** as used herein refers to a single chain polypeptide composed of an ectodomain of a TNF ligand family member fused to a trimerization domain. In addition, the fusion polypeptide may further comprise a moiety capable of specific binding to a target cell antigen, in particular a Fab fragment, more particularly the CH1 and VH domain of a Fab fragment. The fusion may occur by directly linking the N or C-terminal amino acid of the antigen binding moiety via a peptide linker to the C- or N-terminal amino acid of the ectodomain of said TNF ligand family member.

By "fused" or "connected" is meant that the components (e.g. a polypeptide and an ectodomain of said TNF ligand family member) are linked by peptide bonds, either directly or via one or more peptide linkers.

The term **"trimerization domain"** refers to an amino acid sequence within a polypeptide that promotes self-assembly by associating with two other trimerization domains to form a trimer. The term is also use to refer to the polynucleotide encoding said amino acid sequence. Typically, the trimerization domain comprises an amino acid sequence able to form an alpha-helicial coiled-coil domain or an isoleucine zipper domain. Suitable trimerization domains include TRAF2 (UniProt accession no. Q12933, SEQ ID NO:66), in particular amino acids 299 to 348 or amino acids 310 to 349), Thrombospondin 1 (UniProt accession no. P07996, SEQ ID NO:67), in particular amino acids 291 to 314), Matrilin-4 (UniProt accession no. 095460, SEQ ID NO:68), in particular amino acids 594 to 618; CMP (matrilin-1) (Uniprot accession No. P21941, SEQ ID NO:1), in particular amino acids 454 to 496, and Cubilin (UniProt accession no. 060494, SEQ ID NO: 69), in particular amino acids 104 to 138. An exemplary isoleucine zipper domain is the engineered yeast GCN4 isoleucine variant described by Harbury et al. (1993) Science 262, 1401-1407 comprising the amino acid sequence of SEQ ID NO:70.

A particular trimerization domain is **"huCMP"** or **"CMP"** or "human cartilage matrix protein", a protein that is also known as matulin-1 or MATN1 or CRTM (UniProt accession no. P21941, SEQ ID NO:1). The term "huCMP trimerization domain" or "trimerization domain derived from human cartilage matrix protein (CMP)" refers to a polypeptide structure capable of associating with two similar or identical polypeptides to form a stable trimer.The trimerisation is mediated through ionic bonds and other non-covalent bonds formed between adjacent charged amino acids of the polypeptide chains. The huCMP trimerization domain has been been described e.g. in Beck et al (1996), J. Mol. Biol. 256, 909-923. A huCMP trimerization domain of particular interes comprises a sequence having at least 95% identity and most preferably at least 98% identity to SEQ ID NO 2. In one embodiment said trimerization domain comprises the sequence of SEQ ID NO. 2.

**"Percent (%) amino acid sequence identity"** with respect to a reference polypeptide (protein) sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN. SAWI or Megalign (DNASTAR) software. Those skilled in the art can determine appropriate parameters for aligning sequences, including any algorithms needed to achieve maximal alignment over the full length of the sequences being compared. For purposes herein, however, % amino acid sequence identity values are generated using the sequence comparison computer program ALIGN-2. The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087. The ALIGN-2 program is publicly available from Genentech, Inc., South San Francisco, California, or may be compiled from the source code. The ALIGN-2 program should be compiled for use on a UNIX operating system, including digital UNIX V4.0D. All sequence comparison parameters are set by the ALIGN-2 program and do not vary. In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:

$$100 \text{ times the fraction } X/Y$$

where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. Unless specifically stated otherwise, all % amino acid sequence identity values used herein are obtained as described in the immediately preceding paragraph using the ALIGN-2 computer program.

In certain embodiments, **amino acid sequence variants** of the TNF ligand trimer-containing antigen binding molecules provided herein are contemplated. For example, it may be desirable to improve the binding affinity and/or other biological properties of the TNF ligand trimer-containing antigen binding molecules. Amino acid sequence variants of the TNF ligand trimer-containing antigen binding molecules may be prepared by introducing appropriate modifications into the nucleotide sequence encoding the molecules, or by peptide synthesis. Such modifications include, for example, deletions from, and/or insertions into and/or substitutions of residues within the amino acid sequences of the antibody. Any combination of deletion, insertion, and substitution can be made to arrive at the final construct, provided that the final construct possesses the desired characteristics, e.g., antigen-binding. Sites of interest for substitutional mutagenesis include the HVRs and Framework (FRs). Conservative substitutions are provided in Table B under the heading "Preferred Substitutions" and further described below in reference to amino acid side chain classes (1) to (6). Amino acid substitutions may be introduced into the molecule of interest and the products screened for a desired activity, e.g., retained/improved antigen binding, decreased immunogenicity, or improved ADCC or CDC.

TABLE B

| Original Residue | Exemplary Substitutions | Preferred Substitutions |
|---|---|---|
| Ala (A) | Val; Leu; He | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| He (I) | Leu; Val; Met; Ala; Phe; Norleucine | Leu |
| Leu (L) | Norleucine; Ile; Val; Met; Ala; Phe | He |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids may be grouped according to common side-chain properties:

(1) hydrophobic: Norleucine, Met, Ala, Val, Leu, Ile;

(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe.

Non-conservative substitutions will entail exchanging a member of one of these classes for another class.

The term **"amino acid sequence variants"** includes substantial variants wherein there are amino acid substitutions in one or more hypervariable region residues of a parent antigen binding molecule (*e.g.* a humanized or human antibody). Generally, the resulting variant(s) selected for further study will have modifications (e.g., improvements) in certain biological properties (e.g., increased affinity, reduced immunogenicity) relative to the parent antigen binding molecule and/or will have substantially retained certain biological properties of the parent antigen binding molecule. An exemplary substitutional variant is an affinity matured antibody, which may be conveniently generated, e.g., using phage display-based affinity maturation techniques such as those described herein. Briefly, one or more HVR residues are mutated and the variant antigen binding molecules displayed on phage and screened for a particular biological activity (e.g. binding affinity). In certain embodiments, substitutions, insertions, or deletions may occur within one or more HVRs so long as such alterations do not substantially reduce the ability of the antigen binding molecule to bind antigen. For example, conservative alterations (e.g., conservative substitutions as provided herein) that do not substantially reduce binding affinity may be made in HVRs. A useful method for identification of residues or regions of an antibody that may be targeted for mutagenesis is called "alanine scanning mutagenesis" as described by Cunningham and Wells (1989) Science, 244:1081-1085. In this method, a residue or group of target residues (e.g., charged residues such as Arg, Asp, His, Lys, and Glu) are identified and replaced by a neutral or negatively charged amino acid (e.g., alanine or polyalanine) to determine whether the interaction of the antibody with antigen is affected. Further substitutions may be introduced at the amino acid locations demonstrating functional sensitivity to the initial substitutions. Alternatively, or additionally, a crystal structure of an antigen-antigen binding molecule complex to identify contact points between the antibody and antigen. Such contact residues and neighboring residues may be targeted or eliminated as candidates for substitution. Variants may be screened to determine whether they contain the desired properties.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include the fusion to the N- or C-terminus to a polypeptide which increases the serum half-life of the trimeric costimulatory TNF family ligand-containing antigen binding molecules.

In certain aspects, the trimeric costimulatory TNF family ligand-containing antigen binding molecules provided herein are altered to increase or decrease the extent to which the antibody is glycosylated. Glycosylation variants of the molecules may be conveniently obtained by altering the amino acid sequence such that one or more glycosylation sites is created or removed. In certain aspects, the trimeric costimulatory TNF family ligand-containing antigen binding molecules provided herein may be further modified to contain additional non-proteinaceous moieties that are known in the art and readily available. The moieties suitable for derivatization of the antigen binding molecule include but are not limited to water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1, 3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, propropylene glycol homopolymers, prolypropylene oxide/ethylene oxide co-polymers, polyoxyethylated polyols (e.g., glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antibody may vary, and if more than one polymer is attached, they can be the same or different molecules. In general, the number and/or type of polymers used for derivatization can be determined based on considerations including, but not limited to, the particular properties or functions of the antigen binding molecule to be improved.

The term **"polynucleotide"** refers to an isolated nucleic acid molecule or construct, e.g. messenger RNA (mRNA), virally-derived RNA, or plasmid DNA (pDNA). A polynucleotide may comprise a conventional phosphodiester bond or a non-conventional bond (e.g. an amide bond, such as found in peptide nucleic acids (PNA). The term "nucleic acid molecule" refers to any one or more nucleic acid segments, e.g. DNA or RNA fragments, present in a polynucleotide.

By **"isolated"** nucleic acid molecule or polynucleotide is intended a nucleic acid molecule, DNA or RNA, which has been removed from its native environment. For example, a recombinant polynucleotide encoding a polypeptide contained in a vector is considered isolated for the purposes of the present invention. Further examples of an isolated polynucleotide include recombinant polynucleotides maintained in heterologous host cells or purified (partially or substantially) polynucleotides in solution. An isolated polynucleotide includes a polynucleotide molecule contained in cells that ordinarily contain the polynucleotide molecule, but the polynucleotide molecule is present extrachromosomally or at a chromosomal location that is different from its natural chromosomal location. Isolated RNA molecules include in vivo or in vitro RNA transcripts of the present invention, as well as positive and negative strand forms, and double-stranded forms. Isolated polynucleotides or nucleic acids according to the present invention further include such molecules produced synthetically. In addition, a polynucleotide or a nucleic acid may be or may include a regulatory element such as a promoter, ribosome binding site, or a transcription terminator.

By a nucleic acid or polynucleotide having a nucleotide sequence at least, for example, 95% "identical" to a reference nucleotide sequence of the present invention, it is intended that the nucleotide sequence of the polynucleotide is identical to the reference sequence except that the polynucleotide sequence may include up to five point mutations per each 100 nucleotides of the reference nucleotide sequence. In other words, to obtain a polynucleotide having a nucleotide sequence at least 95% identical to a reference nucleotide sequence, up to 5% of the nucleotides in the reference sequence may be deleted or substituted with another nucleotide, or a number of nucleotides up to 5% of the total nucleotides in the reference sequence may be inserted into the reference sequence. These alterations of the reference sequence may occur at the 5' or 3' terminal positions of the reference nucleotide sequence or anywhere between those terminal positions, interspersed either individually among residues in the reference sequence or in one or more contiguous groups within the reference sequence. As a practical matter, whether any particular polynucleotide sequence is at least 80%, 85%, 90%, 95%, 96%, 97%, 98% or 99% identical to a nucleotide sequence of the present invention can be determined conventionally using known computer programs, such as the ones discussed above for polypeptides (e.g. ALIGN-2).

The term **"expression cassette"** refers to a polynucleotide generated recombinantly or synthetically, with a series of specified nucleic acid elements that permit transcription of a particular nucleic acid in a target cell. The recombinant expression cassette can be incorporated into a plasmid, chromosome, mitochondrial DNA, plastid DNA, virus, or nucleic acid fragment. Typically, the recombinant expression cassette portion of an expression vector includes, among other sequences, a nucleic acid sequence to be transcribed and a promoter. In certain embodiments, the expression cassette of the invention comprises polynucleotide sequences that encode bispecific antigen binding molecules of the invention or fragments thereof.

The term **"vector"** or "expression vector" is synonymous with "expression construct" and refers to a DNA molecule that is used to introduce and direct the expression of a specific gene to which it is operably associated in a target cell. The term includes the vector as a self-replicating nucleic acid structure as well as the vector incorporated into the genome of a host cell into which it has been introduced. The expression vector of the present invention comprises an expression cassette. Expression vectors allow transcription of large amounts of stable mRNA. Once the expression vector is inside the target cell, the ribonucleic acid molecule or protein that is encoded by the gene is produced by the cellular transcription and/or translation machinery. In one embodiment, the expression vector of the invention comprises an expression cassette that comprises polynucleotide sequences that encode bispecific antigen binding molecules of the invention or fragments thereof.

The terms **"host cell",** "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein. A host cell is any type of cellular system that can be used to generate the bispecific antigen binding molecules of the present invention. Host cells include cultured cells, e.g. mammalian cultured cells, such as CHO cells, BHK cells, NSO cells, SP2/0 cells, YO myeloma cells, P3X63 mouse myeloma cells, PER cells, PER.C6 cells or hybridoma cells, yeast cells, insect cells, and plant cells, to name only a few, but also cells comprised within a transgenic animal, transgenic plant or cultured plant or animal tissue.

An **"effective amount"** of an agent refers to the amount that is necessary to result in a physiological change in the cell or tissue to which it is administered.

A **"therapeutically effective amount"** of an agent, e.g. a pharmaceutical composition, refers to an amount effective, at dosages and for periods of time necessary, to achieve the desired therapeutic or prophylactic result. A therapeutically effective amount of an agent for example eliminates, decreases, delays, minimizes or prevents adverse effects of a disease.

An **"individual"** or "subject" is a mammal. Mammals include, but are not limited to, domesticated animals (e.g. cows, sheep, cats, dogs, and horses), primates (e.g. humans and non-human primates such as monkeys), rabbits, and rodents (e.g. mice and rats). Particularly, the individual or subject is a human.

The term **"pharmaceutical composition"** refers to a preparation which is in such form as to permit the biological activity of an active ingredient contained therein to be effective, and which contains no additional components which are unacceptably toxic to a subject to which the formulation would be administered.

A **"pharmaceutically acceptable excipient"** refers to an ingredient in a pharmaceutical composition, other than an active ingredient, which is nontoxic to a subject. A pharmaceutically acceptable excipient includes, but is not limited to, a buffer, a stabilizer, or a preservative.

As used herein, **"treatment"** (and grammatical variations thereof such as "treat" or "treating") refers to clinical intervention in an attempt to alter the natural course of the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, the molecules of the invention are used to delay development of a disease or to slow the progression of a disease.

The term **"cancer"** as used herein refers to proliferative diseases, such as lymphomas, lymphocytic leukemias, lung cancer, non-small cell lung (NSCL) cancer, bronchioloalviolar cell lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, gastric cancer, colon cancer, breast cancer, uterine cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, cancer of the bladder, cancer of the kidney or ureter, renal cell carcinoma, carcinoma of the renal pelvis, mesothelioma, hepatocellular cancer, biliary cancer, neoplasms of the central nervous system (CNS), spinal axis tumors, brain stem glioma, glioblastoma multiforme, astrocytomas, schwanomas, ependymonas, medulloblastomas, meningiomas, squamous cell carcinomas, pituitary adenoma and Ewings sarcoma, including refractory versions of any of the above cancers, or a combination of one or more of the above cancers.

**Trimeric costimulatoryTNF family ligand-containing antigen binding molecules of the invention**

The invention provides novel trimeric costimulatory TNF family ligand-containing antigen binding molecules with particularly advantageous properties such as producibility, stability, binding affinity, biological activity, targeting efficiency and reduced toxicity.

The invention relates to a trimeric costimulatory TNF family ligand-containing antigen binding molecule comprising three fusion polypeptides, each of the three fusion polypeptides comprising

(a) an ectodomain of a costimulatory TNF ligand family member or fragments thereof,
(b) a trimerization domain, and
(c) a moiety capable of specific binding to a target cell antigen.

The trimerization domain can be derived from a polypeptide derived from a protein selected from the group consisting of human TRAF2, human thrombospondin 1, human matrilin-4, human cartilage matrix protein (huCMP) and human cubilin. It can also be an isoleucine zipper domain, for example the peptide with the amino acid sequence of SEQ ID NO:70.

In one aspect, the invention provides a trimeric costimulatory TNF family ligand-containing antigen binding molecule comprising three fusion polypeptides, each of the three fusion polypeptides comprising

(a) an ectodomain of a costimulatory TNF ligand family member or fragments thereof,
(b) a trimerization domain derived from human cartilage matrix protein (huCMP) of amino acid sequence of SEQ ID NO:1, and
(c) a moiety capable of specific binding to a target cell antigen.

The trimerization domain derived from human cartilage matrix protein (huCMP) comprises at least a part of SEQ ID NO.: 1. In one aspect, the trimerization domain comprises an amino acid sequence having at least 95% identity and most preferably at least 98% identity to SEQ ID NO:2. More particularly, the trimerization domain comprises the amino acid sequence of SEQ ID NO:2. The trimerization domain derived from human cartilage matrix protein (CMP) is herein further referred to as "huCMP trimerization domain".

In a particular aspect, the trimeric costimulatory TNF family ligand-containing antigen binding molecule comprises three fusion polypeptides, each of the three fusion polypeptides comprising a trimerization domain comprising an amino acid sequence having at least 95% identity and most preferably at least 98% identity to SEQ ID NO:2. More particularly, each of the three fusion polypeptides comprises one trimerization domain comprising the amino acid sequence of SEQ ID NO:2.

In particular, the costimulatory TNF family ligand member is selected from the group consisting of 4-1BBL, OX40L, GITRL, CD70, CD30L and LIGHT, more particularly the costimulatory TNF family ligand member is selected from 4-1BBL and OX40L.

In one aspect, the costimulatory TNF ligand family member is 4-1BBL. In particular, the trimeric costimulatory TNF family **ligand-containing** antigen binding molecule comprises three fusion polypeptides, wherein in each of the three fusion polypeptides the ectodomain of a TNF ligand family member comprises the amino acid sequence selected from the group consisting of SEQ ID NO:3, SEQ ID NO:4, SEQ ID NO:5, SEQ ID NO:6, SEQ ID NO:7, SEQ ID NO:8, SEQ ID NO:9 and SEQ ID NO: 10, particularly the amino acid sequence of SEQ ID NO:3 or SEQ ID NO:7. In a further aspect, the trimeric costimulatory TNF family ligand-containing antigen binding molecule comprises three fusion polypeptides, each of the three fusion polypeptides comprising (a) an ectodomain of a TNF ligand family member comprising the amino acid sequence selected from the group consisting of SEQ ID NO:3, SEQ ID NO:4, SEQ ID NO:5, SEQ ID NO:6, SEQ ID NO:7, SEQ ID NO:8, SEQ ID NO:9 and SEQ ID NO: 10, (b) a trimerization domain comprising the amino acid sequence of SEQ ID NO:2 and (c) a moiety capable of specific binding to a target cell antigen.

In another aspect, the costimulatory TNF ligand family member is OX40L. In particular, the trimeric costimulatory TNF family ligand-containing antigen binding molecule comprises three fusion polypeptides, wherein in each of the three fusion polypeptides the ectodomain of a TNF ligand family member comprises the amino acid sequence selected from the group consisting of SEQ ID NO: 11, SEQ ID NO: 12 and SEQ ID NO: 13. In a further aspect, the trimeric costimulatory TNF family **ligand-containing** antigen binding molecule comprises three fusion polypeptides, wherein each of the three fusion polypeptides comprises (a) an ectodomain of a TNF ligand family member comprising the amino acid sequence selected from the group consisting of SEQ ID NO: 11, SEQ ID NO: 12 and SEQ ID NO: 13, (b) a trimerization domain comprising the amino acid sequence of SEQ ID NO:2 and (c) a moiety capable of specific binding to a target cell antigen.

In another aspect, provided is a trimeric costimulatory TNF family ligand-containing antigen binding molecule comprising three fusion polypeptides as described before, wherein the ectodomain of the TNF ligand family member or a fragment thereof is fused at the N-terminal amino acid to the C-terminal amino acid of the trimerization domain, optionally through a peptide linker. The fusion can be a direct bond between the ectodomain of the TNF ligand family member and the trimerization domain, or the ectodomain of the TNF ligand family member and the trimerization domain are connected through a peptide linker. In a particular aspect, the ectodomain of the TNF ligand family member and the trimerization domain are connected through a peptide linker.

Typically, the peptide linker is a peptide comprising 2 to 20 amino acids. In particular, the peptide linker is a peptide selected from the group consisting of GGGGS (SEQ ID NO:51), GGGGSGGGGS (SEQ ID NO:52), SGGGGSGGGG (SEQ ID NO:53), $(G_4S)_3$ or GGGGSGGGGSGGGGS (SEQ ID NO:54), GGGGSGGGGSGGGG or $G4(SG4)_2$ (SEQ ID NO:55), and $(G_4S)_4$ or GGGGSGGGGSGGGGSGGGGS (SEQ ID NO:56), but also include the sequences GSPGSSSSGS (SEQ ID NO:57), GSGSGSGS (SEQ ID NO:58), GSGSGNGS (SEQ ID NO:59), GGSGSGSG (SEQ ID NO:60), GGSGSG (SEQ ID NO:61), GGSG (SEQ ID NO:62), GGSGNGSG (SEQ ID NO:63), GGNGSGSG (SEQ ID NO:64) and GGNGSG (SEQ ID NO:65). More particularly, the peptide linker is selected from $(G4S)_1$ or GGGGS (SEQ ID NO:51), $(G_4S)_2$ or GGGGSGGGGS (SEQ ID NO:52), $(G_4S)_3$ or GGGGSGGGGSGGGGS (SEQ ID NO:54) and $(G_4S)_4$ or GGGGSGGGGSGGGGSGGGGS (SEQ ID NO:56). Most particularly, the ectodomain of the costimulatory TNF ligand family member or fragments thereof and the huCMP trimerization domain are connected by a peptide linker of SEQ ID NO:56.

In a further aspect, provided is a trimeric costimulatory TNF family ligand-containing antigen binding molecule comprising three fusion polypeptides as described before, wherein the moiety capable of specific binding to a target cell antigen is fused at the C-terminal amino acid to the N-terminal amino acid of the trimerization domain, optionally through a peptide linker. In a particular aspect, the trimerization domain and the moiety capable of specific binding to a target cell antigen are connected through a peptide linker.

In particular, the peptide linker is a peptide selected from the group consisting of GGGGS (SEQ ID NO:51), GGGGSGGGGS (SEQ ID NO:52), SGGGGSGGGG (SEQ ID NO:53), $(G_4S)_3$ or GGGGSGGGGSGGGGS (SEQ ID NO:54), GGGGSGGGGSGGGG or $G4(SG4)_2$ (SEQ ID NO:55), and $(G_4S)_4$ or GGGGSGGGGSGGGGSGGGGS (SEQ ID NO:56), but also include the sequences GSPGSSSSGS (SEQ ID NO:57), GSGSGSGS (SEQ ID NO:58), GSGSGNGS (SEQ ID NO:59), GGSGSGSG (SEQ ID NO:60), GGSGSG (SEQ ID NO:61), GGSG (SEQ ID NO:62), GGSGNGSG (SEQ ID NO:63), GGNGSGSG (SEQ ID NO:64) and GGNGSG (SEQ ID NO:65). More particularly, the peptide linker is selected from $(G4S)_1$ or GGGGS (SEQ ID NO:51), $(G_4S)_2$ or GGGGSGGGGS (SEQ ID NO:52), $(G_4S)_3$ or GGGGSGGGGSGGGGS (SEQ ID NO:54) and $(G_4S)_4$ or GGGGSGGGGSGGGGSGGGGS (SEQ ID NO:56). Most particularly, the huCMP trimerization domain and the moiety capable of specific binding to a target cell antigen are connected by a peptide linker of SEQ ID NO:52.

In another aspect, the invention provides a trimeric costimulatory TNF family ligand-containing antigen binding molecule comprising three fusion polypeptides as described before, wherein the moiety capable of specific binding to a target cell antigen is selected from the group consisting of is selected from the group consisting of an antibody, an antibody fragment and a scaffold antigen binding protein. In one aspect, the moiety capable of specific binding to a target cell antigen is selected from the group consisting of an antibody fragment, a Fab molecule, a crossover Fab molecule, a single chain Fab molecule, a Fv molecule, a scFv molecule, a single domain antibody, an aVH and a scaffold antigen binding protein.

In one aspect, the invention provides a trimeric costimulatory TNF family ligand-containing antigen binding molecule as defined herein before, wherein the moiety capable of specific binding to a target cell antigen is an antibody fragment. In particular, the antibody fragment is selected from the group consisting of a Fab molecule, a crossover Fab molecule, a single chain Fab molecule, a Fv molecule, a scFv molecule, a single domain antibody, and aVH.

In a further aspect, the invention provides a trimeric costimulatory TNF family ligand-containing antigen binding molecule as defined herein before, wherein the moiety capable of specific binding to a target cell antigen is a scaffold antigen binding protein. In particular, the moiety capable of specific binding to a target cell antigen can be a specifically designed ankyrin repeat protein.

In a particular aspect, the invention is concerned with a trimeric costimulatory TNF family ligand-containing antigen binding molecule as defined herein before, wherein the moiety capable of specific binding to a target cell antigen is a Fab molecule capable of specific binding to a target cell antigen. Thus, the invention provides a TNF family ligand trimer-containing antigen binding molecule comprising (a) an ectodomain of a costimulatory TNF ligand family member or fragments thereof, (b) a trimerization domain derived from human cartilage matrix protein (huCMP) of amino acid sequence of SEQ ID NO:1, and (c) a Fab molecule capable of specific binding to a target cell antigen.

In a further aspect, said Fab molecule is fused at the C-terminal amino acid of the CH1 domain to the N-terminal amino acid of the trimerization domain, optionally through a peptide linker as defined above.

In a further aspect, provided is a trimeric costimulatory TNF family ligand-containing antigen binding molecule comprising three fusion polypeptides as described before, wherein the target cell antigen is selected from the group consisting of Fibroblast Activation Protein (FAP), Melanoma-associated Chondroitin Sulfate Proteoglycan (MCSP), Epidermal Growth Factor Receptor (EGFR), Carcinoembryonic Antigen (CEA), CD 19, CD20 and CD33. In a particular aspect, the target cell antigen is Fibroblast Activation Protein (FAP).

In a further aspect, provided is a trimeric costimulatory TNF family ligand-containing antigen binding molecule comprising three fusion polypeptides as described before, wherein the moiety capable of specific binding to FAP comprises

(a) a VH domain comprising (i) CDR-H1 comprising the amino acid sequence of SEQ ID NO:14, (ii) CDR-H2 comprising the amino acid sequence of SEQ ID NO:15 and (iii) CDR-H3 comprising the amino acid sequence of SEQ ID NO:16, and a VL domain comprising (iv) CDR-L1 comprising the amino acid sequence of SEQ ID NO: 17, (v) CDR-L2 comprising the amino acid sequence of SEQ ID NO:18 and (vi) CDR-L3 comprising the amino acid sequence of SEQ ID NO: 19 or

(b) a VH domain comprising (i) CDR-H1 comprising the amino acid sequence of SEQ ID NO:20, (ii) CDR-H2 comprising the amino acid sequence of SEQ ID NO:21 and (iii) CDR-H3 comprising the amino acid sequence of SEQ ID NO:22, and a VL domain comprising (iv) CDR-L1 comprising the amino acid sequence of SEQ ID NO:23, (v) CDR-L2 comprising the amino acid sequence of SEQ ID NO:24 and (vi) CDR-L3 comprising the amino acid sequence of SEQ ID NO:25.

In a further aspect, the moiety capable of specific binding to FAP comprises a heavy chain variable region comprising an amino acid sequence that is at least about 95%, 96%, 97%, 98%, 99% or 100% identical to the amino acid sequence of SEQ ID NO:26 and a light chain variable region comprising an amino acid sequence that is at least about 95%, 96%, 97%, 98%, 99% or 100% identical to the amino acid sequence of SEQ ID NO:27.

In another aspect, the moiety capable of specific binding to FAP comprises a heavy chain variable region comprising an amino acid sequence that is at least about 95%, 96%, 97%, 98%, 99% or 100% identical to the amino acid sequence of SEQ ID NO:28 and a light chain variable region comprising an amino acid sequence that is at least about 95%, 96%, 97%, 98%, 99% or 100% identical to the amino acid sequence of SEQ ID NO:29.

In a further aspect, the moiety capable of specific binding to FAP comprises

(a) a VH domain comprising the amino acid sequence of SEQ ID NO:26 and a VL domain comprising the amino acid sequence of SEQ ID NO:27, or (b) a VH domain comprising the amino acid sequence of SEQ ID NO:28 and a VL domain comprising the amino acid sequence of SEQ ID NO:29.

In a further aspect, provided is a trimeric costimulatory TNF family ligand-containing antigen binding molecule comprising three fusion polypeptides as described before, wherein said three fusion polypeptides are identical.

In another aspect, the invention provides a fusion polypeptide comprising (a) an ectodomain of a costimulatory TNF ligand family member or a fragment thereof and (b) a trimerization domain derived from human cartilage matrix protein (huCMP) of the amino acid sequence of SEQ ID NO:2, wherein said trimerization domain is capable of mediating stable association of said fusion polypeptide with two further such fusion polypeptides. In a further aspect, the fusion polypeptide further comprises (c) a moiety capable of specific binding to a target cell antigen.

In yet a further aspect, provided is a fusion polypeptide, wherein the fusion polypeptide comprises

(a) an ectodomain of a TNF ligand family comprising the amino acid sequence selected from the group consisting of SEQ ID NO:3, SEQ ID NO:4, SEQ ID NO:5, SEQ ID NO:6, SEQ ID NO:7, SEQ ID NO:8, SEQ ID NO:9 and SEQ ID NO: 10,
(b) a trimerization domain comprising the amino acid sequence of SEQ ID NO:2 and
(c) a VH domain comprising (i) CDR-H1 comprising the amino acid sequence of SEQ ID NO:14, (ii) CDR-H2 comprising the amino acid sequence of SEQ ID NO:15 and (iii) CDR-H3 comprising the amino acid sequence of SEQ ID NO:16 or a VH domain comprising (i) CDR-H1 comprising the amino acid sequence of SEQ ID NO:20,(ii) CDR-H2 comprising the amino acid sequence of SEQ ID NO:21 and (iii) CDR-H3 comprising the amino acid sequence of SEQ ID NO:22.

In a particular aspect, provided is a fusion polypeptide comprising the amino acid sequence of SEQ ID NO:71.
In another aspect, provided is a fusion polypeptide, wherein the fusion polypeptide comprises

(a) an ectodomain of a TNF ligand family comprising the amino acid sequence selected from the group consisting of SEQ ID NO: 11, SEQ ID NO:12, and SEQ ID NO: 13,
(b) a trimerization domain comprising the amino acid sequence of SEQ ID NO:2 and
(c) a VH domain comprising (i) CDR-H1 comprising the amino acid sequence of SEQ ID NO:14, (ii) CDR-H2 comprising the amino acid sequence of SEQ ID NO:15 and (iii) CDR-H3 comprising the amino acid sequence of SEQ ID NO:16 or a VH domain comprising (i) CDR-H1 comprising the amino acid sequence of SEQ ID NO:20,(ii) CDR-H2 comprising the amino acid sequence of SEQ ID NO:21 and (iii) CDR-H3 comprising the amino acid sequence of SEQ ID NO:22.

In a particular aspect, provided is a fusion polypeptide comprising the amino acid sequence of SEQ ID NO:72.

## Polynucleotides

The invention further provides isolated polynucleotides encoding a trimeric costimulatory TNF family ligand-containing antigen binding molecule as described herein or a fragment thereof.
The isolated polynucleotides encoding trimeric costimulatory TNF family ligand-containing antigen binding molecules of the invention may be expressed as a single polynucleotide that encodes the entire antigen binding molecule or as multiple (e.g., two or more) polynucleotides that are co-expressed. Polypeptides encoded by polynucleotides that are co-expressed may associate through, e.g., disulfide bonds or other means to form a functional antigen binding molecule. For example, the light chain portion of a moiety capable of specific binding to a target cell antigen may be encoded by a separate polynucleotide from the heavy chain portion of the capable of specific binding to a target cell antigen. When co-expressed, the heavy chain polypeptides will associate with the light chain polypeptides to form the moiety capable of specific binding to a target cell antigen.
In a particular aspect, the invention relates to an isolated polynucleotide encoding a fusion polypeptide as described herein before. The invention thus relates to a polynucleotide encoding a fusion polypeptide comprising (a) an ectodomain of a TNF ligand family comprising the amino acid sequence selected from the group consisting of SEQ ID NO:3, SEQ ID NO:4, SEQ ID NO:5, SEQ ID NO:6, SEQ ID NO:7, SEQ ID NO:8, SEQ ID NO:9 and SEQ ID NO: 10, (b) a trimerization domain comprising the amino acid sequence of SEQ ID NO:2 and (c) a VH domain comprising (i) CDR-H1 comprising the amino acid sequence of SEQ ID NO:14,(ii) CDR-H2 comprising the amino acid sequence of SEQ ID NO:15 and (iii) CDR-H3 comprising the amino acid sequence of SEQ ID NO:16 or a VH domain comprising (i) CDR-H1 comprising the amino acid sequence of SEQ ID NO:20,(ii) CDR-H2 comprising the amino acid sequence of SEQ ID NO:21 and (iii) CDR-H3 comprising the amino acid sequence of SEQ ID NO:22.
In a particular aspect, provided is a polynucleotide encoding a fusion polypeptide comprising the amino acid sequence of SEQ ID NO:71. More particularly, provided is a polynucleotide comprising the sequence of SEQ ID NO:73.
In another aspect, provided is a polynucleotide encoding a fusion polypeptide comprising (a) an ectodomain of a TNF ligand family comprising the amino acid sequence selected from the group consisting of SEQ ID NO: 11, SEQ ID NO: 12, and SEQ ID NO: 13, (b) a trimerization domain comprising the amino acid sequence of SEQ ID NO:2 and (c) a VH domain comprising (i) CDR-H1 comprising the amino acid sequence of SEQ ID NO:14,(ii) CDR-H2 comprising the amino acid sequence of SEQ ID NO:15 and (iii) CDR-H3 comprising the amino acid sequence of SEQ ID NO:16 or a VH domain comprising (i) CDR-H1 comprising the amino acid sequence of SEQ ID NO:20,(ii) CDR-H2 comprising the amino acid sequence of SEQ ID NO:21 and (iii) CDR-H3 comprising the amino acid sequence of SEQ ID NO:22.
In a particular aspect, provided is a polynucleotide encoding a fusion polypeptide comprising the amino acid sequence of SEQ ID NO:72. More particularly, provided is a polynucleotide comprising the sequence of SEQ ID NO:74.
In certain embodiments the polynucleotide or nucleic acid is DNA. In other embodiments, a polynucleotide of the present invention is RNA, for example, in the form of messenger RNA (mRNA). RNA of the present invention may be single stranded or double stranded.

According to another aspect of the invention, there is provided an isolated polynucleotide encoding a trimeric costimulatory TNF family ligand-containing antigen binding molecule as defined herein before or a fusion polypeptide as described herein before. The invention further provides a vector, particularly an expression vector, comprising the isolated polynucleotide of the invention and a host cell comprising the isolated polynucleotide or the vector of the invention. In some embodiments the host cell is a eukaryotic cell, particularly a mammalian cell.

In another aspect, provided is a method for producing the trimeric costimulatory TNF family ligand-containing antigen binding molecule of the invention, comprising the steps of (i) culturing the host cell of the invention under conditions suitable for expression of said antigen binding molecule, and (ii) isolating said trimeric antigen binding molecule. The invention also encompasses a trimeric costimulatory TNF family ligand-containing antigen binding molecule produced by the method of the invention.

The invention further provides a pharmaceutical composition comprising trimeric costimulatory TNF family ligand-containing antigen binding molecule of the invention and at least one pharmaceutically acceptable excipient.

Also encompassed by the invention is the trimeric costimulatory TNF family ligand-containing antigen binding molecule of the invention, or the pharmaceutical composition of the invention, for use as medicament. In one aspect, provided is a trimeric costimulatory TNF family ligand-containing antigen binding molecule of the invention, or the pharmaceutical composition of the invention, for use in the treatment of a disease in an individual in need thereof. In a specific embodiment, provided is the trimeric costimulatory TNF family ligand-containing antigen binding molecule of the invention, or the pharmaceutical composition of the invention, for use in the treatment of cancer.

Also provided is the use of the trimeric costimulatory TNF family ligand-containing antigen binding molecule of the invention for the manufacture of a medicament for the treatment of a disease in an individual in need thereof, in particular for the manufacture of a medicament for the treatment of cancer, as well as a method of treating a disease in an individual, comprising administering to said individual a therapeutically effective amount of a composition comprising the trimeric costimulatory TNF family ligand-containing antigen binding molecule of the invention in a pharmaceutically acceptable form. In a specific embodiment, the disease is cancer. In any of the above embodiments the individual is preferably a mammal, particularly a human.

**Recombinant Methods**

Trimeric costimulatory TNF family ligand-containing antigen binding molecules of the invention may be obtained, for example, by solid-state peptide synthesis (e.g. Merrifield solid phase synthesis) or recombinant production. For recombinant production one or more polynucleotide encoding the TNF family ligand trimer-containing antigen binding molecule or polypeptide fragments thereof, e.g., as described above, is isolated and inserted into one or more vectors for further cloning and/or expression in a host cell. Such polynucleotide may be readily isolated and sequenced using conventional procedures. In one aspect of the invention, a vector, preferably an expression vector, comprising one or more of the polynucleotides of the invention is provided. Methods which are well known to those skilled in the art can be used to construct expression vectors containing the coding sequence of the trimeric costimulatory TNF family ligand-containing antigen binding molecule (fragment) along with appropriate transcriptional/translational control signals. These methods include in vitro recombinant DNA techniques, synthetic techniques and in vivo recombination/genetic recombination. See, for example, the techniques described in Maniatis et al., MOLECULAR CLONING: A LABORATORY MANUAL, Cold Spring Harbor Laboratory, N.Y. (1989); and Ausubel et al., CURRENT PROTOCOLS IN MOLECULAR BIOLOGY, Greene Publishing Associates and Wiley Interscience, N.Y. (1989). The expression vector can be part of a plasmid, virus, or may be a nucleic acid fragment. The expression vector includes an expression cassette into which the polynucleotide encoding the trimeric costimulatory TNF family ligand-containing antigen binding molecule or polypeptide fragments thereof (i.e. the coding region) is cloned in operable association with a promoter and/or other transcription or translation control elements. As used herein, a "coding region" is a portion of nucleic acid which consists of codons translated into amino acids. Although a "stop codon" (TAG, TGA, or TAA) is not translated into an amino acid, it may be considered to be part of a coding region, if present, but any flanking sequences, for example promoters, ribosome binding sites, transcriptional terminators, introns, 5' and 3' untranslated regions, and the like, are not part of a coding region. Two or more coding regions can be present in a single polynucleotide construct, e.g. on a single vector, or in separate polynucleotide constructs, e.g. on separate (different) vectors. Furthermore, any vector may contain a single coding region, or may comprise two or more coding regions, e.g. a vector of the present invention may encode one or more polypeptides, which are post- or co-translationally separated into the final proteins via proteolytic cleavage. In addition, a vector, polynucleotide, or nucleic acid of the invention may encode heterologous coding regions, either fused or unfused to a polynucleotide encoding the trimeric costimulatory TNF family ligand-containing antigen binding molecule of the invention or polypeptide fragments thereof, or variants or derivatives thereof. Heterologous coding regions include without limitation specialized elements or motifs, such as a secretory signal peptide or a heterologous functional domain. An operable association is when a coding region for a gene product, e.g. a polypeptide, is associated with one or more regulatory sequences in such a way as to place expression of the gene product under the influence or control of the regulatory sequence(s). Two DNA fragments (such as a polypeptide coding region and a promoter associated therewith) are "operably associated" if induction of promoter function results in the transcription of mRNA encoding the desired gene product and if the nature of the linkage between the two DNA fragments does not interfere with the ability of the expression regulatory sequences to direct the expression of the gene product or interfere with the ability of the DNA template to be transcribed. Thus, a promoter region would be operably associated with a nucleic acid encoding a polypeptide if the promoter was capable of effecting transcription of that nucleic acid. The promoter may be a cell-specific promoter that directs substantial transcription of the DNA only in predetermined cells. Other transcription control elements, besides a promoter, for example enhancers, operators, repressors, and transcription termination signals, can be operably associated with the polynucleotide to direct cell-specific transcription.

Suitable promoters and other transcription control regions are disclosed herein. A variety of transcription control regions are known to those skilled in the art. These include, without limitation, transcription control regions, which function in vertebrate cells, such as, but not limited to, promoter and enhancer segments from cytomegaloviruses (e.g. the immediate early promoter, in conjunction with intron-A), simian virus 40 (e.g. the early promoter), and retroviruses (such as, e.g. Rous sarcoma virus). Other transcription control regions include those derived from vertebrate genes such as actin, heat shock protein, bovine growth hormone and rabbit â-globin, as well as other sequences capable of controlling gene expression in eukaryotic cells. Additional suitable transcription control regions include tissue-specific promoters and enhancers as well as inducible promoters (e.g. promoters inducible tetracyclins). Similarly, a variety of translation control elements are known to those of ordinary skill in the art. These include, but are not limited to ribosome binding sites, translation initiation and termination codons, and elements derived from viral systems (particularly an internal ribosome entry site, or IRES, also referred to as a CITE sequence). The expression cassette may also include other features such as an origin of replication, and/or chromosome integration elements such as retroviral long terminal repeats (LTRs), or adeno-associated viral (AAV) inverted terminal repeats (ITRs).

Polynucleotide and nucleic acid coding regions of the present invention may be associated with additional coding regions which encode secretory or signal peptides, which direct the secretion of a polypeptide encoded by a polynucleotide of the present invention. For example, if secretion of the trimeric costimulatory TNF family ligand-containing antigen binding molecule or polypeptide fragments thereof is desired, DNA encoding a signal sequence may be placed upstream of the nucleic acid encoding a trimeric costimulatory TNF family ligand-containing antigen binding molecule of the invention or polypeptide fragments thereof. According to the signal hypothesis, proteins secreted by mammalian cells have a signal peptide or secretory leader sequence which is cleaved from the mature protein once export of the growing protein chain across the rough endoplasmic reticulum has been initiated. Those of ordinary skill in the art are aware that polypeptides secreted by vertebrate cells generally have a signal peptide fused to the N-terminus of the polypeptide, which is cleaved from the translated polypeptide to produce a secreted or "mature" form of the polypeptide. In certain embodiments, the native signal peptide, e.g. an immunoglobulin heavy chain or light chain signal peptide is used, or a functional derivative of that sequence that retains the ability to direct the secretion of the polypeptide that is operably associated with it. Alternatively, a heterologous mammalian signal peptide, or a functional derivative thereof, may be used. For example, the wild-type leader sequence may be substituted with the leader sequence of human tissue plasminogen activator (TPA) or mouse β-glucuronidase.

DNA encoding a short protein sequence that could be used to facilitate later purification (e.g. a histidine tag) or assist in labeling the fusion protein may be included within or at the ends of the polynucleotide encoding a trimeric costimulatory TNF family ligand-containing antigen binding molecule of the invention or polypeptide fragments thereof.

In a further aspect of the invention, a host cell comprising one or more polynucleotides of the invention is provided. In certain embodiments a host cell comprising one or more vectors of the invention is provided. The polynucleotides and vectors may incorporate any of the features, singly or in combination, described herein in relation to polynucleotides and vectors, respectively. In one aspect, a host cell comprises (e.g. has been transformed or transfected with) a vector comprising a polynucleotide that encodes (part of) a trimeric costimulatory TNF family ligand-containing antigen binding molecule of the invention of the invention. As used herein, the term "host cell" refers to any kind of cellular system which can be engineered to generate the fusion proteins of the invention or fragments thereof. Host cells suitable for replicating and for supporting expression of antigen binding molecules are well known in the art. Such cells may be transfected or transduced as appropriate with the particular expression vector and large quantities of vector containing cells can be grown for seeding large scale fermenters to obtain sufficient quantities of the antigen binding molecule for clinical applications. Suitable host cells include prokaryotic microorganisms, such as E. coli, or various eukaryotic cells, such as Chinese hamster ovary cells (CHO), insect cells, or the like. For example, polypeptides may be produced in bacteria in particular when glycosylation is not needed. After expression, the polypeptide may be isolated from the bacterial cell paste in a soluble fraction and can be further purified. In addition to prokaryotes, eukaryotic microbes such as filamentous fungi or yeast are suitable cloning or expression hosts for polypeptide-encoding vectors, including fungi and yeast strains whose glycosylation pathways have been "humanized", resulting in the production of a polypeptide with a partially or fully human glycosylation pattern. See Gerngross, Nat Biotech 22, 1409-1414 (2004), and Li et al., Nat Biotech 24, 210-215 (2006).

Suitable host cells for the expression of (glycosylated) polypeptides are also derived from multicellular organisms (invertebrates and vertebrates). Examples of invertebrate cells include plant and insect cells. Numerous baculoviral strains have been identified which may be used in conjunction with insect cells, particularly for transfection of Spodoptera frugiperda cells. Plant cell cultures can also be utilized as hosts. See e.g. US Patent Nos. 5,959,177, 6,040,498, 6,420,548, 7,125,978, and 6,417,429 (describing PLANTIBODIES™ technology for producing antibodies in transgenic plants). Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293T cells as described, e.g., in Graham et al., J Gen Virol 36, 59 (1977)), baby hamster kidney cells (BHK), mouse sertoli cells (TM4 cells as described, e.g., in Mather, Biol Reprod 23, 243-251 (1980)), monkey kidney cells (CV1), African green monkey kidney cells (VERO-76), human cervical carcinoma cells (HELA), canine kidney cells (MDCK), buffalo rat liver cells (BRL 3A), human lung cells (W138), human liver cells (Hep G2), mouse mammary tumor cells (MMT 060562), TRI cells (as described, e.g., in Mather et al., Annals N.Y. Acad Sci 383, 44-68 (1982)), MRC 5 cells, and FS4 cells. Other useful mammalian host cell lines include Chinese hamster ovary (CHO) cells, including dhfr- CHO cells (Urlaub et al., Proc Natl Acad Sci USA 77, 4216 (1980)); and myeloma cell lines such as YO, NS0, P3X63 and Sp2/0. For a review of certain mammalian host cell lines suitable for protein production, see, e.g., Yazaki and Wu, Methods in Molecular Biology, Vol. 248 (B.K.C. Lo, ed., Humana Press, Totowa, NJ), pp. 255-268 (2003). Host cells include cultured cells, e.g., mammalian cultured cells, yeast cells, insect cells, bacterial cells and plant cells, to name only a few, but also cells comprised within a transgenic animal, transgenic plant or cultured plant or animal tissue. In one embodiment, the host cell is a eukaryotic cell, preferably a mammalian cell, such as a Chinese Hamster Ovary (CHO) cell, a human embryonic kidney (HEK) cell or a lymphoid cell (e.g., Y0, NS0, Sp20 cell). Standard technologies are known in the art to express foreign genes in these systems. Cells expressing a polypeptide comprising either the heavy or the light chain of an antigen binding domain, may be engineered so as to also express the other of the immunoglobulin chains such that the expressed product is an antigen binding domain that has both a heavy and a light chain.

In another aspect, provided is a method for producing the trimeric costimulatory TNF family ligand-containing antigen binding molecule of the invention, comprising the steps of (i) culturing the host cell of the invention under conditions suitable for expression of said antigen binding molecule, and (ii) isolating said trimeric antigen binding molecule form the host cell or host cell culture medium.

The components of the trimeric antigen binding molecule are genetically fused to each other. Trimeric antigen binding molecules can be designed such that its components are fused directly to each other or indirectly through a linker sequence. The composition and length of the linker may be determined in accordance with methods well known in the art and may be tested for efficacy. Examples of linker sequences between different components of trimeric antigen binding molecules are found in the sequences provided herein. Additional sequences may also be included to incorporate a cleavage site to separate the individual components of the fusion if desired, for example an endopeptidase recognition sequence.

In certain embodiments the moieties capable of specific binding to a target cell antigen (e.g. Fab fragments) forming part of the antigen binding molecule comprise at least an immunoglobulin variable region capable of binding to an antigen. Variable regions can form part of and be derived from naturally or non-naturally occurring antibodies and fragments thereof. Methods to produce polyclonal antibodies and monoclonal antibodies are well known in the art (see e.g. Harlow and Lane, "Antibodies, a laboratory manual", Cold Spring Harbor Laboratory, 1988). Non-naturally occurring antibodies can be constructed using solid phase-peptide synthesis, can be produced recombinantly (e.g. as described in U.S. patent No. 4,186,567) or can be obtained, for example, by screening combinatorial libraries comprising variable heavy chains and variable light chains (see e.g. U.S. Patent. No. 5,969,108 to McCafferty).

Any animal species of immunoglobulin can be used in the invention. Non-limiting immunoglobulins useful in the present invention can be of murine, primate, or human origin. If the fusion protein is intended for human use, a chimeric form of immunoglobulin may be used wherein the constant regions of the immunoglobulin are from a human. A humanized or fully human form of the immunoglobulin can also be prepared in accordance with methods well known in the art (see e. g. U.S. Patent No. 5,565,332 to Winter). Humanization may be achieved by various methods including, but not limited to (a) grafting the non-human (e.g., donor antibody) CDRs onto human (e.g. recipient antibody) framework and constant regions with or without retention of critical framework residues (e.g. those that are important for retaining good antigen binding affinity or antibody functions), (b) grafting only the non-human specificity-determining regions (SDRs or a-CDRs; the residues critical for the antibody-antigen interaction) onto human framework and constant regions, or (c) transplanting the entire non-human variable domains, but "cloaking" them with a human-like section by replacement of surface residues. Humanized antibodies and methods of making them are reviewed, e.g., in Almagro and Fransson, Front Biosci 13, 1619-1633 (2008), and are further described, e.g., in Riechmann et al., Nature 332, 323-329 (1988); Queen et al., Proc Natl Acad Sci USA 86, 10029-10033 (1989); US Patent Nos. 5,821,337, 7,527,791, 6,982,321, and 7,087,409; Jones et al., Nature 321, 522-525 (1986); Morrison et al., Proc Natl Acad Sci 81, 6851-6855 (1984); Morrison and Oi, Adv Immunol 44, 65-92 (1988); Verhoeyen et al., Science 239, 1534-1536 (1988); Padlan, Molec Immun 31(3), 169-217 (1994); Kashmiri et al., Methods 36, 25-34 (2005) (describing SDR (a-CDR) grafting); Padlan, Mol Immunol 28, 489-498 (1991) (describing "resurfacing"); Dall'Acqua et al., Methods 36, 43-60 (2005) (describing "FR shuffling"); and Osbourn et al., Methods 36, 61-68 (2005) and Klimka et al., Br J Cancer 83, 252-260 (2000) (describing the "guided selection" approach to FR shuffling). Particular immunoglobulins according to the invention are human immunoglobulins. Human antibodies and human variable regions can be produced using various techniques known in the art. Human antibodies are described generally in van Dijk and van de Winkel, Curr Opin Pharmacol 5, 368-74 (2001) and Lonberg, Curr Opin Immunol 20, 450-459 (2008). Human variable regions can form part of and be derived from human monoclonal antibodies made by the hybridoma method (see e.g. Monoclonal Antibody Production Techniques and Applications, pp. 51-63 (Marcel Dekker, Inc., New York, 1987)). Human antibodies and human variable regions may also be prepared by administering an immunogen to a transgenic animal that has been modified to produce intact human antibodies or intact antibodies with human variable regions in response to antigenic challenge (see e.g. Lonberg, Nat Biotech 23, 1117-1125 (2005). Human antibodies and human variable regions may also be generated by isolating Fv clone variable region sequences selected from human-derived phage display libraries (see e.g., Hoogenboom et al. in Methods in Molecular Biology 178, 1-37 (O'Brien et al., ed., Human Press, Totowa, NJ, 2001); and McCafferty et al., Nature 348, 552-554; Clackson et al., Nature 352, 624-628 (1991)). Phage typically display antibody fragments, either as single-chain Fv (scFv) fragments or as Fab fragments.

In certain aspects, the moieties capable of specific binding to a target cell antigen (e.g. Fab fragments) comprised in the antigen binding molecules of the present invention are engineered to have enhanced binding affinity according to, for example, the methods disclosed in PCT publication WO 2012/020006 (see Examples relating to affinity maturation) or U.S. Pat. Appl. Publ. No. 2004/0132066. The ability of the antigen binding molecules of the invention to bind to a specific antigenic determinant can be measured either through an enzyme-linked immunosorbent assay (ELISA) or other techniques familiar to one of skill in the art, e.g. surface plasmon resonance technique (Liljeblad, et al., Glyco J 17, 323-329 (2000)), and traditional binding assays (Heeley, Endocr Res 28, 217-229 (2002)). Competition assays may be used to identify an antigen binding molecule that competes with a reference antibody for binding to a particular antigen. In certain embodiments, such a competing antigen binding molecule binds to the same epitope (e.g. a linear or a conformational epitope) that is bound by the reference antigen binding molecule. Detailed exemplary methods for mapping an epitope to which an antigen binding molecule binds are provided in Morris (1996) "Epitope Mapping Protocols", in Methods in Molecular Biology vol. 66 (Humana Press, Totowa, NJ). In an exemplary competition assay, immobilized antigen is incubated in a solution comprising a first labeled antigen binding molecule that binds to the antigen and a second unlabeled antigen binding molecule that is being tested for its ability to compete with the first antigen binding molecule for binding to the antigen. The second antigen binding molecule may be present in a hybridoma supernatant. As a control, immobilized antigen is incubated in a solution comprising the first labeled antigen binding molecule but not the second unlabeled antigen binding molecule. After incubation under conditions permissive for binding of the first antibody to the antigen, excess unbound antibody is removed, and the amount of label associated with immobilized antigen is measured. If the amount of label associated with immobilized antigen is substantially reduced in the test sample relative to the control sample, then that indicates that the second antigen binding molecule is competing with the first antigen binding molecule for binding to the antigen. See Harlow and Lane (1988) Antibodies: A Laboratory Manual ch.14 (Cold Spring Harbor Laboratory, Cold Spring Harbor, NY).

Trimeric costimulatory TNF family ligand-containing antigen binding molecule of the invention prepared as described herein may be purified by art-known techniques such as high performance liquid chromatography, ion exchange chromatography, gel electrophoresis, affinity chromatography, size exclusion chromatography, and the like. The actual conditions used to purify a particular protein will depend, in part, on factors such as net charge, hydrophobicity, hydrophilicity etc., and will be apparent to those having skill in the art. For affinity chromatography purification an antibody, ligand, receptor or antigen can be used to which the trimeric costimulatory TNF family ligand-containing antigen binding molecule binds. For example, for affinity chromatography purification of fusion proteins of the invention, a matrix with protein A or protein G may be used. Sequential Protein A or G affinity chromatography and size exclusion chromatography can be used to isolate an antigen binding molecule essentially as described in the Examples. The purity of the trimeric costimulatory TNF family ligand-containing antigen binding molecule or fragments thereof can be determined by any of a variety of well-known analytical methods including gel electrophoresis, high pressure liquid chromatography, and the like. For example, the trimeric costimulatory TNF family ligand-containing antigen binding molecule expressed as described in the Examples were shown to be intact and properly assembled as demonstrated by reducing and non-reducing SDS-PAGE.

The invention also encompasses a trimeric costimulatory TNF family ligand-containing antigen binding molecule produced by the method of the invention.

**Assays**

The trimeric costimulatory TNF family ligand-containing antigen binding molecules provided herein may be identified, screened for, or characterized for their physical/chemical properties and/or biological activities by various assays known in the art.

**1. Affinity assays**

The affinity of the trimeric costimulatory TNF family ligand-containing antigen binding molecule provided herein for the corresponding TNF receptor can be determined in accordance with the methods set forth in the Examples by surface plasmon resonance (SPR), using standard instrumentation such as a BIAcore instrument (GE Healthcare), and receptors or target proteins such as may be obtained by recombinant expression. The affinity of the trimeric costimulatory TNF family ligand-containing antigen binding molecule for the target cell antigen can also be determined by surface plasmon resonance (SPR), using standard instrumentation such as a BIAcore instrument (GE Healthcare), and receptors or target proteins such as may be obtained by recombinant expression. A specific illustrative and exemplary embodiment for measuring binding affinity is described in Example 3.2. According to one aspect, $K_D$ is measured by surface plasmon resonance using a BIACORE® T100 machine (GE Healthcare) at 25 °C.

**2. Binding assays and other assays**

Binding of the trimeric costimulatory TNF family ligand-containing antigen binding molecule provided herein to the corresponding receptor expressing cells may be evaluated using cell lines expressing the particular receptor or target antigen, for example by flow cytometry (FACS). In one aspect, fresh peripheral blood mononuclear cells (PBMCs) expressing the TNF receptor are used in the binding assay. These cells are used directly after isolation (naive PMBCs) or after stimulation (activated PMBCs). In another aspect, activated mouse splenocytes (expressing the TNF receptor molecule) were used to demonstrate the binding of trimeric costimulatory TNF family ligand-containing antigen binding molecule of the invention to the corresponding TNF receptor expressing cells.

In a further aspect, cancer cell lines expressing the target cell antigen, for example FAP, were used to demonstrate the binding of the antigen binding molecules to the target cell antigen.

In another aspect, competition assays may be used to identify an antigen binding molecule that competes with a specific antibody or antigen binding molecule for binding to the target or TNF receptor, respectively. In certain embodiments, such a competing antigen binding molecule binds to the same epitope (e.g., a linear or a conformational epitope) that is bound by a specific anti-target antibody or a specific anti-TNF receptor antibody. Detailed exemplary methods for mapping an epitope to which an antibody binds are provided in Morris (1996) "Epitope Mapping Protocols," in Methods in Molecular Biology vol. 66 (Humana Press, Totowa, NJ).

**3. Activity assays**

In one aspect, assays are provided for identifying trimeric costimulatory TNF family ligand-containing antigen binding molecules that bind to a specific target cell antigen and to a specific TNF receptor having biological activity. Biological activity may include, e.g., agonistic signalling through the TNF receptor on cells expressing the target cell antigen. TNF family ligand trimer-containing antigen binding molecules identified by the assays as having such biological activity in vitro are also provided. In particular, a reporter cell assay detecting NF-κB activation in Hela cells expressing human 4-1BB or human OX40 and co-cultured with FAP-expressing tumor cells is provided (see e.g. Example 5.1.2.).

In certain aspects, a trimeric costimulatory TNF family ligand-containing antigen binding molecules of the invention is tested for such biological activity. Assays for detecting the biological activity of the molecules of the invention are those described in Example 5 or Example 6.3. Furthermore, assays for detecting cell lysis (e.g. by measurement of LDH release), induced apoptosis kinetics (e.g. by measurement of Caspase 3/7 activity) or apoptosis (e.g. using the TUNEL assay) are well known in the art. In addition the biological activity of such complexes can be assessed by evaluating their effects on survival, proliferation and lymphokine secretion of various lymphocyte subsets such as NK cells, NKT-cells or γδ T-cells or assessing their capacity to modulate phenotype and function of antigen presenting cells such as dendritic cells, monocytes/macrophages or B-cells.

**Pharmaceutical Compositions, Formulations and Routes of Administation**

In a further aspect, the invention provides pharmaceutical compositions comprising any of the trimeric costimulatory TNF family ligand-containing antigen binding molecules provided herein, e.g., for use in any of the below therapeutic methods. In one embodiment, a pharmaceutical composition comprises any of the TNF family ligand trimer-containing antigen binding molecules provided herein and at least one pharmaceutically acceptable excipient. In another embodiment, a pharmaceutical composition comprises any of the TNF family ligand trimer-containing antigen binding molecules provided herein and at least one additional therapeutic agent, e.g., as described below.

Pharmaceutical compositions of the present invention comprise a therapeutically effective amount of one or more trimeric costimulatory TNF family ligand-containing antigen binding molecule dissolved or dispersed in a pharmaceutically acceptable excipient. The phrases Pharmaceutical or pharmacologically acceptable" refers to molecular entities and compositions that are generally non-toxic to recipients at the dosages and concentrations employed, i.e. do not produce an adverse, allergic or other untoward reaction when administered to an animal, such as, for example, a human, as appropriate. The preparation of a pharmaceutical composition that contains at least one trimeric costimulatory TNF family ligand-containing antigen binding molecules and optionally an additional active ingredient will be known to those of skill in the art in light of the present disclosure, as exemplified by Remington's Pharmaceutical Sciences, 18th Ed. Mack Printing Company, 1990, incorporated herein by reference. In particular, the compositions are lyophilized formulations or aqueous solutions. As used herein, "pharmaceutically acceptable excipient" includes any and all solvents, buffers, dispersion media, coatings, surfactants, antioxidants, preservatives (e.g. antibacterial agents, antifungal agents), isotonic agents, salts, stabilizers and combinations thereof, as would be known to one of ordinary skill in the art.

Parenteral compositions include those designed for administration by injection, e.g. subcutaneous, intradermal, intralesional, intravenous, intraarterial intramuscular, intrathecal or intraperitoneal injection. For injection, the trimeric costimulatory TNF family ligand-containing antigen binding molecules of the invention may be formulated in aqueous solutions, preferably in physiologically compatible buffers such as Hanks' solution, Ringer's solution, or physiological saline buffer. The solution may contain formulatory agents such as suspending, stabilizing and/or dispersing agents. Alternatively, the fusion proteins may be in powder form for constitution with a suitable vehicle, e.g., sterile pyrogen-free water, before use. Sterile injectable solutions are prepared by incorporating the fusion proteins of the invention in the required amount in the appropriate solvent with various of the other ingredients enumerated below, as required. Sterility may be readily accomplished, e.g., by filtration through sterile filtration membranes. Generally, dispersions are prepared by incorporating the various sterilized active ingredients into a sterile vehicle which contains the basic dispersion medium and/or the other ingredients. In the case of sterile powders for the preparation of sterile injectable solutions, suspensions or emulsion, the preferred methods of preparation are vacuum-drying or freeze-drying techniques which yield a powder of the active ingredient plus any additional desired ingredient from a previously sterile-filtered liquid medium thereof. The liquid medium should be suitably buffered if necessary and the liquid diluent first rendered isotonic prior to injection with sufficient saline or glucose. The composition must be stable under the conditions of manufacture and storage, and preserved against the contaminating action of microorganisms, such as bacteria and fungi. It will be appreciated that endotoxin contamination should be kept minimally at a safe level, for example, less that 0.5 ng/mg protein. Suitable pharmaceutically acceptable excipients include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as polyethylene glycol (PEG). Aqueous injection suspensions may contain compounds which increase the viscosity of the suspension, such as sodium carboxymethyl cellulose, sorbitol, dextran, or the like. Optionally, the suspension may also contain suitable stabilizers or agents which increase the solubility of the compounds to allow for the preparation of highly concentrated solutions. Additionally, suspensions of the active compounds may be prepared as appropriate oily injection suspensions. Suitable lipophilic solvents or vehicles include fatty oils such as sesame oil, or synthetic fatty acid esters, such as ethyl cleats or triglycerides, or liposomes.

Active ingredients may be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nanoparticles and nanocapsules) or in macroemulsions. Such techniques are disclosed in Remington's Pharmaceutical Sciences (18th Ed. Mack Printing Company, 1990). Sustained-release preparations may be prepared. Suitable examples of sustained-release preparations include semipermeable matrices of solid hydrophobic polymers containing the polypeptide, which matrices are in the form of shaped articles, e.g. films, or microcapsules. In particular embodiments, prolonged absorption of an injectable composition can be brought about by the use in the compositions of agents delaying absorption, such as, for example, aluminum monostearate, gelatin or combinations thereof.

Exemplary pharmaceutically acceptable excipients herein further include insterstitial drug dispersion agents such as soluble neutral-active hyaluronidase glycoproteins (sHASEGP), for example, human soluble PH-20 hyaluronidase glycoproteins, such as rHuPH20 (HYLENEX®, Baxter International, Inc.). Certain exemplary sHASEGPs and methods of use, including rHuPH20, are described in US Patent Publication Nos. 2005/0260186 and 2006/0104968. In one aspect, a sHASEGP is combined with one or more additional glycosaminoglycanases such as chondroitinases.

Exemplary lyophilized antibody formulations are described in US Patent No. 6,267,958. Aqueous antibody formulations include those described in US Patent No. 6,171,586 and WO2006/044908, the latter formulations including a histidine-acetate buffer.

In addition to the compositions described previously, the fusion proteins may also be formulated as a depot preparation. Such long acting formulations may be administered by implantation (for example subcutaneously or intramuscularly) or by intramuscular injection. Thus, for example, the fusion proteins may be formulated with suitable polymeric or hydrophobic materials (for example as emulsion in a pharmaceutically acceptable oil) or ion exchange resins, or as sparingly soluble derivatives, for example, as a sparingly soluble salt.

Pharmaceutical compositions comprising the trimeric costimulatory TNF family ligand-containing antigen binding molecules of the invention may be manufactured by means of conventional mixing, dissolving, emulsifying, encapsulating, entrapping or lyophilizing processes. Pharmaceutical compositions may be formulated in conventional manner using one or more physiologically acceptable carriers, diluents, excipients or auxiliaries which facilitate processing of the proteins into preparations that can be used pharmaceutically. Proper formulation is dependent upon the route of administration chosen.

The trimeric costimulatory TNF family ligand-containing antigen binding molecules may be formulated into a composition in a free acid or base, neutral or salt form. Pharmaceutically acceptable salts are salts that substantially retain the biological activity of the free acid or base. These include the acid addition salts, e.g. those formed with the free amino groups of a proteinaceous composition, or which are formed with inorganic acids such as for example, hydrochloric or phosphoric acids, or such organic acids as acetic, oxalic, tartaric or mandelic acid. Salts formed with the free carboxyl groups can also be derived from inorganic bases such as for example, sodium, potassium, ammonium, calcium or ferric hydroxides; or such organic bases as isopropylamine, trimethylamine, histidine or procaine. Pharmaceutical salts tend to be more soluble in aqueous and other protic solvents than are the corresponding free base forms.

The composition herein may also contain more than one active ingredients as necessary for the particular indication being treated, preferably those with complementary activities that do not adversely affect each other. Such active ingredients are suitably present in combination in amounts that are effective for the purpose intended.

The formulations to be used for in vivo administration are generally sterile. Sterility may be readily accomplished, e.g., by filtration through sterile filtration membranes.

**Therapeutic methods and compositions**

Any of the trimeric costimulatory TNF family ligand-containing antigen binding molecules provided herein may be used in therapeutic methods.

For use in therapeutic methods, TNF family ligand trimer-containing antigen binding molecules of the invention can be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners.

In one aspect, trimeric costimulatory TNF family ligand-containing antigen binding molecules of the invention for use as a medicament are provided. In further aspects, trimeric costimulatory TNF family ligand-containing antigen binding molecules of the invention for use in treating a disease, in particular for use in the treatment of cancer, are provided. In certain embodiments, trimeric costimulatory TNF family ligand-containing antigen binding molecules of the invention for use in a method of treatment are provided. In one embodiment, the invention provides a trimeric costimulatory TNF family ligand-containing antigen binding molecule as described herein for use in the treatment of a disease in an individual in need thereof. In certain embodiments, the invention provides a trimeric costimulatory TNF family ligand-containing antigen binding molecule for use in a method of treating an individual having a disease comprising administering to the individual a therapeutically effective amount of the fusion protein. In certain embodiments the disease to be treated is cancer. The subject, patient, or "individual" in need of treatment is typically a mammal, more specifically a human.

In a further aspect, the invention provides for the use of a trimeric costimulatory TNF family ligand-containing antigen binding molecule of the invention in the manufacture or preparation of a medicament for the treatment of a disease in an individual in need thereof. In one aspect, the medicament is for use in a method of treating a disease comprising administering to an individual having the disease a therapeutically effective amount of the medicament. In certain embodiments the disease to be treated is a proliferative disorder, particularly cancer. Examples of cancers include bladder cancer, brain cancer, head and neck cancer, pancreatic cancer, lung cancer, breast cancer, ovarian cancer, uterine cancer, cervical cancer, endometrial cancer, esophageal cancer, colon cancer, colorectal cancer, rectal cancer, gastric cancer, prostate cancer, blood cancer, skin cancer, squamous cell carcinoma, bone cancer, and kidney cancer. Other cell proliferation disorders that can be treated using a trimeric costimulatory TNF family ligand-containing antigen binding molecule of the present invention include, but are not limited to neoplasms located in the: abdomen, bone, breast, digestive system, liver, pancreas, peritoneum, endocrine glands (adrenal, parathyroid, pituitary, testicles, ovary, thymus, thyroid), eye, head and neck, nervous system (central and peripheral), lymphatic system, pelvic, skin, soft tissue, spleen, thoracic region, and urogenital system. Also included are pre-cancerous conditions or lesions and cancer metastases. In certain embodiments the cancer is chosen from the group consisting of renal cell cancer, skin cancer, lung cancer, colorectal cancer, breast cancer, brain cancer, head and neck cancer. A skilled artisan readily recognizes that in many cases the trimeric costimulatory TNF family ligand-containing antigen binding molecule may not provide a cure but may only provide partial benefit. In some embodiments, a physiological change having some benefit is also considered therapeutically beneficial. Thus, in some embodiments, an amount of trimeric costimulatory TNF family ligand-containing antigen binding molecule that provides a physiological change is considered an "effective amount" or a "therapeutically effective amount".

In a further aspect, the invention provides a method for treating a disease in an individual, comprising administering to said individual a therapeutically effective amount of a trimeric costimulatory TNF family ligand-containing antigen binding molecule of the invention. In one embodiment a composition is administered to said individual, comprising a fusion protein of the invention in a pharmaceutically acceptable form. In certain embodiments the disease to be treated is a proliferative disorder. In a particular embodiment the disease is cancer. In certain embodiments the method further comprises administering to the individual a therapeutically effective amount of at least one additional therapeutic agent, e.g. an anti-cancer agent if the disease to be treated is cancer. An "individual" according to any of the above embodiments may be a mammal, preferably a human.

For the prevention or treatment of disease, the appropriate dosage of a trimeric costimulatory TNF family ligand-containing antigen binding molecule of the invention (when used alone or in combination with one or more other additional therapeutic agents) will depend on the type of disease to be treated, the route of administration, the body weight of the patient, the type of fusion protein, the severity and course of the disease, whether the fusion protein is administered for preventive or therapeutic purposes, previous or concurrent therapeutic interventions, the patient's clinical history and response to the fusion protein, and the discretion of the attending physician. The practitioner responsible for administration will, in any event, determine the concentration of active ingredient(s) in a composition and appropriate dose(s) for the individual subject. Various dosing schedules including but not limited to single or multiple administrations over various time-points, bolus administration, and pulse infusion are contemplated herein.

The trimeric costimulatory TNF family ligand-containing antigen binding molecule is suitably administered to the patient at one time or over a series of treatments. Depending on the type and severity of the disease, about 1 μg/kg to 15 mg/kg (e.g. 0.1 mg/kg - 10 mg/kg) of TNF family ligand trimer-containing antigen binding molecule can be an initial candidate dosage for administration to the patient, whether, for example, by one or more separate administrations, or by continuous infusion. One typical daily dosage might range from about 1 μg/kg to 100 mg/kg or more, depending on the factors mentioned above. For repeated administrations over several days or longer, depending on the condition, the treatment would generally be sustained until a desired suppression of disease symptoms occurs. One exemplary dosage of the fusion protein would be in the range from about 0.005 mg/kg to about 10 mg/kg. In other examples, a dose may also comprise from about 1 μg/kg body weight, about 5 μg/kg body weight, about 10 μg/kg body weight, about 50 μg/kg body weight, about 100 μg/kg body weight, about 200 μg/kg body weight, about 350 μg/kg body weight, about 500 μg/kg body weight, about 1 mg/kg body weight, about 5 mg/kg body weight, about 10 mg/kg body weight, about 50 mg/kg body weight, about 100 mg/kg body weight, about 200 mg/kg body weight, about 350 mg/kg body weight, about 500 mg/kg body weight, to about 1000 mg/kg body weight or more per administration, and any range derivable therein. In examples of a derivable range from the numbers listed herein, a range of about 5 mg/kg body weight to about 100 mg/kg body weight, about 5 μg/kg body weight to about 500 mg/kg body weight etc., can be administered, based on the numbers described above. Thus, one or more doses of about 0.5 mg/kg, 2.0 mg/kg, 5.0 mg/kg or 10 mg/kg (or any combination thereof) may be administered to the patient. Such doses may be administered intermittently, e.g. every week or every three weeks (e.g. such that the patient receives from about two to about twenty, or e.g. about six doses of the fusion protein). An initial higher loading dose, followed by one or more lower doses may be administered. However, other dosage regimens may be useful. The progress of this therapy is easily monitored by conventional techniques and assays.

The trimeric costimulatory TNF family ligand-containing antigen binding molecule of the invention will generally be used in an amount effective to achieve the intended purpose. For use to treat or prevent a disease condition, the trimeric costimulatory TNF family ligand-containing antigen binding molecules of the invention, or pharmaceutical compositions thereof, are administered or applied in a therapeutically effective amount. Determination of a therapeutically effective amount is well within the capabilities of those skilled in the art, especially in light of the detailed disclosure provided herein.

For systemic administration, a therapeutically effective dose can be estimated initially from in vitro assays, such as cell culture assays. A dose can then be formulated in animal models to achieve a circulating concentration range that includes the $IC_{50}$ as determined in cell culture. Such information can be used to more accurately determine useful doses in humans.

Initial dosages can also be estimated from in vivo data, e.g., animal models, using techniques that are well known in the art. One having ordinary skill in the art could readily optimize administration to humans based on animal data.

Dosage amount and interval may be adjusted individually to provide plasma levels of the trimeric costimulatory TNF family ligand-containing antigen binding molecules which are sufficient to maintain therapeutic effect. Usual patient dosages for administration by injection range from about 0.1 to 50 mg/kg/day, typically from about 0.5 to 1 mg/kg/day. Therapeutically effective plasma levels may be achieved by administering multiple doses each day. Levels in plasma may be measured, for example, by HPLC.

In cases of local administration or selective uptake, the effective local concentration of the trimeric costimulatory TNF family ligand-containing antigen binding molecules may not be related to plasma concentration. One skilled in the art will be able to optimize therapeutically effective local dosages without undue experimentation.

A therapeutically effective dose of the trimeric costimulatory TNF family ligand-containing antigen binding molecules described herein will generally provide therapeutic benefit without causing substantial toxicity. Toxicity and therapeutic efficacy of a fusion protein can be determined by standard pharmaceutical procedures in cell culture or experimental animals. Cell culture assays and animal studies can be used to determine the $LD_{50}$ (the dose lethal to 50% of a population) and the $ED_{50}$ (the dose therapeutically effective in 50% of a population). The dose ratio between toxic and therapeutic effects is the therapeutic index, which can be expressed as the ratio $LD_{50}/ED_{50}$. Trimeric costimulatory TNF family ligand-containing antigen binding molecules that exhibit large therapeutic indices are preferred. In one embodiment, the TNF family ligand trimer-containing antigen binding molecule according to the present invention exhibits a high therapeutic index. The data obtained from cell culture assays and animal studies can be used in formulating a range of dosages suitable for use in humans. The dosage lies preferably within a range of circulating concentrations that include the ED50 with little or no toxicity. The dosage may vary within this range depending upon a variety of factors, e.g., the dosage form employed, the route of administration utilized, the condition of the subject, and the like. The exact formulation, route of administration and dosage can be chosen by the individual physician in view of the patient's condition (see, e.g., Fingl et al., 1975, in: The Pharmacological Basis of Therapeutics, Ch. 1, p. 1, incorporated herein by reference in its entirety).

The attending physician for patients treated with fusion proteins of the invention would know how and when to terminate, interrupt, or adjust administration due to toxicity, organ dysfunction, and the like. Conversely, the attending physician would also know to adjust treatment to higher levels if the clinical response were not adequate (precluding toxicity). The magnitude of an administered dose in the management of the disorder of interest will vary with the severity of the condition to be treated, with the route of administration, and the like. The severity of the condition may, for example, be evaluated, in part, by standard prognostic evaluation methods. Further, the dose and perhaps dose frequency will also vary according to the age, body weight, and response of the individual patient.

**Other agents and treatments**

The trimeric costimulatory TNF family ligand-containing antigen binding molecules of the invention may be administered in combination with one or more other agents in therapy. For instance, a fusion protein of the invention may be co-administered with at least one additional therapeutic agent. The term "therapeutic agent" encompasses any agent that can be administered for treating a symptom or disease in an individual in need of such treatment. Such additional therapeutic agent may comprise any active ingredients suitable for the particular indication being treated, preferably those with complementary activities that do not adversely affect each other. In certain embodiments, an additional therapeutic agent is another anti-cancer agent.

Such other agents are suitably present in combination in amounts that are effective for the purpose intended. The effective amount of such other agents depends on the amount of fusion protein used, the type of disorder or treatment, and other factors discussed above. The trimeric costimulatory TNF family ligand-containing antigen binding molecules are generally used in the same dosages and with administration routes as described herein, or about from 1 to 99% of the dosages described herein, or in any dosage and by any route that is empirically/clinically determined to be appropriate.

Such combination therapies noted above encompass combined administration (where two or more therapeutic agents are included in the same or separate compositions), and separate administration, in which case, administration of the trimeric costimulatory TNF family ligand-containing antigen binding molecule of the invention can occur prior to, simultaneously, and/or following, administration of the additional therapeutic agent and/or adjuvant.

**Articles of Manufacture**

In another aspect of the invention, an article of manufacture containing materials useful for the treatment, prevention and/or diagnosis of the disorders described above is provided. The article of manufacture comprises a container and a label or package insert on or associated with the container. Suitable containers include, for example, bottles, vials, syringes, IV solution bags, etc. The containers may be formed from a variety of materials such as glass or plastic. The container holds a composition which is by itself or combined with another composition effective for treating, preventing and/or diagnosing the condition and may have a sterile access port (for example the container may be an intravenous solution bag or a vial having a stopper that is pierceable by a hypodermic injection needle). At least one active agent in the composition is a trimeric costimulatory TNF family ligand-containing antigen binding molecule of the invention.

The label or package insert indicates that the composition is used for treating the condition of choice. Moreover, the article of manufacture may comprise (a) a first container with a composition contained therein, wherein the composition comprises a trimeric costimulatory TNF family ligand-containing antigen binding molecule of the invention; and (b) a second container with a composition contained therein, wherein the composition comprises a further cytotoxic or otherwise therapeutic agent. The article of manufacture in this embodiment of the invention may further comprise a package insert indicating that the compositions can be used to treat a particular condition.

Alternatively, or additionally, the article of manufacture may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

**Table C (Sequences):**

| SEQ ID NO: | Name | Sequence |
|---|---|---|
| 1 | Full length human CMP | MRVLSGTSLMLCSLLLLLQALCSPGLAPQSRGHLCR TRPTDLVFVVDSSRSVRPVEFEKVKVFLSQVIESLD VGPNATRVGMVNYASTVKQEFSLRAHVSKAALLQ AVRRIQPLSTGTMTGLAIQFAITKAFGDAEGGRSRS PDISKVVIVVTDGRPQDSVQDVSARARASGVELFAI GVGSVDKATLRQIASEPQDEHVDYVESYSVIEKLSR KFQEAFCVVSDLCATGDHDCEQVCISSPGSYTCAC HEGFTLNSDGKTCNVCSGGGGSSATDLVFLIDGSKS VRPENFELVKKFISQIVDTLDVSDKLAQVGLVQYSS SVRQEFPLGRFHTKKDIKAAVRNMSYMEKGTMTG AALKYLIDNSFTVSSGARPGAQKVGIVFTDGRSQDY INDAAKKAKDLGFKMFAVGVGNAVEDELREIASEP VAEHYFYTADFKTINQIGKKLQKKICVEEDPCACES LVKFQAKVEGLLQALTRKLEAVSKRLAILENTVV |
| 2 | CMP trimerization domain | CACESLVKFQAKVEGLLQALTRKLEAVSKRLAILE NTVV |
| 3 | Human (hu) 4-1BBL (71-254) | REGPELSPDDPAGLLDLRQGMFAQLVAQNVLLIDG PLSWYSDPGLAGVSLTGGLSYKEDTKELVVAKAGV YYVFFQLELRRVVAGEGSGSVSLALHLQPLRSAAG AAALALTVDLPPASSEARNSAFGFQGRLLHLSAGQ RLGVHLHTEARARHAWQLTQGATVLGLFRVTPEIP AGLPSPRSE |
| 4 | hu 4-1BBL (85-254) | LDLRQGMFAQLVAQNVLLIDGPLSWYSDPGLAGVS LTGGLSYKEDTKELVVAKAGVYYVFFQLELRRVV AGEGSGSVSLALHLQPLRSAAGAAALALTVDLPPA SSEARNSAFGFQGRLLHLSAGQRLGVHLHTEARAR HAWQLTQGATVLGLFRVTPEIPAGLPSPRSE |
| 5 | hu 4-1BBL (80-254) | DPAGLLDLRQGMFAQLVAQNVLLIDGPLSWYSDPG LAGVSLTGGLSYKEDTKELVVAKAGVYYVFFQLEL RRVVAGEGSGSVSLALHLQPLRSAAGAAALALTVD LPPASSEARNSAFGFQGRLLHLSAGQRLGVHLHTEA RARHAWQLTQGATVLGLFRVTPEIPAGLPSPRSE |

(continued)

| SEQ ID NO: | Name | Sequence |
|---|---|---|
| 6 | hu 4-1BBL (52-254) | PWAVSGARASPGSAASPRLREGPELSPDDPAGLLDLRQGMFAQLVAQNVLLIDGPLSWYSDPGLAGVSLTGGLSYKEDTKELVVAKAGVYYVFFQLELRRVVAGEGSGSVSLALHLQPLRSAAGAAALALTVDLPPASSEARNSAFGFQGRLLHLSAGQRLGVHLHTEARARHAWQLTQGATVLGLFRVTPEIPAGLPSPRSE |
| 7 | Human (hu) 4-1BBL (71-248) | REGPELSPDDPAGLLDLRQGMFAQLVAQNVLLIDGPLSWYSDPGLAGVSLTGGLSYKEDTKELVVAKAGVYYVFFQLELRRVVAGEGSGSVSLALHLQPLRSAAGAAALALTVDLPPASSEARNSAFGFQGRLLHLSAGQRLGVHLHTEARARHAWQLTQGATVLGLFRVTPEIPAGL |
| 8 | hu 4-1BBL (85-248) | LDLRQGMFAQLVAQNVLLIDGPLSWYSDPGLAGVSLTGGLSYKEDTKELVVAKAGVYYVFFQLELRRVVAGEGSGSVSLALHLQPLRSAAGAAALALTVDLPPASSEARNSAFGFQGRLLHLSAGQRLGVHLHTEARARHAWQLTQGATVLGLFRVTPEIPAGL |
| 9 | hu 4-1BBL (80-248) | DPAGLLDLRQGMFAQLVAQNVLLIDGPLSWYSDPGLAGVSLTGGLSYKEDTKELVVAKAGVYYVFFQLELRRVVAGEGSGSVSLALHLQPLRSAAGAAALALTVDLPPASSEARNSAFGFQGRLLHLSAGQRLGVHLHTEARARHAWQLTQGATVLGLFRVTPEIPAGL |
| 10 | hu 4-1BBL (52-248) | PWAVSGARASPGSAASPRLREGPELSPDDPAGLLDLRQGMFAQLVAQNVLLIDGPLSWYSDPGLAGVSLTGGLSYKEDTKELVVAKAGVYYVFFQLELRRVVAGEGSGSVSLALHLQPLRSAAGAAALALTVDLPPASSEARNSAFGFQGRLLHLSAGQRLGVHLHTEARARHAWQLTQGATVLGLFRVTPEIPAGL |
| 11 | hu OX40L (51-183) | QVSHRYPRIQSIKVQFTEYKKEKGFILTSQKEDEIMKVQNNSVIINCDGFYLISLKGYFSQEVNISLHYQKDEEPLFQLKKVRSVNSLMVASLTYKDKVYLNVTTDNTSLDDFHVNGGELILIHQNPGEFCVL |
| 12 | hu OX40L (51-183) N90D, N114D | QVSHRYPRIQSIKVQFTEYKKEKGFILTSQKEDEIMKVQDNSVIINCDGFYLISLKGYFSQEVDISLHYQKDEEPLFQLKKVRSVNSLMVASLTYKDKVYLNVTTDNTSLDDFHVNGGELILIHQNPGEFCVL |
| 13 | hu OX40L (52-183) | VSHRYPRIQSIKVQFTEYKKEKGFILTSQKEDEIMKVQNNSVIINCDGFYLISLKGYFSQEVNISLHYQKDEEPLFQLKKVRSVNSLMVASLTYKDKVYLNVTTDNTSLDDFHVNGGELILIHQNPGEFCVL |
| 14 | FAP(28H1) CDR-H1 | SHAMS |
| 15 | FAP(28H1) CDR-H2 | AIWASGEQYYADSVKG |
| 16 | FAP(28H1) CDR-H3 | GWLGNFDY |
| 17 | FAP(28H1) CDR-L1 | RASQSVSRSYLA |
| 18 | FAP(28H1) CDR-L2 | GASTRAT |
| 19 | FAP(28H1) CDR-L3 | QQGQVIPPT |
| 20 | FAP(4B9) CDR-H1 | SYAMS |

(continued)

| SEQ ID NO: | Name | Sequence |
|---|---|---|
| 21 | FAP(4B9) CDR-H2 | AIIGSGASTYYADSVKG |
| 22 | FAP(4B9) CDR-H3 | GWFGGFNY |
| 23 | FAP(4B9) CDR-L1 | RASQSVTSSYLA |
| 24 | FAP(4B9) CDR-L2 | VGSRRAT |
| 25 | FAP(4B9) CDR-L3 | QQGIMLPPT |
| 26 | FAP(28H1) VH | EVQLLESGGGLVQPGGSLRLSCAASGFTFSSHA MSWVRQAPGKGLEWVSAIWASGEQYYADSV KGRFTISRDNSKNTLYLQMNSLRAEDTAVYYC AKGWLGNFDYWGQGTLVTVSS |
| 27 | FAP(28H1) VL | EIVLTQSPGTLSLSPGERATLSCRASQSVSRSYL AWYQQKPGQAPRLLIIGASTRATGIPDRFSGSG SGTDFTLTISRLEPEDFAVYYCQQGQVIPPTFGQ GTKVEIK |
| 28 | FAP(4B9) VH | EVQLLESGGGLVQPGGSLRLSCAASGFTFSSYAMS WVRQAPGKGLEWVSAIIGSGASTYYADSVKGRFTI SRDNSKNTLYLQMNSLRAEDTAVYYCAKGWFGGF NYWGQGTLVTVSS |
| 29 | FAP(4B9) VL | EIVLTQSPGTLSLSPGERATLSCRASQSVTSSYLAWY QQKPGQAPRLLINVGSRRATGIPDRFSGSGSGTDFT LTISRLEPEDFAVYYCQQGIMLPPTFGQGTKVEIK |
| 30 | Human (hu) FAP | UniProt no. Q12884 |
| 31 | hu FAP ectodomain+poly-lys-tag+$his_6$-tag | RPSRVHNSEENTMRALTLKDILNGTFSYKTFFPNWI SGQEYLHQSADNNIVLYNIETGQSYTILSNRTMKSV NASNYGLSPDRQFVYLESDYSKLWRYSYTATYYIY DLSNGEFVRGNELPRPIQYLCWSPVGSKLAYVYQN NIYLKQRPGDPPFQITFNGRENKIFNGIPDWVYEEE MLATKYALWWSPNGKFLAYAEFNDTDIPVIAYSYY GDEQYPRTINIPYPKAGAKNPVVRIFIIDTTYPAYVG PQEVPVPAMIASSDYYFSWLTWVTDERVCLQWLK RVQNVSVLSICDFREDWQTWDCPKTQEHIEESRTG WAGGFFVSTPVFSYDAISYYKIFSDKDGYKHIHYIK DTVENAIQITSGKWEAINIFRVTQDSLFYSSNEFEEY PGRRNIYRISIGSYPPSKKCVTCHLRKERCQYYTASF SDYAKYYALVCYGPGIPISTLHDGRTDQEIKILEENK ELENALKNIQLPKEEIKKLEVDEITLWYKMILPPQFD RSKKYPLLIQVYGGPCSQSVRSVFAVNWISYLASKE GMVIALVDGRGTAFQGDKLLYAVYRKLGVYEVED QITAVRKFIEMGFIDEKRIAIWGWSYGGYVSSLALA SGTGLFKCGIAVAPVSSWEYYASVYTERFMGLPTK DDNLEHYKNSTVMARAEYFRNVDYLLIHGTADDN VHFQNSAQIAKALVNAQVDFQAMWYSDQNHGLSG LSTNHLYTHMTHFLKQCFSLSDGKKKKKKGHHHH HH |

(continued)

| SEQ ID NO: | Name | Sequence |
|---|---|---|
| 32 | nucleotide sequence hu FAP ectodomain+poly-lys-tag+his$_6$-tag | CGCCCTTCAAGAGTTCATAACTCTGAAGAAAATA<br>CAATGAGAGCACTCACACTGAAGGATATTTTAAA<br>TG<br>GAACATTTTCTTATAAAACATTTTTTCCAAACTGG<br>ATTTCAGGACAAGAATATCTTCATCAATCTGCAG<br>ATAACAATATAGTACTTTATAATATTGAAACAGG<br>ACAATCATATACCATTTTGAGTAATAGAACCATG<br>AAAAGTGTGAATGCTTCAAATTACGGCTTATCAC<br>CTGATCGGCAATTTGTATATCTAGAAAGTGATTA<br>TTCAAAGCTTTGGAGATACTCTTACACAGCAACA<br>TATTACATCTATGACCTTAGCAATGGAGAATTTG<br>TAAGAGGAAATGAGCTTCCTCGTCCAATTCAGTA<br>TTTATGCTGGTCGCCTGTTGGGAGTAAATTAGCA<br>TATGTCTATCAAAACAATATCTATTTGAAACAAA<br>GACCAGGAGATCCACCTTTTCAAATAACATTTAA<br>TGGAAGAGAAAATAAAATATTTAATGGAATCCCA<br>GACTGGGTTTATGAAGAGGAAATGCTTGCTACAA<br>AATATGCTCTCTGGTGGTCTCCTAATGGAAAATTT<br>TTGGCATATGCGGAATTTAATGATACGGATATAC<br>CAGTTATTGCCTATTCCTATTATGGCGATGAACA<br>ATATCCTAGAACAATAAATATTCCATACCCAAAG<br>GCTGGAGCTAAGAATCCCGTTGTTCGGATATTTA<br>TTATCGATACCACTTACCCTGCGTATGTAGGTCCC<br>CAGGAAGTGCCTGTTCCAGCAATGATAGCCTCAA<br>GTGATTATTATTTCAGTTGGCTCACGTGGGTTACT<br>GATGAACGAGTATGTTTGCAGTGGCTAAAAAGAG<br>TCCAGAATGTTTCGGTCCTGTCTATATGTGACTTC<br>AGGGAAGACTGGCAGACATGGGATTGTCCAAAG<br>ACCCAGGAGCATATAGAAGAAAGCAGAACTGGA |

(continued)

| SEQ ID NO: | Name | Sequence |
|---|---|---|
| | | TGGGCTGGTGGATTCTTTGTTTCAACACCAGTTTT CAGCTATGATGCCATTTCGTACTACAAAATATTT AGTGACAAGGATGGCTACAAACATATTCACTATA TCAAAGACACTGTGGAAAATGCTATTCAAATTAC AAGTGGCAAGTGGGAGGCCATAAATATATTCAG AGTAACACAGGATTCACTGTTTTATTCTAGCAAT GAATTTGAAGAATACCCTGGAAGAAGAAACATCT ACAGAATTAGCATTGGAAGCTATCCTCCAAGCAA GAAGTGTGTTACTTGCCATCTAAGGAAAGAAAGG TGCCAATATTACACAGCAAGTTTCAGCGACTACG CCAAGTACTATGCACTTGTCTGCTACGGCCCAGG CATCCCCATTTCCACCCTTCATGATGGACGCACTG ATCAAGAAATTAAAATCCTGGAAGAAAACAAGG AATTGGAAAATGCTTTGAAAAATATCCAGCTGCC TAAAGAGGAAATTAAGAAACTTGAAGTAGATGA AATTACTTTATGGTACAAGATGATTCTTCCTCCTC AATTTGACAGATCAAAGAAGTATCCCTTGCTAAT TCAAGTGTATGGTGGTCCCTGCAGTCAGAGTGTA AGGTCTGTATTTGCTGTTAATTGGATATCTTATCT TGCAAGTAAGGAAGGGATGGTCATTGCCTTGGTG GATGGTCGAGGAACAGCTTTCCAAGGTGACAAAC TCCTCTATGCAGTGTATCGAAAGCTGGGTGTTTAT GAAGTTGAAGACCAGATTACAGCTGTCAGAAAAT TCATAGAAATGGGTTTCATTGATGAAAAAAGAAT AGCCATATGGGGCTGGTCCTATGGAGGATACGTT TCATCACTGGCCCTTGCATCTGGAACTGGTCTTTT CAAATGTGGTATAGCAGTGGCTCCAGTCTCCAGC TGGGAATATTACGCGTCTGTCTACACAGAGAGAT TCATGGGTCTCCCAACAAAGGATGATAATCTTGA GCACTATAAGAATTCAACTGTGATGGCAAGAGCA GAATATTTCAGAAATGTAGACTATCTTCTCATCC ACGGAACAGCAGATGATAATGTGCACTTTCAAAA CTCAGCACAGATTGCTAAAGCTCTGGTTAATGCA CAAGTGGATTTCCAGGCAATGTGGTACTCTGACC AGAACCACGGCTTATCCGGCCTGTCCACGAACCA CTTATACACCCACATGACCCACTTCCTAAAGCAG TGTTTCTCTTTGTCAGACGGCAAAAAGAAAAAGA AAAAGGGCCACCACCATCACCATCAC |
| 33 | mouse FAP | UniProt no. P97321 |
| 34 | Murine FAP ectodomain+poly-lys-tag+$his_6$-tag | RPSRVYKPEGNTKRALTLKDILNGTFSYKTYFPNWI SEQEYLIIQSEDDNIVFYNIETRESYIILSNSTMKSVN ATDYGLSPDRQFVYLESDYSKLWRYSYTATYYIYD LQNGEFVRGYELPRPIQYLCWSPVGSKLAYVYQNN IYLKQRPGDPPFQITYTGRENRIFNGIPDWVYEEML ATKYALWWSPDGKFLAYVEFNDSDIPIIAYSYYGD GQYPRTINIPYPKAGAKNPVVRVFIVDTTYPHHVGP MEVPVPEMIASSDYYFSWLTWVSSERVCLQWLKR VQNVSVLSICDFREDWHAWECPKNQEHVEESRTG WAGGFFVSTPAFSQDATSYYKIFSDKDGYKHIHYIK DTVENAIQITSGKWEAIYIFRVTQDSLFYSSNEFEGY PGRRNIYRISIGNSPPSKKCVTCHLRKERCQYYTASF SYKAKYYALVCYGPGLPISTLHDGRTDQEIQVLEEN |

(continued)

| SEQ ID NO: | Name | Sequence |
|---|---|---|
| | | KELENSLRNIQLPKVEIKKLKDGGLTFWYKMILPPQ FDRSKKYPLLIQVYGGPCSQSVKSVFAVNWITYLAS KEGIVIALVDGRGTAFQGDKFLHAVYRKLGVYEVE DQLTAVRKFIEMGFIDEERIAIWGWSYGGYVSSLAL ASGTGLFKCGIAVAPVSSWEYYASIYSERFMGLPTK DDNLEHYKNSTVMARAEYFRNVDYLLIHGTADDN VHFQNSAQIAKALVNAQVDFQAMWYSDQNHGILS GRSQNHLYTHMTHFLKQCFSLSDGKKKKKKGHHH HHH |
| 35 | nucleotide sequence of murine FAP ectodomain+poly-lys-tag+his$_6$-tag | CGTCCCTCAAGAGTTTACAAACCTGAAGGAAACA CAAAGAGAGCTCTTACCTTGAAGGATATTTTAAA TGGAACATTCTCATATAAAACATATTTTCCCAACT GGATTTCAGAACAAGAATATCTTCATCAATCTGA GGATGATAACATAGTATTTTATAATATTGAAACA AGAGAATCATATATCATTTTGAGTAATAGCACCA TGAAAAGTGTGAATGCTACAGATTATGGTTTGTC ACCTGATCGGCAATTTGTGTATCTAGAAAGTGAT TATTCAAAGCTCTGGCGATATTCATACACAGCGA CATACTACATCTACGACCTTCAGAATGGGGAATT TGTAAGAGGATACGAGCTCCCTCGTCCAATTCAG TATCTATGCTGGTCGCCTGTTGGGAGTAAATTAG CATATGTATATCAAAACAATATTTATTTGAAACA AAGACCAGGAGATCCACCTTTTCAAATAACTTAT ACTGGAAGAGAAAATAGAATATTTAATGGAATA CCAGACTGGGTTTATGAAGAGGAAATGCTTGCCA CAAAATATGCTCTTTGGTGGTCTCCAGATGGAAA ATTTTTGGCATATGTAGAATTTAATGATTCAGATA TACCAATTATTGCCTATTCTTATTATGGTGATGGA CAGTATCCTAGAACTATAAATATTCCATATCCAA AGGCTGGGGCTAAGAATCCGGTTGTTCGTGTTTT TATTGTTGACACCACCTACCCTCACCACGTGGGC CCAATGGAAGTGCCAGTTCCAGAAATGATAGCCT CAAGTGACTATTATTTCAGCTGGCTCACATGGGT GTCCAGTGAACGAGTATGCTTGCAGTGGCTAAAA AGAGTGCAGAATGTCTCAGTCCTGTCTATATGTG ATTTCAGGGAAGACTGGCATGCATGGGAATGTCC AAAGAACCAGGAGCATGTAGAAGAAAGCAGAAC AGGATGGGCTGGTGGATTCTTTGTTTCGACACCA GCTTTTAGCCAGGATGCCACTTCTTACTACAAAA TATTTAGCGACAAGGATGGTTACAAACATATTCA CTACATCAAAGACACTGTGGAAAATGCTATTCAA ATTACAAGTGGCAAGTGGGAGGCCATATATATAT TCCGCGTAACACAGGATTCACTGTTTTATTCTAGC AATGAATTTGAAGGTTACCCTGGAAGAAGAAAC ATCTACAGAATTAGCATTGGAAACTCTCCTCCGA GCAAGAAGTGTGTTACTTGCCATCTAAGGAAAGA AAGGTGCCAATATTACACAGCAAGTTTCAGCTAC AAAGCCAAGTACTATGCACTCGTCTGCTATGGCC CTGGCCTCCCCATTTCCACCCTCCATGATGGCCGC ACAGACCAAGAAATACAAGTATTAGAAGAAAAC AAAGAACTGGAAAATTCTCTGAGAAATATCCAGC TGCCTAAAGTGGAGATTAAGAAGCTCAAAGACG |

(continued)

| SEQ ID NO: | Name | Sequence |
|---|---|---|
| | | GGGGACTGACTTTCTGGTACAAGATGATTCTGCC TCCTCAGTTTGACAGATCAAAGAAGTACCCTTTG CTAATTCAAGTGTATGGTGGTCCTTGTAGCCAGA GTGTTAAGTCTGTGTTTGCTGTTAATTGGATAACT TATCTCGCAAGTAAGGAGGGGATAGTCATTGCCC TGGTAGATGGTCGGGGCACTGCTTTCCAAGGTGA CAAATTCCTGCATGCCGTGTATCGAAAACTGGGT GTATATGAAGTTGAGGACCAGCTCACAGCTGTCA GAAAATTCATAGAAATGGGTTTCATTGATGAAGA AAGAATAGCCATATGGGGCTGGTCCTACGGAGGT TATGTTTCATCCCTGGCCCTTGCATCTGGAACTGG TCTTTTCAAATGTGGCATAGCAGTGGCTCCAGTCT CCAGCTGGGAATATTACGCATCTATCTACTCAGA GAGATTCATGGGCCTCCCAACAAAGGACGACAAT CTCGAACACTATAAAAATTCAACTGTGATGGCAA GAGCAGAATATTTCAGAAATGTAGACTATCTTCT CATCCACGGAACAGCAGATGATAATGTGCACTTT CAGAACTCAGCACAGATTGCTAAAGCTTTGGTTA ATGCACAAGTGGATTTCCAGGCGATGTGGTACTC TGACCAGAACCATGGTATATTATCTGGGCGCTCC CAGAATCATTTATATACCCACATGACGCACTTCC TCAAGCAATGCTTTTCTTTATCAGACGGCAAAAA GAAAAAGAAAAAGGGCCACCACCATCACCATCA C |
| 36 | Cynomolgus FAP ectodomain+poly-lys-tag+his$_6$-tag | RPPRVHNSEENTMRALTLKDILNGTFSYKTFFPNWI SGQEYLHQSADNNIVLYNIETGQSYTILSNRTMKSV NASNYGLSPDRQFVYLESDYSKLWRYSYTATYYIY DLSNGEFVRGNELPRPIQYLCWSPVGSKLAYVYQN NIYLKQRPGDPPFQITFNGRENKIFNGIPDWVYEEE MLATKYALWWSPNGKFLAYAEFNDTDIPVIAYSYY GDEQYPRTINIPYPKAGAKNPFVRIFIIDTTYPAYVG PQEVPVPAMIASSDYYFSWLTWVTDERVCLQWLK RVQNVSVLSICDFREDWQTWDCPKTQEHIEESRTG WAGGFFVSTPVFSYDAISYYKIFSDKDGYKHIHYIK DTVENAIQITSGKWEAINIFRVTQDSLFYSSNEFEDY PGRRNIYRISIGSYPPSKKCVTCHLRKERCQYYTASF SDYAKYYALVCYGPGIPISTLHDGRTDQEIKILEENK ELENALKNIQLPKEEIKKLEVDEITLWYKMILPPQFD RSKKYPLLIQVYGGPCSQSVRSVFAVNWISYLASKE GMVIALVDGRGTAFQGDKLLYAVYRKLGVYEVED QITAVRKFIEMGFIDEKRIAIWGWSYGGYVSSLALA SGTGLFKCGIAVAPVSSWEYYASVYTERFMGLPTK DDNLEHYKNSTVMARAEYFRNVDYLLIHGTADDN VHFQNSAQIAKALVNAQVDFQAMWYSDQNHGLSG LSTNHLYTHMTHFLKQCFSLSDGKKKKKKGHHHH HH |
| 37 | nucleotide sequence of cynomolgus FAP ectodomain+poly-lys-tag+his$_6$-tag | CGCCCTCCAAGAGTTCATAACTCTGAAGAAAATA CAATGAGAGCACTCACACTGAAGGATATTTTAAA TGGGACATTTTCTTATAAAACATTTTTTCCAAACT GGATTTCAGGACAAGAATATCTTCATCAATCTGC AGATAACAATATAGTACTTTATAATATTGAAACA GGACAATCATATACCATTTTGAGTAACAGAACCA |

(continued)

| SEQ ID NO: | Name | Sequence |
|---|---|---|
| | | TGAAAAGTGTGAATGCTTCAAATTATGGCTTATC ACCTGATCGGCAATTTGTATATCTAGAAAGTGAT TATTCAAAGCTTTGGAGATACTCTTACACAGCAA CATATTACATCTATGACCTTAGCAATGGAGAATT TGTAAGAGGAAATGAGCTTCCTCGTCCAATTCAG TATTTATGCTGGTCGCCTGTTGGGAGTAAATTAG CATATGTCTATCAAAACAATATCTATTTGAAACA AAGACCAGGAGATCCACCTTTTCAAATAACATTT AATGGAAGAGAAAATAAAATATTTAATGGAATC CCAGACTGGGTTTATGAAGAGGAAATGCTTGCTA CAAAATATGCTCTCTGGTGGTCTCCTAATGGAAA ATTTTTGGCATATGCGGAATTTAATGATACAGAT ATACCAGTTATTGCCTATTCCTATTATGGCGATGA ACAATATCCCAGAACAATAAATATTCCATACCCA AAGGCCGGAGCTAAGAATCCTTTTGTTCGGATAT TTATTATCGATACCACTTACCCTGCGTATGTAGGT CCCCAGGAAGTGCCTGTTCCAGCAATGATAGCCT CAAGTGATTATTATTTCAGTTGGCTCACGTGGGTT ACTGATGAACGAGTATGTTTGCAGTGGCTAAAAA GAGTCCAGAATGTTTCGGTCTTGTCTATATGTGAT TTCAGGGAAGACTGGCAGACATGGGATTGTCCAA AGACCCAGGAGCATATAGAAGAAAGCAGAACTG GATGGGCTGGTGGATTCTTTGTTTCAACACCAGTT TTCAGCTATGATGCCATTTCATACTACAAAATATT TAGTGACAAGGATGGCTACAAACATATTCACTAT ATCAAAGACACTGTGGAAAATGCTATTCAAATTA CAAGTGGCAAGTGGGAGGCCATAAATATATTCAG AGTAACACAGGATTCACTGTTTTATTCTAGCAAT GAATTTGAAGATTACCCTGGAAGAAGAAACATCT ACAGAATTAGCATTGGAAGCTATCCTCCAAGCAA GAAGTGTGTTACTTGCCATCTAAGGAAAGAAAGG TGCCAATATTACACAGCAAGTTTCAGCGACTACG CCAAGTACTATGCACTTGTCTGCTATGGCCCAGG CATCCCCATTTCCACCCTTCATGACGGACGCACT GATCAAGAAATTAAAATCCTGGAAGAAAACAAG GAATTGGAAAATGCTTTGAAAAATATCCAGCTGC CTAAAGAGGAAATTAAGAAACTTGAAGTAGATG AAATTACTTTATGGTACAAGATGATTCTTCCTCCT CAATTTGACAGATCAAAGAAGTATCCCTTGCTAA TTCAAGTGTATGGTGGTCCCTGCAGTCAGAGTGT AAGGTCTGTATTTGCTGTTAATTGGATATCTTATC TTGCAAGTAAGGAAGGGATGGTCATTGCCTTGGT GGATGGTCGGGGAACAGCTTTCCAAGGTGACAA ACTCCTGTATGCAGTGTATCGAAAGCTGGGTGTT TATGAAGTTGAAGACCAGATTACAGCTGTCAGAA AATTCATAGAAATGGGTTTCATTGATGAAAAAAG AATAGCCATATGGGGCTGGTCCTATGGAGGATAT GTTTCATCACTGGCCCTTGCATCTGGAACTGGTCT TTTCAAATGTGGGATAGCAGTGGCTCCAGTCTCC AGCTGGGAATATTACGCGTCTGTCTACACAGAGA GATTCATGGGTCTCCCAACAAAGGATGATAATCT TGAGCACTATAAGAATTCAACTGTGATGGCAAGA |
| | | GCAGAATATTTCAGAAATGTAGACTATCTTCTCA TCCACGGAACAGCAGATGATAATGTGCACTTTCA AAACTCAGCACAGATTGCTAAAGCTCTGGTTAAT GCACAAGTGGATTTCCAGGCAATGTGGTACTCTG ACCAGAACCACGGCTTATCCGGCCTGTCCACGAA CCACTTATACACCCACATGACCCACTTCCTAAAG CAGTGTTTCTCTTTGTCAGACGGCAAAAAGAAAA AGAAAAAGGGCCACCACCATCACCATCAC |

(continued)

| SEQ ID NO: | Name | Sequence |
|---|---|---|
| 38 | human CEA | UniProt no. P06731 |
| 39 | human MCSP | UniProt no. Q6UVK1 |
| 40 | human EGFR | UniProt no. P00533 |
| 41 | human CD 19 | UniProt no. P15391 |
| 42 | human CD20 | Uniprot no. P11836 |
| 43 | human CD33 | UniProt no. P20138 |
| 44 | human OX40L | UniProt no. P23510 |
| 45 | human CD27L | UniProt no. P32970 |
| 46 | human CD30L | UniProt no. P32971 |
| 47 | human 4-1BBL | UniProt no. P41273 |
| 48 | human LIGHT | UniProt no. 043557 |
| 49 | human GITRL | UniProt no. Q9UNG2 |
| 50 | hu 4-1BBL (50-254) | ACPWAVSGARASPGSAASPRLREGPELSPDDPAGL<br>LDLRQGMFAQLVAQNVLLIDGPLSWYSDPGLAGVS<br>LTGGLSYKEDTKELVVAKAGVYYVFFQLELRRVV<br>AGEGSGSVSLALHLQPLRSAAGAAALALTVDLPPA<br>SSEARNSAFGFQGRLLHLSAGQRLGVHLHTEARAR<br>HAWQLTQGATVLGLFRVTPEIPAGLPSPRSE |
| 51 | Petpide linker G4S | GGGGS |
| 52 | Peptide linker $(G4S)_2$ | GGGGSGGGGS |
| 53 | Peptide linker $(SG4)_2$ | SGGGGSGGGG |
| 54 | Peptide linker $(G_4S)_3$ | GGGGSGGGGSGGGGS |
| 55 | Peptide linker $G4(SG4)_2$ | GGGGSGGGGSGGGG |
| 56 | Peptide linker $(G_4S)_4$ | GGGGSGGGGSGGGGSGGGGS |
| 57 | Peptide linker | GSPGSSSSGS |
| 58 | Peptide linker | GSGSGSGS |
| 59 | Peptide linker | GSGSGNGS |
| 60 | Peptide linker | GGSGSGSG |
| 61 | Peptide linker | GGSGSG |
| 62 | Peptide linker | GGSG |
| 63 | Peptide linker | GGSGNGSG |
| 64 | Peptide linker | GGNGSGSG |
| 65 | Peptide linker | GGNGSG |
| 66 | human TRAF2 | UniProt no. Q12933 |
| 67 | human Thrombospondin 1 | UniProt no. P07996 |
| 68 | human Matrilin-4 | UniProt no. 095460 |
| 69 | human Cubilin | UniProt no. 060494 |
| 70 | isoleucine zipper domain | IKQIEDKIEE ILSKIYHIEN EIARIKKLIG ER |
| 71 | FAP(28H1)(VHCH1)-CMPtd-4-1BBL | see Table 1 |

(continued)

| SEQ ID NO: | Name | Sequence |
|---|---|---|
| 72 | FAP(28H1)(VHCH1)-CMPtd-Ox40L(52-183) | see Table 8 |
| 73 | Nucleotide sequence of FAP(28H1)(VHCH1)-CMPtd-4-1BBL | see Table 1 |
| 74 | Nucleotide sequence of FAP(28H1)(VHCH1)-CMPtd-Ox40L(52-183) | see Table 8 |
| 75 | Nucleotide sequence of anti-FAP(28H1) light chain | see Table 1 |
| 76 | anti-FAP(28H1) light chain | see Table 1 |
| 77 | Nucleotide sequence of DP47 (VHCH1)-CMPtd-4-1BBL(71-254) | see Table 4 |
| 78 | Nucleotide sequence of Germline control (DP47) light chain | see Table 4 |
| 79 | DP47(VHCH1)-CMPtd-4-1BBL(71-254) | see Table 4 |
| 80 | Germline control (DP47) light chain | see Table 4 |
| 81 | Nucleotide sequence of DP47 heavy chain (hu IgG1 PG LALA) | see Table 6 |
| 82 | DP47 heavy chain (hu IgG1 PG LALA) | see Table 6 |
| 83 | Human 4-1BB ECD | Uniprot No. Q07011, aa 24-186 |
| 84 | Cynomolgus 4-1BB ECD | aa 24-186 |
| 85 | Murine 4-1BB ECD | Uniprot No. P20334, aa 24-187 |
| 86 | Nucleotide sequence of Fc hole chain | see Table 11 |
| 87 | Nucleotide sequence of human 4-1BB antigen Fc knob chain | see Table 11 |
| 88 | Nucleotide sequence of cynomolgus 4-1BB antigen Fc knob chain | see Table 11 |
| 89 | Nucleotide sequence of murine 4-1BB antigen Fc knob chain | see Table 11 |
| 90 | Fc hole chain | see Table 11 |
| 91 | human 4-1BB antigen Fc knob chain | see Table 11 |
| 92 | cynomolgus 4-1BB antigen Fc knob chain | see Table 11 |
| 93 | murine 4-1BB antigen Fc knob chain | see Table 11 |

Aspects of the invention are the following:

- A trimeric costimulatory TNF family ligand-containing antigen binding molecule comprising three fusion polypeptides, each of the three fusion polypeptides comprising

  (a) an ectodomain of a costimulatory TNF ligand family member or fragments thereof,
  (b) a trimerization domain, and
  (c) a moiety capable of specific binding to a target cell antigen

- The trimeric costimulatory TNF family ligand-containing antigen binding molecule as defined herein before comprising three fusion polypeptides, each of the three fusion polypeptides comprising

(a) an ectodomain of a costimulatory TNF ligand family member or fragments thereof,
(b) a trimerization domain derived from human cartilage matrix protein (huCMP) of amino acid sequence of SEQ ID NO:1, and
(c) a moiety capable of specific binding to a target cell antigen.

- The trimeric costimulatory TNF family ligand-containing antigen binding molecule as defined herein before, wherein the trimerization domain comprises an amino acid sequence having at least 95% identity to SEQ ID NO:2.

- The trimeric costimulatory TNF family ligand-containing antigen binding molecule as defined herein before, wherein the trimerization domain comprises the amino acid sequence of SEQ ID NO:2.

- The trimeric costimulatory TNF family ligand-containing antigen binding molecule as defined herein before, wherein the costimulatory TNF ligand family member is selected from 4-1BBL and OX40L.

- The trimeric costimulatory TNF family ligand-containing antigen binding molecule as defined herein before, wherein the costimulatory TNF ligand family member is 4-1BBL.

- The trimeric costimulatory TNF family ligand-containing antigen binding molecule as defined herein before, wherein the ectodomain of a TNF ligand family member comprises the amino acid sequence selected from the group consisting of SEQ ID NO:3, SEQ ID NO:4, SEQ ID NO:5, SEQ ID NO:6, SEQ ID NO:7, SEQ ID NO:8, SEQ ID NO:9 and SEQ ID NO: 10, particularly the amino acid sequence of SEQ ID NO:3 or SEQ ID NO:7.

- The trimeric costimulatory TNF family ligand-containing antigen binding molecule as defined herein before, comprising three fusion polypeptides, each of the three fusion polypeptides comprising

  (a) an ectodomain of a TNF ligand family comprising the amino acid sequence selected from the group consisting of SEQ ID NO:3, SEQ ID NO:4, SEQ ID NO:5, SEQ ID NO:6, SEQ ID NO:7, SEQ ID NO:8, SEQ ID NO:9 and SEQ ID NO: 10,
  (b) a trimerization domain comprising the amino acid sequence of SEQ ID NO:2 and
  (c) a moiety capable of specific binding to a target cell antigen.

- The trimeric costimulatory TNF family ligand-containing antigen binding molecule as defined herein before, wherein the costimulatory TNF ligand family member is OX40L.

- The trimeric costimulatory TNF family ligand-containing antigen binding molecule as defined herein before, wherein the ectodomain of a TNF ligand family member comprises the amino acid sequence of SEQ ID NO: 11 or SEQ ID NO: 12.

- The trimeric costimulatory TNF family ligand-containing antigen binding molecule as defined herein before, comprising three fusion polypeptides, wherein each of the three fusion polypeptides comprises

  (a) an ectodomain of a TNF ligand family comprising the amino acid sequence of SEQ ID NO: 11 or SEQ ID NO: 12,
  (b) a trimerization domain comprising the amino acid sequence of SEQ ID NO:2 and
  (c) a moiety capable of specific binding to a target cell antigen.

- The trimeric costimulatory TNF family ligand-containing antigen binding molecule as defined herein before, wherein the ectodomain of a TNF ligand family member or a fragment thereof is fused at the N-terminal amino acid to the C-terminal amino acid of the trimerization domain, optionally through a peptide linker.

- The trimeric costimulatory TNF family ligand-containing antigen binding molecule as defined herein before, wherein the moiety capable of specific binding to a target cell antigen is fused at the C-terminal amino acid to the N-terminal amino acid of the trimerization domain, optionally through a peptide linker.

- The trimeric costimulatory TNF family ligand-containing antigen binding molecule as defined herein before, wherein the moiety capable of specific binding to a target cell antigen is selected from the group consisting of an antibody fragment, a Fab molecule, a crossover Fab molecule, a single chain Fab molecule, a Fv molecule, a scFv molecule, a single domain antibody, an aVH and a scaffold antigen binding protein.

- The trimeric costimulatory TNF family ligand-containing antigen binding molecule as defined herein before, wherein the moiety capable of specific binding to a target cell antigen is a Fab molecule capable of specific binding to a target cell antigen.

- The trimeric costimulatory TNF family ligand-containing antigen binding molecule as defined herein before, wherein said Fab molecule is fused at the C-terminal amino acid of the CH1 domain to the N-terminal amino acid of the trimerization domain, optionally through a peptide linker.

- The trimeric costimulatory TNF family ligand-containing antigen binding molecule as defined herein before, wherein the target cell antigen is selected from the group consisting of Fibroblast Activation Protein (FAP), Melanoma-associated Chondroitin Sulfate Proteoglycan (MCSP), Epidermal Growth Factor Receptor (EGFR), Carcinoembryonic Antigen (CEA), CD 19, CD20 and CD33.

- The trimeric costimulatory TNF family ligand-containing antigen binding molecule as defined herein before, wherein the target cell antigen is Fibroblast Activation Protein (FAP).

- The trimeric costimulatory TNF family ligand-containing antigen binding molecule as defined herein before, wherein the moiety capable of specific binding to FAP comprises

  (a) a VH domain comprising (i) CDR-H1 comprising the amino acid sequence of SEQ ID NO: 13, (ii) CDR-H2 comprising the amino acid sequence of SEQ ID NO:14 and (iii) CDR-H3 comprising the amino acid sequence of SEQ ID NO:15, and a VL domain comprising (iv) CDR-L1 comprising the amino acid sequence of SEQ ID NO:16, (v) CDR-L2 comprising the amino acid sequence of SEQ ID NO: 17 and (vi) CDR-L3 comprising the amino acid sequence of SEQ ID NO:18 or
  (b) a VH domain comprising (i) CDR-H1 comprising the amino acid sequence of SEQ ID NO:19, (ii) CDR-H2 comprising the amino acid sequence of SEQ ID NO:20 and (iii) CDR-H3 comprising the amino acid sequence of SEQ ID NO:21, and a VL domain comprising (iv) CDR-L1 comprising the amino acid sequence of SEQ ID NO:22, (v) CDR-L2 comprising the amino acid sequence of SEQ ID NO:23 and (vi) CDR-L3 comprising the amino acid sequence of SEQ ID NO:24.

- The trimeric costimulatory TNF family ligand-containing antigen binding molecule as defined herein before, wherein said three fusion polypeptides are identical.

- A fusion polypeptide comprising (a) an ectodomain of a costimulatory TNF ligand family member or a fragment thereof and (b) a trimerization domain derived from human cartilage matrix protein (huCMP) comprising the amino acid sequence of SEQ ID NO:2, wherein said trimerization domain is capable of mediating stable association of said fusion polypeptide with two further such fusion polypeptides.

- The fusion polypeptide as defined herein before, wherein the fusion polypeptide further comprises (c) a moiety capable of specific binding to a target cell antigen.

- The fusion polypeptide as defined herein before, wherein the fusion polypeptide comprises

  (a) an ectodomain of a TNF ligand family comprising the amino acid sequence selected from the group consisting of SEQ ID NO:3, SEQ ID NO:4, SEQ ID NO:5, SEQ ID NO:6, SEQ ID NO:7, SEQ ID NO:8, SEQ ID NO:9 and SEQ ID NO: 10,
  (b) a trimerization domain comprising the amino acid sequence of SEQ ID NO:2 and
  (c) a VH domain comprising (i) CDR-H1 comprising the amino acid sequence of SEQ ID NO:13, (ii) CDR-H2 comprising the amino acid sequence of SEQ ID NO:14 and (iii) CDR-H3 comprising the amino acid sequence of SEQ ID NO:15 or a VH domain comprising (i) CDR-H1 comprising the amino acid sequence of SEQ ID NO:19, (ii) CDR-H2 comprising the amino acid sequence of SEQ ID NO:20and (iii) CDR-H3 comprising the amino acid sequence of SEQ ID NO:21.

- A polynucleotide encoding the trimeric costimulatory TNF family ligand-containing antigen binding molecule as defined herein before or the fusion polypeptide as defined herein before.

- An expression vector comprising the polynucleotide as defined herein before.

- A host cell comprising the polynucleotide as defined herein before or the expression vector as defined herein before.
- A method of producing a trimeric costimulatory TNF family ligand-containing antigen binding molecule, comprising culturing the host cell as defined herein before under conditions suitable for the expression of said trimeric antigen binding molecule and isolating said trimeric antigen binding molecule.
- A trimeric costimulatory TNF family ligand-containing antigen binding molecule produced by the method as defined herein before.
- A pharmaceutical composition comprising the trimeric costimulatory TNF family ligand-containing antigen binding molecule as defined herein before and at least one pharmaceutically acceptable excipient.
- The trimeric costimulatory TNF family ligand-containing antigen binding molecule as defined herein before, or the pharmaceutical composition of claim 29, for use as a medicament.
- The trimeric costimulatory TNF family ligand-containing antigen binding molecule as defined herein before, or the pharmaceutical composition as defined herein before, for use in the treatment of cancer.

## EXAMPLES

The following are examples of methods and compositions of the invention. It is understood that various other embodiments may be practiced, given the general description provided above.

### Recombinant DNA techniques

Standard methods were used to manipulate DNA as described in Sambrook et al., Molecular cloning: A laboratory manual; Cold Spring Harbor Laboratory Press, Cold Spring Harbor, New York, 1989. The molecular biological reagents were used according to the manufacturer's instructions. General information regarding the nucleotide sequences of human immunoglobulin light and heavy chains is given in: Kabat, E.A. et al., (1991) Sequences of Proteins of Immunological Interest, Fifth Ed., NIH Publication No 91-3242.

### DNA sequencing

DNA sequences were determined by double strand sequencing.

### Gene synthesis

Desired gene segments were either generated by PCR using appropriate templates or were synthesized by Geneart AG (Regensburg, Germany) from synthetic oligonucleotides and PCR products by automated gene synthesis. In cases where no exact gene sequence was available, oligonucleotide primers were designed based on sequences from closest homologues and the genes were isolated by RT-PCR from RNA originating from the appropriate tissue. The gene segments flanked by singular restriction endonuclease cleavage sites were cloned into standard cloning / sequencing vectors. The plasmid DNA was purified from transformed bacteria and concentration determined by UV spectroscopy. The DNA sequence of the subcloned gene fragments was confirmed by DNA sequencing. Gene segments were designed with suitable restriction sites to allow sub-cloning into the respective expression vectors. All constructs were designed with a 5'-end DNA sequence coding for a leader peptide which targets proteins for secretion in eukaryotic cells.

### Example 1

#### 1.1. Preparation and purification of targeted huCMP trimeric 4-1BB ligand-containing antigen binding molecules

To enhance binding of Fab molecules to specific members of the TNF receptor superfamily and thereby enhanced cross-linking of these receptors, trimerized Fab molecules targeting 4-1BB were generated.

The Fab genes (VH-CH1) against fibroblast activation protein (FAP clone 28H1) were fused via a $(G4S)_2$ linker to a short trimerization domain derived from human cartilage matrix protein (huCMP) (Uniprot Accession: P21941, SEQ ID NO:1; Residues 454 to 496, SEQ ID NO.:2) by standard recombinant DNA technologies. The cysteine residues forming interchain disulfide bridges at positions 458 and 460 were used together with the coiled coil domain comprising residues 467 to 495.

The DNA sequence encoding part of the ectodomain (amino acids 71-254 or 71-248) of human **4-1BB** ligand was synthetized according to P41273 (Uniprot Accession) and fused via a $(G_4S)_4$ linker downstream of the trimerization domain.

The chain encoding FAP-targeted 4-1BB ligand and the light chain with FAP specificity are shown in **Figure 1a.** Disulphide bonds are formed between three FAP-targeted 4-1BB ligand chains leading to the formation of covalently linked CMP-trimeric 4-1BB ligand antigen binding molecules, targeted to FAP. **Figure 1b** shows the trimeric form.

**Table 1** shows, respectively, the cDNA and amino acid sequences of the trimeric FAP-targeted 4-1BBL-containing fusion polypeptide, which is trimerizing via CMP trimerization domain (CMPtd) to form a trimeric FAP-targeted 4-1BBL-containing antigen binding molecule of the invention.

**Table 1: cDNA and amino acid sequences of FAP(28H1)-targeted hu CMP (CMPtd) trimeric human 4-1BBL(71-254)-containing antigen binding molecule**

| SEQ ID NO: | Description | Sequence |
|---|---|---|
| 73 | nucleotide sequence of FAP(28H1)(VHCH1)-CMPtd-4-1BBL (71-254) | GAAGTGCAGCTGCTGGAATCCGGCGGAGGCCTG GTGCAGCCTGGCGGATCTCTGAGACTGTCCTGC GCCGCCTCCGGCTTCACCTTCTCCTCCCACGCCA TGTCCTGGGTCCGACAGGCTCCTGGCAAAGGCC TGGAATGGGTGTCCGCCATCTGGGCCTCCGGCG AGCAGTACTACGCCGACTCTGTGAAGGGCCGGT TCACCATCTCCCGGGACAACTCCAAGAACACCC TGTACCTGCAGATGAACTCCCTGCGGGCCGAGG |

(continued)

| SEQ ID NO: | Description | Sequence |
|---|---|---|
| | | ACACCGCCGTGTACTACTGTGCCAAGGGCTGGC TGGGCAACTTCGACTACTGGGGACAGGGCACCC TGGTCACCGTGTCCAGCGCTAGCACAAAGGGAC CTAGCGTGTTCCCCCTGGCCCCCAGCAGCAAGT CTACATCTGGCGGAACAGCCGCCCTGGGCTGCC TCGTGAAGGACTACTTTCCCGAGCCCGTGACCG TGTCCTGGAACTCTGGCGCTCTGACAAGCGGCG TGCACACCTTTCCAGCCGTGCTGCAGAGCAGCG GCCTGTACTCTCTGAGCAGCGTCGTGACAGTGC CCAGCAGCTCTCTGGGCACCCAGACCTACATCT GCAACGTGAACCACAAGCCCAGCAACACCAAG GTGGACAAGAAGGTGGAACCCAAGAGCTGCGA CGGCGGAGGGGGATCTGGCGGCGGAGGATCCG AGGAAGATCCTTGCGCCTGCGAGAGCCTCGTGA AGTTCCAGGCCAAGGTGGAAGGACTGCTGCAG GCCCTGACCCGGAAACTGGAAGCCGTGTCCAAG CGGCTGGCCATCCTGGAAAACACCGTGGTGTCC GGAGGCGGAGGATCTGGCGGCGGAGGAAGTGG CGGAGGCGGATCTGGCGGCGGAGGATCTAGAG AGGGACCCGAACTGTCCCCTGACGATCCAGCCG GGCTGCTGGATCTGAGACAGGGAATGTTCGCCC AGCTGGTGGCTCAGAATGTGCTGCTGATTGACG GACCTCTGAGCTGGTACTCCGACCCAGGGCTGG CAGGGGTGTCCCTGACTGGGGGACTGTCCTACA AAGAAGATACAAAAGAACTGGTGGTGGCTAAA GCTGGGGTGTACTATGTGTTTTTTCAGCTGGAAC TGAGGCGGGTGGTGGCTGGGGAGGGCTCAGGA TCTGTGTCCCTGGCTCTGCATCTGCAGCCACTGC GCTCTGCTGCTGGCGCAGCTGCACTGGCTCTGA CTGTGGACCTGCCACCAGCCTCTAGCGAGGCCA GAAACAGCGCCTTCGGGTTCCAAGGACGCCTGC TGCATCTGAGCGCCGGACAGCGCCTGGGAGTGC ATCTGCATACTGAAGCCAGAGCCCGGCATGCTT GGCAGCTGACTCAGGGGGCAACTGTGCTGGGAC TGTTTCGCGTGACACCTGAGATCCCTGCCGGAC TGCCAAGCCCTAGATCAGAA |

(continued)

| SEQ ID NO: | Description | Sequence |
|---|---|---|
| 75 | nucleotide sequence of anti-FAP(28H1) light chain | GAGATCGTGCTGACCCAGTCCCCCGGCACCCTG TCTCTGAGCCCTGGCGAGAGAGCCACCCTGTCC TGCAGAGCCTCCCAGTCCGTGTCCCGGTCCTAC CTCGCCTGGTATCAGCAGAAGCCCGGCCAGGCC CCTCGGCTGCTGATCATCGGCGCCTCTACCAGA GCCACCGGCATCCCTGACCGGTTCTCCGGCTCT GGCTCCGGCACCGACTTCACCCTGACCATCTCC CGGCTGGAACCCGAGGACTTCGCCGTGTACTAC TGCCAGCAGGGCCAGGTCATCCCTCCCACCTTT GGCCAGGGCACCAAGGTGGAAATCAAGCGTAC GGTGGCTGCACCATCTGTCTTCATCTTCCCGCCA TCTGATGAGCAGTTGAAATCTGGAACTGCCTCT GTTGTGTGCCTGCTGAATAACTTCTATCCCAGA GAGGCCAAAGTACAGTGGAAGGTGGATAACGC CCTCCAATCGGGTAACTCCCAGGAGAGTGTCAC AGAGCAGGACAGCAAGGACAGCACCTACAGCC TCAGCAGCACCCTGACGCTGAGCAAAGCAGACT ACGAGAAACACAAAGTCTACGCCTGCGAAGTC |
| | | ACCCATCAGGGCCTGAGCTCGCCCGTCACAAAG AGCTTCAACAGGGGAGAGTGT |
| 71 | FAP(28H1)(VHCH1)-CMPtd-4-1BBL(71-254) | EVQLLESGGGLVQPGGSLRLSCAASGFTFSSHAMS WVRQAPGKGLEWVSAIWASGEQYYADSVKGRFT ISRDNSKNTLYLQMNSLRAEDTAVYYCAKGWLG NFDYWGQGTLVTVSSASTKGPSVFPLAPSSKSTSG GTAALGCLVKDYFPEPVTVSWNSGALTSGVHTFP AVLQSSGLYSLSSVVTVPSSSLGTQTYICNVNHKP SNTKVDKKVEPKSCDGGGGSGGGGSEEDPCACES LVKFQAKVEGLLQALTRKLEAVSKRLAILENTVV SGGGGSGGGGSGGGGSGGGGSREGPELSPDDPAG LLDLRQGMFAQLVAQNVLLIDGPLSWYSDPGLAG VSLTGGLSYKEDTKELVVAKAGVYYVFFQLELRR VVAGEGSGSVSLALHLQPLRSAAGAAALALTVDL PPASSEARNSAFGFQGRLLHLSAGQRLGVHLHTE ARARHAWQLTQGATVLGLFRVTPEIPAGLPSPRSE |
| 76 | Anti-FAP(28H1) light chain | EIVLTQSPGTLSLSPGERATLSCRASQSVSRSYLAW YQQKPGQAPRLLIIGASTRATGIPDRFSGSGSGTDF TLTISRLEPEDFAVYYCQQGQVIPPTFGQGTKVEIK RTVAAPSVFIFPPSDEQLKSGTASVVCLLNNFYPRE AKVQWKVDNALQSGNSQESVTEQDSKDSTYSLSS TLTLSKADYEKHKVYACEVTHQGLSSPVTKSFNR GEC |

The sequences encoding the trimeric FAP-targeted 4-1BBL-containing antigen binding molecule were cloned into a plasmid vector, which drives expression of the insert from an MPSV promoter and contains a synthetic polyA signal sequence located at the 3' end of the CDS. In addition, the vector contains an EBV OriP sequence for episomal maintenance of the plasmid.

The trimeric FAP-targeted 4-1BBL-containing antigen binding molecule was produced by co-transfecting HEK293-EBNA cells with the mammalian expression vectors using polyethylenimine. The cells were transfected with the corresponding expression vectors in a 1:1 ratio ("vector FAP(VHCH1)-CMPtd-4-1BBL": "vector light chain").

For production in 500 mL shake flasks, 400 million HEK293 EBNA cells were seeded 24 hours before transfection. For transfection cells were centrifuged for 5 minutes by 210 x g, and supernatant was replaced by pre-warmed CD CHO medium. Expression vectors were mixed in 20 mL CD CHO medium to a final amount of 200 μg DNA. After

addition of 540 μL PEI, the solution was vortexed for 15 seconds and incubated for 10 minutes at room temperature. Afterwards, cells were mixed with the DNA/PEI solution, transferred to a 500 mL shake flask and incubated for 3 hours at 37 °C in an incubator with a 5% $CO_2$ atmosphere. After the incubation, 160 mL F17 medium was added and cells were cultured for 24 hours. One day after transfection 1 mM valproic acid and 7% Feed with supplements were added. After culturing for 7 days, the cell supernatant was collected by centrifugation for 15 minutes at 210 x g. The solution was sterile filtered (0.22 μm filter), supplemented with sodium azide to a final concentration of 0.01 % (w/v), and kept at 4 °C.

The trimeric FAP-targeted huCMP trimeric 4-1BBL-containing antigen binding molecule was purified from cell culture supernatants by affinity chromatography via CH1 domain of human IgG antibodies, followed by size exclusion chromatography. For affinity chromatography, the supernatant was loaded on a column packed with CaptureSelect IgG-CH1 matrix (Column Volume = 1 ml; BAC, The Netherlands) and equilibrated with with 5 ml 50 mM Tris(hydroxymethyl)-aminomethan (TRIS), 100 mM Glycine, 150 mM sodium chloride, pH 8.0. Unbound protein was removed by washing with at least 10 column volumes of the same buffer. The bound protein was eluted by applying a linear pH-gradient over 20 column volumes from 50 mM TRIS, 100 mM Glycine, 150 mM sodium chloridepH 8.0 to pH 2.0. The column was then washed with 10 column volumes of 50 mM TRIS, 100 mM Glycine, 150 mM sodium chloride, pH 2.0.

The pH of collected fractions was neutralized by adding 1/40 (v/v) of 2M Tris, pH8.0. The protein was concentrated prior to loading on a HiLoad Superdex 200 column (GE Healthcare) equilibrated with 20mM Histidine, 140 mM sodium chloride, 0.01% (v/v) Tween/20 solution of pH 6.0.

The protein concentration was determined by measuring the optical density (OD) at 280 nm, using the molar extinction coefficient calculated on the basis of the amino acid sequence. Purity and molecular weight of the trimeric FAP-targeted 4-1BBL-containing antigen binding molecule was analyzed by SDS-PAGE in the presence and absence of a reducing agent (5 mM 1,4-dithiotreitol) and staining with Coomassie SimpleBlue™ SafeStain (Invitrogen USA). The aggregate content of samples was analyzed using a TSKgel G3000 SW XL analytical size-exclusion column (Tosoh) equilibrated in 25 mM $K_2HPO_4$, 125 mM NaCl, 200mM L-Arginine Monohydrocloride, 0.02 % (w/v) NaN3, pH 6.7 running buffer at 25 °C.

The molecular weight of the huCMP containing construct was determined under non-reducing and reducing conditions by LC-MS using a Agilent HPLC 1200 coupled to a TOF 6441 mass spectrometer (Agilent). For analyses under reduced conditions sample was incubated for 30 minutes at 37 °C in 10 μl 8 M Guanidine-HCl and 10 μl 0.5 mM TCEP diluted in 4 M Guanidine-HCl. Non-reduced samples were directly used for LC-MS analyses. The chromatographic separation was performed on a Macherey & Nagel Polysterene column; RP1000-8 (8 μm particle size, 4.6 x 250 mm; cat. No. 719510) using the program shown in **Table 2.** Eluent A was 5 % acetonitrile and 0.05 % (v/v) formic acid in water, eluent B was 95 % acetonitrile, 5 % water and 0.05 % formic acid. The separation was performed at 40 °C with a flow of 1 ml/min and 7 μg (15 μl) of the sample was injected.

During the first 4 minutes the eluate is directed into the waste to prevent the mass spectrometer from salt contamination. The ESI-source was running with a drying gas flow of 12 1/min, a temperature of 350 °C and a nebulizer pressure of 60 psi. The MS spectra are acquired using a fragmentor voltage of 350 V and a mass range 700 to 3200 m/z in positive ion mode. MS data are acquired by the instrument software from 4 to 17 minutes.

**Table 2: Mixture of solvents in HPLC chromatography for separation of individual compounds for Mass Spectrometry analysis.**

| Time (min.) | % solvent A | % solvent B |
|---|---|---|
| 0.5 | 85 | 15 |
| 10 | 40 | 60 |
| 12.5 | 0 | 100 |
| 14.5 | 0 | 100 |
| 14.6 | 85 | 15 |
| 16 | 85 | 15 |
| 16.1 | 0 | 100 |
| Solvent A is: 5 % acetonitrile and 0.05 % (v/v) formic acid in water, and solvent B is: 95 % acetonitrile, 5 % water and 0.05 % formic acid | | |

All used analytical methods confirmed homogeneous preparation of the trimerized molecules. **Table 3** summarizes yield, final monomer content and mass of the FAP-targeted trimeric 4-1BBL-containing antigen binding molecule.

**Table 3: Biochemical analysis of targeted CMP-trimeric 4-1BBL-containing antigen binding molecule**

| **Construct** | **Trimer [%] (SEC)** | **CE-SDS non red [%]** | **CE-SDS red [%]** | **LC/MS (non red)** | **LC/MS (red)** | **Thermal stability [°C]** |
|---|---|---|---|---|---|---|
| trimeric FAP(28H1)-targeted 4-1BBL(71-254)-containing antigen binding molecule | 100 % (439.2 kDa) | 92.2% (218 kDa) | 31.1 % (27.4 kDa) 67.1 % (55.7 kDa) | *Theoretical:* 218375.2 Da *Experimental:* 218404.2 Da (98.1%) | **(VL-CL)** *Theoretical:* 23367.13 Da *Experimental:* 23371.8 Da **(VH-CH1-huCMP-flag)** *Theoretical:* 49424.61 Da | 51°C |
| | | | | | *Experimental:* 49431.7 Da | |

Trimer content of the purified molecules was determined by size exclusion chromatography (SEC) and liquid chromatography mass spectrometry (LC/MS). Thermal stability of the molecules was analyzed by Dynamic Light Scattering (DLS) experiment. In brief, 30 μg of filtered protein sample with a protein concentration of 1 mg/ml is applied in duplicate to a Dynapro plate reader (Wyatt Technology Corporation; USA). The temperature is ramped from 25 to75 °C at 0.05 °C/min, with the radius and total scattering intensity being collected.

### 1.2. Preparation and purification of untargeted huCMP trimeric 4-1BB ligand-containing antigen binding molecules

The Fab genes (VH-CH1) from a germline control (DP47) were fused via a $(G4S)_2$ linker to a short trimerization domain derived from human CMP (Uniprot Accession: P21941; Residues 454 to 496, SEQ ID NO:2) by standard recombinant DNA technologies, as described for the trimeric FAP-targeted 4-1BB ligand-containing antigen binding molecules.

Table 4 shows, respectively, the cDNA and amino acid sequences of the trimeric untargeted 4-1BBL(71-254)-containing antigen binding molecule, which is trimerizing via CMP trimerization domain.

**Table 4: cDNA and amino acid sequences of DP47 untargeted human CMP (CMPtd) trimeric 4-1BBL(71-254)-containing antigen binding molecule**

| SEQ ID NO: | Description | Sequence |
|---|---|---|
| 77 | nucleotide sequence of DP47 (VHCH1)-CMPtd-4-1BBL(71-254) | GAGGTGCAATTGTTGGAGTCTGGGGGAGGC TTGGTACAGCCTGGGGGGTCCCTGAGACTC TCCTGTGCAGCCTCCGGATTCACCTTTAGCA GTTATGCCATGAGCTGGGTCCGCCAGGCTC CAGGGAAGGGGCTGGAGTGGGTCTCAGCTA TTAGTGGTAGTGGTGGTAGCACATACTACG CAGACTCCGTGAAGGGCCGGTTCACCATCT CCAGAGACAATTCCAAGAACACGCTGTATC TGCAGATGAACAGCCTGAGAGCCGAGGACA CGGCCGTATATTACTGTGCGAAAGGCAGCG GATTTGACTACTGGGGCCAAGGAACCCTGG TCACCGTCTCGAGTGCTAGCACAAAGGGAC CTAGCGTGTTCCCCCTGGCCCCCAGCAGCA AGTCTACATCTGGCGGAACAGCCGCCCTGG GCTGCCTCGTGAAGGACTACTTTCCCGAGC CCGTGACCGTGTCCTGGAACTCTGGCGCTCT |

(continued)

| SEQ ID NO: | Description | Sequence |
|---|---|---|
| | | GACAAGCGGCGTGCACACCTTTCCAGCCGT GCTGCAGAGCAGCGGCCTGTACTCTCTGAG CAGCGTCGTGACAGTGCCCAGCAGCTCTCT GGGCACCCAGACCTACATCTGCAACGTGAA CCACAAGCCCAGCAACACCAAGGTGGACAA GAAGGTGGAACCCAAGAGCTGCGACGGCG GAGGGGGATCTGGCGGCGGAGGATCCGAG GAAGATCCTTGCGCCTGCGAGAGCCTCGTG AAGTTCCAGGCCAAGGTGGAAGGACTGCTG CAGGCCCTGACCCGGAAACTGGAAGCCGTG TCCAAGCGGCTGGCCATCCTGGAAAACACC GTGGTGTCCGGAGGCGGAGGATCTGGCGGC GGAGGAAGTGGCGGAGGCGGATCTGGCGG CGGAGGATCTAGAGAGGGACCCGAACTGTC CCCTGACGATCCAGCCGGGCTGCTGGATCT GAGACAGGGAATGTTCGCCCAGCTGGTGGC TCAGAATGTGCTGCTGATTGACGGACCTCT GAGCTGGTACTCCGACCCAGGGCTGGCAGG GGTGTCCCTGACTGGGGGACTGTCCTACAA AGAAGATACAAAAGAACTGGTGGTGGCTAA AGCTGGGGTGTACTATGTGTTTTTCAGCTG GAACTGAGGCGGGTGGTGGCTGGGGAGGG CTCAGGATCTGTGTCCCTGGCTCTGCATCTG CAGCCACTGCGCTCTGCTGCTGGCGCAGCT GCACTGGCTCTGACTGTGGACCTGCCACCA GCCTCTAGCGAGGCCAGAAACAGCGCCTTC GGGTTCCAAGGACGCCTGCTGCATCTGAGC GCCGGACAGCGCCTGGGAGTGCATCTGCAT ACTGAAGCCAGAGCCCGGCATGCTTGGCAG CTGACTCAGGGGGCAACTGTGCTGGGACTG TTTCGCGTGACACCTGAGATCCCTGCCGGA CTGCCAAGCCCTAGATCAGAA |

(continued)

| SEQ ID NO: | Description | Sequence |
|---|---|---|
| 78 | nucleotide sequence of Germline control (DP47) light chain | GAAATCGTGTTAACGCAGTCTCCAGGCACC CTGTCTTTGTCTCCAGGGGAAAGAGCCACC CTCTCTTGCAGGGCCAGTCAGAGTGTTAGC AGCAGCTACTTAGCCTGGTACCAGCAGAAA CCTGGCCAGGCTCCCAGGCTCCTCATCTATG GAGCATCCAGCAGGGCCACTGGCATCCCAG ACAGGTTCAGTGGCAGTGGATCCGGGACAG ACTTCACTCTCACCATCAGCAGACTGGAGC CTGAAGATTTTGCAGTGTATTACTGTCAGCA GTATGGTAGCTCACCGCTGACGTTCGGCCA GGGGACCAAAGTGGAAATCAAACGTACGGT GGCTGCACCATCTGTCTTCATCTTCCCGCCA TCTGATGAGCAGTTGAAATCTGGAACTGCC TCTGTTGTGTGCCTGCTGAATAACTTCTATC CCAGAGAGGCCAAAGTACAGTGGAAGGTG GATAACGCCCTCCAATCGGGTAACTCCCAG GAGAGTGTCACAGAGCAGGACAGCAAGGA CAGCACCTACAGCCTCAGCAGCACCCTGAC |
| | | GCTGAGCAAAGCAGACTACGAGAAACACA AAGTCTACGCCTGCGAAGTCACCCATCAGG GCCTGAGCTCGCCCGTCACAAAGAGCTTCA ACAGGGGAGAGTGT |
| 79 | DP47(VHCH1)-CMPtd-4-1BBL(71-254) | EVQLLESGGGLVQPGGSLRLSCAASGFTFSSY AMSWVRQAPGKGLEWVSAISGSGGSTYYAD SVKGRFTISRDNSKNTLYLQMNSLRAEDTAV YYCAKGSGFDYWGQGTLVTVSSASTKGPSVF PLAPSSKSTSGGTAALGCLVKDYFPEPVTVSW NSGALTSGVHTFPAVLQSSGLYSLSSVVTVPS SSLGTQTYICNVNHKPSNTKVDKKVEPKSCD GGGGSGGGGSEEDPCACESLVKFQAKVEGLL QALTRKLEAVSKRLAILENTVVSGGGGSGGG GSGGGGSGGGGSREGPELSPDDPAGLLDLRQ GMFAQLVAQNVLLIDGPLSWYSDPGLAGVSL TGGLSYKEDTKELVVAKAGVYYVFFQLELRR VVAGEGSGSVSLALHLQPLRSAAGAAALALT VDLPPASSEARNSAFGFQGRLLHLSAGQRLGV HLHTEARARHAWQLTQGATVLGLFRVTPEIP AGLPSPRSE |

(continued)

| SEQ ID NO: | Description | Sequence |
|---|---|---|
| 80 | Germline control (DP47) light chain | EIVLTQSPGTLSLSPGERATLSCRASQSVSSSY LAWYQQKPGQAPRLLIYGASSRATGIPDRFSG SGSGTDFTLTISRLEPEDFAVYYCQQYGSSPLT FGQGTKVEIKRTVAAPSVFIFPPSDEQLKSGTA SVVCLLNNFYPREAKVQWKVDNALQSGNSQ ESVTEQDSKDSTYSLSSTLTLSKADYEKHKVY ACEVTHQGLSSPVTKSFNRGEC |

The sequences encoding the untargeted huCMP trimeric 4-1BB ligand-containing antigen binding molecule were cloned into a plasmid vector, which drives expression of the insert from an MPSV promoter and contains a synthetic polyA signal sequence located at the 3' end of the CDS. In addition, the vector contains an EBV OriP sequence for episomal maintenance of the plasmid.

The untargeted huCMP trimeric 4-1BB ligand-containing antigen binding molecule was produced by co-transfecting HEK293-EBNA cells with the mammalian expression vectors using polyethylenimine. The cells were transfected with the corresponding expression vectors in a 1:1 ratio ("vector DP47(VHCH1)-CMPtd-4-1BBL": "vector light chain").

Production, purification and characterization of the untargeted CMP trimeric 4-1BB ligand-containing antigen binding molecule was performed as described above for the FAP-targeted CMP trimeric 4-1BB ligand-containing antigen binding molecule.

All used analytical methods confirmed homogeneous preparation of trimerized molecules. **Table 5** summarizes the final monomer content of the untargeted CMP-trimeric 4-1BB ligand.

**Table 5: Biochemical analysis of untargeted CMP-trimeric 4-1BBL-containing antigen binding molecule**

| Construct | Trimer [%] (SEC) | CE-SDS non red [%] | CE-SDS red [%] |
|---|---|---|---|
| untargeted CMP-trimeric 4-1BB ligand-containing antigen binding molecule | 99.1% (468 kDa) | 91.3% (217 kDa) | 33.9% (27.5 kDa)<br>63.4% (55.7 kDa) |

### 1.3 Preparation of untargeted human IgG1 as Control F

An additional control molecule used in the assays was an untargeted DP47, germline control, human IgG1, containing the Pro329Gly, Leu234Ala and Leu235Ala mutations, to abrogate binding to Fc gamma receptors according to the method described in International Patent Appl. Publ. No. WO 2012/130831).

Table 6 shows the cDNA and amino acid sequences of the cDNA and amino acid sequences of the untargeted DP47 huIgG1 PGLALA (Control F).

**Table 6: Sequences of untargeted DP47 huIgG1 (Control F)**

| SEQ ID NO: | Description | Sequence |
|---|---|---|
| 81 | nucleotide sequence of DP47 heavy chain (hu IgG1 PGLALA) | GAGGTGCAATTGTTGGAGTCTGGGGGAGGCTTGGT ACAGCCTGGGGGGTCCCTGAGACTCTCCTGTGCAG CCTCCGGATTCACCTTTAGCAGTTATGCCATGAGCT GGGTCCGCCAGGCTCCAGGGAAGGGGCTGGAGTGG GTCTCAGCTATTAGTGGTAGTGGTGGTAGCACATAC TACGCAGACTCCGTGAAGGGCCGGTTCACCATCTC CAGAGACAATTCCAAGAACACGCTGTATCTGCAGA TGAACAGCCTGAGAGCCGAGGACACGGCCGTATAT TACTGTGCGAAAGGCAGCGGATTTGACTACTGGGG CCAAGGAACCCTGGTCACCGTCTCGAGTGCTAGCA CCAAGGGCCCATCGGTCTTCCCCCTGGCACCCTCCT CCAAGAGCACCTCTGGGGGCACAGCGGCCCTGGGC TGCCTGGTCAAGGACTACTTCCCCGAACCGGTGAC GGTGTCGTGGAACTCAGGCGCCCTGACCAGCGGCG TGCACACCTTCCCGGCTGTCCTACAGTCCTCAGGAC TCTACTCCCTCAGCAGCGTGGTGACCGTGCCCTCCA GCAGCTTGGGCACCCAGACCTACATCTGCAACGTG AATCACAAGCCCAGCAACACCAAGGTGGACAAGA |
| | | AAGTTGAGCCCAAATCTTGTGACAAAACTCACACA TGCCCACCGTGCCCAGCACCTGAAGCTGCAGGGGG ACCGTCAGTCTTCCTCTTCCCCCCAAAACCCAAGGA CACCCTCATGATCTCCCGGACCCCTGAGGTCACATG CGTGGTGGTGGACGTGAGCCACGAAGACCCTGAGG TCAAGTTCAACTGGTACGTGGACGGCGTGGAGGTG CATAATGCCAAGACAAAGCCGCGGGAGGAGCAGT ACAACAGCACGTACCGTGTGGTCAGCGTCCTCACC GTCCTGCACCAGGACTGGCTGAATGGCAAGGAGTA CAAGTGCAAGGTCTCCAACAAAGCCCTCGGCGCCC CCATCGAGAAAACCATCTCCAAAGCCAAAGGGCAG CCCCGAGAACCACAGGTGTACACCCTGCCCCCATC CCGGGATGAGCTGACCAAGAACCAGGTCAGCCTGA CCTGCCTGGTCAAAGGCTTCTATCCCAGCGACATCG CCGTGGAGTGGGAGAGCAATGGGCAGCCGGAGAA CAACTACAAGACCACGCCTCCCGTGCTGGACTCCG ACGGCTCCTTCTTCCTCTACAGCAAGCTCACCGTGG ACAAGAGCAGGTGGCAGCAGGGGAACGTCTTCTCA TGCTCCGTGATGCATGAGGCTCTGCACAACCACTAC ACGCAGAAGAGCCTCTCCCTGTCTCCGGGTAAA |
| 78 | nucleotide sequence of Germline control (DP47) light chain | see Table 4 |

(continued)

| SEQ ID NO: | Description | Sequence |
|---|---|---|
| 82 | DP47 heavy chain (hu IgG1 PGLALA) | EVQLLESGGGLVQPGGSLRLSCAASGFTFSSYAMSWV RQAPGKGLEWVSAISGSGGSTYYADSVKGRFTISRDN SKNTLYLQMNSLRAEDTAVYYCAKGSGFDYWGQGT LVTVSSASTKGPSVFPLAPSSKSTSGGTAALGCLVKD YFPEPVTVSWNSGALTSGVHTFPAVLQSSGLYSLSSV VTVPSSSLGTQTYICNVNHKPSNTKVDKKVEPKSCDK THTCPPCPAPEAAGGPSVFLFPPKPKDTLMISRTPEVT CVVVDVSHEDPEVKFNWYVDGVEVHNAKTKPREEQ YNSTYRVVSVLTVLHQDWLNGKEYKCKVSNKALGA PIEKTISKAKGQPREPQVYTLPPSRDELTKNQVSLTCL VKGFYPSDIAVEWESNGQPENNYKTTPPVLDSDGSFF LYSKLTVDKSRWQQGNVFSCSVMHEALHNHYTQKS LSLSPGK |
| 80 | Germline control (DP47) light chain | see Table 4 |

Table 7 summarizes the yield and final monomer content of untargeted DP47 huIgG1 (Control F).

**Table 7 Biochemical analysis of Control F**

| Construct | Monomer [%] (SEC) | Yield [mg/l] |
|---|---|---|
| Control F (germline DP47 human IgG1 PGLALA | 100 | 50 |

**Example 2**

**Preparation and purification of targeted huCMP trimeric OX40 ligand-containing antigen binding molecules**

**A** targeted huCMP-trimeric OX40 ligand-containing antigen binding molecule was prepared similarly to the 4-1BBL-containing antigen binding molecule. For human OX40 ligand, the DNA sequence encoding part of the ectodomain (amino acids 51-183, SEQ ID NO: 11) was synthetized according to P23510 (Uniprot Accession) and fused via a $(G_4S)_4$ linker downstream of the trimerization domain. Two Asn-linked glycosylation sites (N90 and N114) were replaced by aspartic acid (Asp) by mutagenesis.

The chain encoding FAP-targeted OX40 ligand and the light chain with FAP specificity are similar to those shown for 4-1BBL in **Figure 1A.** Disulfide bonds are formed between three FAP-targeted OX40 ligand chains leading to the formation of covalently linked CMP-trimeric OX40 ligand-containing antigen binding molecule, targeted to FAP.

**Table 8** shows, respectively, the cDNA and amino acid sequences of the FAP-targeted human OX40 ligand-containing antigen binding molecule, which is trimerizing via huCMP trimerization domain.

**Table 8: cDNA and amino acid sequences of FAP(28H1)-targeted human CMP trimeric OX40L-containing antigen binding molecule**

| SEQ ID NO: | Description | Sequence |
|---|---|---|
| 74 | nucleotide sequence of FAP(28H1)(VHCH1)-CMPtd-Ox40L(51-183) | GAAGTGCAGCTGCTGGAATCCGGCGGAGGC CTGGTGCAGCCTGGCGGATCTCTGAGACTG TCCTGCGCCGCCTCCGGCTTCACCTTCTCCT CCCACGCCATGTCCTGGGTCCGACAGGCTC CTGGCAAAGGCCTGGAATGGGTGTCCGCCA TCTGGGCCTCCGGCGAGCAGTACTACGCCG ACTCTGTGAAGGGCCGGTTCACCATCTCCC GGGACAACTCCAAGAACACCCTGTACCTGC |
| | | AGATGAACTCCCTGCGGGCCGAGGACACCG CCGTGTACTACTGTGCCAAGGGCTGGCTGG GCAACTTCGACTACTGGGGACAGGGCACCC TGGTCACCGTGTCCAGCGCTAGCACAAAGG GACCTAGCGTGTTCCCCCTGGCCCCCAGCA GCAAGTCTACATCTGGCGGAACAGCCGCCC TGGGCTGCCTCGTGAAGGACTACTTTCCCG AGCCCGTGACCGTGTCCTGGAACTCTGGCG CTCTGACAAGCGGCGTGCACACCTTTCCAG CCGTGCTGCAGAGCAGCGGCCTGTACTCTC TGAGCAGCGTCGTGACAGTGCCCAGCAGCT CTCTGGGCACCCAGACCTACATCTGCAACG TGAACCACAAGCCCAGCAACACCAAGGTGG ACAAGAAGGTGGAACCCAAGAGCTGCGAC GGCGGAGGGGGATCTGGCGGCGGAGGATC CGAGGAAGATCCTTGCGCCTGCGAGAGCCT CGTGAAGTTCCAGGCCAAGGTGGAAGGACT GCTGCAGGCCCTGACCCGGAAACTGGAAGC CGTGTCCAAGCGGCTGGCCATCCTGGAAAA CACCGTGGTGTCCGGAGGCGGAGGATCTGG CGGCGGAGGAAGTGGCGGAGGGGGATCTG GGGGAGGCGGATCTCAGGTGTCCCACAGAT ACCCCCGGATCCAGAGCATCAAGGTGCAGT TCACCGAGTACAAGAAAGAGAAGGGCTTCA TCCTGACCAGCCAGAAAGAGGACGAGATCA TGAAGGTGCAGGACAACAGCGTGATCATCA ACTGCGACGGCTTCTACCTGATCAGCCTGA AGGGCTACTTCAGCCAGGAAGTGGACATCA GCCTGCACTACCAGAAGGACGAGGAACCCC TGTTCCAGCTGAAGAAAGTGCGGAGCGTGA ACAGCCTGATGGTGGCCAGCCTGACCTACA AGGACAAGGTGTACCTGAACGTGACCACCG ACAACACCAGCCTGGACGACTTCCACGTGA ACGGCGGCGAGCTGATCCTGATTCACCAGA ACCCCGGCGAGTTCTGCGTGCTC |
| 75 | nucleotide sequence of anti-FAP(28H1) light chain | See Table 1 |

(continued)

| SEQ ID NO: | Description | Sequence |
|---|---|---|
| 72 | FAP(VHCH1)-CMPtd-Ox40L(51-183) | EVQLLESGGGLVQPGGSLRLSCAASGFTFSSH AMSWVRQAPGKGLEWVSAIWASGEQYYADS VKGRFTISRDNSKNTLYLQMNSLRAEDTAVY YCAKGWLGNFDYWGQGTLVTVSSASTKGPS VFPLAPSSKSTSGGTAALGCLVKDYFPEPVTV SWNSGALTSGVHTFPAVLQSSGLYSLSSVVTV PSSSLGTQTYICNVNHKPSNTKVDKKVEPKSC DGGGGSGGGGSEEDPCACESLVKFQAKVEGL LQALTRKLEAVSKRLAILENTVVSGGGGSGG GGSGGGGSGGGGSQVSHRYPRIQSIKVQFTEY KKEKGFILTSQKEDEIMKVQDNSVIINCDGFY LISLKGYFSQEVDISLHYQKDEEPLFQLKKVRS |
| | | VNSLMVASLTYKDKVYLNVTTDNTSLDDFH VNGGELILIHQNPGEFCVL |
| 76 | Anti-FAP (28H1) light chain | See Table 1 |

Production, purification and characterization of the FAP-targeted huCMP-trimeric Ox40 ligand-containing antigen binding molecule was performed as described above for the FAP-targeted human 4-1BB ligand-containing antigen binding molecule.

All used analytical methods confirmed homogeneous preparation of trimerized molecules. **Table 9** summarizes yield and final monomer content of the FAP-targeted CMP-trimeric Ox40 ligand-containing antigen binding molecule.

**Table 9: Biochemical analysis of FAP-targeted CMP-trimeric OX40L-containing antigen binding molecule**

| Construct | Trimer [%] (SEC) | CE-SDS non red [%] |
|---|---|---|
| FAP-targeted CMP-trimeric Ox40 ligand-containing antigen binding molecule | 85 % | 89.4% (226 kDa) |

**Example** 3

**Biochemical characterization of FAP-targeted huCMP trimeric 4-1BB ligand-containing antigen binding molecule by surface plasmon resonance**

**3.1. Preparation, purification and characterization of 4-1BB**

DNA sequences encoding the ectodomains of human, mouse or cynomolgus **4-1BB (Table 10)** were subcloned in frame with the human IgG1 heavy chain CH2 and CH3 domains on the knob (Merchant et al., 1998). An AcTEV protease cleavage site was introduced between an antigen ectodomain and the Fc of human IgG1. An Avi tag for directed biotinylation was introduced at the C-terminus of the antigen-Fc knob. Combination of the antigen-Fc knob chain containing the S354C/T366W mutations, with a Fc hole chain containing the Y349C/T366S/L368A/Y407V mutations allows generation of a heterodimer which includes a single copy of the 4-1BB ectodomain containing chain, thus creating a monomeric form of Fc-linked antigen **(Figure 1C). Table 11** lists the cDNA and amino acid sequences of monomeric antigen Fc(kih) fusion molecules as depicted in **Figure 1C.**

**Table 10:** Amino acid numbering of antigen ectodomains (ECD) and their Origin

| SEQ ID NO: | Construct | Origin | ECD |
|---|---|---|---|
| 83 | human 4-1BB ECD | Synthetized according to Q07011 | aa 24-186 |
| 84 | cynomolgus 4-1BB ECD | isolated from cynomolgus blood | aa 24-186 |
| 85 | murine 4-1BB ECD | Synthetized according to P20334 | aa 24-187 |

**Table 11: cDNA and Amino acid sequences of monomeric antigen Fc(kih) fusion molecules** (produced by combination of one Fc hole chain with one antigen Fc knob chain)

| SEQ ID NO: | Antigen | Sequence |
|---|---|---|
| 86 | Nucleotide sequence of Fc hole chain | GACAAAACTCACACATGCCCACCGTGCCCAGCACCTGA<br>ACTCCTGGGGGGACCGTCAGTCTTCCTCTTCCCCCCAAA<br>ACCCAAGGACACCCTCATGATCTCCCGGACCCCTGAGGT<br>CACATGCGTGGTGGTGGACGTGAGCCACGAAGACCCTG<br>AGGTCAAGTTCAACTGGTACGTGGACGGCGTGGAGGTG<br>CATAATGCCAAGACAAAGCCGCGGGAGGAGCAGTACAA<br>CAGCACGTACCGTGTGGTCAGCGTCCTCACCGTCCTGCA<br>CCAGGACTGGCTGAATGGCAAGGAGTACAAGTGCAAGG<br>TCTCCAACAAAGCCCTCCCAGCCCCCATCGAGAAAACC<br>ATCTCCAAAGCCAAAGGGCAGCCCCGAGAACCACAGGT<br>GTGCACCCTGCCCCCATCCCGGGATGAGCTGACCAAGA<br>ACCAGGTCAGCCTCTCGTGCGCAGTCAAAGGCTTCTATC<br>CCAGCGACATCGCCGTGGAGTGGGAGAGCAATGGGCAG<br>CCGGAGAACAACTACAAGACCACGCCTCCCGTGCTGGA<br>CTCCGACGGCTCCTTCTTCCTCGTGAGCAAGCTCACCGT<br>GGACAAGAGCAGGTGGCAGCAGGGGAACGTCTTCTCAT<br>GCTCCGTGATGCATGAGGCTCTGCACAACCACTACACGC<br>AGAAGAGCCTCTCCCTGTCTCCGGGTAAA |
| 87 | Nucleotide sequence of human 4-1BB antigen Fc knob chain | CTGCAGGACCCCTGCAGCAACTGCCCTGCCGGCACCTTC<br>TGCGACAACAACCGGAACCAGATCTGCAGCCCCTGCCC<br>CCCCAACAGCTTCAGCTCTGCCGGCGGACAGCGGACCT<br>GCGACATCTGCAGACAGTGCAAGGGCGTGTTCAGAACC<br>CGGAAAGAGTGCAGCAGCACCAGCAACGCCGAGTGCGA<br>CTGCACCCCCGGCTTCCATTGTCTGGGAGCCGGCTGCAG<br>CATGTGCGAGCAGGACTGCAAGCAGGGCCAGGAACTGA<br>CCAAGAAGGGCTGCAAGGACTGCTGCTTCGGCACCTTC<br>AACGACCAGAAGCGGGGCATCTGCCGGCCCTGGACCAA<br>CTGTAGCCTGGACGGCAAGAGCGTGCTGGTCAACGGCA<br>CCAAAGAACGGGACGTCGTGTGCGGCCCCAGCCCTGCT<br>GATCTGTCTCCTGGGGCCAGCAGCGTGACCCCTCCTGCC<br>CCTGCCAGAGAGCCTGGCCACTCTCCTCAGGTCGACGAA<br>CAGTTATATTTTCAGGGCGGCTCACCCAAATCTGCAGAC<br>AAAACTCACACATGCCCACCGTGCCCAGCACCTGAACTC<br>CTGGGGGGACCGTCAGTCTTCCTCTTCCCCCCAAAACCC<br>AAGGACACCCTCATGATCTCCCGGACCCCTGAGGTCACA<br>TGCGTGGTGGTGGACGTGAGCCACGAAGACCCTGAGGT<br>CAAGTTCAACTGGTACGTGGACGGCGTGGAGGTGCATA<br>ATGCCAAGACAAAGCCGCGGGAGGAGCAGTACAACAGC<br>ACGTACCGTGTGGTCAGCGTCCTCACCGTCCTGCACCAG |

(continued)

| SEQ ID NO: | Antigen | Sequence |
|---|---|---|
| | | GACTGGCTGAATGGCAAGGAGTACAAGTGCAAGGTCTC CAACAAAGCCCTCCCAGCCCCCATCGAGAAAACCATCT CCAAAGCCAAAGGGCAGCCCCGAGAACCACAGGTGTAC ACCCTGCCCCCATGCCGGGATGAGCTGACCAAGAACCA GGTCAGCCTGTGGTGCCTGGTCAAAGGCTTCTATCCCAG CGACATCGCCGTGGAGTGGGAGAGCAATGGGCAGCCGG AGAACAACTACAAGACCACGCCTCCCGTGCTGGACTCC GACGGCTCCTTCTTCCTCTACAGCAAGCTCACCGTGGAC AAGAGCAGGTGGCAGCAGGGGAACGTCTTCTCATGCTC CGTGATGCATGAGGCTCTGCACAACCACTACACGCAGA AGAGCCTCTCCCTGTCTCCGGGTAAATCCGGAGGCCTGA ACGACATCTTCGAGGCCCAGAAGATTGAATGGCACGAG |
| 88 | Nucleotide sequence of cynomolgus 4-1BB antigen Fc knob chain | TTGCAGGATCTGTGTAGTAACTGCCCAGCTGGTACATTC TGTGATAATAACAGGAGTCAGATTTGCAGTCCCTGTCCT CCAAATAGTTTCTCCAGCGCAGGTGGACAAAGGACCTGT GACATATGCAGGCAGTGTAAAGGTGTTTTCAAGACCAG GAAGGAGTGTTCCTCCACCAGCAATGCAGAGTGTGACT GCATTTCAGGGTATCACTGCCTGGGGGCAGAGTGCAGC ATGTGTGAACAGGATTGTAAACAAGGTCAAGAATTGAC AAAAAAAGGTTGTAAAGACTGTTGCTTTGGGACATTTAA TGACCAGAAACGTGGCATCTGTCGCCCCTGGACAAACT GTTCTTTGGATGGAAAGTCTGTGCTTGTGAATGGGACGA AGGAGAGGGACGTGGTCTGCGGACCATCTCCAGCCGAC CTCTCTCCAGGAGCATCCTCTGCGACCCCGCCTGCCCCT GCGAGAGAGCCAGGACACTCTCCGCAGGTCGACGAACA GTTATATTTTCAGGGCGGCTCACCCAAATCTGCAGACAA AACTCACACATGCCCACCGTGCCCAGCACCTGAACTCCT GGGGGGACCGTCAGTCTTCCTCTTCCCCCCAAAACCCAA GGACACCCTCATGATCTCCCGGACCCCTGAGGTCACATG CGTGGTGGTGGACGTGAGCCACGAAGACCCTGAGGTCA AGTTCAACTGGTACGTGGACGGCGTGGAGGTGCATAAT GCCAAGACAAAGCCGCGGGAGGAGCAGTACAACAGCA CGTACCGTGTGGTCAGCGTCCTCACCGTCCTGCACCAGG ACTGGCTGAATGGCAAGGAGTACAAGTGCAAGGTCTCC AACAAAGCCCTCCCAGCCCCCATCGAGAAAACCATCTC CAAAGCCAAAGGGCAGCCCCGAGAACCACAGGTGTACA CCCTGCCCCCATGCCGGGATGAGCTGACCAAGAACCAG GTCAGCCTGTGGTGCCTGGTCAAAGGCTTCTATCCCAGC GACATCGCCGTGGAGTGGGAGAGCAATGGGCAGCCGGA GAACAACTACAAGACCACGCCTCCCGTGCTGGACTCCG ACGGCTCCTTCTTCCTCTACAGCAAGCTCACCGTGGACA AGAGCAGGTGGCAGCAGGGGAACGTCTTCTCATGCTCC GTGATGCATGAGGCTCTGCACAACCACTACACGCAGAA GAGCCTCTCCCTGTCTCCGGGTAAATCCGGAGGCCTGAA CGACATCTTCGAGGCCCAGAAGATTGAATGGCACGAG |

(continued)

| SEQ ID NO: | Antigen | Sequence |
|---|---|---|
| 89 | Nucleotide sequence of murine 4-1BB antigen Fc knob chain | GTGCAGAACAGCTGCGACAACTGCCAGCCCGGCACCTT CTGCCGGAAGTACAACCCCGTGTGCAAGAGCTGCCCCC CCAGCACCTTCAGCAGCATCGGCGGCCAGCCCAACTGC AACATCTGCAGAGTGTGCGCCGGCTACTTCCGGTTCAAG AAGTTCTGCAGCAGCACCCACAACGCCGAGTGCGAGTG CATCGAGGGCTTCCACTGCCTGGGCCCCCAGTGCACCAG ATGCGAGAAGGACTGCAGACCCGGCCAGGAACTGACCA AGCAGGGCTGTAAGACCTGCAGCCTGGGCACCTTCAAC GACCAGAACGGGACCGGCGTGTGCCGGCCTTGGACCAA |
| | | TTGCAGCCTGGACGGGAGAAGCGTGCTGAAAACCGGCA CCACCGAGAAGGACGTCGTGTGCGGCCCTCCCGTGGTGT CCTTCAGCCCTAGCACCACCATCAGCGTGACCCCTGAAG GCGGCCCTGGCGGACACTCTCTGCAGGTCCTGGTCGACG AACAGTTATATTTTCAGGGCGGCTCACCCAAATCTGCAG ACAAAACTCACACATGCCCACCGTGCCCAGCACCTGAA CTCCTGGGGGGACCGTCAGTCTTCCTCTTCCCCCCAAAA CCCAAGGACACCCTCATGATCTCCCGGACCCCTGAGGTC ACATGCGTGGTGGTGGACGTGAGCCACGAAGACCCTGA GGTCAAGTTCAACTGGTACGTGGACGGCGTGGAGGTGC ATAATGCCAAGACAAAGCCGCGGGAGGAGCAGTACAAC AGCACGTACCGTGTGGTCAGCGTCCTCACCGTCCTGCAC CAGGACTGGCTGAATGGCAAGGAGTACAAGTGCAAGGT CTCCAACAAAGCCCTCCCAGCCCCCATCGAGAAAACCA TCTCCAAAGCCAAAGGGCAGCCCCGAGAACCACAGGTG TACACCCTGCCCCCATGCCGGGATGAGCTGACCAAGAA CCAGGTCAGCCTGTGGTGCCTGGTCAAAGGCTTCTATCC CAGCGACATCGCCGTGGAGTGGGAGAGCAATGGGCAGC CGGAGAACAACTACAAGACCACGCCTCCCGTGCTGGAC TCCGACGGCTCCTTCTTCCTCTACAGCAAGCTCACCGTG GACAAGAGCAGGTGGCAGCAGGGGAACGTCTTCTCATG CTCCGTGATGCATGAGGCTCTGCACAACCACTACACGCA GAAGAGCCTCTCCCTGTCTCCGGGTAAATCCGGAGGCCT GAACGACATCTTCGAGGCCCAGAAGATTGAATGGCACG AG |
| 90 | Fc hole chain | DKTHTCPPCPAPELLGGPSVFLFPPKPKDTLMISRTPEVTCV VVDVSHEDPEVKFNWYVDGVEVHNAKTKPREEQYNSTYR VVSVLTVLHQDWLNGKEYKCKVSNKALPAPIEKTISKAKG QPREPQVCTLPPSRDELTKNQVSLSCAVKGFYPSDIAVEWE SNGQPENNYKTTPPVLDSDGSFFLVSKLTVDKSRWQQGNV FSCSVMHEALHNHYTQKSLSLSPGK |

(continued)

| SEQ ID NO: | Antigen | Sequence |
|---|---|---|
| 91 | human 4-1BB antigen Fc knob chain | LQDPCSNCPAGTFCDNNRNQICSPCPPNSFSSAGGQRTCDI CRQCKGVFRTRKECSSTSNAECDCTPGFHCLGAGCSMCEQ DCKQGQELTKKGCKDCCFGTFNDQKRGICRPWTNCSLDG KSVLVNGTKERDVVCGPSPADLSPGASSVTPPAPAREPGHS PQVDEQLYFQGGSPKSADKTHTCPPCPAPELLGGPSVFLFP PKPKDTLMISRTPEVTCVVVDVSHEDPEVKFNWYVDGVE VHNAKTKPREEQYNSTYRVVSVLTVLHQDWLNGKEYKCK VSNKALPAPIEKTISKAKGQPREPQVYTLPPCRDELTKNQV SLWCLVKGFYPSDIAVEWESNGQPENNYKTTPPVLDSDGS FFLYSKLTVDKSRWQQGNVFSCSVMHEALHNHYTQKSLS LSPGKSGGLNDIFEAQKIEWHE |
| 92 | cynomolgus 4-1BB antigen Fc knob chain | LQDLCSNCPAGTFCDNNRSQICSPCPPNSFSSAGGQRTCDIC RQCKGVFKTRKECSSTSNAECDCISGYHCLGAECSMCEQD CKQGQELTKKGCKDCCFGTFNDQKRGICRPWTNCSLDGK SVLVNGTKERDVVCGPSPADLSPGASSATPPAPAREPGHSP QVDEQLYFQGGSPKSADKTHTCPPCPAPELLGGPSVFLFPP KPKDTLMISRTPEVTCVVVDVSHEDPEVKFNWYVDGVEV HNAKTKPREEQYNSTYRVVSVLTVLHQDWLNGKEYKCKV SNKALPAPIEKTISKAKGQPREPQVYTLPPCRDELTKNQVS LWCLVKGFYPSDIAVEWESNGQPENNYKTTPPVLDSDGSF FLYSKLTVDKSRWQQGNVFSCSVMHEALHNHYTQKSLSL SPGKSGGLNDIFEAQKIEWHE |
| 93 | murine 4-1BB antigen Fc knob chain | VQNSCDNCQPGTFCRKYNPVCKSCPPSTFSSIGGQPNCNIC RVCAGYFRFKKFCSSTHNAECECIEGFHCLGPQCTRCEKDC RPGQELTKQGCKTCSLGTFNDQNGTGVCRPWTNCSLDGR SVLKTGTTEKDVVCGPPVVSFSPSTTISVTPEGGPGGHSLQ VLVDEQLYFQGGSPKSADKTHTCPPCPAPELLGGPSVFLFP PKPKDTLMISRTPEVTCVVVDVSHEDPEVKFNWYVDGVE VHNAKTKPREEQYNSTYRVVSVLTVLHQDWLNGKEYKCK VSNKALPAPIEKTISKAKGQPREPQVYTLPPCRDELTKNQV SLWCLVKGFYPSDIAVEWESNGQPENNYKTTPPVLDSDGS FFLYSKLTVDKSRWQQGNVFSCSVMHEALHNHYTQKSLS LSPGKSGGLNDIFEAQKIEWHE |

All 4-1BB-Fc-fusion encoding sequences were cloned into a plasmid vector, which drives expression of the insert from an MPSV promoter and contains a synthetic polyA signal sequence located at the 3' end of the CDS. In addition, the vector contains an EBV OriP sequence for episomal maintenance of the plasmid.

For preparation of the biotinylated monomeric antigen/Fc fusion molecules, exponentially growing suspension HEK293 EBNA cells were co-transfected with three vectors encoding the two components of fusion protein (knob and hole chains) as well as BirA, an enzyme necessary for the biotinylation reaction. The corresponding vectors were used at a 1 : 1 : 0.05 ratio ("antigen ECD-AcTEV- Fc knob" : "Fc hole" : "BirA").

For protein production in 500 ml shake flasks, 400 million HEK293 EBNA cells were seeded 24 hours before transfection. For transfection cells were centrifuged for 5 minutes at 210 g, and supernatant was replaced by pre-warmed CD CHO medium. Expression vectors were resuspended in 20 mL of CD CHO medium containing 200 μg of vector DNA. After addition of 540 μL of polyethylenimine (PEI), the solution was vortexed for 15 seconds and incubated for 10 minutes at room temperature. Afterwards, cells were mixed with the DNA/PEI solution, transferred to a 500 mL shake flask and incubated for 3 hours at 37°C in an incubator with a 5% $CO_2$ atmosphere. After the incubation, 160 mL of F17 medium was added and cells were cultured for 24 hours. One day after transfection, 1 mM valproic acid and 7% Feed were added to the culture. After 7 days of culturing, the cell supernatant was collected by spinning down cells for 15 min at 210 g. The solution was sterile filtered (0.22 μm filter), supplemented with sodium azide to a final concentration of 0.01 % (w/v), and kept at 4°C.

Secreted proteins were purified from cell culture supernatants by affinity chromatography using Protein A, followed by size exclusion chromatography. For affinity chromatography, the supernatant was loaded on a HiTrap ProteinA HP column (CV = 5 mL, GE Healthcare) equilibrated with 40 mL 20 mM sodium phosphate, 20 mM sodium citrate pH 7.5. Unbound protein was removed by washing with at least 10 column volumes of 20 mM sodium phosphate, 20 mM sodium citrate, 0.5 M sodium chloride containing buffer (pH 7.5). The bound protein was eluted using a linear pH-gradient of sodium chloride (from 0 to 500 mM) created over 20 column volumes of 20 mM sodium citrate, 0.01% (v/v) Tween-20, pH 2.5 . The column was then washed with 10 column volumes of 20 mM sodium citrate, 500 mM sodium chloride, 0.01% (v/v) Tween-20, pH 2.5.

The pH of collected fractions was adjusted by adding 1/40 (v/v) of 2M Tris, pH8.0. The protein was concentrated and filtered prior to loading on a HiLoad Superdex 200 column (GE Healthcare) equilibrated with 2 mM MOPS, 150 mM sodium chloride, 0.02% (w/v) sodium azide solution of pH 7.4.

### 3.2. Biochemical Characterization by surface plasmon resonance

Binding of FAP-targeted huCMP-trimeric 4-1BB ligand-containing antigen binding molecule to the recombinant **4-1BB** Fc (kih) was assessed by surface plasmon resonance (SPR). All SPR experiments were performed on a Biacore T100 at 25 °C with HBS-EP as running buffer (0.01 M HEPES pH 7.4, 0.15 M NaCl, 3 mM EDTA, 0.005% Surfactant P20, Biacore, Freiburg/Germany).

The avidity of the interaction between the huCMP-trimeric human **4-1BB** ligand-containing antigen binding molecule and recombinant 4-1BB (human, cyno and murine) was determined as described below and shown in **Figures 2A to 2F.**

Anti-human Fc antibody (Biacore, Freiburg/Germany) was directly coupled on a CM5 chip at pH 5.0 using the standard amine coupling kit (Biacore, Freiburg/Germany). The immobilization level was about 6800 RU. Recombinant human, cynomolgus and murine 4-1BB Fc(kih) were captured for 60 seconds at 200 nM. FAP-targeted CMP-trimeric 4-1BB ligand-containing antigen binding molecule was passed at 200 nM with a flow of 30 μL/minutes through the flow cells over 120 seconds. The dissociation was monitored for 120 seconds. Bulk refractive index differences were corrected for by subtracting the response obtained on reference flow cell. Here, the FAP-targeted CMP-trimeric 4-1BB ligand-containing antigen binding molecule was flown over a surface with immobilized anti-human Fc antibody but on which HBS-EP has been injected rather than recombinant human, cynomolgus and murine 4-1BB Fc(kih).

The affinity of the interaction between FAP-targeted huCMP-trimeric **4-1BB** ligand-containing antigen binding molecule and recombinant human and cynomolgus **4-1BB** was determined as described below and in **Figures 3A to 3C.**

Anti-penta his antibody (Qiagen) was directly coupled on a CM5 chip at pH 5.0 using the standard amine coupling kit (Biacore, Freiburg/Germany). The immobilization level was about 9200 RU. FAP was captured for 90 seconds at 200 nM. FAP-targeted CMP-trimeric **4-1BB** ligand-containing antigen binding molecule was passed at 100 nM through the flow cells over 120 seconds. Recombinant human, cynomolgus and murine **4-1BB** Fc(kih) were passed at a concentration range from 0.49 to 1000 nM with a flow of 30 μL/minutes through the flow cells over 180 seconds. The dissociation was monitored for 180 seconds. Bulk refractive index differences were corrected for by subtracting the response obtained on reference flow cell. Here, recombinant 4-1BB Fc(kih) was flown over a surface with immobilized anti-penta His antibody but on which HBS-EP has been injected rather than FAP and targeted huCMP-trimeric 4-1BB ligand.

Kinetic constants were derived using the Biacore T100 Evaluation Software (vAA, Biacore AB, Uppsala/Sweden), to fit rate equations for 1:1 Langmuir binding by numerical integration **(Table 12).**

**Table 12: Affinity constants determined by fitting rate equations for 1:1 Langmuir binding**

| Ligand | Analyte | $K_{on}$ (1/Ms) | $K_{off}$ (1/s) | $K_D$ (M) |
|---|---|---|---|---|
| FAP-targeted huCMP-trimeric 4-1BB ligand-containing antigen binding molecule | hu4-1BB Fc(kih) | 1.1E+05 | 5.2E-02 | 4.8E-07 |
| FAP-targeted huCMP-trimeric 4-1BB ligand-containing antigen binding molecule | cy4-1BB Fc(kih) | 3.9E+04 | 1.0E-02 | 2.7E-07 |

**Example 4**

**Functional characterization of targeted huCMP trimeric 4-1BB ligand-containing antigen binding molecules**

**4.1. Binding on 4-1BB expressing cells: Binding on resting (naïve) and activated human PBMCs**

Buffy coats were obtained from the Zurich blood donation center. To isolate fresh peripheral blood mononuclear cells (PBMCs) the buffy coat was diluted with the same volume of DPBS (Gibco by Life Technologies, Cat. No. 14190 326). 50 mL polypropylene centrifuge tubes (TPP, Cat.-No. 91050) were supplied with 15 mL Histopaque 1077 (SIGMA Life Science, Cat.-No. 10771, polysucrose and sodium diatrizoate, adjusted to a density of 1.077 g/mL) and the diluted buffy coat solution was layered above the Histopaque 1077. The tubes were centrifuged for 30 min at 400 x g. PBMCs were then collected from the interface, washed three times with DPBS and resuspended in T cell medium consisting of RPMI 1640 medium (Gibco by Life Technology, Cat. No. 42401-042) supplied with 10 % Fetal Bovine Serum (FBS, Gibco by Life Technology, Cat. No. 16000-044, Lot 941273, gamma-irradiated, mycoplasma-free and heat inactivated at 56 °C for 35 min), 1 % (v/v) GlutaMAX-I (GIBCO by Life Technologies, Cat. No. 35050 038), 1 mM Sodium Pyruvate (SIGMA, Cat. No. S8636), 1 % (v/v) MEM non-essential amino acids (SIGMA, Cat.-No. M7145) and 50 μM β-Mercaptoethanol (SIGMA, M3148).

PBMCs were used directly after isolation (naive cells) or stimulated to induce 4-1BB expression at the cell surface of T cells by culturing for 4 days in T cell medium supplemented with 200 U/mL Proleukin (Novartis Pharma Schweiz AG, CHCLB-P-476-700-10340) and 2 ug/mL PHA-L (SIGMA Cat.-No. L2769) in a 6-well tissue culture plate and then 1 day in a 6-well tissue culture plate coated with 10 ug/mL anti-human CD3 (clone OKT3, BioLegend, Cat.-No. 317315) and 2 ug/mL anti-human CD28 (clone CD28.2, BioLegend, Cat.-No.: 302928) in T cell medium supplied with 200 U/mL Proleukin at 37 °C and 5% $CO_2$.

To determine binding of huCMP trimeric 4-1BB ligand-containing antigen binding molecules to human PBMCs, 0.1 x $10^6$ naive or activated PBMCs were added to each well of a round-bottom suspension cell 96-well plates (Greiner bio-one, cellstar, Cat. No. 650185). Plates were centrifuged 4 minutes with 400 x g and at 4 °C and supernatant was discarded. If cells were fixed before acquisition by flow cytometry they were washed once with 200 μL/well DPBS and then stained for 30 min at 4°C with 100 μL/mL DPBS containing 1:1000 diluted LIVE/DEAD Fixable Aqua Dead Cell Stain (Life technologies, Molecular Probes, Cat-No.: L34957). If cells were acquired the same day and DAPI was used to discriminate dead cells this staining step was skipped. Afterwards cells were washed once with 200 μL cold FACS buffer (DPBS supplied with 2 % (v/v) FBS, 5 mM EDTA pH8 (Amresco, Cat. No. E177) and 7.5 mM sodium azide (Sigma-Aldrich S2002)). Next, 50 μL/well of 4 °C cold FACS buffer containing FAP-targeted CMP trimeric 4-1BB ligand-containing antigen binding molecule, DP47-targeted CMP trimeric 4-1BB ligand-containing antigen binding molecule or not-specific binding huIgG1 P329G LALA control antibody were added and cells were incubated for 120 minutes at 4 °C. Cells were washed four times with 200 μL/well 4 °C FACS buffer. Afterwards cells were further stained with 50 μL/well of 4 °C cold FACS buffer containing 0.25 μg/mL anti-human CD4-BV421 (clone RPA-T4, mouse IgG1κ, BioLegend, Cat.-No. 300532), 0.25 μL anti-human CD8a-APC (clone RPA-T8, mouse IgG1κ, BD Pharmingen, Cat.-No. 555369) or 1:200 diluted anti-human CD8a-AF488 (clone RPA-T8, mouse IgG1κ, BD Pharmingen, Cat.-No. 557704), 0.12 μg/mL anti-human CD56-FITC (clone NCAM16.2, mouse IgG2bκ, BD Pharmingen, Cat.-No. 345811) and 2.5 μg/mL PE-conjugated AffiniPure anti-human IgG F(ab')2-fragment-specific goat F(ab')2 fragment (Jackson ImmunoResearch, Cat. No. 109 116 097) and incubated for 30 minutes at 4 °C. Cells were washed twice with 200 uL FACS buffer/well. If cells were stained with LIVE/DEAD Fixable Aqua Dead Cell Stain, they were fixed with 50 μL/well DPBS containing 1 % Formaldehyde (Sigma, HT501320-9.5L) and stored overnight at 4 °C. Cells were resuspended in FACS buffer and acquired the next day using a 5-laser LSR-Fortessa (BD Bioscience with DIVA software). If DAPI staining was used to detect dead cells, cells were resuspended in 80 μL/well FACS buffer containing 0.2 μg/mL DAPI (Santa Cruz Biotec, Cat. No. Sc-3598) and acquired the same day using a 5-laser LSR-Fortessa (BD Bioscience with DIVA software).

As shown in Figure 4, neither FAP-targeted huCMP trimeric 4-1BB ligand-containing antigen binding molecules (trimeric FAP-huCMP-4-1BBL, filled circles) nor untargeted (DP47-targeted) huCMP-trimeric 4-1BB ligand-containing antigen binding molecules (trimeric DP47-huCMP-4-1BBL) did bind to resting (naive) human $CD4^+$ T-cells or $CD8^+$ T-cells. In contrast, both constructs bound strongly to activated $CD8^+$ or $CD4^+$ T-cells, although the latter showed approximately 6-fold lower intensity of specific fluorescence as compared to $CD8^+$ T-cells. This is consistent with the fact, that human 4-1BB is normally 10-20 fold higher expressed on $CD8^+$ T cells compared to $CD4^+$ T cells (depends on donor and activation protocol).

**4.2. Binding on FAP-expressing tumor cells**

For binding assays on FAP expressing cells, the NIH/3T3-huFAP clone 39 cell line was used. To generate this cell line, NIH/3T3 cells were transfected with human FAP (NIH/3T3-huFAP clone 39). The cells were generated by transfection of mouse embryonic fibroblast NIH/3T3 cells (ATCC CRL-1658) with the expression pETR4921 plasmid encoding human FAP under a CMV promoter. Cells were maintained in the presence of 1.5 μg/mL puromycin (InvivoGen, Cat.-No.: ant-pr-5). 0.1 x $10^6$ of FAP expressing tumor cells were added to each well of a round-bottom suspension cell 96-well plates (Greiner bio-one, cellstar, Cat.-No. 650185). Cells were washed once with 200 μL/well 4 °C cold FACS buffer and resuspended in 50 μL/well of 4 °C cold FACS buffer containing different concentrations of titrated FAP-targeted or DP47-targeted CMP-trimeric 4-1BB ligand-containing antigen binding molecule. Resuspended cells were incubated for 2 hour at 4 °C. After washing four times with 200 μL/well FACS buffer, cells were stained with 50 μL/well of 4°C cold FACS buffer containing either 68 μg/mL polyclonal Fluorescein (FITC)-conjugated AffiniPure Fab Fragment Goat Anti-Human IgG (H+L) or 0.25 ug/mL monoclonal Fluorescein (FITC)-conjugated anti-human IgG-specific mouse IgG1κ (clone G18-145) or 8.3 ug/mL monoclonal phycoerythrin (PE)-labeled anti-human 4-1BB ligand mouse IgG2b (Clone 282220) for 1 h at 4°C. Cells were washed twice with 200 μL/well cold FACS buffer and resuspended in 100 μL/well FACS buffer containing 0.2 μg/mL DAPI (Santa Cruz Biotec, Cat. No. Sc-3598) and acquired the same day using a 5-laser LSR-Fortessa (BD Bioscience with DIVA software).

As shown in **Figure 5,** the FAP-targeted CMP-trimeric 4-1BB ligand-containing antigen binding molecule, but not the untargeted, DP47-Fab-containing construct (DP47-targeted CMP-trimeric 4-1BB ligand), efficiently bound to human FAP-expressing cells. At concentrations above 10nM the DP47-targeted CMP-trimeric 4-1BB ligand-containing antigen binding molecule shows unspecific binding if detected with anti-huIgG (H+L) or anti-huIgGκ-specific antibodies.

**Example 5**

**Biological Activity of targeted CMP trimeric 4-1BB ligand-containing antigen binding molecules**

**5.1. NF-κB-Luciferase activation in transgenic reporter cell line HeLa cells expressing human 4-1BB**

5.1.1. Generation of HeLa cells expressing human 4-1BB and 3NF-κB-luciferase

The cervix carcinoma cell line HeLa (ATCC CCL-2) was transduced with a plasmid based on the expression vector pETR10829, which contains the sequence of human 4-1BB (Uniprot accession Q07011) under control of a CMV-promoter and a puromycin resistance gene. Cells were cultured in DMEM medium supplemented with 10 % (v/v) FBS, 1 % (v/v) GlutaMAX-I and 3 μg/mL Puromycin.

4-1BB-transduced HeLa cells were tested for 4-1BB expression by flow cytometry: 0.2x$10^6$ living cells were resuspended in 100 μL FACS buffer containing 0.1 μg PerCP/Cy5.5 conjugated anti-human 4-1BB mouse IgG1κ clone 4B4-1 (BioLegend Cat.-No.309814) or its isotype control (PerCP/Cy5.5 conjugated mouse IgG1κ isotype control antibody clone MOPC-21, BioLegend Cat.-No.400150) and incubated for 30 minutes at 4 °C. Cells were washed twice with FACS buffer, resuspended in 300 μL FACS buffer containing 0.06 μg DAPI (Santa Cruz Biotec, Cat. No. Sc-3598) and acquired using a 5-laser LSR-Fortessa (BD Bioscience, DIVA software). Limited dilutions were performed to generate single clones as described: human-4-1BB-transduced HeLa cells were resuspended in medium to a density of 10, 5 and 2.5 cells/ml and 200 μl of cell suspensions were transferred to round bottom tissue-culture treated 96-well plates (6 plates/cell concentration, TPP Cat.-No. 92697). Single clones were harvested, expanded and tested for 4-1BB expression as described above. The clone with the highest expression of 4-1BB (clone 5) was chosen for subsequent transfection with the NFκB-luciferase expression-vector 5495p Tranlucent HygB. The vector confers transfected cells both with resistance to Hygromycin B and capacity to express luciferase under control of NF-κB-response element (Panomics, Cat.-No. LR0051). Human-4-1BB HeLa clone 5 cells were cultured to 70 % confluence. 50 μg (40 μL) linearized (restriction enzymes AseI and SalI) 5495p Tranlucent HygB expression vector were added to a sterile 0.4 cm Gene Pulser/Micro-Pulser Cuvette (Biorad, Cat.-No, 165-2081). 2.5x$10^6$ human-4-1BB HeLa clone 5 cells in 400 μl supplement-free DMEM medium were added and mixed carefully with the plasmid solution. Transfection of cells was performed using a Gene Pulser Xcell total system (Biorad, Cat-No. 165-2660) under the following settings: exponential pulse, capacitance 500 μF, voltage 160 V, resistance ∞. Immediately after the pulse transfected cells were transferred to a 75 $cm^2$ tissue culture flask (TPP, Cat.-No. 90075) with 15 mL 37 °C warm DMEM medium supplied with 10 % (v/v) FBS and 1 % (v/v) GlutaMAX-I. Next day, culture medium containing 3 (μg/mL Puromycin and 200 (μg/mL Hygromycin B (Roche, Cat.-No. 10843555001) was added. Surviving cells were expanded and limited dilution was performed as described above to generate single clones.

Clones were tested for 4-1BB expression as described above and for NF-κB-Luciferase activity as following: Clones were harvested in selection medium and counted using a Cell Counter Vi-cell xr 2.03 (Beckman Coulter, Cat.-No. 731050). Cells were set to a cell density of 0.33x$10^6$ cells/mL and 150 μL of this cell suspension were transferred to each well of a sterile white 96-well flat bottom tissue culture plate with lid (greiner bio-one, Cat.-No. 655083) and - as a control - to normal 96-well flat bottom tissue culture plate (TPP Cat.-No. 92096) to test survival and cell density the next day. Cells were incubated at 37°C and 5 % $CO_2$ overnight. The next day 50 μL of medium containing different concentrations of recombinant human tumor necrosis factor alpha (rhTNF-α, PeproTech, Cat.-No. 300-01A) were added to each well of a 96-well plate resulting in final concentration of rhTNF-α of 100, 50, 25, 12.5, 6.25 and 0 ng/well. Cells were incubated for 6 hours at 37°C and 5 % $CO_2$ and then washed three times with 200μL/well DPBS. Reporter Lysis Buffer (Promega, Cat-No: E3971) was added to each well (40 μl) and the plates were stored over night at -20°C. The next day frozen cell plates and Detection Buffer (Luciferase 1000 Assay System, Promega, Cat.-No. E4550) were thawed to room temperature. 100 uL of detection buffer were added to each well and the plate was measured as fast as possible using a SpectraMax M5/M5e microplate reader and the SoftMax Pro Software (Molecular Devices). Measured units of released light for 500 ms/well (URLs) above control (no rhTNF-α added) were taken as luciferase activity. The NF-κB-luc-4-1BB-HeLa clone 26 exhibiting the highest luciferase activity and a considerable level of 4-1BB-expression and was chosen for further use.

5.1.2. NF-κB activation in Hela cells expressing human 4-1BB co-cultured with FAP-expressing tumor cells

NF-κB-luciferase human-4-1BB HeLa cells were harvested and resuspended in DMEM medium supplied with 10 % (v/v) FBS and 1 % (v/v) GlutaMAX-I to a concentration of 0.2 x $10^6$ cells/ml. 100 μl (2 x $10^4$ cells) of this cell suspension were transferred to each well of a sterile white 96-well flat bottom tissue culture plate with lid (greiner bio-one, Cat. No. 655083) and the plate were incubated at 37 °C and 5 % $CO_2$ overnight. The next day 50 μL of medium containing different concentrations of titrated FAP-targeted CMP-trimeric 4-1BB ligand-containing molecule or DP47-untargeted CMP-trimeric 4-1BB ligand-containing molecule were added. FAP-expressing tumor cells were resuspended in DMEM medium supplied with 10 % (v/v) FBS and 1 % (v/v) GlutaMAX-I to a concentration of 2 x $10^6$ cells/ml.

Following FAP-expressing tumor cells were used:

- human melanoma cell line MV3 (first published: van Muijen GN, Jansen KF, Cornelissen IM, Smeets DF, Beck JL, Ruiter DJ. Establishment and characterization of a human melanoma cell line (MV3) which is highly metastatic in nude mice. Int J Cancer. 1991 Apr 22;48(1):85-91)

- human female melanoma WM-266-4 cell line (ATCC CRL-1676).

- mouse embryonic fibroblast NIH/3T3 (ATCC CRL-1658) transfected with expression vector pETR4921 to express human FAP and Puromycin resistance: NIH/3T3-huFAP clone 39 as described in Example 4.2.

50 μL of FAP-expressing tumor cell suspension were added to each well and plates were incubated for 6 hours at 37 °C and 5 % $CO_2$. Cells were washed three times with 200 μL/well DPBS. 40 μl freshly prepared Reporter Lysis Buffer (Promega, Cat-No: E3971) were added to each well and the plate were stored over night at -20 °C. The next day frozen cell plate and Detection Buffer (Luciferase 1000 Assay System, Promega, Cat. No. E4550) were thawed at room temperature. 100 μL of detection buffer were added to each well and luciferase activity was measured as fast as possible using a SpectraMax M5/M5e microplate reader and a SoftMax Pro Software (Molecular Devices).

As shown in **Figure 7,** FAP-targeted huCMP-trimeric 4-1BB ligand-containing antigen binding molecule (filled circles) triggered activation of the NF-κB signaling pathway in the reporter cell line in the presence of FAP-expressing tumor cells. In contrast, the untargeted variant of the same molecule failed to trigger such an effect at any of the tested concentrations (open circles). This activity of targeted 4-1BBL was strictly dependent on the expression of FAP at the cell surface of tumor cells as no NFkB activation could be detected upon culturing of the NF-κB reporter cell line with FAP-negative tumor cells even in the presence of FAP-targeted huCMP-trimeric 4-1BB ligand-containing antigen binding molecule (data not shown). Further activation depends on an optimal concentration window supporting a maximal crosslinking of the FAP-targeted huCMP-trimeric 4-1BB ligand-containing antigen binding molecule (bell-shape curves). The maximal activation is induced at a concentration around 0.3-0.4 nM.

5.1.3. Antigen-specific $CD8^+$ T cell based assay

Cells of the human FAP-expressing melanoma cell line MV3 cell line (described in Example 5.1.2.) were harvested and washed with DPBS and 2 x $10^7$ cells were resuspended in 250 μL C diluent of the PKH-26 Red Fluorescence Cell linker Kit (Sigma, Cat.-No. PKH26GL). 1 μL PKH26-red-stain solution was diluted with 250 μL C diluent and added to the suspension of MV3 cells which were then incubated for 5 min at room temperature in the dark. 0.5 mL FBS were added, cells were incubated for 1 minute and washed once with T cell medium consisting of RPMI 1640 medium supplemented with 10 % (v/v) FBS, 1 % (v/v) GlutaMAX-I, 1 mM Sodium-Pyruvate, 1 % (v/v) MEM non-essential amino acids and 50 μM (β-Mercaptoethanol. 1 x $10^6$ MV3 cells/mL were resuspended in T cell medium and separated into three tubes. Synthetic NLVPMVATV peptide (obtained from thinkpeptides) was added to a final concentration of $1x10^{-9}$ M or $1x10^{-8}$ M and cells were incubated for 90 min under rotation at 37°C and 5% $CO_2$. MV3 cells were washed once with T cell medium and resuspended to a density of 0.5 x $10^6$ cells/mL, distributed (100 μL/well) to a 96-well round bottom cell-suspension plate (Greiner bio-one, cellstar, Cat. No. 650185) and incubated over night at 37°C and 5 % $CO_2$.

The next day, different concentrations of FAP-targeted or DP47-untargeted huCMP-trimeric 4-1BB ligand-containing antigen binding molecules were added with 50 μL of T-cell medium (final concentrations of FAP-targeted or DP47-targeted single chain tumeric 4-1BB ligand were between 5 to 0.002 nM). NLV-specific CD8 T cells were harvested, CFSE-labeled and added in 50 μL medium to each well (final tumor: CD8 T cell ratio 0.125). Cells were incubated for 24 h, 50 μL/well T cell medium containing 2.64 μL/mL Golgi stop (Protein Transport Inhibitor containing Monesin, BD Bioscience, Cat.-No. 554724) were added to each well. Cells were incubated for further 4 h, washed with 200 μL/well DPBS and stained with 100 μL 4 °C DPBS containing 1:5000 diluted Fixable Viability Dye-eF450 (eBioscience, Cat. No. 65-0864) for 30 minutes at 4 °C. Cells were washed with DPBS and the stained in 40 μL/well FACS buffer containing following fluorescent dye-conjugated antibodies: anti-human CD137-PerCP/Cy5.5 (clone 4B4-1, mouse IgG1κ, BioLegend, Cat.-No. 309814), anti-human CD70-PE (clone 113-16, mouse IgG1κ, BioLegend, Cat.-No. 355104) and anti-human PD-1-PE/Cy7 (clone EH12.2H7, mouse IgG1κ, BioLegend, Cat.-No. 329918). After incubation for 30 min at 4 °C, cells were washed twice with 200 μL/well FACS buffer, resuspended in 50 μL/well freshly prepared FoxP3 Fix/Perm buffer (eBioscience Cat.-No. 00-5123 and 00-5223) and incubated for 30 min at 4 °C. Plates were washed twice with 200 μL/well Perm-Buffer (DPBS supplied with 2 % (v/v) FBS, 1 % (w/v) saponin (Sigma Life Science, S7900) and 1 % (w/v) sodium azide (Sigma-Aldrich, S2002) and stained with 50 μL/well Perm-Buffer (eBioscience, Cat.-No. 00-8333-56) containing 0.25 μg/mL anti-human IFNγ-APC (clone B27, mouse IgG1κ, BioLegend, Cat. No. 506510). Plates were incubated for 1 h at 4°C and washed twice with 200 μL/well Perm-Buffer. For fixation, 50 μL/well DPBS containing 1 % formaldehyde were added and cells were stored overnight at 4°C. The next day, cells were resuspended in 100 μL/well FACS buffer and acquired using a 5-laser Fortessa flow cytometer (BD Bioscience with DIVA software).

As shown in **Figure 9,** antigen-specific $CD8^+$ T cells, but not unstimulated controls, exhibited increased levels of surface 4-1BB expression in the presence of FAP-targeted huCMP trimeric 4-1BB ligand-containing antigen binding molecules (filled circles). This effect of FAP-targeted huCMP trimeric 4-1BB ligand-containing antigen binding molecules was dose dependent and required FAP-targeting as addition of the untargeted control molecule did not affect the level of 4-1BB expression. Furthermore, T-cells activated with peptide showed sustained secretion of INFγ in the presence of FAP-targeted huCMP trimeric 4-1BB ligand-containing antigen binding molecules. Collectively, these data demonstrate that the FAP-targeted huCMP trimeric 4-1BB ligand-containing antigen binding molecule modulates the surface phenotype and responsiveness of antigen specific T-cells in a targeting dependent manner.

**Example 6**

**Functional Characterization of targeted huCMP trimeric OX40 ligand-containing antigen binding molecules**

**6.1. Binding on human FAP positive tumor cells**

The binding to cell surface FAP was tested using WM-266-4 cells (ATCC CRL-1676). 5 x $10^4$ WM-266-4 cells were added to each well of a round-bottom suspension cell 96-well plates (greiner bio-one, cellstar, Cat. No. 650185). Cells were stained for 120 minutes at 4°C in the dark in 50 μL/well 4 °C cold FACS buffer (DPBS (Gibco by Life Technologies, Cat. No. 14190 326) w/ BSA (0.1 % v/w, Sigma-Aldrich, Cat. No. A9418) containing titrated huCMP trimeric OX40 ligand-containing antigen binding molecules. After three times washing with excess FACS buffer, cells were stained for 60 minutes at 4°C in the dark in 25 μL/well 4 °C cold FACS buffer containing Fluorescein isothiocyanate (FITC)-conjugated AffiniPure anti-human IgG F(ab')$_2$-fragment-specific goat IgG F(ab')2 fragment (Jackson ImmunoResearch, Cat.-No. 109-096-097).

Plates were finally resuspended in 90 μL/well FACS-buffer containing 0.2 μg/mL DAPI (Santa Cruz Biotec, Cat. No. Sc-3598) and acquired the same day using 5-laser LSR-Fortessa (BD Bioscience with DIVA software).

As shown in **Figure 10,** the FAP(28H1)-targeted huCMP trimeric Ox40 ligand-containing antigen binding molecule (trimeric FAP-huCMP-OX40L) but not the negative control (an unspecific DP47 hu IgG1 P329G LALA antibody termed control F) efficiently bound to human FAP-expressing target cells. $EC_{50}$ value of binding to FAP positive WM-266-4 was 17.3 nM.

**6.2. Binding to human Ox40 expressing cells: naïve and activated human peripheral mononuclear blood leukocytes (PBMC)**

Buffy coats were obtained from the Zurich blood donation center. To isolate fresh peripheral blood mononuclear cells (PBMCs) the buffy coat was diluted with the same volume of DPBS (Gibco by Life Technologies, Cat. No. 14190 326). 50 mL polypropylene centrifuge tubes (TPP, Cat.-No. 91050) were supplied with 15 mL Histopaque 1077 (SIGMA Life Science, Cat.-No. 10771, polysucrose and sodium diatrizoate, adjusted to a density of 1.077 g/mL) and the buffy coat solution was layered above the Histopaque 1077. The tubes were centrifuged for 30 min at 400 x g, room temperature and with low acceleration and no break. Afterwards the PBMCs were collected from the interface, washed three times with DPBS and resuspended in T cell medium consisting of RPMI 1640 medium (Gibco by Life Technology, Cat. No. 42401-042) supplied with 10 % Fetal Bovine Serum (FBS, Gibco by Life Technology, Cat. No. 16000-044, Lot 941273, gamma-irradiated, mycoplasma-free and heat inactivated at 56 °C for 35 min), 1 % (v/v) GlutaMAX I (GIBCO by Life Technologies, Cat. No. 35050 038), 1 mM Sodium-Pyruvat (SIGMA, Cat. No. S8636), 1 % (v/v) MEM non-essential amino acids (SIGMA, Cat.-No. M7145) and 50 μM β-Mercaptoethanol (SIGMA, M3148).

PBMCs were used directly after isolation (binding on resting human PBMCs) or they were stimulated to receive a strong human Ox40 expression on the cell surface of T cells (binding on activated human PBMCs). Therefore naive PBMCs were cultured for three days in T cell medium supplied with 200 U/mL Proleukin (Novartis) and 2 μg/mL PHA-L (Sigma-Aldrich, L2769-10) in 6-well tissue culture plate at 37 °C and 5% $CO_2$.

Cells were stained for 120 minutes at 4 °C in the dark in 50 μL/well 4 °C cold FACS buffer containing titrated anti -Ox40 antibody constructs. After three times washing with excess FACS buffer, cells were stained for 1 h at 4°C in the dark in 25 μL/well 4 °C cold FACS buffer containing a mixture of fluorescently labeled anti-human CD4 (clone OKT4, mouse IgG1 k, BioLegend, Cat.-No. 317428), anti-human CD8 (clone RPa-T8, mouse IgG1k, BD Pharmingen, Cat.-No. 555368) and Fluorescein isothiocyanate (FITC)-conjugated AffiniPure anti-human IgG F(ab')$_2$-fragment-specific goat IgG F(ab')2 fragment (Jackson ImmunoResearch, Cat.-No. 109-096-097). Plates were finally resuspended in 90 μL/well FACS-buffer containing 0.2 μg/mL DAPI (Santa Cruz Biotec, Cat. No. Sc-3598) and acquired the same day using 5-laser LSR-Fortessa (BD Bioscience with DIVA software).

As shown in Figures 11A to 11D, FAP-targeted huCMP trimeric OX40 ligand-containing antigen binding molecules did not bind to resting human $CD4^+$ T-cells or $CD8^+$ T-cells, which are negative for OX40. In contrast, FAP-targeted huCMP trimeric Ox40 ligand-containing antigen binding molecules bound to activated $CD8^+$ or $CD4^+$ T-cells, which do express OX40. Binding to $CD4^+$ T-cells was much stronger than that to $CD8^+$ T cells. Activated human $CD8^+$ T cells do express only a fraction of the OX40 levels detected on activated $CD4^+$ T cells. Expression levels for Ox40 are depending on kinetic and strength of stimulation and conditions were here optimized for Ox40 expression on $CD4^+$ T cells but not for $CD8^+$ T cells. Thus, only little OX40 expression was induced on CD8 T cells. The $EC_{50}$ value of binding to OX40 positive $CD4^+$ T cells was 10.2 nM and the $EC_{50}$ value of binding to OX40 positive $CD8^+$ T cells was 3.0 nM.

**6.3. HeLa cells expressing human OX40 and reporter gene NF-κB-luciferase**

Agonstic binding of OX40 to its ligand induces downstream signaling via activation of nuclear factor kappa B (NFκB) (A. D. Weinberg et al., J. Leukoc. Biol. 2004, 75(6), 962-972). The recombinant reporter cell line HeLa_hOx40_NFkB_Luc1 was generated to express human OX40 on its surface. Additionally, it harbors a reporter plasmid containing the luciferase gene under the control of an NFκB-sensitive enhancer segment. OX40 triggering induces dose-dependent activation of NFκB, which translocates in the nucleus, where it binds on the NFκB sensitive enhancer of the reporter plasmid to increase expression of the luciferase protein. Luciferase catalyzes luciferin-oxidation resulting in oxyluciferin which emits light. This can be quantified by a luminometer. Thus, the capacity of the various anti-OX40 molecules to induce NFκB activation in HeLa_hOx40_NFkB_Luc1 reporter cells was analyzed as a measure for bioactivity.

Adherent HeLa_hOx40_NFkB_Luc1 cells were harvested using cell dissociation buffer (Invitrogen, Cat.-No. 13151-014) for 10 minutes at 37 °C. Cells were washed once with DPBS and were adjusted to a cell density of $2x10^5$ in assay media comprising of MEM (Invitrogen, Cat.-No. 22561-021), 10 % (v/v) heat-inactivated FBS, 1 mM Sodium-Pyruvat and 1% (v/v) non-essential amino acids. Cells were seeded in a density of $0.3*10^5$ cells per well in a sterile white 96-well flat bottom tissue culture plate with lid (greiner bio-one, Cat. No. 655083) and kept over night at 37 °C and 5% $CO_2$ in an incubator (Hera Cell 150).

The next day, HeLa_hOx40_NFkB_Luc1 were stimulated for 6 hours by adding assay medium containing titrated FAP-Ox40L or negative control F. For testing the effect of hyper-crosslinking on anti-Ox40 antibodies, 25 μL/well of medium containing FAP+ tumor cell line NIH/3T3-huFAP clone 39 was added in a 1:2 ratio (2 times more NIH/3T3-huFAP clone 39 than HeLa_hOx40_NFkB_Luc1 cells).

NIH/3T3-huFAP clone 39 was generated by the transfection of the mouse embryonic fibroblast NIH/3T3 cell line (ATCC CRL-1658) with the expression vector pETR4921 to express huFAP under 1.5 μg/mL Puromycin selection. The surface expression of FAP was quantified using the Quifikit (Dako Cat. No. K0078) according to manufactures instructions. The primary antibody used to detect cell surface FAP expression was the human/ mouse crossreactive clone F11-24 (mouse IgG1, Calbiochem, Ca. No. OP188). The surface expression on NIH/3T3-huFAP clone 39 was app. 90000 huFAP per cell.

After incubation, supernatant was aspirated and plates washed two times with DPBS. Quantification of light emission was done using the luciferase 100 assay system and the reporter lysis buffer (both Promega, Cat.-No. E4550 and Cat-No: E3971) according to manufacturer instructions. Briefly, cells were lysed for 10 minutes at -20 °C by addition of 30 uL per well 1x lysis buffer. Cells were thawed for 20 minutes at 37 °C before 90 uL per well provided luciferase assay reagent was added. Light emission was quantified immediately with a SpectraMax M5/M5e microplate reader (Molecular Devices, USA) using 500ms integration time, without any filter to collect all wavelengths. Emitted relative light units (URL) were corrected by basal luminescence of HeLa_hOx40_NFkB_Luc1 cells and were blotted against the logarithmic primary antibody concentration using Prism4 (GraphPad Software, USA). Curves were fitted using the inbuilt sigmoidal dose response.

As shown in Figures 12A and 12B, limited, dose dependent NFκB activation was induced already by addition of FAP-targeted huCMP trimeric OX40 ligand-containing antigen binding molecules to the reporter cell line (Figure 12A). The presence of FAP-expressing tumor cells strongly increased induction of NFκB-mediated luciferase-activation when FAP-targeted huCMP trimeric OX40 ligand-containing antigen binding molecule was added (Figure 12B).

**Citations:**

Aggarwal B.B. (2003), Signalling pathways of the TNF superfamily: a double-edged sword. Nat. Rev. Immunol. 3(9),745-56.

Banner D. et al (1993), Crystal structure of the soluble human 55 kd TNF receptor-human TNF beta complex: implications for TNF receptor activation. Cell 73, 431-445.

Beck K., Gambee J., Bohan C., Bächinger H.P. (1996), The C-terminal domain of cartilage matrix protein assembles into a triple-stranded α-helical coiled-coil structure. J. Mol. Biol. 256, 909-923.

Bodmer J., Schneider P. and Tschopp, J. (2002), The molecular architecture of the TNF superfamily. Trends in Biochemical Sciences 27(1), 19-26.

Broll, K., Richter, G., Pauly, S., Hofstaedter, F., and Schwarz, H. (2001). CD137 expression in tumor vessel walls. High correlation with malignant tumors. Am J Clin Pathol 115, 543-549.

Buechele, C., Baessler, T., Schmiedel, B.J., Schumacher, C.E., Grosse-Hovest, L., Rittig, K., and Salih, H.R. (2012). 4-1BB ligand modulates direct and Rituximab-induced NK-cell reactivity in chronic lymphocytic leukemia. Eur J Immunol 42, 737-748.

Choi, B.K., Kim, Y.H., Kwon, P.M., Lee, S.C., Kang, S.W., Kim, M.S., Lee, M.J., and Kwon, B.S. (2009). 4-1BB functions as a survival factor in dendritic cells. J Immunol 182, 4107-4115.

Cuadros, C., Dominguez, A.L., Lollini, P.L., Croft, M., Mittler, R.S., Borgstrom, P., and Lustgarten, J. (2005). Vaccination with dendritic cells pulsed with apoptotic tumors in combination with anti-OX40 and anti-4-1BB monoclonal antibodies induces T cell-mediated protective immunity in Her-2/neu transgenic mice. Int J Cancer 116, 934-943.

Curran, M.A., Kim, M., Montalvo, W., Al-Shamkhani, A., and Allison, J.P. (2011). Combination CTLA-4 blockade and 4-1BB activation enhances tumor rejection by increasing T-cell infiltration, proliferation, and cytokine production. PLoS One 6, e19499.

Diehl, L., van Mierlo, G.J., den Boer, A.T., van der Voort, E., Fransen, M., van Bostelen, L., Krimpenfort, P., Melief, C.J., Mittler, R., Toes, R.E., and Offringa, R. (2002). In vivo triggering through 4-1BB enables Th-independent priming of CTL in the presence of an intact CD28 costimulatory pathway. J Immunol 168, 3755-3762.

Dubrot, J., Milheiro, F., Alfaro, C., Palazon, A., Martinez-Forero, I., Perez-Gracia, J.L., Morales-Kastresana, A., Romero-Trevejo, J.L., Ochoa, M.C., Hervas-Stubbs, S., et al. (2010). Treatment with anti-CD 137 mAbs causes intense accumulations of liver T cells without selective antitumor immunotherapeutic effects in this organ. Cancer Immunol Immunother 59, 1223-1233.

Futagawa, T., Akiba, H., Kodama, T., Takeda, K., Hosoda, Y., Yagita, H., and Okumura, K. (2002). Expression and function of 4-1BB and 4-1BB ligand on murine dendritic cells. Int Immunol 14, 275-286.

Guo, Z., Cheng, D., Xia, Z., Luan, M., Wu, L., Wang, G., and Zhang, S. (2013). Combined TIM-3 blockade and CD 137 activation affords the long-term protection in a murine model of ovarian cancer. J Transl Med 11, 215.

Heinisch, I.V., Daigle, I., Knopfli, B., and Simon, H.U. (2000). CD137 activation abrogates granulocyte-macrophage colony-stimulating factor-mediated anti-apoptosis in neutrophils. Eur J Immunol 30, 3441-3446.

Ju, S.A., Cheon, S.H., Park, S.M., Tam, N.Q., Kim, Y.M., An, W.G., and Kim, B.S. (2008). Eradication of established renal cell carcinoma by a combination of 5-fluorouracil and anti-4-1BB monoclonal antibody in mice. Int J Cancer 122, 2784-2790.

Kienzle, G., and von Kempis, J. (2000). CD137 (ILA/4-1BB), expressed by primary human monocytes, induces monocyte activation and apoptosis of B lymphocytes. Int Immunol 12, 73-82.

Kim, D.H., Chang, W.S., Lee, Y.S., Lee, K.A., Kim, Y.K., Kwon, B.S., and Kang, C.Y. (2008). 4-1BB engagement costimulates NKT cell activation and exacerbates NKT cell ligand-induced airway hyperresponsiveness and inflammation. J Immunol 180, 2062-2068.

Kim, Y.H., Choi, B.K., Oh, H.S., Kang, W.J., Mittler, R.S., and Kwon, B.S. (2009). Mechanisms involved in synergistic anticancer effects of anti-4-1BB and cyclophosphamide therapy. Mol Cancer Ther 8, 469-478.

Kwon, B.S., and Weissman, S.M. (1989). cDNA sequences of two inducible T-cell genes. Proc Natl Acad Sci U S A 86, 1963-1967.

Lee, H., Park, H.J., Sohn, H.J., Kim, J.M., and Kim, S.J. (2011). Combinatorial therapy for liver metastatic colon cancer: dendritic cell vaccine and low-dose agonistic anti-4-1BB antibody costimulatory signal. J Surg Res 169, e43-50.

Li, F., and Ravetch, J.V. (2011). Inhibitory Fcgamma receptor engagement drives adjuvant and anti-tumor activities of agonistic CD40 antibodies. Science 333, 1030-1034.

Lin, W., Voskens, C.J., Zhang, X., Schindler, D.G., Wood, A., Burch, E., Wei, Y., Chen, L., Tian, G., Tamada, K., et al. (2008). Fc-dependent expression of CD137 on human NK cells: insights into "agonistic" effects of anti-CD 137 monoclonal antibodies. Blood 112, 699-707.

Melero, I., Johnston, J.V., Shufford, W.W., Mittler, R.S., and Chen, L. (1998). NK1.1 cells express 4-1BB (CDw137) costimulatory molecule and are required for tumor immunity elicited by anti-4-1BB monoclonal antibodies. Cell Immunol 190, 167-172.

Melero, I., Shuford, W.W., Newby, S.A., Aruffo, A., Ledbetter, J.A., Hellstrom, K.E., Mittler, R.S., and Chen, L. (1997). Monoclonal antibodies against the 4-1BB T-cell activation molecule eradicate established tumors. Nat Med 3, 682-685.

Merchant, A.M., Zhu, Z., Yuan, J.Q., Goddard, A., Adams, C.W., Presta, L.G., and Carter, P. (1998). An efficient route to human bispecific IgG. Nat Biotechnol 16, 677-681.

Morales-Kastresana, A., Sanmamed, M.F., Rodriguez, I., Palazon, A., Martinez-Forero, I., Labiano, S., Hervas-Stubbs, S., Sangro, B., Ochoa, C., Rouzaut, A., et al. (2013). Combined immunostimulatory monoclonal antibodies extend survival in an aggressive transgenic hepatocellular carcinoma mouse model. Clin Cancer Res 19, 6151-6162.

Murillo, O., Dubrot, J., Palazon, A., Arina, A., Azpilikueta, A., Alfaro, C., Solano, S., Ochoa, M.C., Berasain, C., Gabari, I., et al. (2009). In vivo depletion of DC impairs the anti-tumor effect of agonistic anti-CD137 mAb. Eur J Immunol 39, 2424-2436.

Narazaki, H., Zhu, Y., Luo, L., Zhu, G., and Chen, L. (2010). CD137 agonist antibody prevents cancer recurrence: contribution of CD 137 on both hematopoietic and nonhematopoietic cells. Blood 115, 1941-1948.

Nishimoto, H., Lee, S.W., Hong, H., Potter, K.G., Maeda-Yamamoto, M., Kinoshita, T., Kawakami, Y., Mittler, R.S., Kwon, B.S., Ware, C.F., et al. (2005). Costimulation of mast cells by 4-1BB, a member of the tumor necrosis factor receptor superfamily, with the high-affinity IgE receptor. Blood 106, 4241-4248.

Olofsson, P.S., Soderstrom, L.A., Wagsater, D., Sheikine, Y., Ocaya, P., Lang, F., Rabu, C., Chen, L., Rudling, M., Aukrust, P., et al. (2008). CD137 is expressed in human atherosclerosis and promotes development of plaque inflammation in hypercholesterolemic mice. Circulation 117, 1292-1301.

Palazon, A., Teijeira, A., Martinez-Forero, I., Hervas-Stubbs, S., Roncal, C., Penuelas, I., Dubrot, J., Morales-Kastresana, A., Perez-Gracia, J.L., Ochoa, M.C., et al. (2011). Agonist anti-CD137 mAb act on tumor endothelial cells to enhance recruitment of activated T lymphocytes. Cancer Res 71, 801-811.

Schwarz, H., Valbracht, J., Tuckwell, J., von Kempis, J., and Lotz, M. (1995). ILA, the human 4-1BB homologue, is inducible in lymphoid and other cell lineages. Blood 85, 1043-1052.

Shao, Z., and Schwarz, H. (2011). CD137 ligand, a member of the tumor necrosis factor family, regulates immune responses via reverse signal transduction. J Leukoc Biol 89, 21-29.

Shi, W., and Siemann, D.W. (2006). Augmented antitumor effects of radiation therapy by 4-1BB antibody (BMS-469492) treatment. Anticancer Res 26, 3445-3453.

Simeone, E., and Ascierto, P.A. (2012). Immunomodulating antibodies in the treatment of metastatic melanoma: the experience with anti-CTLA-4, anti-CD 137, and anti-PD1. J Immunotoxicol 9, 241-247.

Snell, L.M., Lin, G.H., McPherson, A.J., Moraes, T.J., and Watts, T.H. (2011). T-cell intrinsic effects of GITR and 4-1BB during viral infection and cancer immunotherapy. Immunol Rev 244, 197-217.

Stagg, J., Loi, S., Divisekera, U., Ngiow, S.F., Duret, H., Yagita, H., Teng, M.W., and Smyth, M.J. (2011). Anti-ErbB-2 mAb therapy requires type I and II interferons and synergizes with anti-PD-1 or anti-CD137 mAb therapy. Proc Natl Acad Sci U S A 108, 7142-7147.

Teng, M.W., Sharkey, J., McLaughlin, N.M., Exley, M.A., and Smyth, M.J. (2009). CD1d-based combination therapy eradicates established tumors in mice. J Immunol 183, 1911-1920.

von Kempis, J., Schwarz, H., and Lotz, M. (1997). Differentiation-dependent and stimulus-specific expression of ILA, the human 4-1BB-homologue, in cells of mesenchymal origin. Osteoarthritis Cartilage 5, 394-406.

Watts, T.H. (2005). TNF/TNFR family members in costimulation of T cell responses. Annu. Rev. Immunol. 23, 23-68

Wei, H., Zhao, L., Li, W., Fan, K., Qian, W., Hou, S., Wang, H., Dai, M., Hellstrom, I., Hellstrom, K.E., and Guo, Y. (2013). Combinatorial PD-1 blockade and CD137 activation has therapeutic efficacy in murine cancer models and synergizes with cisplatin. PLoS One 8, e84927.

Weinberg A.D., Evans D.E., Thalhofer C, Shi T., and Prell R.A. (2004). The generation of T cell memory: a review describing the molecular and cellular events following OX40 (CD 134) engagement. J. Leukoc. Biol. 75, 962-972.

Wilcox, R.A., Chapoval, A.I., Gorski, K.S., Otsuji, M., Shin, T., Flies, D.B., Tamada, K., Mittler, R.S., Tsuchiya, H., Pardoll, D.M., and Chen, L. (2002). Cutting edge: Expression of functional CD137 receptor by dendritic cells. J Immunol 168, 4262-4267.

Wilcox, R.A., Tamada, K., Flies, D.B., Zhu, G., Chapoval, A.I., Blazar, B.R., Kast, W.M., and Chen, L. (2004). Ligation of CD137 receptor prevents and reverses established anergy of CD8+ cytolytic T lymphocytes in vivo. Blood 103, 177-184.

Wyzgol A., Müller N., Fick A., Munkel S., Grigoleit G.U., Pfizenmaier K., Wajant H. (2009), Trimer Stabilization, Oligomerization, and Antibody-mediated cell surface immobilization improve the activity of soluble trimers of CD27L, CD40L, 41BBL and Glucocorticoid-induced TNF receptor ligand. J. Immunol. 183, 1851-1861.

Zhang, X., Voskens, C.J., Sallin, M., Maniar, A., Montes, C.L., Zhang, Y., Lin, W., Li, G., Burch, E., Tan, M., et al. (2010). CD 137 promotes proliferation and survival of human B cells. J Immunol 184, 787-795.

## Claims

1. A trimeric costimulatory TNF family ligand-containing antigen binding molecule comprising three fusion polypeptides, each of the three fusion polypeptides comprising

   (a) an ectodomain of a costimulatory TNF ligand family member or fragments thereof,
   (b) a trimerization domain, and
   (c) a moiety capable of specific binding to a target cell antigen

2. The trimeric costimulatory TNF family ligand-containing antigen binding molecule of claim 1 comprising three fusion polypeptides, each of the three fusion polypeptides comprising

(a) an ectodomain of a costimulatory TNF ligand family member or fragments thereof,
(b) a trimerization domain derived from human cartilage matrix protein (huCMP) of amino acid sequence of SEQ ID NO:1, and
(c) a moiety capable of specific binding to a target cell antigen.

3. The trimeric costimulatory TNF family ligand-containing antigen binding molecule of claim 1 or 2, wherein the trimerization domain comprises an amino acid sequence having at least 95% identity to SEQ ID NO:2.

4. The trimeric costimulatory TNF family ligand-containing antigen binding molecule of claim 1 or 2, wherein the trimerization domain comprises the amino acid sequence of SEQ ID NO:2.

5. The trimeric costimulatory TNF family ligand-containing antigen binding molecule of claims 1 to 4, wherein the costimulatory TNF ligand family member is selected from 4-1BBL and OX40L.

6. The trimeric costimulatory TNF family ligand-containing antigen binding molecule of any one of claims 1 to 5, wherein the ectodomain of a TNF ligand family member comprises the amino acid sequence selected from the group consisting of SEQ ID NO:3, SEQ ID NO:4, SEQ ID NO:5, SEQ ID NO:6, SEQ ID NO:7, SEQ ID NO:8, SEQ ID NO:9 and SEQ ID NO: 10, particularly the amino acid sequence of SEQ ID NO:3 or SEQ ID NO:7.

7. The trimeric costimulatory TNF family ligand-containing antigen binding molecule of any one of claims 1 to 6, comprising three fusion polypeptides, each of the three fusion polypeptides comprising

(a) an ectodomain of a TNF ligand family comprising the amino acid sequence selected from the group consisting of SEQ ID NO:3, SEQ ID NO:4, SEQ ID NO:5, SEQ ID NO:6, SEQ ID NO:7, SEQ ID NO:8, SEQ ID NO:9 and SEQ ID NO: 10,
(b) a trimerization domain comprising the amino acid sequence of SEQ ID NO:2 and
(c) a moiety capable of specific binding to a target cell antigen.

8. The trimeric costimulatory TNF family ligand-containing antigen binding molecule of any one of claims 1 to 5, wherein the ectodomain of a TNF ligand family member comprises the amino acid sequence of SEQ ID NO: 11 or SEQ ID NO: 12.

9. The trimeric costimulatory TNF family ligand-containing antigen binding molecule of any one of claims 1 to 5 or 8, comprising three fusion polypeptides, wherein each of the three fusion polypeptides comprises

(a) an ectodomain of a TNF ligand family comprising the amino acid sequence of SEQ ID NO: 11 or SEQ ID NO: 12,
(b) a trimerization domain comprising the amino acid sequence of SEQ ID NO:2 and
(c) a moiety capable of specific binding to a target cell antigen.

10. The trimeric costimulatory TNF family ligand-containing antigen binding molecule of any one of claims 1 to 9, wherein the ectodomain of a TNF ligand family member or a fragment thereof is fused at the N-terminal amino acid to the C-terminal amino acid of the trimerization domain, optionally through a peptide linker.

11. The trimeric costimulatory TNF family ligand-containing antigen binding molecule of any one of claims 1 to 10, wherein the moiety capable of specific binding to a target cell antigen is fused at the C-terminal amino acid to the N-terminal amino acid of the trimerization domain, optionally through a peptide linker.

12. The trimeric costimulatory TNF family ligand-containing antigen binding molecule of any one of claims 1 to 11, wherein the moiety capable of specific binding to a target cell antigen is a Fab molecule capable of specific binding to a target cell antigen.

13. The trimeric costimulatory TNF family ligand-containing antigen binding molecule of claim 12, wherein said Fab molecule is fused at the C-terminal amino acid of the CH1 domain to the N-terminal amino acid of the trimerization domain, optionally through a peptide linker.

14. The trimeric costimulatory TNF family ligand-containing antigen binding molecule of any one of claims 1 to 13, wherein the target cell antigen is selected from the group consisting of Fibroblast Activation Protein (FAP), Melanoma-associated Chondroitin Sulfate Proteoglycan (MCSP), Epidermal Growth Factor Receptor (EGFR), Carcinoembryonic Antigen (CEA), CD 19, CD20 and CD33.

**15.** The trimeric costimulatory TNF family ligand-containing antigen binding molecule of any one of claims 1 to 14, wherein the target cell antigen is Fibroblast Activation Protein (FAP).

**16.** The trimeric costimulatory TNF family ligand-containing antigen binding molecule of any one of claims 1 to 18, wherein the moiety capable of specific binding to FAP comprises

(a) a VH domain comprising (i) CDR-H1 comprising the amino acid sequence of SEQ ID NO: 13, (ii) CDR-H2 comprising the amino acid sequence of SEQ ID NO:14 and (iii) CDR-H3 comprising the amino acid sequence of SEQ ID NO: 15, and a VL domain comprising (iv) CDR-L1 comprising the amino acid sequence of SEQ ID NO:16, (v) CDR-L2 comprising the amino acid sequence of SEQ ID NO: 17 and (vi) CDR-L3 comprising the amino acid sequence of SEQ ID NO:18 or
(b) a VH domain comprising (i) CDR-H1 comprising the amino acid sequence of SEQ ID NO:19, (ii) CDR-H2 comprising the amino acid sequence of SEQ ID NO:20 and (iii) CDR-H3 comprising the amino acid sequence of SEQ ID NO:21, and a VL domain comprising (iv) CDR-L1 comprising the amino acid sequence of SEQ ID NO:22, (v) CDR-L2 comprising the amino acid sequence of SEQ ID NO:23 and (vi) CDR-L3 comprising the amino acid sequence of SEQ ID NO:24.

**17.** A fusion polypeptide comprising (a) an ectodomain of a costimulatory TNF ligand family member or a fragment thereof and (b) a trimerization domain derived from human cartilage matrix protein (huCMP) comprising the amino acid sequence of SEQ ID NO:2, wherein said trimerization domain is capable of mediating stable association of said fusion polypeptide with two further such fusion polypeptides.

**18.** The fusion polypeptide of claim 17, wherein the fusion polypeptide further comprises (c) a moiety capable of specific binding to a target cell antigen.

**19.** The fusion polypeptide of claim 17 or 18, wherein the fusion polypeptide comprises

(a) an ectodomain of a TNF ligand family comprising the amino acid sequence selected from the group consisting of SEQ ID NO:3, SEQ ID NO:4, SEQ ID NO:5, SEQ ID NO:6, SEQ ID NO:7, SEQ ID NO:8, SEQ ID NO:9 and SEQ ID NO: 10,
(b) a trimerization domain comprising the amino acid sequence of SEQ ID NO:2 and
(c) a VH domain comprising (i) CDR-H1 comprising the amino acid sequence of SEQ ID NO: 13, (ii) CDR-H2 comprising the amino acid sequence of SEQ ID NO:14 and (iii) CDR-H3 comprising the amino acid sequence of SEQ ID NO: 15 or a VH domain comprising (i) CDR-H1 comprising the amino acid sequence of SEQ ID NO:19, (ii) CDR-H2 comprising the amino acid sequence of SEQ ID NO:20and (iii) CDR-H3 comprising the amino acid sequence of SEQ ID NO:21.

Fig. 1

Fig. 1A

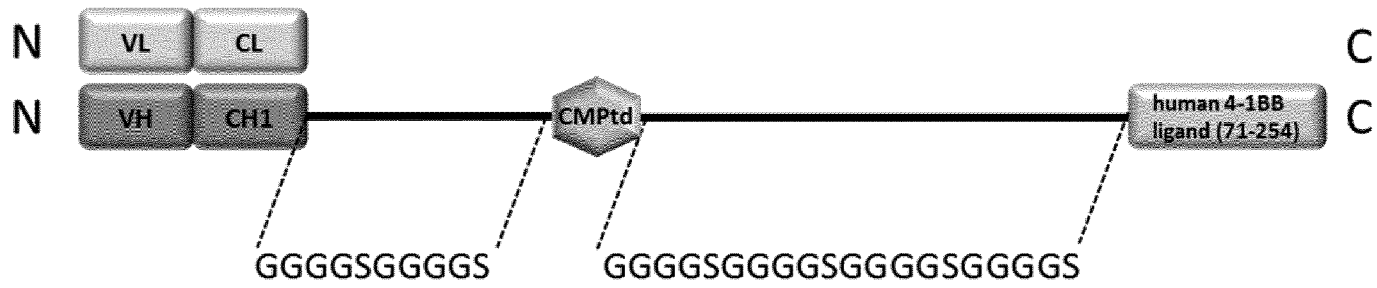


Fig. 1B

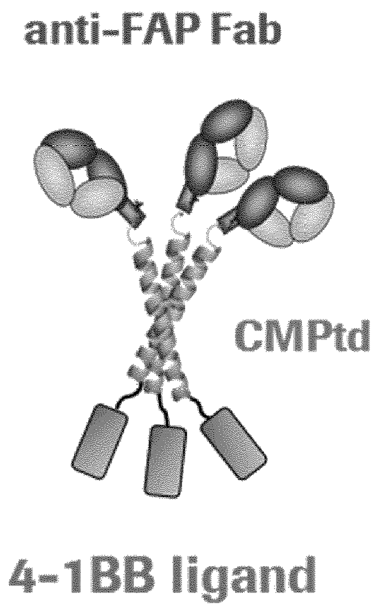


Fig. 1C

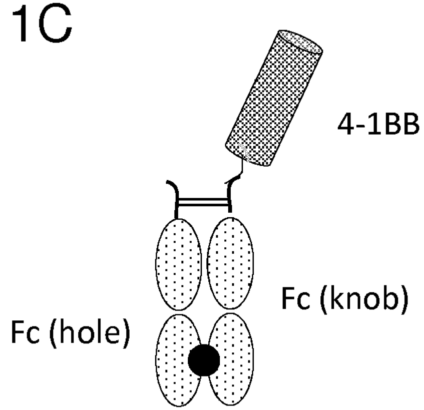

Fig. 2

Fig. 2A

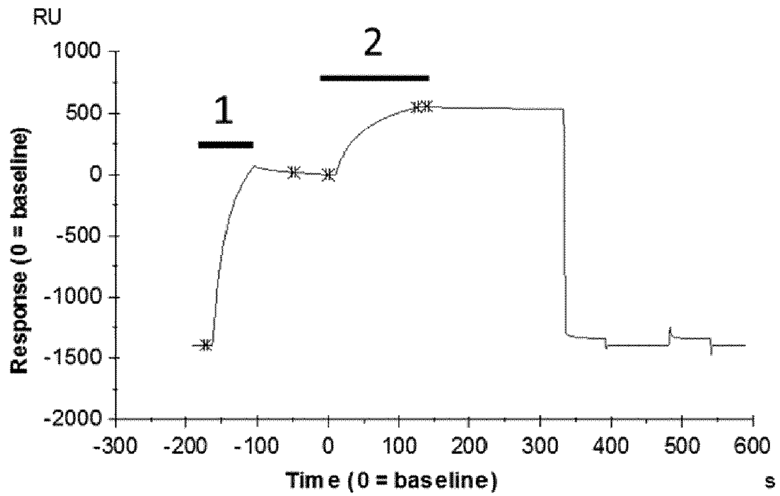


Fig. 2B

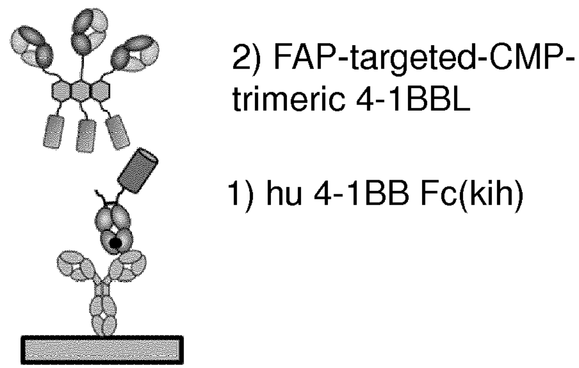

Fig. 2

Fig. 2C

Fig. 2D

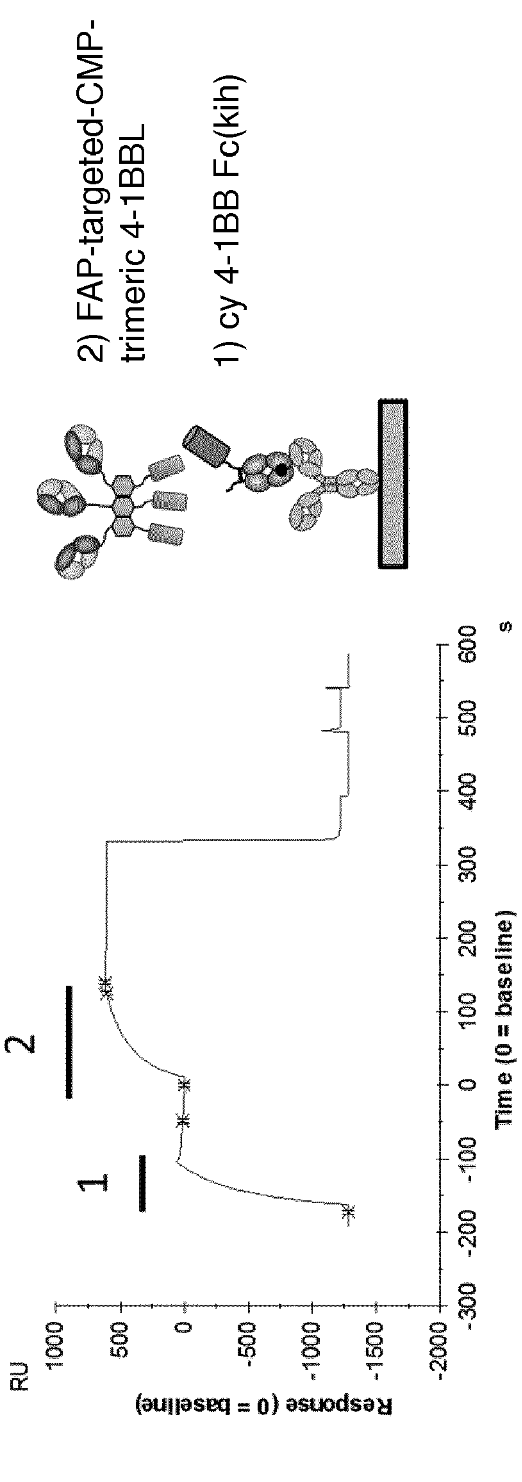

Fig. 2

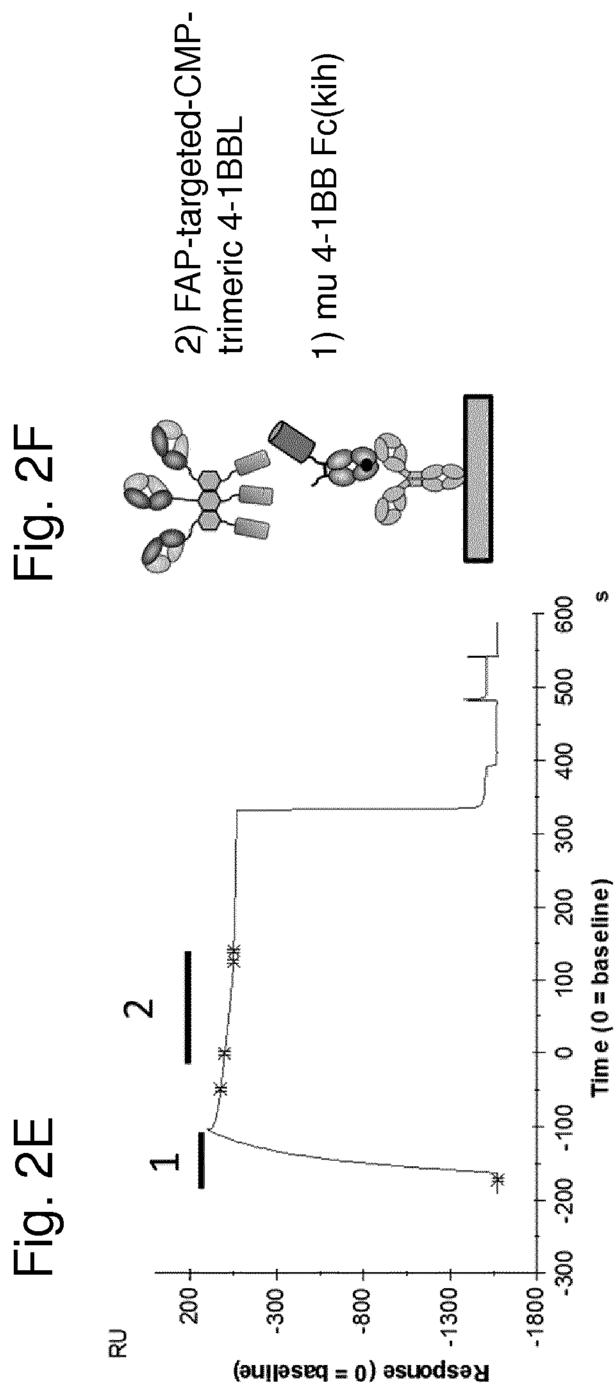
Fig. 2E
1
2
RU
Response (0 = baseline)
200
-300
-800
-1300
-1800
-300
-200
-100
0
100
200
300
400
500
600
s
Time (0 = baseline)
Fig. 2F
2) FAP-targeted-CMP-trimeric 4-1BBL
1) mu 4-1BB Fc(kih)

Fig. 3A

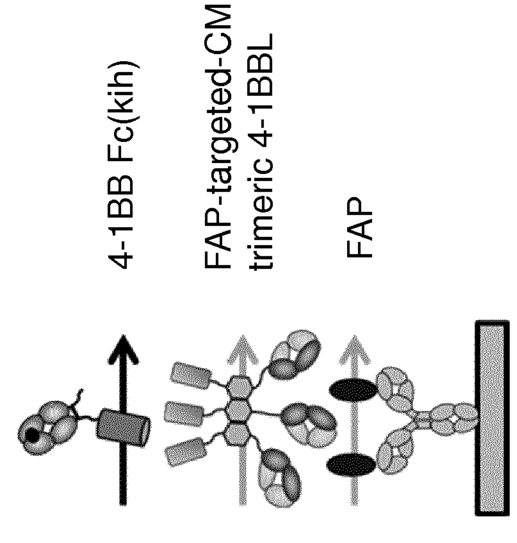


Fig. 3B

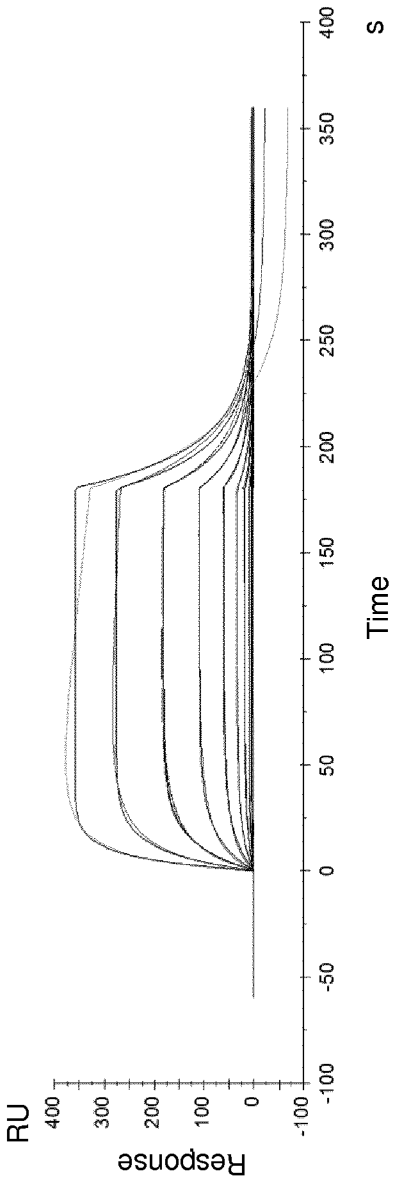


Fig. 3C

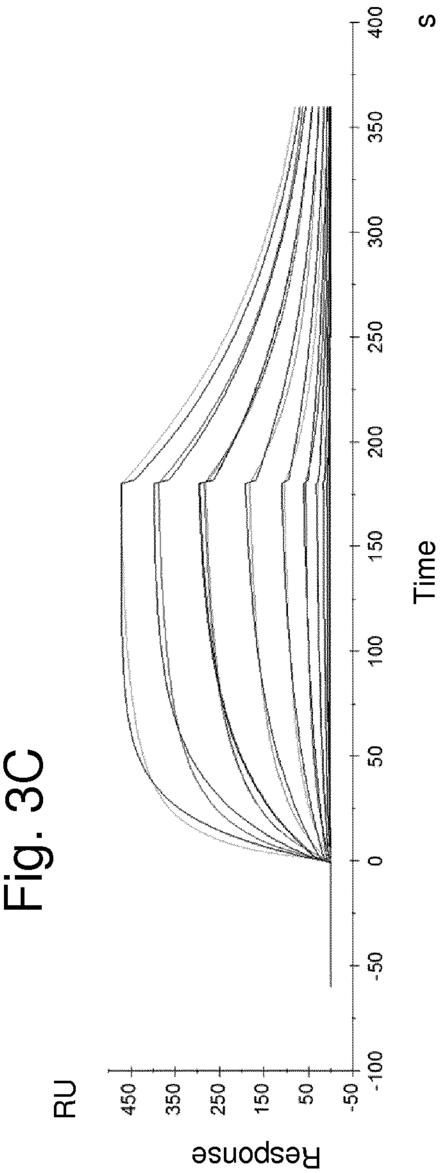

Fig. 4A

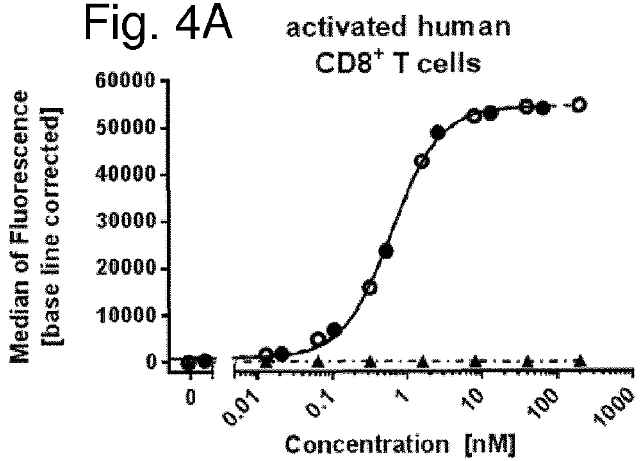


Fig. 4B

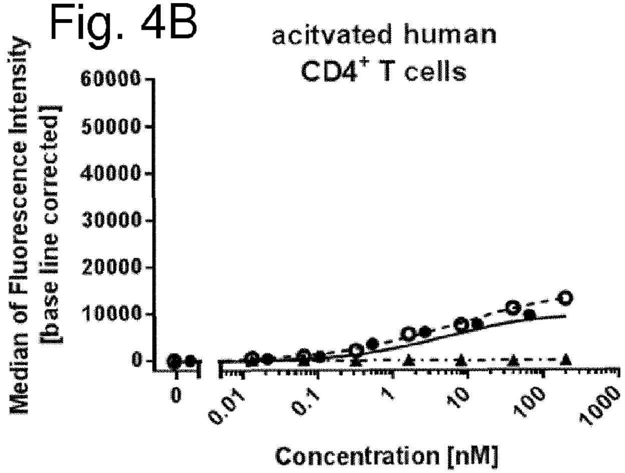


Fig. 4C

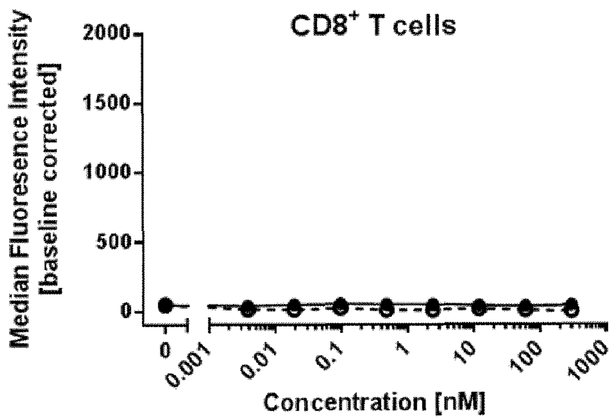


Fig. 4D

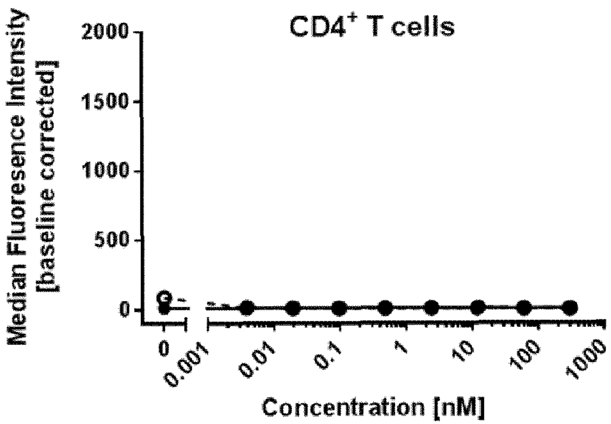


-●- Trimeric FAP-huCMP-4-1BBL -○- Trimeric DP47-huCMP-4-1BBL -▲- DP47 huIgG1 P329G LALA (control)

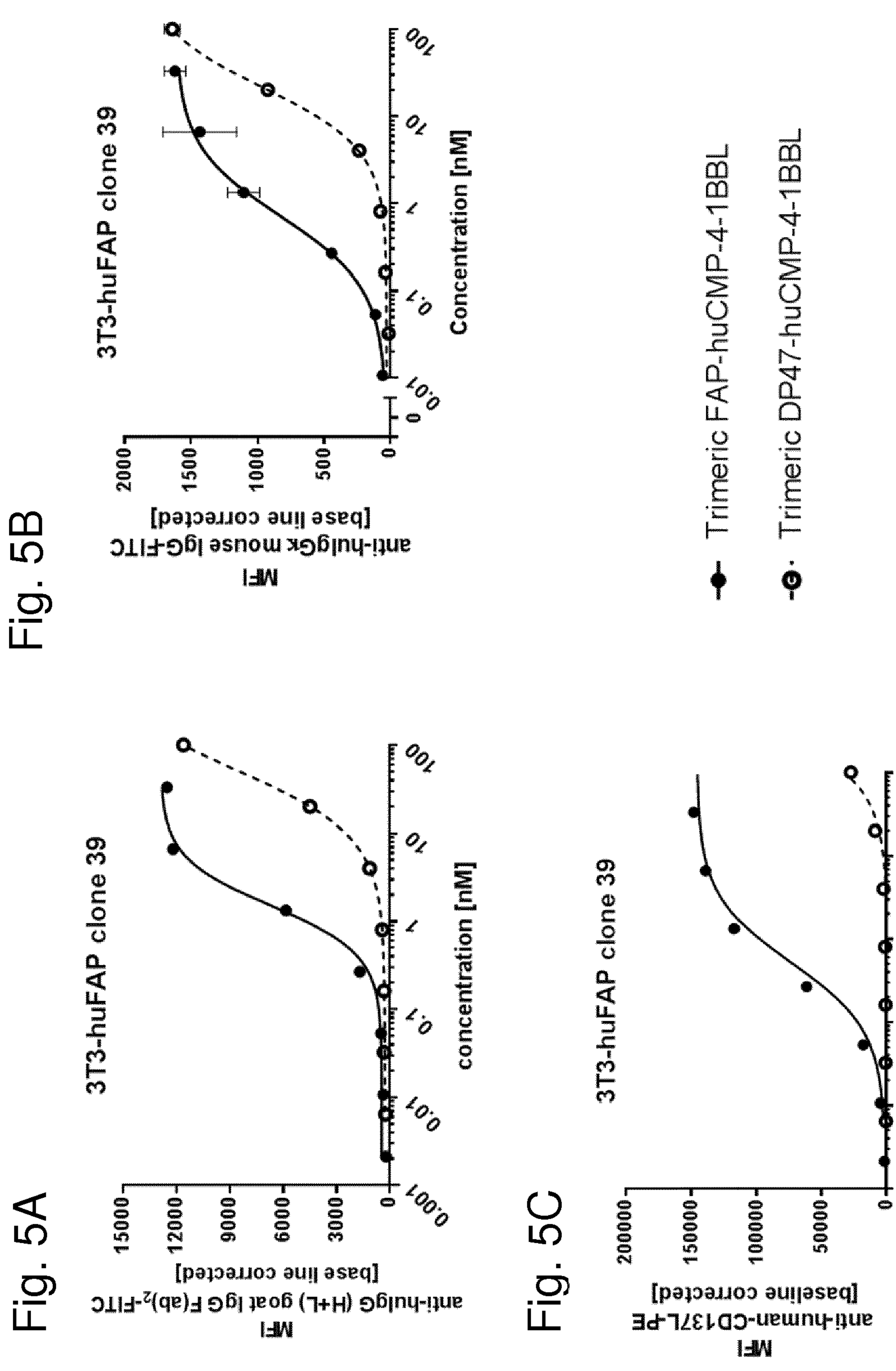


Fig. 5A

Fig. 5B

Fig. 5C

Fig. 6

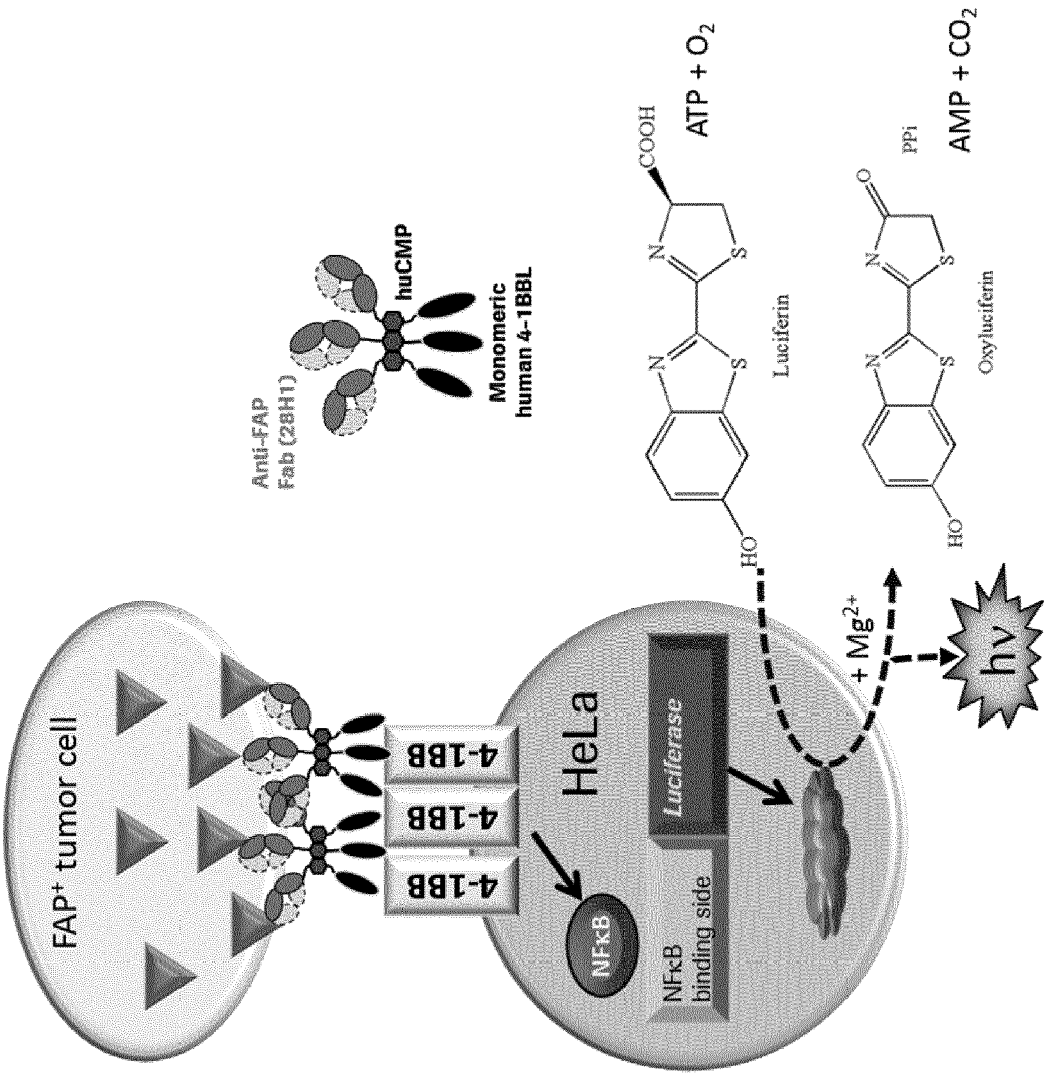
FAP+ tumor cell
4-1BB
4-1BB
4-1BB
HeLa
NFκB
NFκB binding side
Luciferase
+ Mg2+
hv
Anti-FAP Fab (28H1)
huCMP
Monomeric human 4-1BBL
Luciferin
ATP + O2
Oxyluciferin
PPi
AMP + CO2

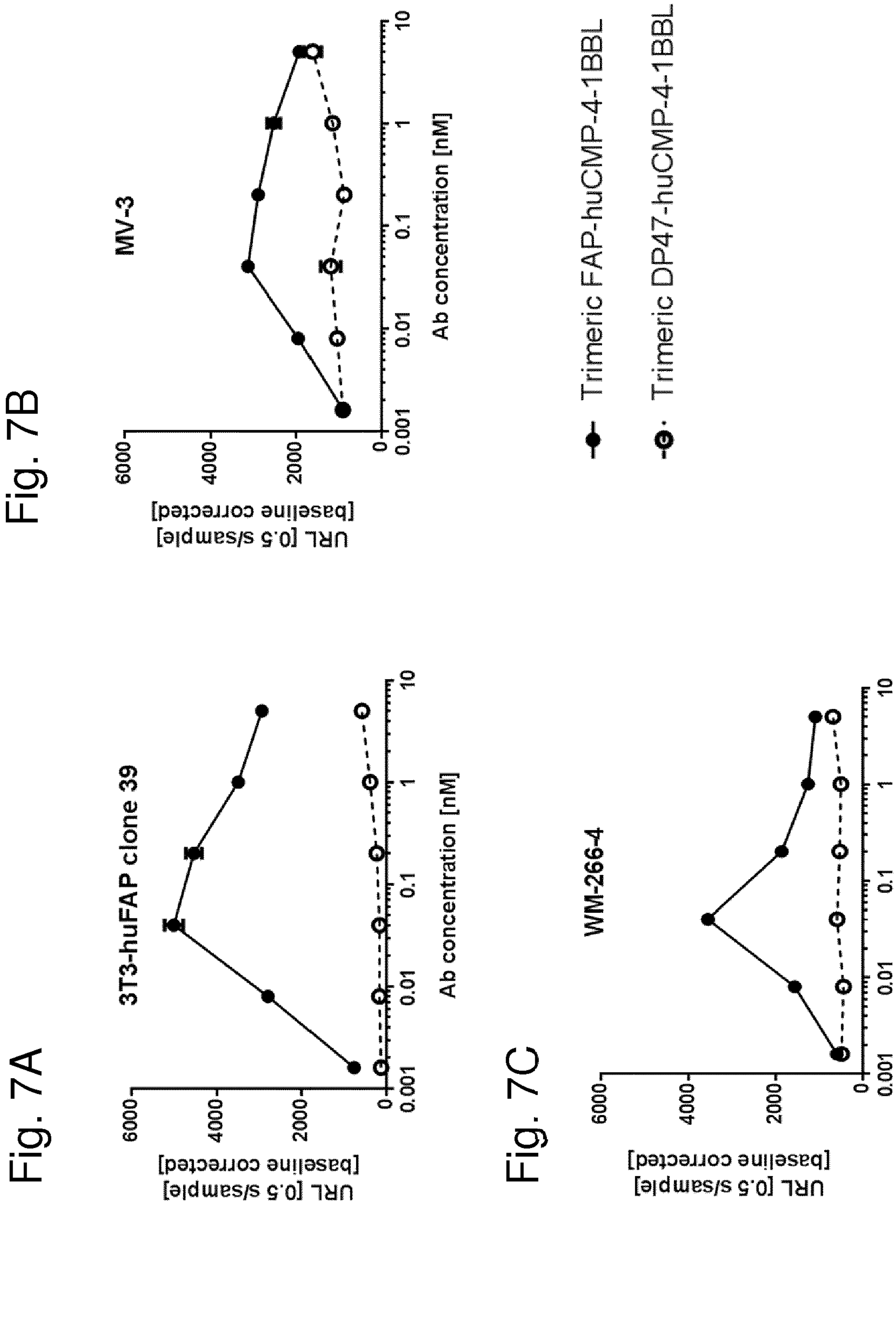


Fig. 7A

Fig. 7B

Fig. 7C

Fig. 8

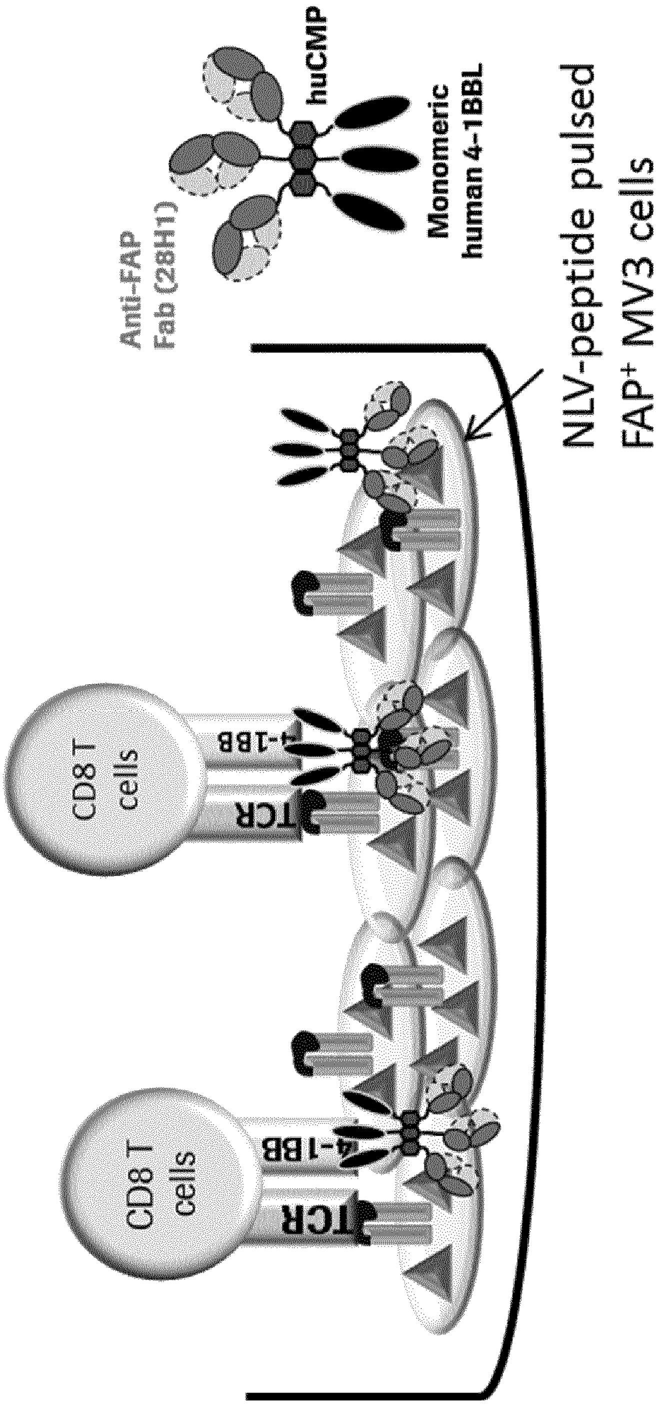
Anti-FAP
Fab (28H1)
huCMP
Monomeric
human 4-1BBL
NLV-peptide pulsed
FAP$^+$ MV3 cells
CD8 T
cells
TCR
CD8 T
cells
TCR

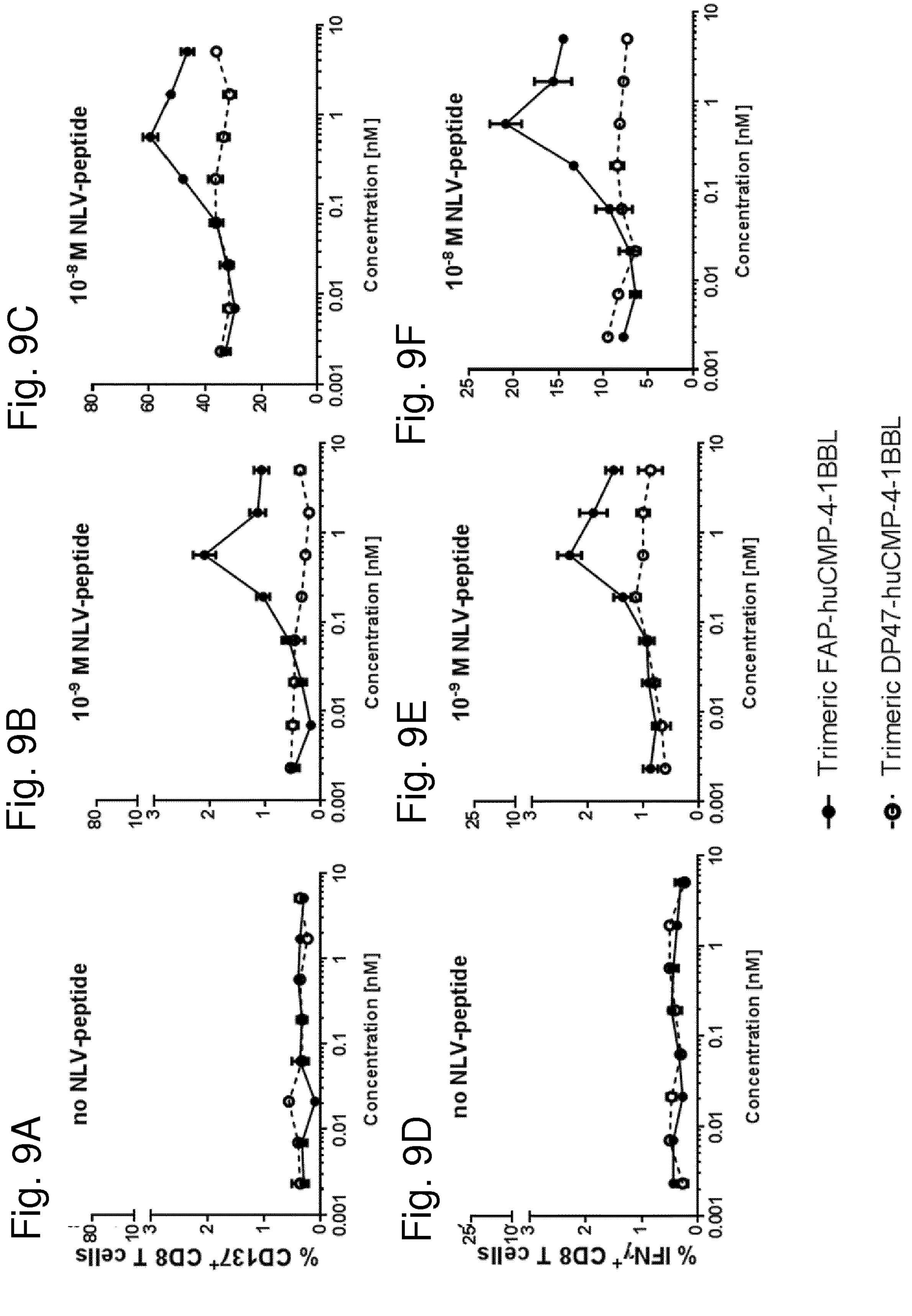

Fig. 9A
no NLV-peptide
Fig. 9B
10^-9 M NLV-peptide
Fig. 9C
10^-8 M NLV-peptide
Fig. 9D
no NLV-peptide
Fig. 9E
10^-9 M NLV-peptide
Fig. 9F
10^-8 M NLV-peptide
% CD137+ CD8 T cells
% IFNγ+ CD8 T cells
Concentration [nM]
Trimeric FAP-huCMP-4-1BBL
Trimeric DP47-huCMP-4-1BBL

Fig. 10

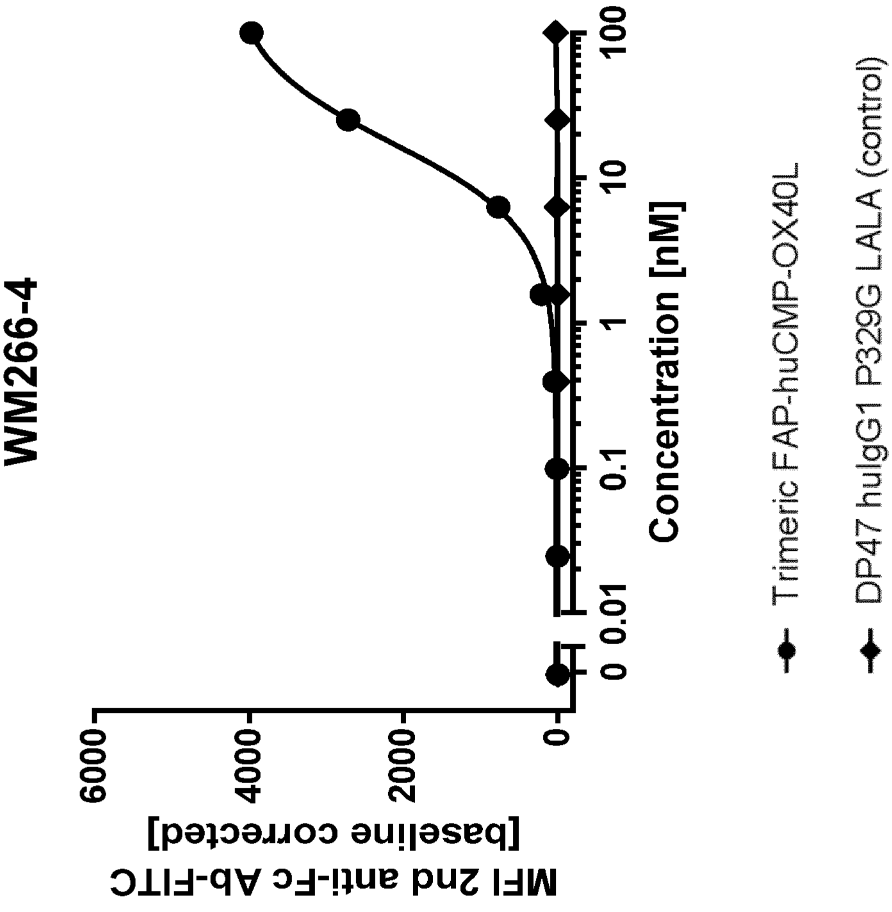
WM266-4
MFI 2nd anti-Fc Ab-FITC [baseline corrected]
6000
4000
2000
0
0
0.01
0.1
1
10
100
Concentration [nM]
Trimeric FAP-huCMP-OX40L
DP47 huIgG1 P329G LALA (control)

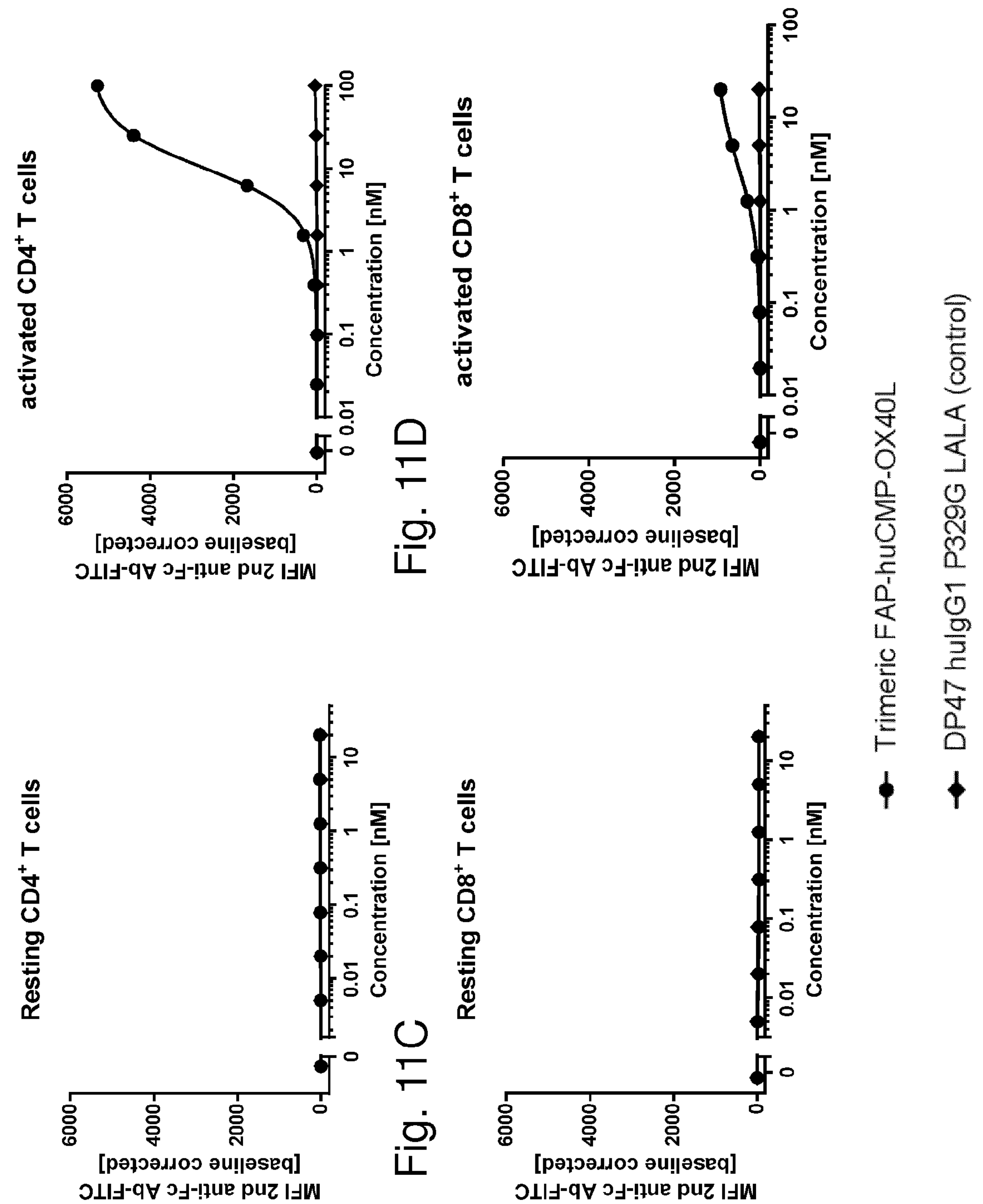


Fig. 11A Fig. 11B Fig. 11C Fig. 11D

Fig. 12A

Fig. 12B

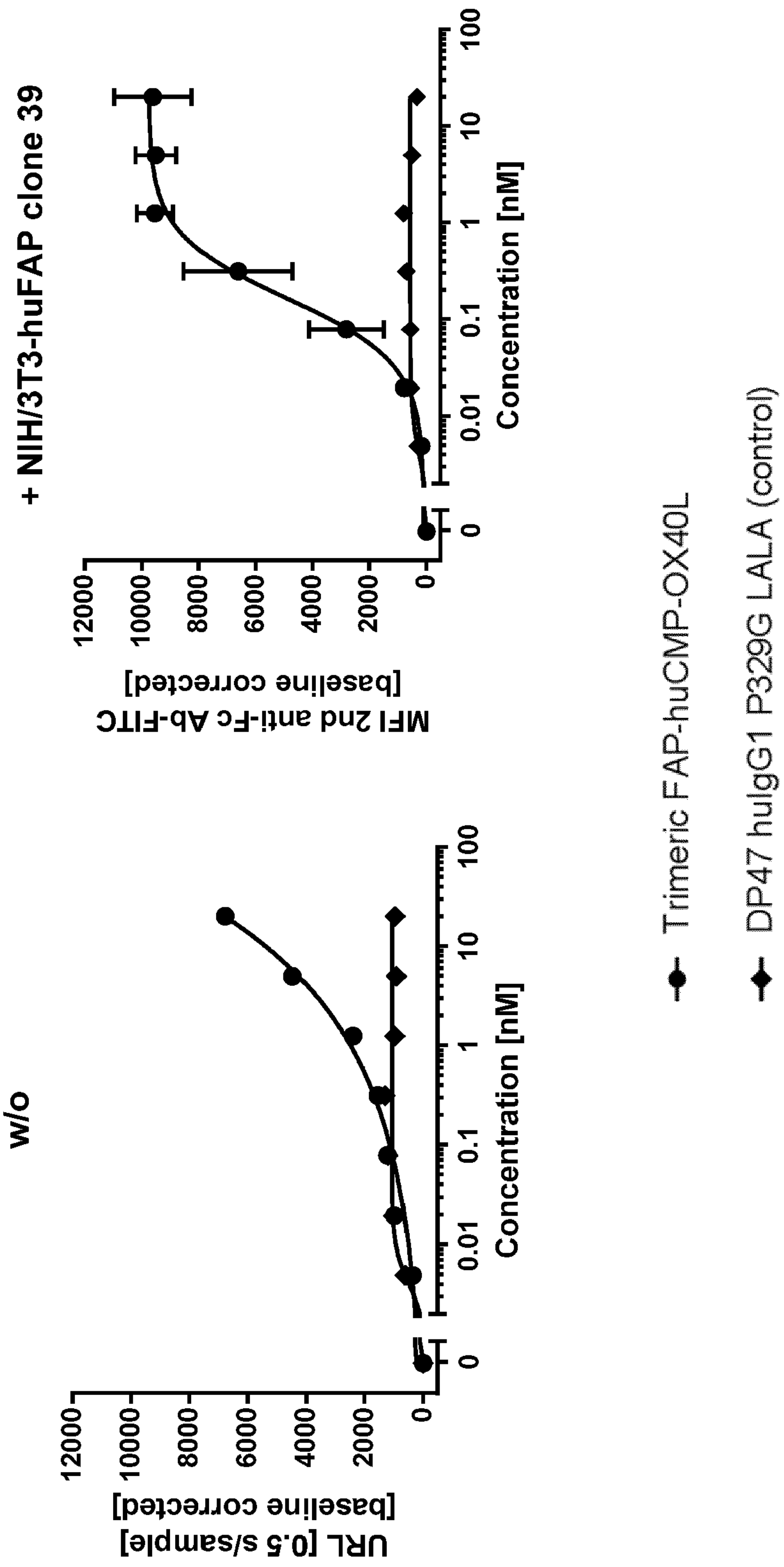

Europäisches Patentamt
European Patent Office
Office européen des brevets

# EUROPEAN SEARCH REPORT

Application Number
EP 16 16 2539

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br><br><br>Y | WO 2007/014744 A2 (UNIV STUTTGART [DE]; UNIV WUERZBURG J MAXIMILIANS [DE]; PFIZENMAIER KL)<br>8 February 2007 (2007-02-08)<br>* page 7, last paragraph - page 8, paragraph 1; claim 1 * | 1-4,12, 14,15,18<br><br><br>5-11,13, 16,17,19 | INV.<br>C07K14/705<br>A61K47/48<br>C07K16/28<br>C07K16/30<br>C12N15/62 |
| X | WO 2004/069876 A2 (MICROMET AG [DE]; KISCHEL ROMAN [DE]; KUFER PETER [DE]; LUTTERBUESE RA)<br>19 August 2004 (2004-08-19)<br>* claims 1,6,11 * | 1-19 | |
| X<br><br>Y | AU 2013 263 717 B2 (PROVIDENCE HEALTH SYSTEM) 19 December 2013 (2013-12-19)<br><br>* claim 1 * | 1-5, 8-11,17, 18<br>6,7, 12-16,19 | |
| Y,D | WO 2009/000538 A1 (APOGENIX GMBH [DE]; PFIZENMAIER KLAUS [DE]; WAJANT HARALD [DE]; HILL O)<br>31 December 2008 (2008-12-31)<br>* claim 1 and 22 * | 1-19 | TECHNICAL FIELDS SEARCHED (IPC)<br>C07K<br>A61K<br>C12N |
| X | WO 2011/109789 A2 (UNIV JOHNS HOPKINS [US]; BEDI ATUL [US]; RAVI RAJANI [US])<br>9 September 2011 (2011-09-09)<br>* claim 1 * | 1-19 | |
| Y,D | WO 2015/183902 A1 (MEDIMMUNE LLC [US])<br>3 December 2015 (2015-12-03)<br>* claim 1 * | 1-19 | |

The present search report has been drawn up for all claims

1

| Place of search | Date of completion of the search | Examiner |
|---|---|---|
| Munich | 7 June 2016 | Griesinger, Irina |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO. EP 16 16 2539

This annex lists the patent family members relating to the patent documents cited in the above-mentioned European search report. The members are as contained in the European Patent Office EDP file on The European Patent Office is in no way liable for these particulars which are merely given for the purpose of information.

07-06-2016

| Patent document cited in search report | Publication date | Patent family member(s) | Publication date |
| --- | --- | --- | --- |
| WO 2007014744 A2 | 08-02-2007 | DE 102005036542 A1 | 08-02-2007 |
| | | WO 2007014744 A2 | 08-02-2007 |
| WO 2004069876 A2 | 19-08-2004 | AU 2004210088 A1 | 19-08-2004 |
| | | BR PI0407293 A | 07-02-2006 |
| | | CA 2515100 A1 | 19-08-2004 |
| | | CN 1756768 A | 05-04-2006 |
| | | EP 1590372 A2 | 02-11-2005 |
| | | JP 2007523602 A | 23-08-2007 |
| | | KR 20050108349 A | 16-11-2005 |
| | | US 2006235201 A1 | 19-10-2006 |
| | | WO 2004069876 A2 | 19-08-2004 |
| | | ZA 200506282 A | 26-04-2006 |
| AU 2013263717 B2 | 19-12-2013 | NONE | |
| WO 2009000538 A1 | 31-12-2008 | EP 2009022 A1 | 31-12-2008 |
| | | WO 2009000538 A1 | 31-12-2008 |
| WO 2011109789 A2 | 09-09-2011 | CA 2791383 A1 | 09-09-2011 |
| | | EP 2542590 A2 | 09-01-2013 |
| | | JP 2013521311 A | 10-06-2013 |
| | | US 2013039911 A1 | 14-02-2013 |
| | | US 2015183881 A1 | 02-07-2015 |
| | | WO 2011109789 A2 | 09-09-2011 |
| WO 2015183902 A1 | 03-12-2015 | GB 2532817 A | 01-06-2016 |
| | | US 2016024176 A1 | 28-01-2016 |
| | | WO 2015183902 A1 | 03-12-2015 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0149866 A
- WO 2009000538 A
- WO 2006121810 A
- WO 2015183902 A
- WO 2002020565 A
- WO 9316185 A
- US 5571894 A
- US 5587458 A
- US 5869046 A
- EP 404097 A
- WO 199301161 A
- US 6248516 B1
- US 7166697 B1
- US 7250297 B1
- US 20070224633 A
- EP 1641818 A1
- US 20040132028 A1
- US 20080139791 A
- WO 2005056764 A
- US 6818418 B1
- WO 2008098796 A
- WO 2012020006 A2
- US 5821333 A
- US 5959177 A
- US 6040498 A
- US 6420548 B
- US 7125978 B
- US 6417429 B
- US 4186567 A
- US 5969108 A, McCafferty
- US 5565332 A
- US 5821337 A
- US 7527791 B
- US 6982321 B
- US 7087409 B
- WO 2012020006 A
- US 20040132066 A
- US 20050260186 A
- US 20060104968 A
- US 6267958 B
- US 6171586 B
- WO 2006044908 A
- WO 2012130831 A


### Non-patent literature cited in the description


- **HUDSON et al.** *Nat Med,* 2003, vol. 9, 129-134
- **PLÜCKTHUN.** The Pharmacology of Monoclonal Antibodies. Springer-Verlag, 1994, vol. 113, 269-315
- **HOLLINGER et al.** *Proc Natl Acad Sci USA,* 1993, vol. 90, 6444-6448
- **HOUSTON, J.S.** *Methods in Enzymol.,* 1991, vol. 203, 46-96
- *Curr Opin Chem Biol,* 2009, vol. 13, 245-255
- **STUMPP et al.** Darpins: A new generation of protein therapeutics. *Drug Discovery Today,* 2008, vol. 13, 695-701
- *Journal of Immunological Methods,* 2001, vol. 248 (1-2), 31-45
- *Biochim Biophys Acta,* 2000, vol. 1482, 337-350
- *Protein Eng. Des. Sel.,* 2004, vol. 17, 455-462
- *Nature Biotechnology,* 2005, vol. 23 (12), 1556-1561
- *Expert Opinion on Investigational Drugs,* June 2007, vol. 16 (6), 909-917
- *J. Biol. Chem,* 1999, vol. 274, 24066-24073
- *J. Mol. Biol.,* 2003, vol. 332, 489-503
- *PNAS,* 2003, vol. 100 (4), 1700-1705
- *J. Mol. Biol.,* 2007, vol. 369, 1015-1028
- *Protein Eng. Des. Sel.,* 2005, vol. 18, 435-444
- *Expert Opin. Biol. Ther.,* 2005, vol. 5, 783-797
- **LILJEBLAD et al.** *Glyco J,* 2000, vol. 17, 323-329
- **HEELEY.** *Endocr Res,* 2002, vol. 28, 217-229
- **GOLD ; FREEDMAN.** *J Exp Med.,* 1965, vol. 121, 439-462
- **BERINSTEIN N. L.** *J Clin Oncol.,* 2002, vol. 20, 2197-2207
- **NAP et al.** *Tumour Biol.,* 1988, vol. 9 (2-3), 145-53
- **NAP et al.** *Cancer Res.,* 1992, vol. 52 (8), 2329-23339
- **HAMMARSTRÖM S.** *Semin Cancer Biol,* 1999, vol. 9 (2), 67-81
- **MARSHALL J.** *Semin Oncol.,* 2003, vol. 30 (8), 30-6
- **GOLDENBERG D M.** *The International Journal of Biological Markers,* 1992, vol. 7, 183-188

- **CHAU I. et al.** *J Clin Oncol.,* 2004, vol. 22, 1420-1429
- **FLAMINI et al.** *Clin Cancer Res,* 2006, vol. 12 (23), 6985-6988
- **KINDT et al.** Kuby Immunology. W.H. Freeman and Co, 2007, 91
- **CHOTHIA ; LESK.** *J. Mol. Biol.,* 1987, vol. 196, 901-917
- **KABAT et al.** Sequences of Proteins of Immunological Interest. National Institutes of Health, 1991
- **KABAT et al.** Sequences of Proteins of Immunological Interest. U.S. Dept. of Health and Human Services, 1983
- **CHOTHIA et al.** *J. Mol. Biol.,* 1987, vol. 196, 901-917
- **KABAT et al.** Sequence of Proteins of Immunological Interest. U.S. Dept. of Health and Human Services, 1983
- **ALMAGRO ; FRANSSON.** *Front. Biosci.,* 2008, vol. 13, 1619-1633
- **BECK et al.** *J. Mol. Biol.,* 1996, vol. 256, 909-923
- **CUNNINGHAM ; WELLS.** *Science,* 1989, vol. 244, 1081-1085
- **MANIATIS et al.** MOLECULAR CLONING: A LABORATORY MANUAL. Cold Spring Harbor Laboratory, 1989
- **AUSUBEL et al.** CURRENT PROTOCOLS IN MOLECULAR BIOLOGY. Greene Publishing Associates and Wiley Interscience, 1989
- **GERNGROSS.** *Nat Biotech,* 2004, vol. 22, 1409-1414
- **LI et al.** *Nat Biotech,* 2006, vol. 24, 210-215
- **GRAHAM et al.** *J Gen Virol,* 1977, vol. 36, 59
- **MATHER.** *Biol Reprod,* 1980, vol. 23, 243-251
- **MATHER et al.** Annals N.Y. Acad Sci. 1982, vol. 383, 44-68
- **URLAUB et al.** *Proc Natl Acad Sci USA,* 1980, vol. 77, 4216
- **YAZAKI ; WU.** Methods in Molecular Biology. Humana Press, 2003, vol. 248, 255-268
- **HARLOW ; LANE.** Antibodies, a laboratory manual. Cold Spring Harbor Laboratory, 1988
- **ALMAGRO ; FRANSSON.** *Front Biosci,* 2008, vol. 13, 1619-1633
- **RIECHMANN et al.** *Nature,* 1988, vol. 332, 323-329
- **QUEEN et al.** *Proc Natl Acad Sci USA,* 1989, vol. 86, 10029-10033
- **JONES et al.** *Nature,* 1986, vol. 321, 522-525
- **MORRISON et al.** *Proc Natl Acad Sci,* 1984, vol. 81, 6851-6855
- **MORRISON ; OI.** *Adv Immunol,* 1988, vol. 44, 65-92
- **VERHOEYEN et al.** *Science,* 1988, vol. 239, 1534-1536
- **PADLAN.** *Molec Immun,* 1994, vol. 31 (3), 169-217
- **KASHMIRI et al.** *Methods,* 2005, vol. 36, 25-34
- **PADLAN.** *Mol Immunol,* 1991, vol. 28, 489-498
- **DALL'ACQUA et al.** *Methods,* 2005, vol. 36, 43-60
- **OSBOURN et al.** *Methods,* 2005, vol. 36, 61-68
- **KLIMKA et al.** *Br J Cancer,* 2000, vol. 83, 252-260
- **VAN DIJK ; VAN DE WINKEL.** *Curr Opin Pharmacol,* 2001, vol. 5, 368-74
- **LONBERG.** *Curr Opin Immunol,* 2008, vol. 20, 450-459
- Monoclonal Antibody Production Techniques and Applications. Marcel Dekker, Inc, 1987, 51-63
- **LONBERG.** *Nat Biotech,* 2005, vol. 23, 1117-1125
- **HOOGENBOOM et al.** Methods in Molecular Biology. Human Press, 2001, vol. 178, 1-37
- **MCCAFFERTY et al.** *Nature,* vol. 348, 552-554
- **CLACKSON et al.** *Nature,* 1991, vol. 352, 624-628
- Epitope Mapping Protocols. **MORRIS.** Methods in Molecular Biology. Humana Press, 1996, vol. 66
- **HARLOW ; LANE.** Antibodies: A Laboratory Manual. Cold Spring Harbor Laboratory, 1988
- Remington's Pharmaceutical Sciences. Mack Printing Company, 1990
- **FINGL et al.** The Pharmacological Basis of Therapeutics. 1975, 1
- **SAMBROOK et al.** Molecular cloning: A laboratory manual. Cold Spring Harbor Laboratory Press, 1989
- **KABAT, E.A. et al.** Sequences of Proteins of Immunological Interest. NIH Publication No 91-3242, 1991
- **VAN MUIJEN GN ; JANSEN KF ; CORNELISSEN IM ; SMEETS DF ; BECK JL ; RUITER DJ.** Establishment and characterization of a human melanoma cell line (MV3) which is highly metastatic in nude mice. *Int J Cancer,* 22 April 1991, vol. 48 (1), 85-91
- **A. D. WEINBERG et al.** *J. Leukoc. Biol,* 2004, vol. 75 (6), 962-972
- **AGGARWAL B.B.** Signalling pathways of the TNF superfamily: a double-edged sword. *Nat. Rev. Immunol.,* 2003, vol. 3 (9), 745-56
- **BANNER D. et al.** Crystal structure of the soluble human 55 kd TNF receptor-human TNF beta complex: implications for TNF receptor activation. *Cell,* 1993, vol. 73, 431-445

- **BECK K. ; GAMBEE J. ; BOHAN C. ; BÄCHINGER H.P.** The C-terminal domain of cartilage matrix protein assembles into a triple-stranded α-helical coiled-coil structure. *J. Mol. Biol.,* 1996, vol. 256, 909-923
- **BODMER J. ; SCHNEIDER P. ; TSCHOPP, J.** The molecular architecture of the TNF superfamily. *Trends in Biochemical Sciences,* 2002, vol. 27 (1), 19-26
- **BROLL, K. ; RICHTER, G. ; PAULY, S. ; HOFSTAEDTER, F. ; SCHWARZ, H.** CD137 expression in tumor vessel walls. High correlation with malignant tumors. *Am J Clin Pathol,* 2001, vol. 115, 543-549
- **BUECHELE, C. ; BAESSLER, T. ; SCHMIEDEL, B.J. ; SCHUMACHER, C.E. ; GROSSE-HOVEST, L. ; RITTIG, K. ; SALIH, H.R.** 4-1BB ligand modulates direct and Rituximab-induced NK-cell reactivity in chronic lymphocytic leukemia. *Eur J Immunol,* 2012, vol. 42, 737-748
- **CHOI, B.K. ; KIM, Y.H. ; KWON, P.M. ; LEE, S.C. ; KANG, S.W. ; KIM, M.S. ; LEE, M.J. ; KWON, B.S.** 4-1BB functions as a survival factor in dendritic cells. *J Immunol,* 2009, vol. 182, 4107-4115
- **CUADROS, C. ; DOMINGUEZ, A.L. ; LOLLINI, P.L. ; CROFT, M. ; MITTLER, R.S. ; BORGSTROM, P. ; LUSTGARTEN, J.** Vaccination with dendritic cells pulsed with apoptotic tumors in combination with anti-OX40 and anti-4-1BB monoclonal antibodies induces T cell-mediated protective immunity in Her-2/neu transgenic mice. *Int J Cancer,* 2005, vol. 116, 934-943
- **CURRAN, M.A. ; KIM, M. ; MONTALVO, W. ; AL-SHAMKHANI, A. ; ALLISON, J.P.** Combination CTLA-4 blockade and 4-1BB activation enhances tumor rejection by increasing T-cell infiltration, proliferation, and cytokine production. *PLoS One,* 2011, vol. 6, e19499
- **DIEHL, L. ; VAN MIERLO, G.J. ; DEN BOER, A.T. ; VAN DER VOORT, E. ; FRANSEN, M. ; VAN BOSTELEN, L. ; KRIMPENFORT, P. ; MELIEF, C.J. ; MITTLER, R. ; TOES, R.E.** In vivo triggering through 4-1BB enables Th-independent priming of CTL in the presence of an intact CD28 costimulatory pathway. *J Immunol,* 2002, vol. 168, 3755-3762
- **DUBROT, J. ; MILHEIRO, F. ; ALFARO, C. ; PALAZON, A. ; MARTINEZ-FORERO, I. ; PEREZ-GRACIA, J.L. ; MORALES-KASTRESANA, A. ; ROMERO-TREVEJO, J.L. ; OCHOA, M.C. ; HERVAS-STUBBS, S. et al.** Treatment with anti-CD 137 mAbs causes intense accumulations of liver T cells without selective antitumor immunotherapeutic effects in this organ. *Cancer Immunol Immunother,* 2010, vol. 59, 1223-1233
- **FUTAGAWA, T. ; AKIBA, H. ; KODAMA, T. ; TAKEDA, K. ; HOSODA, Y. ; YAGITA, H. ; OKUMURA, K.** Expression and function of 4-1BB and 4-1BB ligand on murine dendritic cells. *Int Immunol,* 2002, vol. 14, 275-286
- **GUO, Z. ; CHENG, D. ; XIA, Z. ; LUAN, M. ; WU, L. ; WANG, G. ; ZHANG, S.** Combined TIM-3 blockade and CD 137 activation affords the long-term protection in a murine model of ovarian cancer. *J Transl Med,* 2013, vol. 11, 215
- **HEINISCH, I.V. ; DAIGLE, I. ; KNOPFLI, B. ; SIMON, H.U.** CD137 activation abrogates granulocyte-macrophage colony-stimulating factor-mediated anti-apoptosis in neutrophils. *Eur J Immunol,* 2000, vol. 30, 3441-3446
- **JU, S.A. ; CHEON, S.H. ; PARK, S.M. ; TAM, N.Q. ; KIM, Y.M. ; AN, W.G. ; KIM, B.S.** Eradication of established renal cell carcinoma by a combination of 5-fluorouracil and anti-4-1BB monoclonal antibody in mice. *Int J Cancer,* 2008, vol. 122, 2784-2790
- **KIENZLE, G. ; VON KEMPIS, J.** CD137 (ILA/4-1BB), expressed by primary human monocytes, induces monocyte activation and apoptosis of B lymphocytes. *Int Immunol,* 2000, vol. 12, 73-82
- **KIM, D.H. ; CHANG, W.S. ; LEE, Y.S. ; LEE, K.A. ; KIM, Y.K. ; KWON, B.S. ; KANG, C.Y.** 4-1BB engagement costimulates NKT cell activation and exacerbates NKT cell ligand-induced airway hyperresponsiveness and inflammation. *J Immunol,* 2008, vol. 180, 2062-2068
- **KIM, Y.H. ; CHOI, B.K. ; OH, H.S. ; KANG, W.J. ; MITTLER, R.S. ; KWON, B.S.** Mechanisms involved in synergistic anticancer effects of anti-4-1BB and cyclophosphamide therapy. *Mol Cancer Ther,* 2009, vol. 8, 469-478
- **KWON, B.S. ; WEISSMAN, S.M.** cDNA sequences of two inducible T-cell genes. *Proc Natl Acad Sci U S A,* 1989, vol. 86, 1963-1967
- **LEE, H. ; PARK, H.J. ; SOHN, H.J. ; KIM, J.M. ; KIM, S.J.** Combinatorial therapy for liver metastatic colon cancer: dendritic cell vaccine and low-dose agonistic anti-4-1BB antibody costimulatory signal. *J Surg Res,* 2011, vol. 169, e43-50
- **LI, F. ; RAVETCH, J.V.** Inhibitory Fcgamma receptor engagement drives adjuvant and anti-tumor activities of agonistic CD40 antibodies. *Science,* 2011, vol. 333, 1030-1034
- **LIN, W. ; VOSKENS, C.J. ; ZHANG, X. ; SCHINDLER, D.G. ; WOOD, A. ; BURCH, E. ; WEI, Y. ; CHEN, L. ; TIAN, G. ; TAMADA, K. et al.** Fc-dependent expression of CD137 on human NK cells: insights into ''agonistic'' effects of anti-CD 137 monoclonal antibodies. *Blood,* 2008, vol. 112, 699-707

- **MELERO, I. ; JOHNSTON, J.V. ; SHUFFORD, W.W. ; MITTLER, R.S. ; CHEN, L.** NK1.1 cells express 4-1BB (CDw137) costimulatory molecule and are required for tumor immunity elicited by anti-4-1BB monoclonal antibodies. *Cell Immunol,* 1998, vol. 190, 167-172
- **MELERO, I. ; SHUFORD, W.W. ; NEWBY, S.A. ; ARUFFO, A. ; LEDBETTER, J.A. ; HELLSTROM, K.E. ; MITTLER, R.S. ; CHEN, L.** Monoclonal antibodies against the 4-1BB T-cell activation molecule eradicate established tumors. *Nat Med,* 1997, vol. 3, 682-685
- **MERCHANT, A.M. ; ZHU, Z. ; YUAN, J.Q. ; GODDARD, A. ; ADAMS, C.W. ; PRESTA, L.G. ; CARTER, P.** An efficient route to human bispecific IgG. *Nat Biotechnol,* 1998, vol. 16, 677-681
- **MORALES-KASTRESANA, A. ; SANMAMED, M.F. ; RODRIGUEZ, I. ; PALAZON, A. ; MARTINEZ-FORERO, I. ; LABIANO, S. ; HERVAS-STUBBS, S. ; SANGRO, B. ; OCHOA, C. ; ROUZAUT, A. et al.** Combined immunostimulatory monoclonal antibodies extend survival in an aggressive transgenic hepatocellular carcinoma mouse model. *Clin Cancer Res,* 2013, vol. 19, 6151-6162
- **MURILLO, O. ; DUBROT, J. ; PALAZON, A. ; ARINA, A. ; AZPILIKUETA, A. ; ALFARO, C. ; SOLANO, S. ; OCHOA, M.C. ; BERASAIN, C. ; GABARI, I. et al.** In vivo depletion of DC impairs the anti-tumor effect of agonistic anti-CD137 mAb. *Eur J Immunol,* 2009, vol. 39, 2424-2436
- **NARAZAKI, H. ; ZHU, Y. ; LUO, L. ; ZHU, G. ; CHEN, L.** CD137 agonist antibody prevents cancer recurrence: contribution of CD 137 on both hematopoietic and nonhematopoietic cells. *Blood,* 2010, vol. 115, 1941-1948
- **NISHIMOTO, H. ; LEE, S.W. ; HONG, H. ; POTTER, K.G. ; MAEDA-YAMAMOTO, M. ; KINOSHITA, T. ; KAWAKAMI, Y. ; MITTLER, R.S. ; KWON, B.S. ; WARE, C.F. et al.** Costimulation of mast cells by 4-1BB, a member of the tumor necrosis factor receptor superfamily, with the high-affinity IgE receptor. *Blood,* 2005, vol. 106, 4241-4248
- **CHEN, L. ; RUDLING, M. ; AUKRUST, P. et al.** CD137 is expressed in human atherosclerosis and promotes development of plaque inflammation in hypercholesterolemic mice. *Circulation,* 2008, vol. 117, 1292-1301
- **PALAZON, A. ; TEIJEIRA, A. ; MARTINEZ-FORERO, I. ; HERVAS-STUBBS, S. ; RONCAL, C. ; PENUELAS, I. ; DUBROT, J. ; MORALES-KASTRESANA, A. ; PEREZ-GRACIA, J.L. ; OCHOA, M.C. et al.** Agonist anti-CD137 mAb act on tumor endothelial cells to enhance recruitment of activated T lymphocytes. *Cancer Res,* 2011, vol. 71, 801-811
- **SCHWARZ, H. ; VALBRACHT, J. ; TUCKWELL, J. ; VON KEMPIS, J. ; LOTZ, M.** ILA, the human 4-1BB homologue, is inducible in lymphoid and other cell lineages. *Blood,* 1995, vol. 85, 1043-1052
- **SHAO, Z. ; SCHWARZ, H.** CD137 ligand, a member of the tumor necrosis factor family, regulates immune responses via reverse signal transduction. *J Leukoc Biol,* 2011, vol. 89, 21-29
- **SHI, W. ; SIEMANN, D.W.** Augmented antitumor effects of radiation therapy by 4-1BB antibody (BMS-469492) treatment. *Anticancer Res,* 2006, vol. 26, 3445-3453
- **SIMEONE, E. ; ASCIERTO, P.A.** Immunomodulating antibodies in the treatment of metastatic melanoma: the experience with anti-CTLA-4, anti-CD 137, and anti-PD1. *J Immunotoxicol,* 2012, vol. 9, 241-247
- **SNELL, L.M. ; LIN, G.H. ; MCPHERSON, A.J. ; MORAES, T.J. ; WATTS, T.H.** T-cell intrinsic effects of GITR and 4-1BB during viral infection and cancer immunotherapy. *Immunol Rev,* 2011, vol. 244, 197-217
- **STAGG, J. ; LOI, S. ; DIVISEKERA, U. ; NGIOW, S.F. ; DURET, H. ; YAGITA, H. ; TENG, M.W. ; SMYTH, M.J.** Anti-ErbB-2 mAb therapy requires type I and II interferons and synergizes with anti-PD-1 or anti-CD137 mAb therapy. *Proc Natl Acad Sci U S A,* 2011, vol. 108, 7142-7147
- **TENG, M.W. ; SHARKEY, J. ; MCLAUGHLIN, N.M. ; EXLEY, M.A. ; SMYTH, M.J.** CD1d-based combination therapy eradicates established tumors in mice. *J Immunol,* 2009, vol. 183, 1911-1920
- **VON KEMPIS, J. ; SCHWARZ, H. ; LOTZ, M.** Differentiation-dependent and stimulus-specific expression of ILA, the human 4-1BB-homologue, in cells of mesenchymal origin. *Osteoarthritis Cartilage,* 1997, vol. 5, 394-406
- **WATTS, T.H.** TNF/TNFR family members in costimulation of T cell responses. *Annu. Rev. Immunol,* 2005, vol. 23, 23-68
- **WEI, H. ; ZHAO, L. ; LI, W. ; FAN, K. ; QIAN, W. ; HOU, S. ; WANG, H. ; DAI, M. ; HELLSTROM, I. ; HELLSTROM, K.E.** Combinatorial PD-1 blockade and CD137 activation has therapeutic efficacy in murine cancer models and synergizes with cisplatin. *PLoS One,* 2013, vol. 8, e84927
- **WEINBERG A.D. ; EVANS D.E. ; THALHOFER C ; SHI T. ; PRELL R.A.** The generation of T cell memory: a review describing the molecular and cellular events following OX40 (CD 134) engagement. *J. Leukoc. Biol,* 2004, vol. 75, 962-972
- **WILCOX, R.A. ; CHAPOVAL, A.I. ; GORSKI, K.S. ; OTSUJI, M. ; SHIN, T. ; FLIES, D.B. ; TAMADA, K. ; MITTLER, R.S. ; TSUCHIYA, H. ; PARDOLL, D.M.** Cutting edge: Expression of functional CD137 receptor by dendritic cells. *J Immunol,* 2002, vol. 168, 4262-4267

- **WILCOX, R.A. ; TAMADA, K. ; FLIES, D.B. ; ZHU, G. ; CHAPOVAL, A.I. ; BLAZAR, B.R. ; KAST, W.M. ; CHEN, L.** Ligation of CD137 receptor prevents and reverses established anergy of CD8+ cytolytic T lymphocytes in vivo. *Blood,* 2004, vol. 103, 177-184
- **WYZGOL A. ; MÜLLER N. ; FICK A ; MUNKEL S. ; GRIGOLEIT G.U. ; PFIZENMAIER K. ; WAJANT H.** Trimer Stabilization, Oligomerization, and Antibody-mediated cell surface immobilization improve the activity of soluble trimers of CD27L, CD40L, 41BBL and Glucocorticoid-induced TNF receptor ligand. *J. Immunol.,* 2009, vol. 183, 1851-1861
- **ZHANG, X. ; VOSKENS, C.J. ; SALLIN, M. ; MANIAR, A. ; MONTES, C.L. ; ZHANG, Y. ; LIN, W. ; LI, G. ; BURCH, E. ; TAN, M. et al.** CD 137 promotes proliferation and survival of human B cells. *J Immunol,* 2010, vol. 184, 787-795